# EUROPEAN PATENT SPECIFICATION

(11) **EP 3 328 859 B1**
(45) Date of publication and mention of the grant of the patent: **03.06.2020**
(21) Application number: 16830925.0
(22) Date of filing: 28.07.2016
(51) Int. Cl.: C07D 471/04, C07D 471/10, C07D 471/20, C07D 487/10, C07D 401/06, C07D 401/14, C07D 403/14, A61K 31/4725, A61P 31/14

(54) **3-((4-ISOQUINOLIN-3-YL)METHYL)-1H-IMIDAZO[4,5-C]PYRIDIN-2(3H)-ONE DERIVATIVES AND RELATED COMPOUNDS AS INHIBITORS OF REPLICATION OF THE RESPIRATORY SYNCYTIAL VIRUS (RSV)**
3-((4-ISOQUINOLIN-3-YL)METHYL)-1H-IMIDAZO[4,5-C]PYRIDIN-2(3H)-ON-DERIVATE UND ÄHNLICHE VERBINDUNGEN ALS INHIBITOREN DER REPLIKATION DES RESPIRATORISCHEN SYNZYTIAL VIRUS (RSV)
DÉRIVÉS DU 3-((4-ISOQUINOLIN-3-YL)MÉTHYL)-1H-IMIDAZO[4,5-C]PYRIDIN-2(3H)-ONE ET COMPOSÉS SIMILAIRES EN TANT QU'INHIBITEURS DE LA RÉPLICATION DU VIRUS RESPIRATOIRE SYNCYTIAL (RSV)

(30) Priority: 30.07.2015 SE 1551051; 15.06.2016 SE 1650843
(43) Date of publication of application: 06.06.2018
(73) Proprietor: Boehringer Ingelheim International GmbH, 55216 Ingelheim am Rhein (DE)
(72) Inventor: AYESA, Susana, 141 22 Huddinge (SE); ERSMARK, Karolina, 141 22 Huddinge (SE); KALAYANOV, Gennadiy, 141 22 Huddinge (SE); LEIJONMARCK, Marie, 141 22 Huddinge (SE); SALVADOR ODEN, Lourdes, 141 22 Huddinge (SE); WESTERLIND, Hans, 141 22 Huddinge (SE); WÄHLING, Horst, 141 22 Huddinge (SE); BERTRAND, Megan, 141 22 Huddinge (SE); BROCHU, Christian, 141 22 Huddinge (SE); GHIRO, Elise, 141 22 Huddinge (SE); KUHN, Cyrille, 141 22 Huddinge (SE); STURINO, Claudio, 141 22 Huddinge (SE); BYLUND, Johan, 141 22 Huddinge (SE); SEHGELMEBLE, Fernando, 141 22 Huddinge (SE)
(74) Representative: Schippling, Susanne
(86) International application number: PCT/SE2016/050733
(87) International publication number: WO 2017/018924

(56) References cited:
- WO-A1-2015/065336
- WO-A1-2015/065338
- NAJARRO, PILAR ET AL.: 'The prophylaxis and treatment with antiviral agents of respiratory syncytial virus infections' ANTIVIRAL CHEMISTRY AND CHEMOTHERAPY vol. 22, 2012, pages 139 - 150, XP055349956

## Description

### FIELD OF THE INVENTION

The present invention relates to isoquinoline analogues and their use as inhibitors of replication of the respiratory syncytial virus (RSV), pharmaceutical compositions containing such analogues, and the use of those compounds in a method for the treatment and prevention of RSV infection.

### BACKGROUND OF THE INVENTION

Globally, the annual death rate from RSV is estimated at more than 160,000 and the clinical burden of RSV infection is comparable to that of influenza (Bourgeois et al., 2009; Boyce et al., 2000; Hall et al., 2009; Stockman et al., 2012). The epidemic season for RSV runs from late fall through early spring. The primary populations at risk for poor outcome are children below 5 years of age, immunocompromised patients and older adults, particularly those who are institutionalized or have chronic underlying disease (Hall et al., 2009; Falsey et al., 2005). There is generally no available therapy for RSV infection, except for supportive care. Inhaled ribavirin is approved for the treatment of laboratory-diagnosed RSV infection but is administered only to some bone marrow transplant and immunocompromised patients, because of its limited effectiveness, complexity of administration and mutagenicity potential for patients and staff. Because of the absence of effective therapy for RSV infections and the significance of RSV morbidity and/or morality in at-risk populations, the introduction of an effective RSV agent will be considered a major breakthrough in the care of these patients.

The review by Najarro et al., "The prophylaxis and treatment with antiviral agents of respiratory syncytial virus infections" (Antiviral Chemistry and Chemotherapy, 2012, 22 (4), pages 139-150) considers recent advances in the discovery and development of antiviral agents for respiratory syncytial virus (RSV) infections and provides background information on the various manifestations of human RSV infection and current treatments as well as the technical, clinical and commercial issues surrounding the development of such antiviral agents. Patent application WO2015/065338 discloses quinazolin analogs and WO2015/065336 discloses quinazolinone analogs and their use as inhibitors of replication of RSV.

### SUMMARY OF THE INVENTION

The present invention provides a novel series of compounds that exhibit inhibitory activity on the replication of the RSV.

Further objects of this invention arise for the one skilled in the art from the following description and the examples.

One aspect of the invention provides a compound, represented by Formula (I), or racemate, enantiomer, diastereoisomer or tautomer thereof:
In one embodiment, the invention relates to a compound having Formula (I) or racemate, enantiomer, diastereoisomer or tautomer thereof: 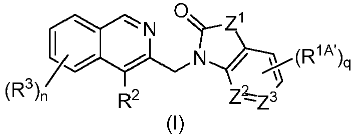 wherein
Z¹ is NR^{1A}, CHR^{1A}, CR^{1B}R^{1B};
one of Z² and Z³ is CH or CR^{1A'}, the other is N, CH or CR^{1A'};
R^{1A} is C₁-C₆alkyl, C₃-C₇cycloalkyl, -S(=O)₂R^{1C}, aryl, heteroaryl or heterocyclyl, wherein each said alkyl, cycloalkyl, aryl, heteroaryl and heterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, -C(=O)OH, -C(=O)R^{1C}, -C(=O)C₁-C₆alkyleneNH₂, -C(=O)OR^{1C}, -C(=O)NHR^{1C}, -C(=O)NR^{1D}R^{1D'}, -S(=O)2R^{1C}, -S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} or -NHS(=O)₂R^{1C};
the two R^{1B} together with the carbon atom to which they are attached combine and form a C₃-C₆cycloalkyl or heterocyclyl, wherein the cycloalkyl and heterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, - C(=O)OH, -C(=O)R^{1C}, -C(=O)OR^{1C}, -C(=O)NHR^{1C}, -C(=O)NR^{1D}R^{1D'}, -S(=O)₂R^{1C}, -S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, - NHC(=O)OR^{1C} or -NHS(=O)₂R^{1C};
each R^{1A'} is independently selected from halo, hydroxy, cyano, C₁-C₃haloalkyl, C₁-C₃alkoxy.
R^{1C} is C₁-C₆alkyl or C₃-C₇cycloalkyl, any of which is optionally substituted with one or two substituents independently selected from halo, hydroxy, cyano, amino, trifluoromethyl, C₁-C₃alkyl, C₁-C₃alkoxy, C₁-C₃haloalkyl, C₁-C₃alkylamino and C₁-C₃dialkylamino;
R^{1D} and R^{1D'} are each independently H or C₁-C₆alkyl, or
R^{1D} and R^{1D'} together with the nitrogen atom to which they are attached form a 4 to 6 membered ring which ring is optionally substituted with one or two substituents independently selected from halo, hydroxy, cyano and amino;
R² is C₁-C₆alkyl which is substituted with one, two or three substituents each independently selected from halo, hydroxy, cyano, trifluoromethyl, amino, -NHR^{2A}, -NR^{2B}R^{2B'}, C₁-C₃alkoxy, S(=O)₂R^{2A}, C₃-C₄cycloalkoxy, heterocycloxy, wherein each said alkoxy, cycloalkoxy and heterocycloxy is optionally mono-, di- or tri-substituted with substituents each independently selected from oxo, halo, hydroxy, cyano, amino, C₁-C₃alkyl, C₁-C₃haloalkyl, C₁-C₃alkoxy, C₁-C₃haloalkoxy, hydroxyC₁-C₃alkoxy, C₁-C₃alkylamino and S(=O)₂R^{2A}, or
R² is C₂-C₆alkyl, C₃-C₇cycloalkylC₀-C₅alkyl, heterocyclylC₀-C₅alkyl, arylC₀-C₅alkyl or heteroarylC₀-C₅alkyl wherein heterocyclyl is a 4 to 8 membered saturated mono-, bi- or spirocyclic ring, and wherein each said cycloalkyl, heterocyclyl, aryl and heteroaryl is optionally mono-, di- or tri-substituted with substituents each independently selected from oxo, halo, hydroxy, cyano, amino, C₁-C₃alkyl, C₁-C₃haloalkyl, C₁-C₃hydroxyalkyl, C₁-C₃alkoxy, C₁-C₃haloalkoxy, hydroxyC₁-C₃alkoxy, C₁-C₃alkylamino and S(=O)₂R^{2A};
R^{2A} is C₁-C₃alkyl, C₃-C₄cycloalkyl, aryl, heteroaryl or heterocyclyl;
R^{2B} and R^{2B'} are each independently C₁-C₃alkyl, or
R^{2B} and R^{2B'} together with the nitrogen atom to which they are attached combine and form a 4 to 6 membered heterocyclyl, which heterocyclyl is optionally substituted with one or two substituents independently selected from amino, halo, C₁-C₃alkyl and trifluoromethyl;
R³ is each independently selected from the group consisting of halo, hydroxy, cyano, amino, C₁-C₆alkyl, C₁-C₆alkoxy, C₃-C₇cycloalkylC₀-C₂alkyl or heterocyclylC₀-C₂alkyl wherein the alkyl, alkoxy, cycloalkyl and heterocyclyl is optionally substituted with 1, 2 or 3 substituents independently selected from -NR^{3A}R^{3B}, halo, hydroxy and trifluoromethyl;
R^{3A} and R^{3B} are each independently H or C₁-C₆alkyl, wherein the alkyl is optionally substituted with one or two halo;
n is 0, 1 or 2;
q is 0, 1 or 2;
heterocyclyl is a saturated 4 to 7 membered mono-, bi- or spirocyclic ring containing 1, 2 or 3 heteroatoms each independently selected from O, S and N, unless otherwise specified;
   or a salt thereof.

In a further embodiment, the invention relates to a compound having Formula (I) or racemate, enantiomer, diastereoisomer or tautomer thereof: 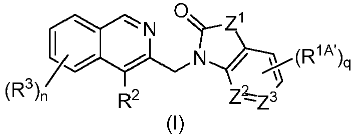 wherein
Z¹ is NR^{1A}, CHR^{1A}, CR^{1B}R^{1B};
one of Z² and Z³ is CH or CR^{1A'}, the other is N, CH or CR^{1A'}; R^{1A} is C₁-C₆alkyl, C₃-C₇cycloalkyl, S(=O)₂R^{1C}, aryl, heteroaryl or heterocyclyl, wherein each said alkyl, cycloalkyl, aryl, heteroaryl and heterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, -C(=O)OH, -C(=O)R^{1C}, -C(=O)C₁-C₆alkyleneNH₂, -C(=O)OR^{1C}, -C(=O)NHR^{1C}, -C(=O)NR^{1D}R^{1D'}, -S(=O)2R^{1C}, -S(=O)₂NHR^{1C}, -S(=O)(=NH)R^{1C}, -OC(=O)R^{1C}, -OC(=O)NHR^{1C}, NHC(=O)R^{1C}, NHC(=^{1C})NHR^{1C}, - NHC(=O)OR^{1C} or -NHS(=O)₂R^{1C};
the two R^{1B} together with the carbon atom to which they are attached combine and form a C₃-C₆cycloalkyl or heterocyclyl, wherein the cycloalkyl and heterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, - C(=O)OH, -C(=O)R^{1C}, -C(=O)OR^{1C}, -C(=O)NHR^{1C}, -C(=O)NR^{1D}R^{1D'}, -S(=O)2R^{1C}, S(=O)₂NHR^{1C}, - S(=O)(=NH)R^{1C}, -OC(=O)R^{1C}, -OC(=O)NHR^{1C}, -NHC(=O)R^{1C}, -NHC(=O)NHR^{1C}, NHC(=O)OR^{1C} or NHS(=O)₂R^{1C};
each R^{1A'} is independently selected from halo, hydroxy, cyano, C₁-C₃haloalkyl, C₁-C₃alkoxy.
R^{1C} is C₁-C₆alkyl, C₃-C₇cycloalkyl or heterocyclyl, any of which is optionally substituted with one or two substituents independently selected from halo, hydroxy, cyano, amino, trifluoromethyl, C₁-C₃alkyl, C₁-C₃alkoxy, C₁-C₃haloalkyl, C₁-C₃alkylamino and C₁-C₃dialkylamino;
R^{1D} and R^{1D'} are each independently H or C₁-C₆alkyl, or
R^{1D} and R^{1D'} together with the nitrogen atom to which they are attached form a 4 to 6 membered ring which ring is optionally substituted with one or two substituents independently selected from halo, hydroxy, cyano and amino;
R² is C₁-C₆alkyl which is substituted with one, two or three substituents each independently selected from halo, hydroxy, cyano, trifluoromethyl, amino, -NHR^{2A}, -NR^{2B}R^{2B'}, C₁-C₃alkoxy, S(=O)₂R^{2A}, S(=O)₂NH₂, C₃-C₄cycloalkoxy, heterocycloxy, wherein each said alkoxy, cycloalkoxy and heterocycloxy is optionally mono-, di- or tri-substituted with substituents each independently selected from oxo, halo, hydroxy, cyano, amino, C₁-C₃alkyl, C₁-C₃haloalkyl, C₁-C₃alkoxy, C₁-C₃haloalkoxy, hydroxyC₁-C₃alkoxy, C₁-C₃alkylamino and S(=O)₂R^{2A} and S(=O)₂NH₂ or
R² is C₂-C₆alkyl, C₃-C₇cycloalkylC₀-C₅alkyl, heterocyclylC₀-C₅alkyl, arylC₀-C₅alkyl or heteroarylC₀-C₅alkyl wherein heterocyclyl is a 4 to 8 membered saturated mono-, bi- or spirocyclic ring, and wherein each said cycloalkyl, heterocyclyl, aryl and heteroaryl is optionally mono-, di- or tri-substituted with substituents each independently selected from oxo, halo, hydroxy, cyano, amino, C₁-C₃alkyl, C₁-C₃haloalkyl, C₁-C₃hydroxyalkyl, C₁-C₃alkoxy, C₁-C₃haloalkoxy, hydroxyC₁-C₃alkoxy, C₁-C₃alkylamino, C₃-C₄cycloalkyl, oxetanyl, S(=O)₂R^{2A}, S(=O)₂NH₂, NHS(=O)₂R^{2A} and C(=O)NH₂, and the cycloalkyl and oxetanyl is optionally substituted with amino or methyl;
R^{2A} is C₁-C₃alkyl, C₃-C₄cycloalkyl, aryl, heteroaryl or heterocyclyl;
R^{2B} and R^{2B'} are each independently C₁-C₃alkyl, or
R^{2B} and R^{2B'} together with the nitrogen atom to which they are attached combine and form a 4 to 6 membered heterocyclyl, which heterocyclyl is optionally substituted with one or two substituents independently selected from amino, halo, C₁-C₃alkyl and trifluoromethyl;
R³ is each independently selected from the group consisting of halo, hydroxy, cyano, amino, C₁-C₆alkyl, C₁-C₆alkoxy, C₃-C₇cycloalkylC₀-C₂alkyl or heterocyclylC₀-C₂alkyl wherein the alkyl, alkoxy, cycloalkyl and heterocyclyl is optionally substituted with 1, 2 or 3 substituents independently selected from -NR^{3A}R^{3B}, halo hydroxy and trifluoromethyl;
R^{3A} and R^{3B} are each independently H or C₁-C₆alkyl, wherein the alkyl is optionally substituted with one or two halo;
n is 0, 1 or 2;
q is 0, 1 or 2;
heterocyclyl is, unless otherwise specified, a saturated 4 to 7 membered mono-, bi- or spirocyclic ring containing 1, 2 or 3 heteroatoms each independently selected from O, S and N;
   or a salt thereof.

In a further embodiment, the invention relates to a compound having Formula (I) or racemate, enantiomer, diastereoisomer or tautomer thereof: 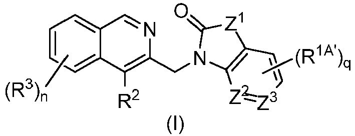 wherein
Z¹ is NR^{1A}, CHR^{1A}, CR^{1B}R^{1B}; one of Z² and Z³ is CH or CR^{1A'}, the other is N, CH or CR^{1A'};
R^{1A} is C₁-C₆alkyl, C₃-C₇cycloalkyl, S(=O)₂R^{1C}, aryl, heteroaryl, heterocyclyl or a 7 or 8-membered spiroheterocyclyl, wherein each said alkyl, cycloalkyl, aryl, heteroaryl, heterocyclyl and spiroheterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, -C(=O)OH, -C(=O)R^{1C}, -C(=O)C₁-C₆alkyleneNH₂, -C(=O)OR^{1C}, -C(=O)NHR^{1C}, -C(=O)NR^{1D}R^{1D'}, -S(=O)₂R^{1C}, S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} or -NHS(=O)₂R^{1C};
the two R^{1B} together with the carbon atom to which they are attached combine and form a C₃-C₆cycloalkyl or heterocyclyl, wherein the cycloalkyl and heterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, - C(=O)OH, -C(=O)R^{1C}, -C(=O)OR^{1C}, -C(=O)NHR^{1C}, -C(=O)NR^{1D}R^{1D'}, -S(=O)2R^{1C}, S(=O)₂NHR^{1C}, - S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} or -NHS(=O)₂R^{1C};
each R^{1A'} is independently selected from halo, hydroxy, cyano, C₁-C₃haloalkyl, C₁-C₃alkoxy;
R^{1C} is C₁-C₆alkyl, C₃-C₇cycloalkyl or heterocyclyl, any of which is optionally substituted with one or two substituents independently selected from halo, hydroxy, cyano, amino, trifluoromethyl, C₁-C₃alkyl, C₁-C₃alkoxy, C₁-C₃haloalkyl, C₁-C₃alkylamino and C₁-C₃dialkylamino;
R^{1D} and R^{1D'} are each independently H or C₁-C₆alkyl, or
R^{1D} and R^{1D'} together with the nitrogen atom to which they are attached form a 4 to 6 membered ring which ring is optionally substituted with one or two substituents independently selected from halo, hydroxy, cyano and amino;
R² is C₁-C₆alkyl which is substituted with one, two or three substituents each independently selected from halo, hydroxy, cyano, trifluoromethyl, amino, -NHR^{2A}, -NR^{2B}R^{2B'}, C₁-C₃alkoxy, S(=O)₂R^{2A}, C₃-C₄cycloalkoxy and heterocycloxy, wherein each said alkoxy, cycloalkoxy and heterocycloxy is optionally mono-, di- or tri-substituted with substituents each independently selected from oxo, halo, hydroxy, cyano, amino, C₁-C₃alkyl, C₁-C₃haloalkyl, C₁-C₃alkoxy, C₁-C₃haloalkoxy, hydroxyC₁-C₃alkoxy, C₁-C₃alkylamino and -S(=O)₂R^{2A}, or
R² is C₂-C₆alkyl, C₃-C₇cycloalkylC₀-C₅alkyl, heterocyclylC₀-C₅alkyl, arylC₀-C₅alkyl or heteroarylC₀-C₅alkyl wherein heterocyclyl is a 4 to 8 membered saturated mono-, bi- or spirocyclic ring, and wherein each said cycloalkyl, heterocyclyl, aryl and heteroaryl is optionally mono-, di- or tri-substituted with substituents each independently selected from oxo, halo, hydroxy, cyano, amino, C₁-C₃alkyl, C₁-C₃haloalkyl, C₁-C₃hydroxyalkyl, C₁-C₃alkoxy, C₁-C₃haloalkoxy, hydroxyC₁-C₃alkoxy, C₁-C₃alkylamino, C₃-C₄cycloalkyl, oxetanyl, -S(=O)₂R^{2A}, - S(=O)₂NH₂, -NHS(=O)₂R^{2A} and -C(=O)NH₂, and the cycloalkyl and oxetanyl is optionally substituted with amino or methyl;
R^{2A} is C₁-C₃alkyl, C₃-C₄cycloalkyl, amino, aryl, heteroaryl or heterocyclyl;
R^{2B} and R^{2B'} are each independently C₁-C₃alkyl, or
R^{2B} and R^{2B'} together with the nitrogen atom to which they are attached combine and form a 4 to 6 membered heterocyclyl, which heterocyclyl is optionally substituted with one or two substituents independently selected from amino, halo, C₁-C₃alkyl and trifluoromethyl;
R³ is each independently selected from the group consisting of halo, hydroxy, cyano, amino, C₁-C₆alkyl, C₁-C₆alkoxy, C₃-C₇cycloalkylC₀-C₂alkyl or heterocyclylC₀-C₂alkyl wherein the alkyl, alkoxy, cycloalkyl and heterocyclyl is optionally substituted with 1, 2 or 3 substituents independently selected from -NR^{3A}R^{3B}, halo, hydroxy and trifluoromethyl;
R^{3A} and R^{3B} are each independently H or C₁-C₆alkyl, wherein the alkyl is optionally substituted with one or two halo;
n is 0, 1 or 2;
q is 0, 1 or 2;
heterocyclyl is a saturated 4 to 7 membered mono- or bi- cyclic ring containing 1, 2 or 3 heteroatoms each independently selected from O, S and N, unless otherwise specified;
   or a salt thereof.

Also within the scope of this invention is the use of a compound of Formula (I), or a pharmaceutically acceptable salt thereof, in a method for the treatment or prevention of RSV infection in a human being.

Included within the scope of this invention is a pharmaceutical composition comprising a compound of Formula (I), or a pharmaceutically acceptable salt thereof, and a pharmaceutically acceptable carrier.

According to a further aspect of this embodiment the pharmaceutical composition according to this invention further comprises a therapeutically effective amount of at least one other antiviral agent.

The invention also provides the use of a pharmaceutical composition as described hereinabove in a method for the treatment of an RSV infection in a human being having or at risk of having the infection.

Another aspect of the invention involves a the use of the compounds in a method of treating or preventing RSV infection in a human being by administering to the human being an anti-RSV virally effective amount of a compound of the invention, a pharmaceutically acceptable salt thereof, or a composition as described above, alone or in combination with at least one other antiviral agent, administered together or separately.

An additional aspect of this invention refers to an article of manufacture comprising a composition effective to treat RSV infection; and packaging material comprising a label which indicates that the composition can be used to treat infection by RSV; wherein the composition comprises a compound of Formula (I) according to this invention or a pharmaceutically acceptable salt thereof.

Still another aspect of this invention relates to a method of inhibiting the replication of RSV comprising exposing the virus to an effective amount of the compound of Formula (I), or a salt thereof, under conditions where replication of RSV is inhibited.

Further included in the scope of the invention is the use of a compound of Formula (I), or a salt thereof, to inhibit the replication of RSV.

### DETAILED DESCRIPTION OF PREFERRED EMBODIMENTS

### DEFINITIONS

Terms not specifically defined herein should be given the meanings that would be given to them by one of skill in the art in light of the disclosure and the context. As used in the specification, however, unless specified to the contrary, the following terms have the meaning indicated and the following conventions are adhered to. In the groups, radicals, or moieties defined below, the number of carbon atoms is often specified preceding the group, for example, C₁-C₆alkyl means an alkyl group or radical having 1 to 6 carbon atoms. In general, for groups comprising two or more subgroups, the last named subgroup is the radical attachment point. For example, the substituent "arylC₁-C₃-alkyl" means an aryl group which is bound to a C₁-C₃alkyl group, with the C₁-C₃alkyl group bound to the core.

The symbol "-" in front of the definition of a radical indicates the radical's point of attachment to the core. For example, the notation "-C(=O)NHC₁-C₆alkyl" represents a primary amide which is linked to the core via the carbonyl carbon, "-C(=O)OC₁-C₆alkyl" indicates an ester linked to the core via the carbonyl carbon, -NHC(=O)C₁-C₆alkyl represents a primary amide linked via the nitrogen atom and "-OC(=O)C₁-C₆alkyl" indicates an ester linked to the core via the oxygen atom.

In case a compound of the present invention is depicted in the form of a chemical name and as a formula in case of any discrepancy the formula shall prevail. The designation, ---- , may be used in partial formulas to indicate the bond which is connected to the core molecule as defined.

Unless specifically indicated, throughout the specification and the appended claims, a given chemical formula or name shall encompass tautomers and all stereo, optical and geometrical isomers (e.g. enantiomers, diastereomers, E/Z isomers, atropisomers) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

One skilled in the art would know how to separate, enrich, or selectively prepare the enantiomers of the compounds of the present invention. Preparation of pure stereoisomers, e.g. enantiomers and diastereomers, or mixtures of desired enantiomeric excess (ee) or enantiomeric purity, are accomplished by one or more of the many methods of (a) separation or resolution of enantiomers, or (b) enantioselective synthesis known to those of skill in the art, or a combination thereof. These resolution methods generally rely on chiral recognition and include but not limited to chromatography using chiral stationary phases, enantioselective host-guest complexation, resolution or synthesis using chiral auxiliaries, enantioselective synthesis, enzymatic and nonenzymatic kinetic resolution, or spontaneous enantioselective crystallization. Such methods are disclosed generally in Chiral Separation Techniques: A Practical Approach (2nd Ed.), G. Subramanian (ed.), Wiley-VCH, 2000; T.E. Beesley and R.P.W. Scott, Chiral Chromatography, John Wiley & Sons, 1999; and Satinder Ahuja, Chiral Separations by Chromatography, Am. Chem. Soc., 2000. Furthermore, there are equally well-known methods for the quantitation of enantiomeric excess or purity, including but not limited to GC, HPLC, CE, or NMR, and assignment of absolute configuration and conformation, including but not limited to CD, ORD, X-ray crystallography, or NMR.

The term "halo" generally denotes fluorine, chlorine, bromine and iodine.

The term "C₁-Cₙalkyl", wherein n is an integer from 2 to n, either alone or in combination with another radical means an acyclic, saturated, branched or linear monovalent hydrocarbon radical with 1 to n C atoms. For example the term C₁₋₃alkyl embraces the radicals H₃C-, H₃C-CH₂-, H₃C-CH₂-CH₂- and H₃C-CH(CH₃)-.

The term C₁-Cₙalkylene wherein n is an integer from 2 to n, means an acyclic, saturated, branched or linear divalent hydrocarbon radical with 1 to n C atoms. For example the term C₁-C₃alkylene embraces the radicals -CH₂-, -CH₂CH₂-, -CH(CH₃)-, -CH₂CH₂CH₂-, -CH₂CH(CH₃)-and -CH(CH₃)CH₂-.The term C₁-Cₙhaloalkyl refers to C₁-Cₙalkyl, wherein at least one C atom is substituted with a halogen, preferably chloro or fluoro. An exemplary C₁-Cₙhaloalkyl is trifluoromethyl.

The term C₁-Cₙalkoxy or C₁-Cₙalkyloxy means a radical -O-C₁-Cₙalkyl which is linked via the oxygen atom, wherein C₁-Cₙalkyl is as defined above, and includes i.a. methoxy, ethoxy, n-propoxy, isopropoxy, t-butoxy, n-butoxy and isobutoxy.

The term "amino" means NH₂.

The term "aminoC₁-Cₙalkyl" means a C₁-Cₙalkyl which is substituted with NH₂, wherein C₁-Cₙalkyl is as defined above.

The term "C₁-Cₙalkylamino" means an amino group which is substituted with C₁-Cₙalkyl, wherein C₁-Cₙalkyl is as defined above.

The term "halo" or "halogen" includes fluoro, chloro, bromo and iodo.

The term "carbocyclyl" or "carbocycle" as used herein, either alone or in combination with another radical, means a mono-, bi- or tricyclic ring structure consisting of 3 to 14 carbon atoms. The term "carbocyclyl" or "carbocycle" refers to fully saturated and aromatic ring systems and partially saturated ring systems. The term "carbocyclyl" or "carbocycle" encompasses fused, bridged and spirocyclic systems.

The term "C₃-Cₘcycloalkyl", wherein m is an integer 3 to m, either alone or in combination with another radical, means a cyclic, saturated, unbranched hydrocarbon radical with 3 to m C atoms. For example the term C₃₋₇cycloalkyl includes cyclopropyl, cyclobutyl, cyclopentyl, cyclohexyl and cycloheptyl.

The term "C₃-Cₘcycloalkxy" means a radical -O-C₃-Cₘcycloalkyl which is linked via the oxygen atom, wherein C₃-Cₘcycloalkyl is as defined above.

The term "oxo" or (=O) is used to indicate an oxygen atom which is double bonded to a carbon or sulfurus atom, thus providing a carbonyl C(=O), sulfoxide S(=O) or sulfonyl S(=O)₂ moiety.

The term "aryl" as used herein, either alone or in combination with another radical, means a carbocyclic aromatic monocyclic group containing 6 carbon atoms which may be further fused to one or more 5- or 6-membered carbocyclic group which may be aromatic, saturated or unsaturated. Aryl includes, but is not limited to, phenyl, indanyl, indenyl, naphthyl, anthracenyl, phenanthrenyl, tetrahydronaphthyl and dihydronaphthyl.

The term "heterocyclyl" or "heterocycle" means a saturated or unsaturated mono-, bi- or tricyclic ring system including aromatic ring systems consisting of 3 to 14 ring atoms and containing one, two, three or four heteroatoms each independently selected from N, O and S. The term "heterocyclyl" or "heterocycle" is intended to include all the possible isomeric forms and all fused, bridged and spiro forms. The "heterocyclyl" may optionally be substituted with one or more substituents.

The term "heterocycloxy" means a radical -O-heterocyclyl which is linked via the oxygen atom, wherein heterocyclyl is as defined above.

The term "heteroaryl" means a mono- bi- or tricyclic ring system containing one, two, three or four heteroatoms each independently selected from N, O and S, consisting of 5 to 14 ring atoms wherein at least one of the heteroatoms is part of an aromatic ring. The term "heteroaryl" is intended to include all the possible isomeric forms and all fused, bridged and spiro forms. Typical heteroaryl are pyridinyl, pyrimidinyl, pyridazinyl, pyrazinyl, thiazolyl, oxazolyl, isoxazolyl, pyrrolyl, pyrazolyl. The "heteroaryl" may be optionally substituted with one or more substituents. The expression "C₃-CₘcycloalkylC₀-Cₙalkyl" wherein m is an integer from 3 to m, and n is an integer from 1 to n as used herein is meant to include a C₃-Cₘcycloalkyl moiety as defined above which is directly bonded (C₀) or bonded through an intermediate C₁-Cₙalkylene linker as defined above.

The expression "carbocyclylC₀-Cₙalkyl" wherein n is an integer from 1 to n as used herein is meant to include a carbocyclyl moiety which is directly bonded (C₀) or bonded through an intermediate C₁-Cₙalkylene linker as defined above.

The expression "heterocyclylC₀-Cₙalkyl" wherein n is an integer from 1 to n as used herein is meant to include a heterocyclyl moiety which is directly bonded (C₀) or bonded through an intermediate C₁-Cₙalkylene linker as defined above.

The expression "heteroarylC₀-Cₙalkyl" wherein n is an integer from 1 to n as used herein is meant to include a heteroaryl moiety which is directly bonded (C₀) or bonded through an intermediate C₁-Cₙalkylene linker as defined above.

The expression "arylC₀-Cₙalkyl" wherein n is an integer from 1 to n as used herein is meant to include a aryl moiety which is directly bonded (C₀) or bonded through an intermediate C₁-Cₙalkylene linker as defined above.

Many of the terms given above may be used repeatedly in the definition of a formula or group and in each case have one of the meanings given above, independently of one another.

The phrase "pharmaceutically acceptable" as used herein refers to compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

The phrase "pharmaceutically acceptable salts" as used herein refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. For example, such salts include acetates, ascorbates, benzenesulfonates, benzoates, besylates, bicarbonates, bitartrates, bromides/hydrobromides, Ca-edetates/edetates, camsylates, carbonates, chlorides/hydrochlorides, citrates, edisylates, ethane disulfonates, estolates esylates, fumarates, gluceptates, gluconates, glutamates, glycolates, glycollylarsnilates, hexylresorcinates, hydrabamines, hydroxymaleates, hydroxynaphthoates, iodides, isothionates, lactates, lactobionates, malates, maleates, mandelates, methanesulfonates, mesylates, methylbromides, methylnitrates, methylsulfates, mucates, napsylates, nitrates, oxalates, pamoates, pantothenates, phenylacetates, phosphates/diphosphates, polygalacturonates, propionates, salicylates, stearates subacetates, succinates, sulfamides, sulfates, tannates, tartrates, teoclates, toluenesulfonates, triethiodides, ammonium, benzathines, chloroprocaines, cholines, diethanolamines, ethylenediamines, meglumines and procaines. Further pharmaceutically acceptable salts can be formed with cations from metals like aluminium, calcium, lithium, magnesium, potassium, sodium, zinc and the like. (also see Pharmaceutical salts, Birge, S.M. et al., J. Pharm. Sci., (1977), 66, 1-19).

The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent like ether, ethyl acetate, ethanol, isopropanol, or acetonitrile, or a mixture thereof.

Salts of other acids than those mentioned above which for example are useful for purifying or isolating the compounds of the present invention also comprise a part of the invention.

As used herein, the term "treatment" means the administration of a compound or composition according to the present invention to alleviate or eliminate symptoms of RSV disease and/or to reduce viral load in a patient.

As used herein, the term "prevention" means the administration of a compound or composition according to the present invention post-exposure of the individual to the virus but before the appearance of symptoms of the disease, and/or prior to the detection of the virus, to prevent the appearance of symptoms of the disease.

The term "therapeutically effective amount" means an amount of a compound according to the invention, which when administered to a patient in need thereof, is sufficient to effect treatment for disease-states, conditions, or disorders for which the compounds have utility. Such an amount would be sufficient to elicit the biological or medical response of a tissue system, or patient that is sought by a researcher or clinician. The amount of a compound according to the invention which constitutes a therapeutically effective amount will vary depending on such factors as the compound and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a therapeutically effective amount can be determined routinely by one of ordinary skill in the art having regard to their own knowledge, the state of the art, and this disclosure.

In the following embodiments, groups and substituents of the compounds of Formula (I) according to this invention are described in detail. Any and each of the definitions below may be combined with each other.

In one embodiment of compounds of formula (I), Z¹ is NR^{1A}, thus providing compounds of formula (Ia):

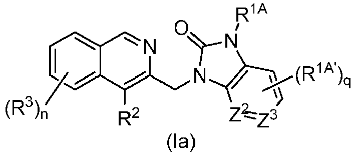

In one configuration, R^{1A} is unsubstituted C₃-C₆cycloalkyl, such as cyclopropyl. In another configuration of compounds of formula (Ia), R^{1A} is C₃-C₆cycloalkyl which is substituted with methyl or fluoro.

In a further configuration of compounds of formula (Ia), R^{1A} is a 4 to 6 membered heterocyclyl which is optionally substituted with -C(=O)R^{1C}, -C(=O)OR^{1C}, -C(=O)NH₂, -C(=O)NHR^{1C}, -C(=O)NR^{1D}R^{1D}' and -S(=O)₂R^{1C};
R^{1C} is C₁-C₄alkyl, C₃-C₆cycloalkyl any of which is optionally substituted with methyl or fluoro; R^{1D} and R^{1D'} are C₁-C₄alkyl, or R^{1D} and R^{1D'} together with the nitrogen atom to which they are attached form a 4 to 6 membered heterocyclyl;

In a typical configuration of compounds of formula (Ia), R^{1A} is azetidinyl or piperidinyl, which is substituted on the N-atom. Typical substituents in this configuration includes -C(=O)OR^{1C} and - S(=O)₂R^{1C}, wherein R^{1C} is C₁-C₄alkyl or C₃-C₆cycloalkyl any of which is optionally substituted with methyl or fluoro;

In an alternative configuration of compounds of formula (Ia), R^{1A} is C₁-C₆alkyl or C₁-C₆haloalkyl.

In an alternative embodiment of compounds of formula (I), Z¹ is CR^{1B}R^{1B}. In this embodiment, the two R^{1B} together with the carbon atom to which they are attached combine and form a C₃-C₆cycloalkyl or a 4 to 7 membered heterocyclyl any of which is optionally substituted, thus providing compounds of the formula (Ib):

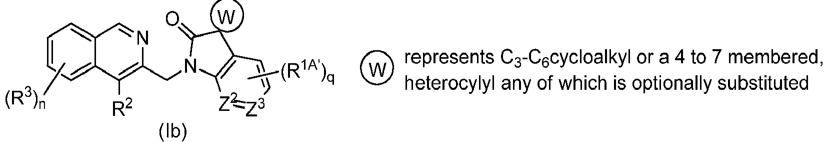

Representative substituents to the ring W are selected from C₁-C₄alkyl, C₁-C₄haloalkyl, halo, hydroxy, C₁-C₄alkoxy, -C(=O)R^{1C}, -C(=O)OR^{1C}, -C(=O)NH₂, -C(=O)NHR^{1C}, -C(=O)NR^{1D}R^{1D'} and -S(=O)₂R^{1C}, wherein
R^{1C} is C₁-C₄alkyl or C₃-C₆cycloalkyl, any of which is optionally substituted with one or two substituents independently selected from methyl, fluoro, amino and hydroxy;
R^{1D} and R^{1D'} are each independently C₁-C₄alkyl, or R^{1D} and R^{1D'} together with the nitrogen atom to which they are attached combine and form a 4 to 6 membered optionally substituted ring;

In one embodiment of compounds of formula (lb), the ring W is an optionally substituted 4 to 6 membered heterocyclyl.

In one embodiment of compounds of formula (lb), the ring W is unsubstituted heterocyclyl.

In one embodiment of compounds of formula (lb), the ring W is optionally substituted C₃-C₆cycloalkyl. Representative substituents according to this embodiment includes hydroxy, NHR^{1C}, -C(=O)OR^{1C}, -OC(=O)NHR^{1C} and -NHC(=O)OR^{1C};
R^{1C} is C₁-C₃alkyl which is optionally substituted with fluoro, or cyclopropyl which is optionally substituted with methyl or fluoro. Typically in this embodiment, R^{1C} is methyl;

Typically, the heterocyclyl in compounds of formula (Ib) is a nitrogen containing ring, such as azetidine or piperidine, which typically is substituted on the nitrogen atom as defined above, thus providing compounds having the structures (Ib') and (Ib") respectively:

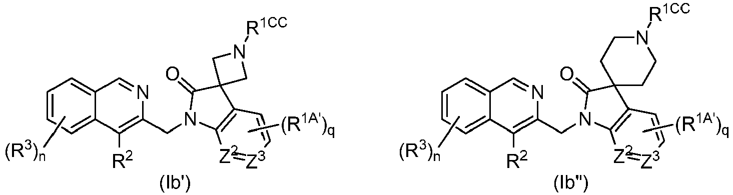

Representative values for R^{1CC} according to this embodiment includes -C(=O)R^{1C}, -C(=O)OR^{1C}, -C(=O)NH₂, -C(=O)NHR^{1C}, -C(=O)NR^{1D}R^{1D'}, -S(=O)2R^{1C}, S(=O)₂NHR^{1C}, -S(=O)(=NH)R^{1C}, especially -C(=O)R^{1C} or -S(=O)₂R^{1C}, wherein
R^{1C} is C₁-C₄alkyl or C₃-C₆cycloalkyl any of which is optionally substituted with methyl amino or fluoro.

R^{1D} and R^{1D'} are C₁-C₄alkyl, such as methyl.

A further group of representative values for R^{1CC} according to this embodiment includes -C(=O)R^{1C}, -C(=O)OR^{1C}, -C(=O)NH₂, -C(=O)NHR^{1C}, -C(=O)NR^{1D}R^{1D'}, -S(=O)2R^{1C}, especially - C(=O)R^{1C}, C(=O)OR^{1C} or -S(=O)₂R^{1C}, wherein
R^{1C} is C₁-C₄alkyl or C₃-C₆cycloalkyl any of which is optionally substituted with methyl amino or trifluoromethyl.

R^{1D} and R^{1D'} are C₁-C₄alkyl, such as methyl.

A further group of representative values for R^{1CC} according to this embodiment includes -C(=O)R^{1C}, -C(=O)OR^{1C}, -S(=O)2R^{1C}, wherein
R^{1C} is C₁-C₄alkyl or C₃-C₆cycloalkyl any of which is optionally substituted with methyl amino or trifluoromethyl.

In one embodiment of compounds of formula (Ib') and (Ib"), R² is heteroaryl, such as pyridinyl, pyrimidinyl, pyridazinyl, pyrazinyl or thiazolyl. Typically in this embodiment, Z² is CH and Z³ is N or CH.

In one embodiment of compounds of formula (Ib') and (Ib"),
Z² is CH;
Z³ is N or CH;
R^{1CC} is -C(=O)R^{1C}, -C(=O)OR^{1C}, -C(=O)NH₂, -C(=O)NHR^{1C}, -C(=O)NR^{1D}R^{1D'}, -S(=O)₂R^{1C}, wherein
R^{1C} is C₁-C₄alkyl or C₃-C₆cycloalkyl any of which is optionally substituted with methyl, amino or trifluoromethyl.
R² is heteroaryl which is optionally substituted with cyano, one or two fluoro, -C(=O)NH₂, -NHS(=O)₂Me or -S(=O)₂NH₂;
n is 0 or 1;
q is 0;

In one embodiment of compounds of formula (Ib') and (Ib"),
Z² is CH;
Z³ is N or CH;
R^{1CC} is -C(=O)R^{1C}, -C(=O)OR^{1C}, -C(=O)NH₂, -C(=O)NHR^{1C}, -C(=O)NR^{1D}R^{1D'}, -S(=O)₂R^{1C}, wherein
R^{1C} is C₁-C₄alkyl or C₃-C₆cycloalkyl any of which is optionally substituted with methyl, amino or trifluoromethyl.
R² is heteroaryl;
n is 0 or 1;
q is 0;

Typically in this embodiment, R^{1CC} is -C(=O)R^{1C}, -C(=O)OR^{1C} or -S(=O)₂R^{1C}, wherein
R^{1C} is methyl or cyclopropyl, wherein cyclopropyl is optionally substituted with amino or trifluoromethyl;
R² is pyridinyl, pyrimidinyl, pyridazinyl, pyrazinyl or thiazolyl.

In a further alternative embodiment of compounds of formula I, Z¹ is CHR^{1A};

In one embodiment of the invention, q is 0.

In one embodiment of the invention, Z² is CR^{1A'} and Z³ is N. Typically, Z² is CH and Z³ is N.

In one embodiment of the invention, Z² is CH and Z³ is N and q is 0.

In an alternative embodiment, Z² and Z³ are both CH.

In one embodiment of the invention, Z² and Z³ both are CH, and q is 0.

In typical embodiments of compounds of formula (I) or any subgroup of formula (I), q is 0.

In alternative embodiments, q is 1 or 2.

In one embodiment of the invention, R² is C₁-C₆alkyl which is substituted with one, two or three substituents each independently selected from halo, hydroxy, trifluoromethyl, amino, -NHR^{2A}, -NR^{2B}R^{2B'}, C₁-C₃alkoxy, C₁-C₃haloalkoxy, hydroxyC₁-C₃alkoxy, S(=O)₂R^{2A};
R^{2A} is C₁-C₃alkyl, C₃-C₄cycloalkyl;
R^{2B} and R^{2B'} are each independently C₁-C₃alkyl.

In one embodiment of the invention, R² is C₁-C₆alkyl which is substituted with hydroxy or trifluoromethyl.

A representative configuration of R² according to this embodiment is C₁-C₆alkyl which is substituted with hydroxy, such as hydroxypropyl, hydroxybutyl or hydroxypentyl, typically hydroxybutyl.

A further representative configuration of R² according to this embodiment is C₁-C₆alkyl which is substituted with one, two or three fluoro.

A further representative configuration of R² according to this embodiment is C₁-C₆alkyl which is substituted with cyano.

In an alternative embodiment of the invention, R² is selected from C₂-C₆alkyl, C₃-C₇cycloalkylC₀-C₅alkyl, heterocyclylC₀-C₅alkyl, arylC₀-C₅alkyl or heteroarylC₀-C₅alkyl wherein each said cycloalkyl, aryl, heteroaryl and heterocyclyl are optionally mono-, di- or tri-substituted with substituents each independently selected from the group consisting of oxo, halo, hydroxy, cyano, amino, C₁-C₃alkyl, C₁-C₃haloalkyl, C₁-C₃hydroxyalkyl, C₁-C₃alkoxy, C₁-C₃haloalkoxy, C₁-C₃alkylamino and -S(=O)₂R^{2A}, wherein R^{2A} is as defined above. Typically in this embodiment, R^{2A} is C₁-C₃alkyl such as methyl.

In one embodiment of the invention, R² is optionally substituted C₃-C₇cycloalkyl, heterocyclyl, phenyl or heteroaryl which is directly linked to the isoquinoline moiety.

A representative configuration of R² according to this embodiment is phenyl which is substituted with cyano, C₁-C₃alkyl such as methyl, or with -S(=O)₂R^{2A}, wherein R^{2A} typically is C₁-C₃alkyl such as methyl.

A further representative configuration of R² according to this embodiment is C₃-C₆cycloalkyl or a 4- to 6-membered heterocyclyl each of which is substituted with oxo, one or two halo, hydroxy, C₁-C₃alkyl or -S(=O)₂R^{2A}, wherein R^{2A} is as defined above. Typically in this embodiment, R^{2A} is C₁-C₃alkyl such as methyl.

In one embodiment of the invention, R² is heteroaryl which is optionally substituted with one or two substituents. Typical heteroaryl according to this embodiment include pyridinyl, pyrimidinyl, pyridazinyl, pyrazinyl, thiazolyl, oxazolyl, isoxazolyl, pyrrolyl, pyrazolyl. Representative substituents according to this embodiment include C₃-C₄cycloalkyl, such as cyclopropyl, C₃-C₄cycloalkyl which is substituted with amino or halo, such as cyclopropyl which is substituted with amino or fluoro. Further representative substituents include halo such as fluoro, cyano, trifluoromethyl, amino, -NHR^{2A}, -NR^{2B}R^{2B'}, -S(=O)₂R^{2A}, -NHS(=O)₂R^{2A}, -S(=O)₂NH₂, -S(=O)₂NHR^{2A}, -C(=O)NHR^{2A}, -C(=O)NR^{2B}R^{2B'}
wherein R^{2A} is C₁-C₃alkyl, C₃-C₄cycloalkyl, such as methyl or cyclopropyl;
R^{2B} and R^{2B'} are each independently C₁-C₃alkyl.

In one embodiment of the invention, R² is heteroaryl.

In a typical embodiment of the invention, R² is heteroaryl selected from pyridinyl, pyrimidinyl, pyridazinyl, pyrazinyl, thiazolyl, oxazolyl, isoxazolyl, pyrrolyl, pyrazolyl. Typically according to this embodiment, R² is pyrimidinyl, pyridinyl or pyridazinyl.

In one embodiment of the invention, R² is thiazolyl or optionally substituted pyridinyl.

In one embodiment of the invention, R² is optionally substituted pyridinyl.

In one embodiment of the invention, R² is pyridinyl.

In a representative embodiment of the invention, R² is pyrid-3-yl or pyrid-4-yl, any of which is optionally substituted.

In a representative configuration according to this embodiment, R² is pyrid-3-yl.

In a further representative configuration according to this embodiment, R² is pyrid-4-yl.

In an alternative embodiment of the invention, R² is thiazolyl. Typically according to this embodiment, R² is thiazol-5-yl.

In one embodiment of the invention where n is 1 or 2, each R³ is independently selected from the group consisting of halo, cyano, hydroxy, C₁-C₆alkyl, C₁-C₆alkoxy, C₃-C₇cycloalkyl and C₁-C₆haloalkyl. Typically according to this embodiment, R³ is fluoro, chloro or cyano.

In one embodiment of the invention, n is 1, R³ is C₁-C₃alkyl, which is substituted with -NR^{3A}R^{3B} and optionally with 1, 2 or 3 fluoro or with C₁-C₃alkoxy. R^{3A} and R^{3B} are independently H or C₁-C₃alkyl. Typically according to this embodiment, R³ is methyl which is substituted with NH₂ and optionally with one fluoro.

In an alternative embodiment of the invention where n is 1, R³ is C₁-C₃alkyl or halo. Typically according to this embodiment, R³ is methyl, chloro or fluoro.

In one embodiment of the invention n is 1, R³ is C₁-C₃alkyl, halo or trifluoromethyl. Typically according to this embodiment, R³ is methyl, chloro, fluoro or trifluoromethyl.

In one embodiment of the invention, n is 1 and R³ is located in the 7-position of the isoquinoline moiety, thus providing compounds of the general formula:

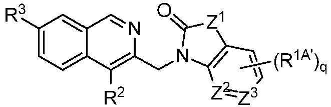

A typical value for R³ according to this embodiment is aminomethyl.

A further typical value for R³ according to this embodiment is halo such as chloro or fluoro, preferably chloro.

A further typical value for R³ according to this embodiment is C₁-C₃alkyl such as methyl.

In one embodiment of the invention, n is 0. In alternative embodiments of the invention, n is 1 or 2. Typically n is 0.

In one embodiment of the invention, the compound of formula I is a compound of formula IIb' or IIb": 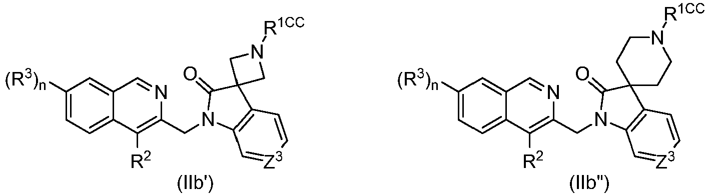 wherein
Z³ is N or CH;
R^{1CC} is -C(=O)R^{1C}, -C(=O)OR^{1C}, -S(=O)₂R^{1C}, wherein
R^{1C} is C₁-C₄alkyl or C₃-C₆cycloalkyl any of which is optionally substituted with methyl, amino or trifluoromethyl;
R² is thiazolyl, pyridinyl or pyridinyl which is substituted with cyano, -NHS(=O)₂Me or fluoro;
R³ is C₁-C₃alkyl, halo, cyano or C₁-C₃haloalkyl;
n is 0 or 1.

Typically according to this embodiment, R^{1C} is methyl, cyclopropyl or cyclopropyl which is substituted with methyl, amino or trifluoromethyl.

Representative values for R^{1CC} according to this embodiment include -C(=O)Me, -S(=O)₂Me.

In one configuration according to this embodiment, n is 1 and R³ is methyl,

In an alternative configuration according to this embodiment, n is 1 and R³ is fluoro.

In a further alternative configuration according to this embodiment, n is 1 and R³ is chloro.

In one configuration according to this embodiment, R² is pyrid-3-yl.

In an alternative configuration according to this embodiment, R² is pyrid-4-yl.

In a further alternative configuration according to this embodiment, R² is thiazolyl, typically thiazol-5-yl.

In one embodiment of compounds of formula IIb' or IIb"
R^{1C} is methyl or cyclopropyl wherein cyclopropyl is optionally substituted with methyl, amino or trifluoromethyl, and
R² is thiazol-5-yl, pyrid-3-yl or pyrid-4-yl;
R³ is methyl, chloro, fluoro or trifluoromethyl.

In a representative configuration of compounds of formula IIb' or IIb", Z³ is N.

### GENERAL SYNTHETIC METHODS

Compounds of the present invention may be prepared by a variety of methods e.g. as depicted in the illustrative synthetic schemes shown and described below. The starting materials and reagents used are available from commercial suppliers or can be prepared according to literature procedures set forth in references using methods well known to those skilled in the art.

For simplicity, in the general synthesis schemes below the following designation will be used:

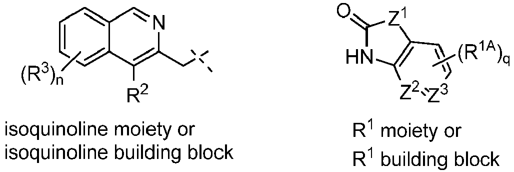

The compounds of the invention are typically obtained by coupling of a chloromethyl or bromomethyl of the isoquinoline moiety (isoquinoline-bb) with an R¹ building block (R¹-bb). The coupling step is typically performed under basic conditions using a base like cesium carbonate, sodium hydride or potassium *tert*.butoxide or similar in an organic solvent like DMF or acetonitrile or the like. The strategy is generally depicted in Scheme 1.

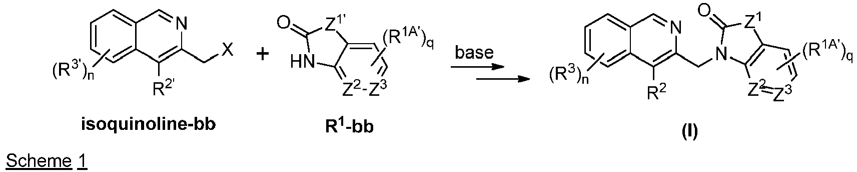

The substituents to the core structure are introduced on the isoquinoline and R¹ building blocks prior to coupling or they can be introduced after the coupling step. Alternatively, precursors to the final substituents may be present on the building blocks and transformed to the desired substituents at a later stage of synthesis of final compounds.

A suitable isoquinoline building block useful for the preparation of compounds of the invention is 4-bromo-3-(bromomethyl)isoquinoline. The building block is commercially available or can be prepared as outlined in Scheme 2.

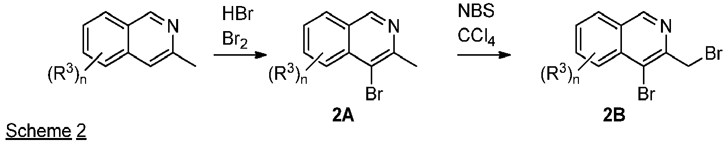

Bromination of commercially available 3-methylisoquinoline effected by treatment with hydrobromic acid and bromine using e.g. the procedures as described in J.Org. Chem., 1991, 56(8), 2805-2809, provides bromo derivative (2A), followed by benzylic bromination effected by treatment with N-bromosuccinimide and carbon tetrachloride provides the desired building block (2B).

An alternative approach to a halo substituted isoquinoline building block is illustrated in Scheme 3.

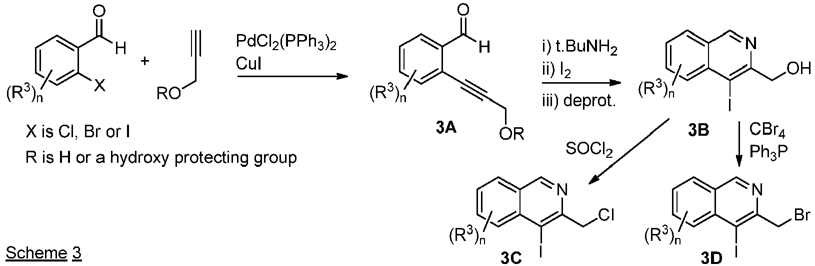

Reaction of optionally substituted benzaldehyde with prop-2-yn-1-ol which may be hydroxy protected, in a cross-coupling reaction under Sonogashira conditions, i.e. using a Pd catalyst such as bis(triphenylphosphine)palladium(II) chloride or equivalent and a copper halide such as copper iodide or the like, in the presence of a base such as triethylamine or similar, provides the acetylene derivative (3A). Reaction with tert. butylamine in the presence of magnesium sulfate or similar followed by iodine, and optionally hydroxy deprotection, provides isoquinoline derivative (3B). The benzylic hydroxy group can then be transformed to a suitable leaving group such as chloro or bromo. Typically, the chloro derivative (3C) is achieved by treatment of the alcohol with phosgene, whereas the bromo derivative (3D) is typically achieved by treatment of the alcohol with carbon tetrachloride in the presence of triphenylphosphine. Alternatively, the iodo derivative (3B) may be further reacted to introduce a desired R² substituent or suitable precursor thereof.

Compounds of the invention wherein R² is optionally substituted aryl or heteroaryl are conveniently prepared using an isoquinoline moiety carrying the desired R² group in the coupling with the R¹-building block. Such isoquinolines can be prepared for instance by a palladium catalysed cross coupling reaction as illustrated in Scheme 4.

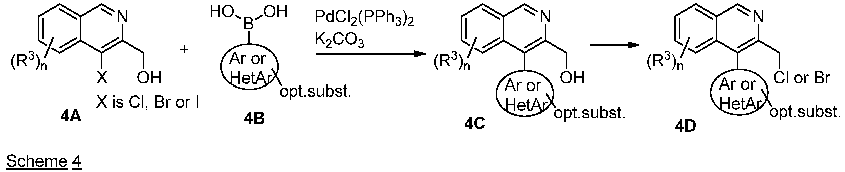

Reaction of halo substituted isoquinoline (4A) and the boronic acid (4B) of the desired aryl or heteroaryl using a catalyst like bis(triphenylphosphine)palladium(II) chloride or equivalent in the presence of a base such as potassium carbonate or similar provides biaryl derivative (4C). Transformation of the benzylic hydroxy group to a chloride or bromide using conditions as described above, provides the desired isoquinoline derivative (4D) ready for coupling with an R¹ building block.

Isoquinolines wherein R² is alkyl or substituted alkyl are obtained e.g. by reaction of an isoquinolinylhalide and a desired olefin under Heck coupling conditions, followed by reduction of the double bond, as shown in Scheme 5.

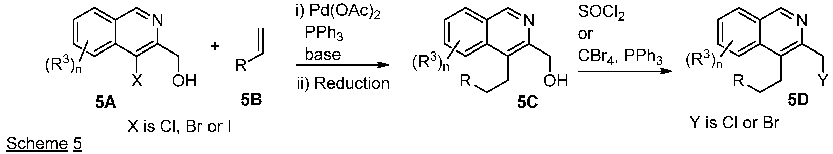

Alternatively, isoquinoline building block (5C) can be obtained by hydroboration of olefin (5B) using 9-BBN or similar followed by a Suzuki coupling of the afforded borate, i.e. with a palladium catalyst in the presence of triphenylphosphine or similar and a base such as triethylamine or potassium carbonate or the like.

A further alternative route to isoquinolines useful for the preparation of compounds of the invention wherein R² is alkyl or substituted alkyl using optionally substituted benzaldehyde as starting material is illustrated in Scheme 6.

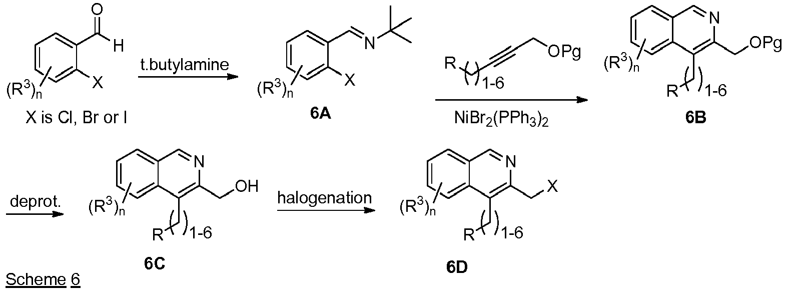

Reaction of imine (6A), prepared from optionally substituted benzaldehyde by reaction with tert.butylamine, with a suitably hydroxy protected acetylene derivative in a cross-coupling reaction using a catalyst like dibromobis(triphenylphosphine)nickel(II) or equivalent provides quinoline derivative (6B). Removal of the hydroxy protecting group, using the appropriate conditions for the protecting group used, e.g. by treatment with acid in the case of a THP group, followed by halogenation as described above, provides the isoquinoline (6D) ready for coupling with a desired R¹ building block. In the case R in compound 6D is hydroxy, the hydroxy groups of the acetylene diol used in the coupling with compound 6A are suitably protected. Preferably in this case with orthogonal protecting groups in order to enable selective deprotection, for example a silyl group e.g. TBDMS, and an acetal group such as tetrahydropyranyl group may be used.

In a similar way, isoquinolines carrying a direct linked optionally substituted cycloalkyl or heterocycloalkyl can be prepared using the substituted acetylene derivative, as illustrated in Scheme 7.

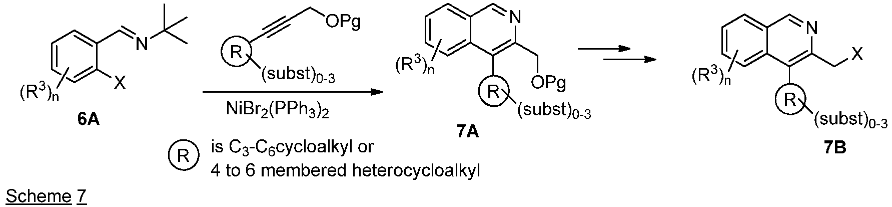

R¹ building blocks can be prepared according to literature procedures or as disclosed herein below. For example, WO2003/053344 and WO2013/186335 disclose the preparation of R¹ building blocks wherein Z¹ is N, Z² is CH and Z³ is CH or N. In J. Org. Chem. 60 (1995) 1565-1582, methods for the preparation of R^{1'} building block wherein q is 0, Z¹ is N, are disclosed. Spirocyclic R¹ building blocks, i.e. wherein Z¹ is CR^{1B}R^{1B} and the two R^{1B} together with the carbon atom to which they are attached form C₃-C₆cycloalkyl or a 4-6 membered heterocyclyl are disclosed in e.g. WO2014/060411 and WO2015/022301. R¹ building blocks wherein Z² is CH and Z³ is CH or N can be prepared according to procedures described in e.g. WO2014/009302 or in Tetrahedron 70(2014) 8413-8418.

For example, R¹ building blocks wherein Z¹ is CR^{1B}R^{1B} and the two R^{1B} together with the carbon atom to which they are attached form a 4 to 6 membered heterocyclyl can be prepared as illustrated in Scheme 8.

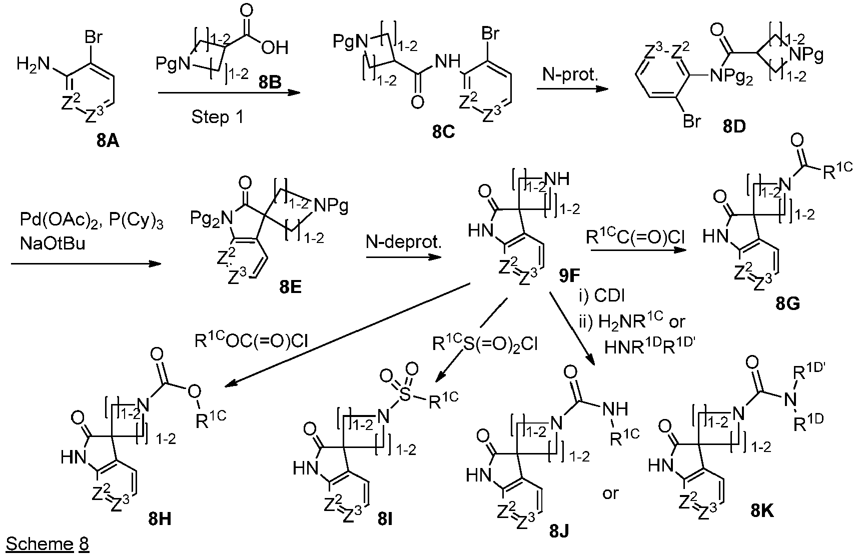

Coupling of the desired amino derivative (8A) and acid (8B) under peptide coupling conditions such as in the presence of coupling agent like HATU, EDC or similar in the presence of an amine like DIEA or the like provides the amide (7c). Protection of the amide nitrogen with for instance a p-methoxy benzyl group which can be introduced by reaction with p-methoxy benzyl chloride in the presence of a base like potassium carbonate or similar, followed by a palladium catalysed ring closure using for instance palladium acetate and tricyclohexylphosphine or similar and a base such as sodium tert.butoxide or the like, provides the bicycle (8E). Removal of the two N-protecting groups using the appropriate conditions according to the protecting group used, such as acidic treatment in the case of Boc and p-methoxybenzyl provides the unprotected spirobicycle (8F). The afforded amine can then be transferred to an amide (8G) by reaction with an acid R^{1C}C(=O)OH under peptide coupling conditions, or with an acid chloride R^{1C}C(=O)Cl, or to a carbamate (8H) by reaction with a chloroformate R^{1C}OC(=O)Cl or anhydride R^{1C}OC(=O)OC(=O)R^{1C} or similar. Reaction of the amine with a sulfonylchloride R^{1C}S(=O)₂Cl provides a sulfonamide(3i) whereas reaction with carbonyl diimidazole or phosgene or similar followed by an amine H₂NR^{1C}C or HNR^{1D}R^{1D'}C provides a urea (8J) or (8K) respectively.

A route to R¹ building block useful for the preparation of compounds of formula I wherein Z¹ is NR^{1A} and R^{1A} is a 4 to 6 membered cyclic amine is depicted in Scheme 9.

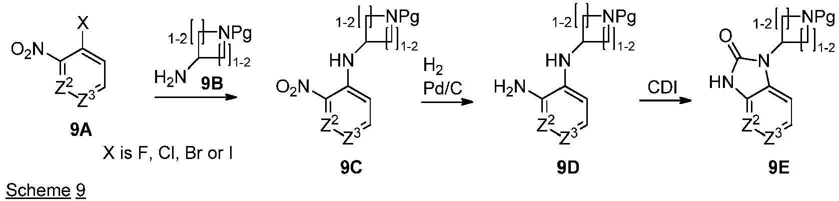

Reaction of nitro substituted aryl halide (9A) in a substitution reaction with a suitably protected heterocyclyl amine (9B) using conditions like in the presence of a base such as diisopropylethylamine or similar in a solvent like DMF and typically at an elevated temperature, provides the substituted aniline derivative (9C). Reduction of the nitro group effected for instance by catalytic hydrogenation using a catalyst like palladium on carbon in a solvent like MeOH or EtOH or the like or similar conditions provides the aniline (9D). Ring formation is then performed by reaction with carbonyl diimidazole or phosgene or triphosgene in the presence of a base like triethylamine or similar, thus providing the bicyclic compound (9E).

Similarly, compounds of the invention wherein Z¹ is NR^{1A} and R^{1A} is optionally substituted C₃-C₇cycloalkyl can be prepared in a similar manner, using the desired substituted cycloalkylamine in the coupling with the aryl halide 9A. A route to this building block is illustrated in Scheme 10.

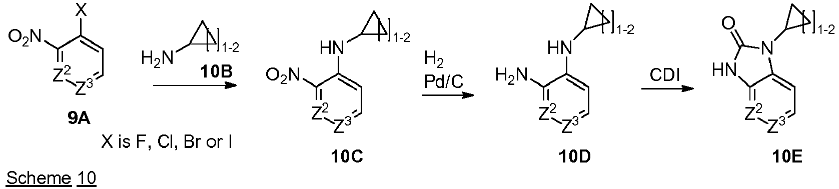

Routes to compounds of the invention wherein Z¹ is NR^{1A} and R^{1A} is a cyclic amine which is substituted on the nitrogen atom are generally depicted in Scheme 11.

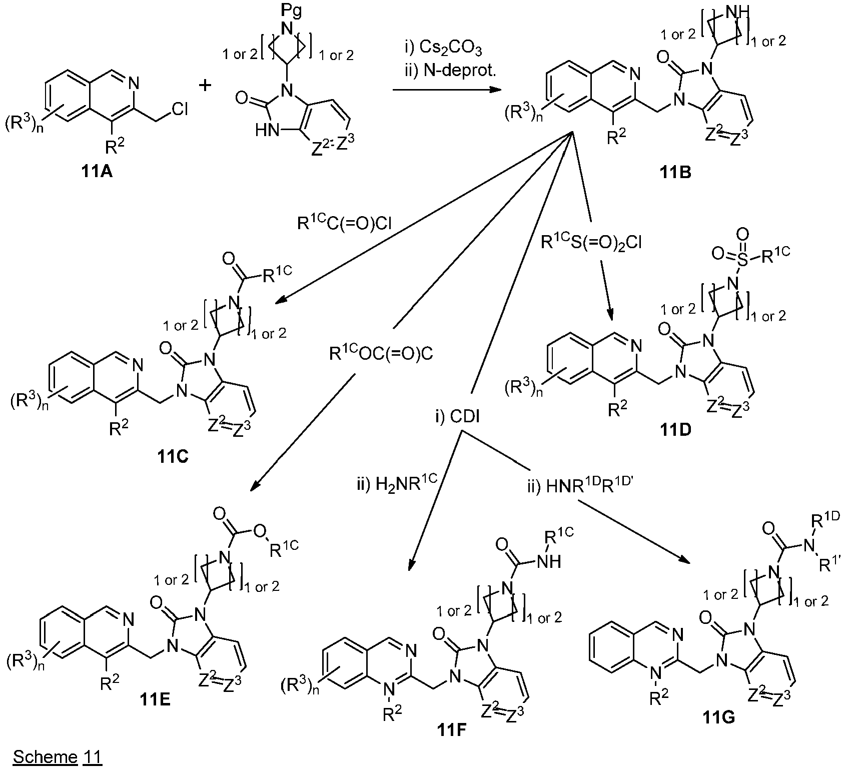

Coupling of the quinoline derivative (11A) with a suitably N-protected R¹-building block effected by treatment with Cs₂CO₃ as described above, followed by removal of the N-protecting group, provides compound (11B), ready for substitution of the cyclic amine with a desired group. For example, the amine can be reacted with an acid R^{1C}C(=O)OH under peptide coupling conditions or with an acid chloride R^{1C}C(=O)Cl thus providing an amide (11C). Reaction with a sulfonylchloride R^{1C}S(=O)₂Cl provides a sulfonamide (11D), whereas
reaction with a chloroformate R^{1C}C(=O)Cl or anhydride R^{1C}C(=O)OC(=O)R^{1C} or similar, provides a carbamate (11E). Reaction with carbonyl diimidazole or phosgene or similar followed by reaction with an amine H²NR^{1C} or HNR^{1D}R^{1D'}C provides urea (11F) or (11G) respectively.

A route to compounds of formula I wherein n is ≥1 and R³ is amino substituted C₃-C₆cycloalkyl or 4 to 6 membered heterocyclyl is generally illustrated in Scheme 12.

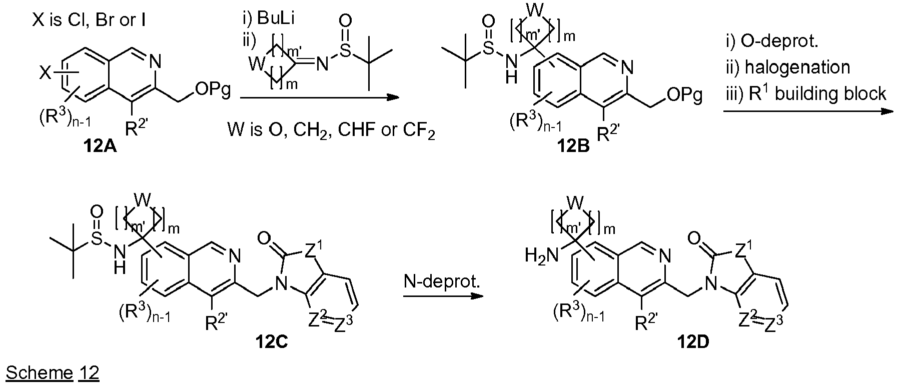

Lithiation of optionally substituted halo substituted isoquinoline (12A) using n-BuLi or tert.BuLi followed by reaction with a sulfinamide derivative of the desired cycloalkyl or heterocyclyl provides the amide (12B). The desired R¹ building block is then introduced as described above, i.e. removal of the hydroxy protecting group, conversion of the thus liberated hydroxy group to bromo or chloro using e.g. CBr₄ or phosgene respectively as described above provides isoquinoline derivative (12C) and finally coupling of the desired R¹ building block.

### PHARMACEUTICAL COMPOSITION

Suitable preparations for administering the compounds of the invention will be apparent to those with ordinary skill in the art and include for example tablets, pills, capsules, suppositories, lozenges, troches, solutions, syrups, elixirs, sachets, injectables, inhalatives and powders, etc. The content of the pharmaceutically active compound(s) should be in the range from 0.05 to 90 wt.-%, preferably 0.1 to 50 wt.-% of the composition as a whole.

Suitable tablets may be obtained, for example, by mixing one or more compounds of the invention with known excipients, for example inert diluents, carriers, binders, disintegrants, adjuvants, surfactants and/or lubricants. The tablets may also consist of several layers.

Suitable inhalatives may be obtained, for example, by administering one or more compounds of the invention in the form of a solution, dry powder or suspension. The compounds of the invention may be administered via inhalation of a solution in nebulized or aerosolized doses.

The dose range of the compounds of the invention applicable per day is usually from 0.01 to 100 mg/kg of body weight, preferably from 0.1 to 50 mg/kg of body weight. Each dosage unit may conveniently contain from 5% to 95% active compound (w/w). Preferably such preparations contain from 20% to 80% active compound.

The actual pharmaceutically effective amount or therapeutic dosage will of course depend on factors known by those skilled in the art such as age and weight of the patient, route of administration and severity of disease. In any case the combination will be administered at dosages and in a manner which allows a pharmaceutically effective amount to be delivered based upon patient's unique condition.

### COMBINATION THERAPY

When the composition of this invention comprises a combination of a compound of the invention and one or more additional therapeutic or prophylactic agent, both the compound and the additional agent should be present at dosage levels of between about 10 to 100%, and more preferably between about 10 and 80% of the dosage normally administered in a monotherapy regimen. Therefore, according to one embodiment, the pharmaceutical composition of this invention additionally comprises one or more antiviral agents.

Antiviral agents contemplated for use in such combination therapy include agents (compounds or biologicals) that are effective to inhibit the production and/or replication of a virus in a human being, including but not limited to agents that interfere with either host or viral mechanisms necessary for the production and/or replication of a virus in a human being. Such agents can be selected from: RSV Fusion inhibitors, such as MDT-637 (MicroDose), BTA-9881 (Biota); RSV Polymerase inhibitors, such as ALS-8112 (Alios), ALS-8176 (Alios) and Virazole (ribavirin); others, such as GS-5806 (Gilead Sciences) and RSV-604 (Novartis); antibodies, such as Synagis® (palimizumab), RespiGam® (RSV-IG), MEDI-557 (Medlmmune/AstraZeneca), ALX-0171 (Ablynx), motavizumab (Medlmmune/AstraZeneca); other biological, such as ALN-RSV-01 (Alnylam) and Vaccines, such as MEDI-559 (Medlmmune/AstraZeneca), RSV F (Novavax), MEDI-534 (Medlmmune/AstraZeneca).

### EXAMPLES

Other features of the present invention will become apparent from the following non-limiting examples which illustrate the principles of the invention. As is well known to a person skilled in the art, reactions are performed in an inert atmosphere (including but not limited to nitrogen or argon) where necessary to protect reaction components from air or moisture. Temperatures are given in degrees Celsius (°C). Solution percentages and ratios express a volume to volume relationship, unless stated otherwise. The reactants used in the examples below may be obtained from commercial sources or they may be prepared from commercially available starting materials as described herein or by methods known in the art.

All of the compounds of the invention are synthesized according to the Examples described herein. It will be apparent to a skilled person that analogous synthetic routes may be used, with appropriate modifications, to prepare the compounds of the invention as described herein. The progress of the reactions described herein were followed as appropriate by e.g. LC, GC or TLC, and as the skilled person will readily realise, reaction times and temperatures may be adjusted accordingly.

In addition to the definitions above, the following abbreviations are used in the synthetic schemes above and the examples below. If an abbreviation used herein is not defined, it has its generally accepted meaning
- ABC: Ammonium bicarbonate
- Ac: Acetyl
- ACN: Acetonitrile
- AcOH: Acetic acid
- Bn: Benzyl
- Boc: *tert*-butyloxycarbonyl
- BOP-CI: Bis(2-oxo-3-oxazolidinyl)phosphinic chloride
- CDI: 1,1'-Carbonyldiimidazole
- DCC: Dicyclohexylcarbodiimide
- DCM: Dichloromethane
- DIEA: Diisopropylethylamine
- DIPEA: Diisopropylethylamine
- DMAP: 4-Dimethylaminopyridine
- DME: 1,2-Dimethoxyethane
- DMEM: Dulbecco's modified Eagle's medium
- DMF: *N*,*N*-Dimethylformamide
- DMSO: Dimethylsulfoxide
- EC₅₀: 50% effective concentration
- EDAC: 1-Ethyl-3-(3-dimethylaminopropyl)carbodiimide
- Et: Ethyl
- EtOAc: Ethyl acetate
- Et₃N: Triethylamine
- EtOH: Ethanol
- Et₂O: Diethyl ether
- LC: Liquid chromatography
- HATU: [O-(7-azabenzotriazol-1-yl)-1,1,3,3-tetramethyluronium hexafluorophosphate]
- HDMS: Hexamethyldisilazane
- HOAc: Acetic acid
- HOBt: Hydroxybenzotriazole
- HPLC: High performance liquid chromatography
- Me: Methyl
- MeCN: Acetonitrile
- MeO: Methoxy
- MeOH: Methanol
- MS: Mass spectrometry
- PCC: Pyridinium chlorochromate
- Pg: Protecting group
- Ph: Phenyl
- PCC: Pyridinium chlorochromate
- rt: Room temperature (18 to 22 °C)
- TBAF: Tetrabutylammonium fluoride
- TEA: Triethylamine
- TEST: bis(triethoxysilyl)propyl-tetrasulfide
- TFA: Trifluoroacetic acid
- TFAA: Trifluoroacetic anhydride
- THF: Tetrahydrofuran

The compounds illustrated in TABLE 1 are described in the literature and used as intermediates in the synthesis of compounds of the invention and referred to as indicated

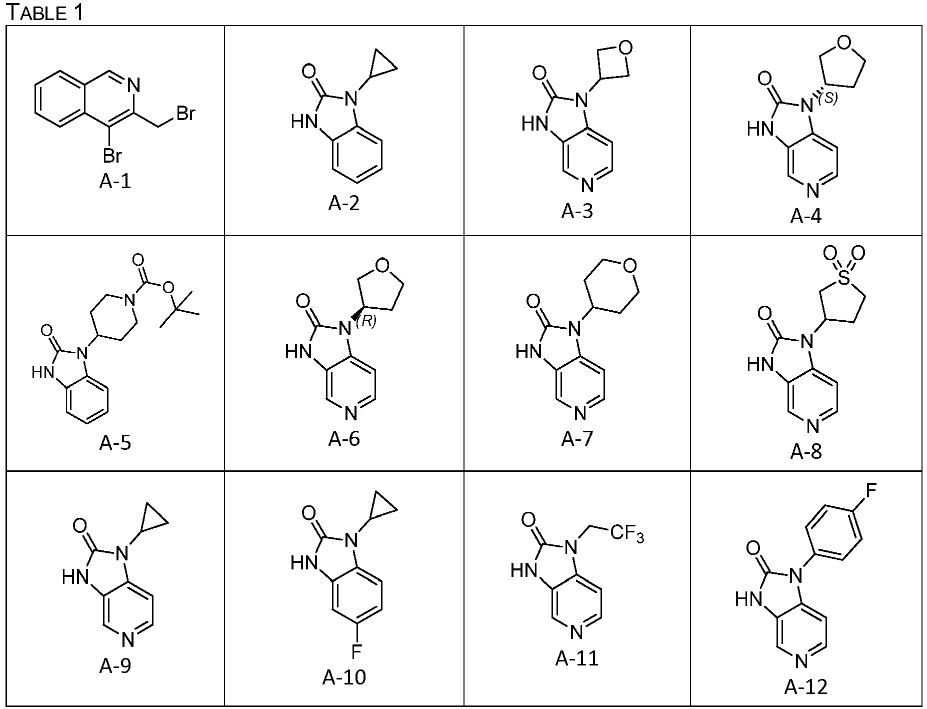

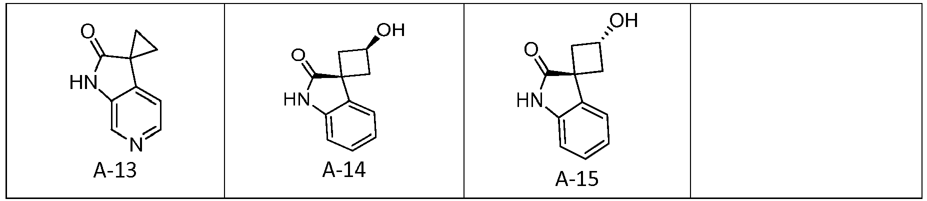

### Preparation of intermediates

### Intermediate 1

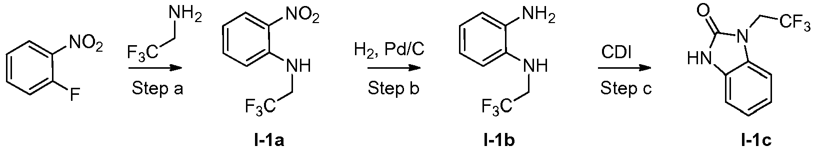

### Step a) 2-nitro-N-(2,2,2-trifluoroethyl)aniline (I-1a)

To a solution of the HCI salt of 2,2,2-trifluoroethylamine (1.0 g, 7.38 mmol, 1 eq) in DMF (8.4 mL) were added DIPEA (1.6 mL, 9.2 mmol, 1.25 eq) and 1-flouro-2-nitro-benzene (0.65 mL, 6.15 mmol, 0.83 eq). The mixture was stirred at 80 °C for 16 h, then poured into an aqueous citric acid solution (10%) and stirred for 5 minutes. The mixture was extracted with EtOAc (3x30 mL) and washed with brine (2x10 mL). The organic layer was dried over anhyd. Na₂SO₄, filtered and concentrated under reduced pressure. The afforded crude material was purified by column chromatography on silica gel (100-200 mesh) using 20% EtOAc/ hexane which gave the title compound (0.84 g, 51.7%) as a solid.

### Step b) N1-(2,2,2-trifluoroethyl)benzene-1,2-diamine (I-1b)

To an ethanolic solution (50 mL) of compound I-1a (5.0 g, 22.7 mmol) was added Pd/C (50% moisture, 500 mg) and the mixture was stirred in Parr shaker at room temperature under 40 psi of hydrogen pressure for 4 h. The reaction mixture was filtered through a pad of celite and the filtrate was concentrated in vacuo. The crude compound was purified by column chromatography (100-200 mesh silica gel) using 30% EtOAc in hexane as eluent which gave the title compound (2.8 g, 65%).

### Step c) 1-(2,2,2-trifluoroethyl)-1H-benzo[d]imidazol-2(3H)-one (I-1c)

To a stirred solution of compound I-1b (4.5 g, 23.5 mmol, 1 eq) in DCM (50 mL) was added DIPEA (12.3 mL, 70 mmol, 3 eq), followed by CDI (6.1 g, 37.7 mmol, 1.6 eq) at ambient temperature under nitrogen atmosphere. The reaction mixture was stirred for 3h, then diluted with water and was extracted with DCM. The organic layer was washed with brine, dried over anhyd. Na₂SO₄ and concentrated under reduced pressure. The afforded crude compound was purified by silica gel (100-200 mesh) column chromatography using 40% EtOAc in hexane which gave the title compound (1.2 g, 23%) as a solid.

¹H NMR 400 MHz, DMSO-d6 δ 4.68-4.75 (q, J = 9.4 Hz, 2H), 7.01-7.10 (m, 3H), 7.21- 7.22 (m, 1H), 11.11 (s, 1H).

### Intermediate 2

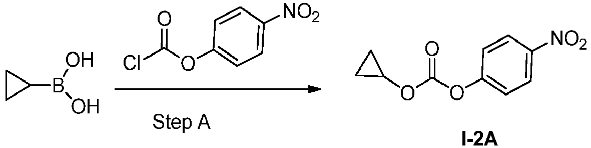

### Step A) cyclopropyl (4-nitrophenyl) carbonate (I-2A)

To a stirred solution of cyclopropylboronic acid (5 g, 58 mmol) in 10% aq. NaOH solution at 0 °C was slowly added 50% H₂O₂ and stirred for 2 h at 0° C. To the reaction mixture was added saturated aq. sodium thiosulphate solution and the product was extracted with diethyl ether and washed the ether layer with saturated aq. sodium thiosulphate solution, water and brine. The organic layer was dried (Na₂SO₄) and concentrated in vacuo at low temp to obtain a colorless liquid, which was directly used without further purification.

The afforded liquid (4.17 g, contains diethyl ether) was dissolved in acetonitrile (40 mL) and cooled to 0 °C. Pyridine (9.3 g, 119 mmol) was added and the mixture was stirred for 15 min at 0 °C and a solution of 4-nitrophenyl carbonochloridate (8 g, 39.8 mmol) in acetonitrile (40 mL) was added under N₂ atmosphere. The reaction mixture was then stirred at the ambient temperature for 12 h. The reaction mixture was diluted with water and the organic components were extracted with EtOAc, washed with brine and dried (Na₂SO₄) and concentrated under reduced pressure to afford crude compound. The crude compound was purified by silica gel (100-200 mesh) column chromatography using 4% EtOAc/ Hexane as eluent to obtain the title compound (1.5 g) as a solid.

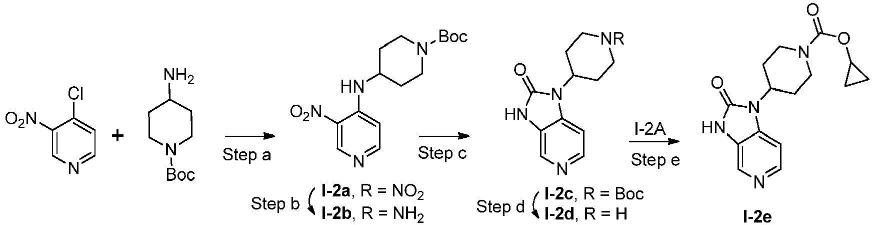

### Step a) tert-butyl 4-((3-nitropyridin-4-yl)amino)piperidine-1-carboxylate (I-2a)

To the stirred solution of 4-chloro-3-nitropyridine (15 g, 94.9 mmol, 1 eq) in DMF (100 mL) was added DIPEA (19.9 mL, 114 mmol, 1.2 eq) followed by tert-butyl 4-aminopiperidine-1-carboxylate (18.9 g, 94.9 mmol, 1 eq) and the resulting mixture was stirred at 80 °C for 16 h. The mixture was cooled to ambient temperature and diluted with chilled water and extracted with EtOAc, the organic phase was washed with water, brine and dried (Na₂SO₄). The organic layer was concentrated under reduced pressure and the residue was purified by silica gel (100-200 mesh) column chromatography using 20% EtOAc/ Hexane to obtain the title compound (10 g, 33%) as a solid. MS 323.3 [M+H]⁺.

### Step b) tert-butyl 4-((3-aminopyridin-4-yl)amino)piperidine-1-carboxylate (I-2b)

To a stirred solution of compound I-2a (12 g, 41.1 mmol) in methanol (100 mL) was added palladium on charcoal (2.5 g, 50% moist) and the mixture was stirred under hydrogen (balloon pressure) for 2 h. The reaction mixture was filtered through celite and the filtrate was concentrated *in vacuo* to obtain crude title compound (8 g) as a solid, which was used in the next step without further purification. MS 292.9 [M+H]⁺.

### Step c) tert-butyl 4-(2-oxo-2,3-dihydro-1H-imidazo[4,5-c]pyridin-1-yl)piperidine-1-carboxylate (I-2c)

To a stirred solution of compound I-2b (5 g, 17.06 mmol, 1 eq) in DCM (60 mL) was added DIPEA (4 mL, 42.7 mmol, 2.5 eq) followed by CDI (4.3 g, 26.6 mmol, 1.6 eq) under nitrogen atmosphere and the resulting reaction mixture was stirred at ambient temp for 12 h. The reaction mixture was diluted with water and the organic components were extracted into DCM. The organic layer was washed with brine, dried (Na₂SO₄) and concentrated under reduced pressure. The crude compound was stirred in 30% EtOAc /hexane for 30 min, then filtered which gave the title compound (3.5 g, 64%) as a solid. MS 319.2 [M+H]⁺.

### Step d) 1-(piperidin-4-yl)-1H-imidazo[4,5-c]pyridin-2(3H)-one (I-2d)

HCI (4M in dioxane, 50 mL) was added at 0 °C to a stirred solution of compound I-2c (8.5 g, 26.7 mmol, 1 eq) in dioxane (20 mL). The solution was stirred at ambient temperature for 16 h, then concentrated *in vacuo* and the afforded solid was triturated with diethyl ether to obtain the title compound (4.8 g, 82%) as a solid. MS 219.3 [M+H]⁺.

### Step e) cyclopropyl 4-(2-oxo-2,3-dihydro-1H-imidazo[4,5-c]pyridin-1-yl)piperidine-1-carboxylate (I-2e)

TEA (4.17 mL, 41.2 mmol, 3 eq) was added at 0 °C to a stirred solution of compound I-2d (3.0 g, 13.76 mmol, 1 eq) in DCM (50 mL), followed by addition of compound I-2A (3.07 g, 13.7 mmol, 1 eq). The mixture was stirred at ambient temperature for 12 h, then diluted with water and extracted with EtOAc. The organic layer was washed with brine, dried (Na₂SO₄) and concentrated under reduced pressure. The afforded crude compound was purified by silica gel (100-200 mesh) column chromatography using 5% MeOH / DCM as eluent to obtain the title compound (1.8 g, 43%) as a solid. MS (ES+) 303.2 [M+H]⁺.

### Intermediate 3

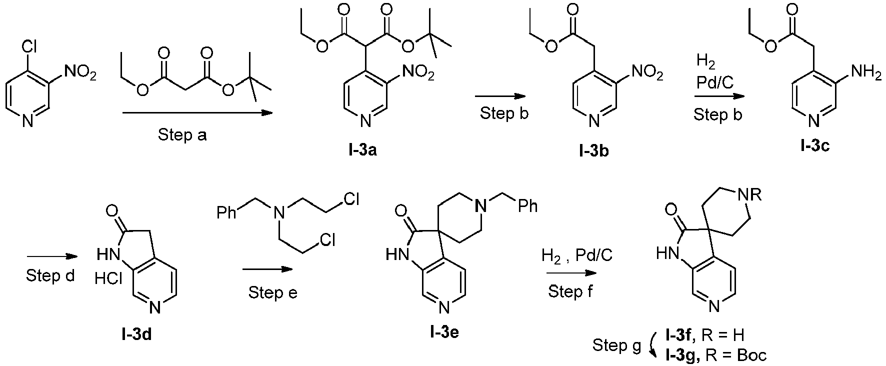

### Step a) 1-tert-butyl 3-ethyl 2-(3-nitropyridin-4-yl)malonate (I-3a)

To the stirred solution 4-chloro-3-nitropyridine (11g, 69.6 mmol) in THF (100 mL) was added NaH (60% in mineral oil, 8.3 g, 208.8 mmol) at 0 °C under N₂ atmosphere. The mixture was stirred at 15 °C for 1h, then cooled to 0 °C and a solution of tert-butyl ethyl malonate (14.5 g, 76.5 mmol) in THF (50 mL) was added drop wise. The resulting reaction mixture was stirred at 15 °C for 1 h and then quenched with saturated aq. solution of NH₄Cl. The reaction mixture was diluted with water and extracted with EtOAc. The organic layer was washed with water, brine, dried (Na₂SO₄) and concentrated under reduced pressure to afford crude title compound (8 g) as an oil which was used for the next step without further purification.

### Step b) ethyl 2-(3-nitropyridin-4-yl)acetate (I-3b)

To a stirred solution of compound I-3a (25 g, 80.64 mmol) in DCM (150 mL) was added TFA (25 mL) dropwise at 0 °C under N₂ atmosphere and the resulting mixture was stirred at 60 °C for 16 h. The reaction mixture was concentrated by distilling out the volatiles completely and slowly diluted by ice cold water. Aq. NaHCO₃ solution was added and the mixture was extracted with EtOAc. The Organic layer was then washed with water and brine, dried (Na₂SO₄) and concentrated *in vacuo.* The afforded crude compound was purified by column chromatography on silica gel (100-200 mesh) using 25% EtOAc/ hexane as eluent to obtain the title compound (12.5 g, 74%) as a liquid. MS (ES+) 211.0 [M+H]⁺.

### Step c) ethyl 2-(3-aminopyridin-4-yl)acetate (I-3c)

10% Pd/C (50% moist) was under nitrogen added to a solution of compound I-3b (1g, 4.7 mmol) in methanol (10 mL). The mixture was stirred under hydrogen atmosphere (50 psi) at ambient temperature in Parr shaker for 4h, then filtered through Celite and the filtrate was concentrated *in vacuo* to afford the title compound (550 mg, 64%) as an oil which was used for the next step without further purification. MS (ES+) 181.2 [M+H]⁺.

### Step d) 1H-pyrrolo[2,3-c]pyridin-2(3H)-one hydrochloride (I-3d)

To a stirred solution of compound I-3c (30 g, 167 mmol) in 1.4 N aq. HCI was added di isopropyl ether and the resulting mixture was stirred at ambient temperature for 16 h. From the reaction mixture, diisopropyl ether was separated and the aqueous part was washed with DCM. The aqueous layer was collected and concentrated in vacuo. The afforded crude compound was triturated with ethyl acetate and filtered to afford the title compound (15 g, 67%) as a solid.

### Step e) 1-benzylspiro[piperidine-4,3'-pyrrolo[2,3-c]pyridin]-2'(1'H)-one (I-3e)

To a stirred solution of compound I-3d (11 g, 82 mmol) in THF (200 mL) was added 1M LiHMDS/ THF solution (234 mL) at -78 °C over a period of 15 min. The resulting reaction mixture was slowly warmed up to 0 °C and was added N-benzyl-2-chloro-N-(2-chloroethyl)ethanamine under nitrogen atmosphere at 0°C. The resulting reaction mixture was then stirred under reflux for 12h. The reaction mixture was quenched with a 10% aq. NH₄Cl solution, diluted with water and the organic components were extracted with EtOAc. The organic part was washed with water and brine, dried (Na₂SO₄) and concentrated under reduced pressure to get crude compound as light brown solid. The crude compound was purified by silica gel (100-200 mesh) column chromatography using 5% MeOH/ DCM to obtain compound 8 (3g, 12%) as an off white solid. MS (ES+) 293.7 [M+H]⁺.

### Step f) spiro[piperidine-4,3'-pyrrolo[2,3-c]pyridin]-2'(1'H)-one (I-3f)

10% Pd/C (50% moist) was added under nitrogen to a solution of compound I-3e (5 g, 17 mmol) in methanol (80 mL). The resulting reaction mixture was stirred under hydrogen atmosphere (50 psi, 50 °C) in Parr shaker for 15 h, then filtered through Celite and the filtrate was concentrated in vacuo to obtain crude title compound (3 g, 86%) as an oil. The crude compound was used in the next step without further purification. MS (ES+) 204.3 [M+H]⁺.

### Step g) tert-butyl 2'-oxo-1',2'-dihydrospiro[piperidine-4,3'-pyrrolo[2,3-c]pyridine]-1-carboxylate (I-3g)

To a stirred solution of compound I-3f (3.5 g, 17.2 mmol) in methanol (40 mL) was added di-tert-butyl dicarbonate (3.9 g, 17.2 mmol) at 0 °C and the mixture was stirred at ambient temperature for 16h. The reaction mixture was concentrated in vacuo and the crude material was purified by silica gel (100-200 mesh) column chromatography using 5% MeOH/ DCM to afford the title compound (3.9 g, 75%) as a solid. MS (ES+) 304.2 [M+H]⁺.

### Intermediate 4

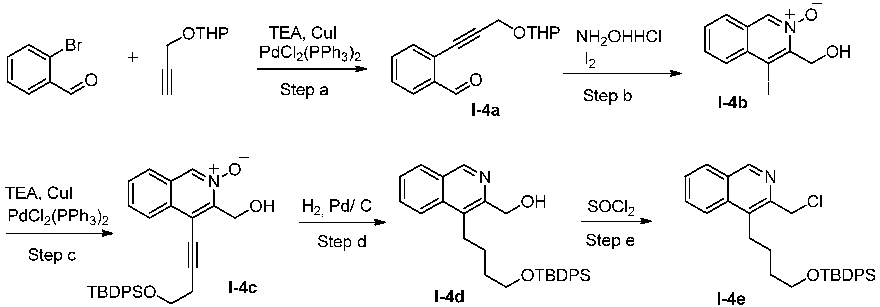

### Step a) 2-(3-((Tetrahydro-2H-pyran-2-yl)oxy)prop-1-yn-1-yl)benzaldehyde (I-4a)

A solution of 2-bromobenzaldehyde (20.0 g, 108.11 mmol, 1 eq) and 2-(prop-2-yn-1-yloxy)tetrahydro-2H-pyran (17.90 g, 129.73 mmol, 1.2 eq) in Et₃N (300 mL) was degassed with argon, then PdCl₂(PPh₃)₂ (1.52 g, 2.16 mmol, 0.02 eq) and Cul (0.205 g, 1.08 mmol, 0.01 eq) were added and the mixture was degassed again with argon for 10 min, then was heated at 80 °C for 4 h under Ar atmosphere. The mixture was concentrated *in vacuo* and the residue was dissolved with water (30 mL) and extracted with ethyl acetate (2 x 250mL). The combined organic layers were washed with water and brine, dried over anhyd. Na₂SO₄ and concentrated under reduced pressure. The afforded residue was purified by silica gel (100-200 mesh) column chromatography using EtOAc which gave the title compound (21 g, 80%).

### Step b) 3-(Hydroxymethyl)-4-iodoisoquinoline 2-oxide (I-4b)

To a stirred suspension of compound I-4a (0.5 g, 2.05 mmol, 1 eq) in EtOH (10 mL) were added NH₂OH (0.213 g, 3.07 mmol, 1.5 eq) and pyridine (0.24 mL, 4.10 mmol, 2.0 eq) and the reaction mixture was stirred for 40 min at ambient temperature. I₂ (2.6 g, 10.2 mmol, 5 eq) was added and the stirring was continued for 15 min. After completion of the reaction as deemed by TLC, the product was extracted into DCM. The crude product was purified by silica gel (100-200 mesh) column chromatography using 2% MeOH in DCM to obtain the title compound (240 mg, 30%) as a solid.

### Step c) 3-(Hydroxymethyl)-4-iodoisoguinoline 2-oxide (I-4c)

To a degassed solution of compound I-4b (8.0 g, 26.6 mmol, 1 eq) and TBDPSCI (9.82 g, 31.9 mmol, 1.2 eq) in TEA (50.0 mL) and DMF (10.0 mL) were added PdCl₂ (PPh₃)₂ (0.187 g, 0.266 mmol, 0.01 eq), and Cul (0.101 g, 0.532 mmol, 0.02 eq) and degassed again with argon for 10 min. The resulting mixture was heated to 80 °C for 4 h under Ar atmosphere. After completion of the reaction (monitored by TLC), the reaction mixture was diluted with DCM and washed with water and brine. The combined organic layer was dried over anhydrous Na₂SO₄, filtered and concentrated under reduced pressure. The afforded crude was purified by silica gel (100-200 mesh) column chromatography using 70% ethyl acetate in hexane to afford the title compound (8.0 g, 62%) as a solid.

### Step d) 3-(Hydroxymethyl)-4-iodoisoguinoline 2-oxide (I-4d)

To a stirred, with argon degassed solution of compound I-4c (3.0 g, 6.2 mmol, 1 eq) in MeOH (70 mL) was added Pd in Charcoal (10%, 4.2 g, 50% wet). The mixture was put under hydrogen gas by balloon pressure and stirred at ambient temperature. After 14 h, the reaction mixture was filtered through Celite. The filtrate was concentrated under reduced pressure and azeotropically distilled with toluene and concentrated which gave the title compound (2.8 g, 96%) as a solid.

### Step e) 4-(4-((Tert-butyldiphenylsilyl)oxy)butyl)-3-(chloromethyl)isoquinoline (I-4e)

Phosgene (2.16 mL, 29.81 mmol, 2 eq) was added drop wise at 0 °C to a stirred solution of compound I-4d (7.0 g, 14.9 mmol, 1 eq) in DCM (200 mL). The reaction mixture was then stirred at ambient temperature for 17 h, then concentrated under reduced pressure, diluted with DCM and washed with water and brine. The combined organic layer was dried over anhyd. Na₂SO₄ and concentrated under reduced pressure. The afforded residue was purified by silica gel (100-200 mesh) column chromatography using 1% MeOH in DCM which gave the title compound (5.5 g, 76%) as a solid. MS (ES+) 488.0 [M+H]⁺.

### Intermediate 5

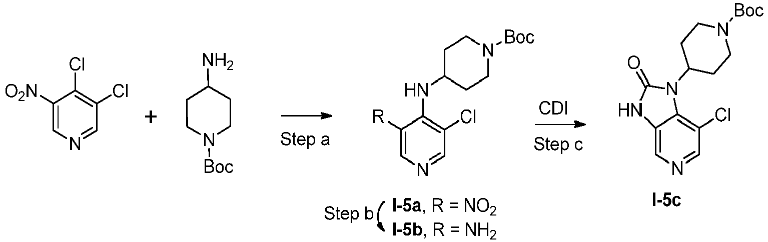

### Step a) tert-butyl 4-((3-chloro-5-nitropyridin-4-yl)amino)piperidine-1-carboxylate (I-5a)

To a stirred solution of tert-butyl 4-aminopiperidine-1-carboxylate (4.47 g, 22.39 mmol) in DMF (60 mL) were added DIPEA (4.68 mL, 26.87 mmol) and 3,4-dichloro-5-nitropyridine (4.3 g, 22.39 mmol) and the resulting reaction mixture was stirred at 80 °C for 16 h. The reaction mixture was cooled to room temperature and diluted with chilled water and the organic components were extracted in EtOAc. The organic layer was washed water, brine and dried over anhyd. Na₂SO₄. The organic layer was then concentrated *in vacuo* to obtain crude product as dark brown oil. The crude material was purified by silica gel (100-200 mesh) column chromatography using 20% EtOAc/Hexane as eluent to afford the title compound (2 g, 25%) as a solid.

### Step b) tert-butyl 4-((3-amino-5-chloropyridin-4-yl)amino)piperidine-1-carboxylate (I-5b)

To a stirred solution of compound I-5a (5 g, 14.1 mmol, 1 eq) in a mixture of THF (80 mL) and water (20 mL) were slowly added NH₄Cl (7.5 g, 141mmol, 10 eq) and Zn powder (7.3 g, 113 mmol, 8 eq) and the resulting reaction mass was stirred at 100 °C for 2 h. The reaction mixture was filtered through Celite and the filtrate was concentrated *in vacuo* and then diluted with water and EtOAc. The organic components were extracted with EtOAc (3 x 150 mL) and then washed with water and brine. The organic layer was dried over anhyd. sodium sulphate and concentrated under reduced pressure to obtain a brown oil as crude title compound (3.5 g, 76%) which was used for the next step without further purification.

### Step c) tert-butyl 4-(7-chloro-2-oxo-2,3-dihydro-1H-imidazo[4,5-c]pyridin-1-yl)piperidine-1-carboxylate (I-5c)

To a stirred solution of compound I-5b (5 g, 15.34 mmol, 1 eq) in DCM (150 mL) were added DIPEA (21 mL, 123 mmol, 8 eq) and CDI (9.9 g, 61.3 mmol, 4 eq) at ambient temperature under nitrogen atmosphere and continued stirring for 12h. The reaction mixture was diluted with water and the organic components were extracted in DCM. The organic layer was washed with brine and dried over anhyd. Na₂SO₄ and concentrated under reduced pressure. The afforded crude material was stirred in 30% EtOAc/ Hexane for 30 min and filtered to obtain the title compound (3 g, 55%) as a solid.

### Intermediate 6

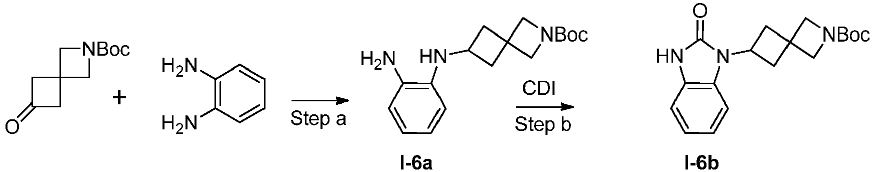

### Step a) Tert-butyl 6-((2-aminophenyl)amino)-2-azaspiro[3.3]heptane-2-carboxylate (I-6a)

To 1,2-phenylenediamine (1,54 g, 14,2 mmol) in methanol (50 mL) under a nitrogen atmosphere were tert-butyl 6-oxo-2-azaspiro[3.3]heptane-2-carboxylate (1.00 g, 4,73 mmol), acetic acid (0,38 mL, 6,64 mmol) and sodium cyanoborohydride (447 mg, 7,11 mmol) successively added. The resulting mixture was stirred at room temperature for 1 h 10 min and was then concentrated under reduced pressure. The residue was partitioned between saturated aqueous sodium bicarbonate and ethyl acetate. The layers were separated and the aqueous phase was extracted with fresh ethyl acetate. The organic extracts were passed through a phase separator and concentrated under reduced pressure. The afforded residue was purified by flash chromatography using a gradient of 0-100 % ethyl acetate in heptane. The desired fractions were pooled and concentrated under reduced pressure and the residue was further purified by flash chromatography using first isocratic elution with dichloromethane (2 CV) then isocratic elution with 5 % methanol in dichloromethane (6 CV). The desired fractions were pooled and concentrated under reduced pressure to give the title compound and a byproduct (957 mg, 57%), which was used as such in next step without further purification.
MS (ES+) m/z 304 [M+H]⁺, (ES-) m/z 362 [M+OAc]⁻.

### Step b) tert-butyl 6-(2-oxo-2,3-dihydro-1H-benzo[d]imidazol-1-yl)-2-azaspiro[3.3]heptane-2-carboxylate (I-6b)

A mixture of I-6a (957 mg, 3,15 mmol) and 1,1'-carbonyldiimidazole (613 mg, 3,78 mmol) in DCM (40 mL) was stirred at room temperature for 5 days. Water was added and the layers were separated. The aqueous phase was extracted with ethyl acetate twice. The organic extracts were passed through a phase separator and concentrated under reduced pressure. Purified by flash chromatography using a gradient of 0-70 % ethyl acetate in heptane. The desired fractions were pooled and concentrated under reduced pressure to give the title comopund (340 mg, 31%). MS (ES-) m/z 328 [M-1]⁻.

### Intermediate 7

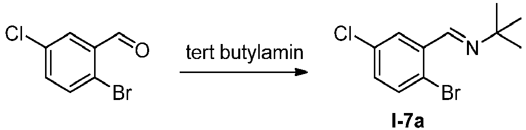

### Step a) (E)-N-(2-bromo-5-chlorobenzylidene)-2-methylpropan-2-amine

A solution of 2-bromo-5-chlorobenzaldehyde (7g, 31.9 mmol) in tert butylamine (25 mL) was heated at 80 °C in a sealed tube for a 17 h, then the reaction mixture was cooled and concentrated under reduced pressure which gave the title compound (8.5 g, 97%). MS (ES+) 273.8 & 275.8 [M+H]⁺.

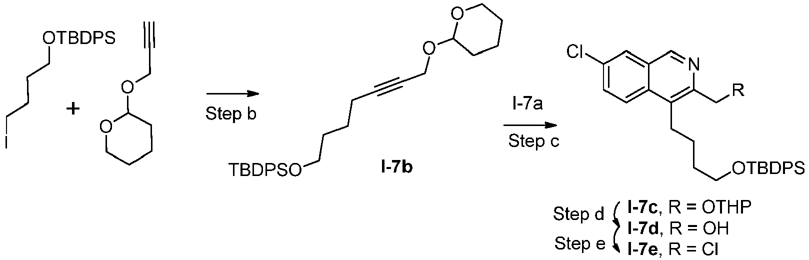

### Step b) tert-butyldiphenyl((7-((tetrahydro-2H-pyran-2-yl)oxy)hept-5-yn-1-yl)oxy)silane (I-7b)

nBuLi (2.2 M in hexane, 6.8 mL, 1.05 eq) was added drop wise to a stirred solution -78 °C of tert-butyl(4-iodobutoxy)diphenylsilane (6.25 g, 14.3 mmol, 1 eq) in THF (80 mL), followed by addition of DMPU (15 mL). After 30 min, the reaction mixture was warmed to -10 °C over a period of 2 h and kept at -10 °C for 20 min. The reaction mixture was then cooled again to -78 °C and a solution of 2-(prop-2-yn-1-yloxy)tetrahydro-2H-pyran (2 g, 14.3 mmol, 1 eq) in THF (10 mL) was added. The resulting mixture was allowed slowly to attain room temperature and was stirred for 17 h. Saturated aqueous ammonium chloride solution was added to the reaction mixture and the organic components were extracted into diethyl ether. The organic layer was washed with water and brine and dried (Na₂SO₄) and concentrated under reduced pressure. The afforded residue was purified by silica gel (100-200 mesh) column chromatography using 10% EtOAc in hexane which gave the title compound (5.5 g, 85%).

### Step c) 4-(4-((tert-butyldiphenylsilyl)oxy)butyl)-7-chloro-3-(((tetrahydro-2H-pyran-2-yl)oxy)methyl)isoquinoline (I-7c)

A round bottom flask containing Zn (43.6 mg, 0.67 mmol, 3 eq) and NiBr₂(PPh₃)₂ (8.25 mg, 0.011 mmol, 0.05 eq) was evacuated and back filled with argon. This process was repeated three times whereafter a degassed solution of compound I-7b (0.1 g, 450 mmol, 0.22 eq) and compound I-7a (0.06 g, 0.22 mmol, 1 eq) in acetonitrile (5 mL) was added. The reaction mixture was heated at 80 °C under argon for 3 h, then filtered through celite. The filtrate was concentrated under reduced pressure and purified by silica gel (100-200 mesh) column chromatography using 0-15% EtOAc in hexane which gave the title compound (60 mg, 46%) and also another regioisomer. Both isomers were carried forward to the next step and the configuration was confirmed by 1D NOE at that stage.

### Step d) (4-(4-((tert-butyldiphenylsilyl)oxy)butyl)-7-chloroisoquinolin-3-yl)methanol (I-7d)

pTSA monohydrate (77.6 mg, 0.4 mmol, 0.3 eq) was added to a stirred solution of compound I-7c (800 mg, 1.36 mmol, 1 eq) in MeOH (10 mL). The mixture was stirred under reflux for 6h, then solid NaHCO₃ was added and the mixture was concentrated under reduced pressure. The residue was diluted with ethyl acetate and washed with saturated aq. NaHCO₃, water and brine. The combined organic layers were dried (Na₂SO₄) and concentrated *in vacuo.* The crude material was purified by silica gel (100-200 mesh) column chromatography using 30% EtOAc in Hexane to obtain the title compound (370 mg, 54%) as a solid. MS (ES+) 504.0 [M+H]⁺.

### Step e) 4-(4-((tert-butyldiphenylsilyl)oxy)butyl)-7-chloro-3-(chloromethyl)isoquinoline (I-7e)

Phosgene (0.76 mL, 10.5 mmol, 2 eq) was added drop wise to an ice-cooled solution of compound I-7d (2.65 g, 5.25 mmol, 1 eq) in DCM (15 mL). The reaction mixture was stirred at ambient temperature for 5 h, then concentrated under reduced pressure, diluted with DCM and washed with saturated aq. sodium bicarbonate solution, water and brine. The combined organic layers were dried (Na₂SO₄) and concentrated under reduced pressure to afford the title compound (2.7g, 98%) as a solid. MS (ES+) 522.1 [M+H]⁺.

### Intermediate 8

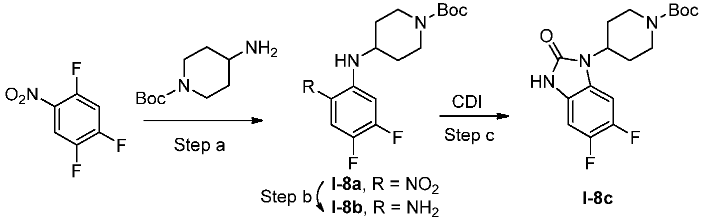

### Step a) tert-butvl 4-((4,5-difluoro-2-nitrophenyl)amino)piperidine-1-carboxylate (I-8a)

K₂CO₃ (4.9 g, 35.6 mmol, 1.26 eq) was added portion wise to a stirred solution of 1,2,4-trifluoro-5-nitrobenzene (5 g, 28.2 mmol, 1 eq) and tert-butyl 4-aminopiperidine-1-carboxylate (7.91 g, 39.5 mmol, 1.4 eq) in 1,4-dioxane (50 mL). The resulting mixture was stirred at ambient temperature for 12 h, then diluted with water and the organic components were extracted into EtOAc. The organic layer was washed with water and brine. The combined organic layer was dried over anhyd. Na₂SO₄ and concentrated under reduced pressure. The afforded crude compound was purified by silica gel (100-200 mesh) column chromatography using 10% EtOAc in hexane which gave the title compound (4.2 g, 42 %) as a solid. MS (ES+) 358.2 [M+H]⁺.

### Step b) Tert-butyl 4-((2-amino-4,5-difluorophenyl)amino)piperidine-1-carboxylate (I-8b)

Pd/C (10%, 350.0 mg, 50% moist) was added to a stirred and with argon degassed solution of compound I-8a (3.5 g, 9.80 mmol, 1.0 eq) in MeOH (60.0 mL). The mixture was shaken under hydrogen atmosphere (50 psi) in a Parr shaker at ambient temperature for 4 h, then filtered through a pad of Celite and washed thoroughly with MeOH. The filtrate was concentrated under reduced pressure which gave the title compound (2.21 g, 69 %) as a solid. MS (ES+) 328.3
[M+H]⁺.

### Step c) Tert-butyl 4-(5,6-difluoro-2-oxo-2,3-dihydro-1H-benzo[d]imidazol-1-yl)piperidine-1-carboxylate (I-8c)

To a stirred solution of compound I-8b (6.0 g, 18.39 mmol, 1 eq) in DCM (50.0 mL) was added DIPEA (8.0 mL, 45.87 mmol, 2.5 eq) followed by CDI (5.94 g, 36.7 mmol, 2.0 eq) at ambient temperature under nitrogen atmosphere. The mixture was stirred at ambient temperature for 12 h, then diluted with water and the organic components were extracted into DCM. The organic layer was washed with water and brine. The combined organic layer was dried over anhyd. Na₂SO₄ and concentrated under reduced pressure. The afforded crude product was dissolved in 30% EtOAc in hexane stirred for 30 min then filtered, which gave the title compound (3.2 g, 49 %) as a solid. MS (ES+) 352.3 [M-H]⁻.
¹H NMR DMSO-d₆ δ 1.426 (s, 9H), 1.644-1.670 (d, 2H), 2.146-2.240 (m, 2H), 2.828 (bs, 2H), 4.074-4.092 (m, 2H), 4.249-4.310 (m, 1H), 7.014-7.058 (t, 1H), 7.425-7.471 (t, 1H), 11.0393 (s, 1H).

### Intermediate 9

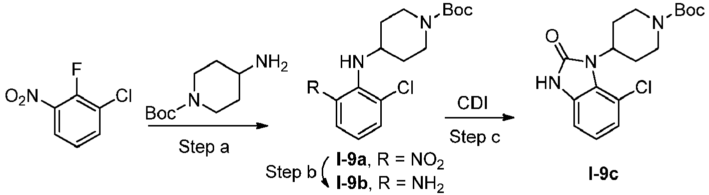

### Step a) tert-butvl 4-((2-chloro-6-nitrophenyl)amino)piperidine-1-carboxylate (I-9a)

To a stirred solution of 1-chloro-2-fluoro-3-nitrobenzene (3.64 g, 20.72 mmol, 1 eq) in DMF (42.0 mL) was added DIPEA (8.7 mL, 49.93 mmol, 2.4 eq) followed by addition of tert-butyl 4-aminopiperidine-1-carboxylate (5.0 g, 24.96 mmol, 1.2 eq). The resulting reaction mixture was stirred at 80 °C for 16 h, then poured into 10% aq. citric acid solution and the organic components were extracted into EtOAc. The organic layer was washed with water and brine. The combined organic layers were dried over anhyd. Na₂SO₄ and concentrated under reduced pressure to afford the desired compound 3 (4.2 g, 47%) as a solid. MS (ES+) 356.2 [M+H]⁺.

### Step b) Tert-butvl 4-((2-amino-6-chlorophenyl)amino)piperidine-1-carboxylate (I-9b)

To a stirred solution of compound I-9a (10.0 g, 28.16 mmol, 1 eq) in THF : water (4:1) (90.0 mL) was added NH₄Cl (15.1 g, 281.7 mmol, 10 eq) followed by addition of zinc powder (14.7 g, 225 mmol, 8 eq). The mixture was stirred at 80 °C for 1 h, then filtered through Celite and the Celite bed was washed with EtOAc. The organic layer was concentrated under reduced pressure and the crude compound was partitioned between water and ethyl acetate. The combined organic layers were washed with water and brine and dried over anhyd. Na₂SO₄ and concentrated under reduced pressure to afford the title compound (8.0 g, 87%) as a liquid.

### Step c) Tert-butvl 4-(7-chloro-2-oxo-2,3-dihydro-1H-benzo[d]imidazol-1-yl)piperidine-1-carboxylate (I-9c)

To the stirred solution of compound I-9b (8.0 mg, 24.62 mmol, 1 eq) in DCM (80.0 mL) was added DIPEA (15.9 mL, 123 mmol, 5.0 eq) followed by addition of CDI (11.97 g, 73.8 mmol, 3.0 eq) at ambient temperature under nitrogen atmosphere. The mixture was stirred for 3h, then diluted with water and the organic components were extracted into DCM. The organic layer was washed with water and brine, dried over anhyd. Na₂SO₄ and concentrated under reduced pressure. The afforded crude material was purified by silica gel (100-200 mesh) column chromatography using 30% ethyl acetate in hexane to obtain pure product which was triturated with diethyl ether and pentane to afford the title compound (5.6 g, 65 %) as a solid. MS (ES+) 352.1 [M+H]⁺. ¹H NMR DMSO-d₆ δ 1.421 (s, 9H), 1.719-1.749 (m, 2H), 2.413-2.438 (m, 2H), 2.796 (m, 2H), 4.071-4.091 (m, 2H), 5.0274 (bs, 1H), 6.931-7.023 (m, 3H), 11.1873 (s, 1H).

### Intermediate 10

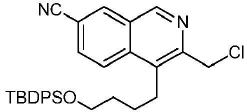

### 4-(4-((tert-butyldiphenylsilyl)oxy)butyl)-3-(chloromethyl)isoquinoline-7-carbonitrite (I-10)

The title compound was prepared according to the procedure described for Intermediate 7, but using 4-bromo-3-formylbenzonitrile instead of 2-Bromo-5-chlorobenzaldehyde. MS (ES+) 513.1 [M+H]⁺. ¹H NMR DMSO-d₆ δ 0.95 (s, 9H), 1.73 (m, 4H), 3.17 (m, 2H), 3.71(m, 2H), 5.02 (s, 2H), 7.40-7.45 (m, 6H), 7.57-7.59 (m, 4H), 8.05-8.07 (d, 1H), 8.31-8.33 (d, 1H), 8.82 (s, 1H), 9.31 (s, 1H).

### Intermediate 11

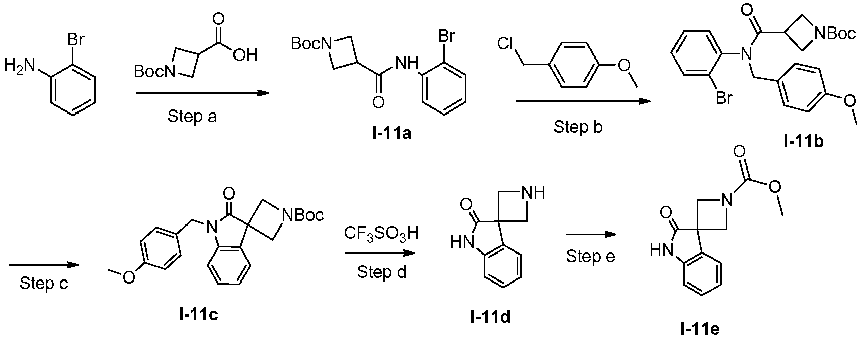

### Step a) tert-butvl 3-((2-bromophenyl)carbamoyl)azetidine-1-carboxylate (I-11a)

To a stirred solution of 2-bromoaniline (5 g, 29.42 mmol, 1 eq) and DMAP (4.6 g, 37.5 mmol, 1.3 eq) in DCM (83 mL) were added 1-(tert-butoxycarbonyl)azetidine-3-carboxylic acid (5.85 g, 29.10 mmol, 1 eq) followed by EDClxHCl (7.24 g, 37.93 mmol, 1.3 eq) at 23 °C and the resulting reaction mixture was stirred at ambient temperature for 12 h. The reaction mixture was then washed with 10% citric acid aqueous solution, water, saturated aqueous Na₂CO₃ solution, brine, and the organic components were extracted into DCM. The organic layer was dried over anhyd. Na₂SO₄ and evaporated under reduced pressure to afford crude compound. The crude compound was purified by silica gel (100-200 mesh) column chromatography using 15% EtOAc in Hexane as the eluent to afford the title compound (6 g, 57.6%) as an off white solid.

### Step b) tert-butyl 3-((2-bromophenyl)(4-methoxybenzyl)carbamoyl)azetidine-1-carboxylate (I-11b)

To a stirred solution of compound I-11a (6 g, 16.9 mmol, 1 eq) and 1-(chloromethyl)-4-methoxybenzene (4.02 g, 25.7 mmol) in acetonitrile (80 mL) was added K₂CO₃ (7.24 g, 50.72 mmol, 3 eq) and the resulting reaction mixture was stirred under reflux for 12 h. The reaction mixture was filtered and the solid residue was washed with acetonitrile. The filtrate was concentrated *in vacuo* and the crude product was triturated with hexane/ethyl acetate (30:1), which gave the title compound (6.5g, 81.25%) as an off white solid.

### Step c) tert-butyl 1'-(4-methoxybenzyl)-2'-oxospiro[azetidine-3,3'-indoline]-1-carboxylate (I-11c)

To the stirred, degassed with argon solution of compound I-11b (0.5 g, 1.27 mmol, 1 eq) and ^{t}BuONa (0.182 g, 1.9mmol, 1.5 eq) in dioxane (4mL) were added Pd(OAc)₂ (0.0071 g, 0.03 mmol, 0.025 eq) and PCy₃ (0.0088 g, 0.03 mmol, 0.025 eq) and the resulting mixture was further degassed with argon for 5 min. The reaction mixture was then stirred under microwave at 120 °C for 1h. The reaction mixture was then filtered through Celite and the filtrate was concentrated under reduced pressure to obtain a crude mass which was washed with water and brine. The organic component was extracted into EtOAc and dried over anhyd. sodium sulphate and concentrated *in vacuo* to dryness. The crude material was then purified on silica gel (230-400 mesh) gravity column using 7% EtOAc-Hexane as eluent which gave the title compound.

### Step d) spiro[azetidine-3,3'-indolin]-2'-one (I-11d)

To a solution of compound I-11c (0.3 g, 0.76 mmol, 1 eq) in TFA (1.2mL) was added CF₃SO₃H (0.20 mL, 2.28 mmol, 3 eq) and the resulting mixture was stirred at 23 °C for 12 h. The reaction mixture was then concentrated under reduced pressure and the residue was dissolved in water and washed with DCM. From the aqueous layer, water was completely distilled out to obtain corresponding salt of the title compound which was used directly in the next step.

### Step e) methyl 2'-oxospiro[azetidine-3,3'-indoline]-1-carboxylate (I-11e)

To a stirred solution of compound I-11d (0.167 g, 0.96 mmol, 1 eq) in DMF, were added TEA (0.404 mL 2.89 mmol, 3 eq) and Methyl chloroformate (0.075 mL, 0.95 mmol, 1eq) at 0 °C and the resulting reaction mixture was stirred at room temperature for 4 h, The reaction mixture then diluted with water and the organic components were extracted with ethyl acetate. The organic layer was washed with brine and dried over anhyd. sodium sulphate and concentrated *in vacuo* to obtain a crude title compound.

### Intermediate 12

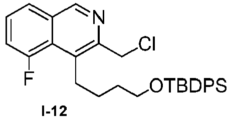

### 4-(4-((Tert-butyldiphenylsilyl)oxy)butyl)-3-(chloromethyl)-5-fluoroisoquinoline (I-12)

The title compound was prepared according to the procedure described for Intermediate 7, but using 2-bromo-3-fluorobenzaldehyde instead of 2-bromo-5-chlorobenzaldehyde. MS (ES+) 506.2 [M+H]⁺. ¹H NMR DMSO-d₆ δ 0.954 (s, 9H), 1.73 (m, 4H), 3.18 (m, 2H), 3.72-3.73 (m, 2H),5.00-5.02 (s, 2H), 7.38-7.46 (m, 6H), 7.58-7.74 (m, 6H), 8.00-8.02 (d, 1H), 9.24 (d, 1H).

### Intermediate 13

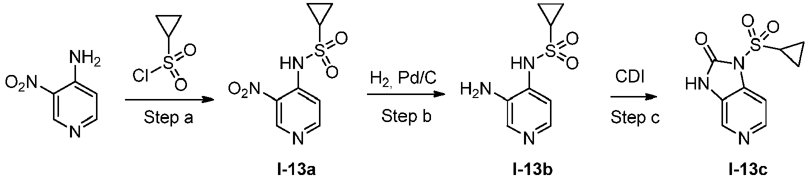

### Step a) N-(3-nitropyridin-4-yl)cyclopropanesulfonamide (I-13a)

To a stirred solution of compound 3-nitropyridin-4-amine (3.0 g, 24.8 mmol, 1 eq) in DMSO (45.0 mL) was added NaH (60%, 1.2 g, 29.8 mmol, 1.2 eq) and the reaction mixture was stirred at ambient temperature for 15 min. To the reaction mixture was added compound cyclopropanesulfonyl chloride (3.92 g, 24.8 mmol, 1 eq). The mixture was stirred at 80 °C for 16 h, then cooled to ambient temperature, ice cold water was added and the organic components were extracted into EtOAc. The organic layer was washed with water and brine, dried (Na₂SO₄), filtered and concentrated under reduced pressure. The afforded crude material was purified by silica gel (100-200 mesh) column chromatography using 50 % EtOAc in hexane which gave the title compound (2 g, 33 %) as a solid. MS (ES+) 243.8 [M+H]⁺.

### Step b) N-(3-aminopyridin-4-yl)cyclopropanesulfonamide (I-13b)

To a stirred with argon degassed solution of compound I-13a (2.5 g, 10.28 mmol, 1 eq) in MeOH (50 mL) was added Pd/C (10%, 300 mg, 50% moist) and the mixture was shaken under hydrogen atmosphere (40 psi) in a Parr shaker at ambient temperature for 12 h. The reaction mixture was then filtered through Celite and washed thoroughly with MeOH. The filtrate was concentrated under reduced pressure which gave the crude title compound 4 (2.1 g, 96%) as a solid which was used directly in next step without any purification. MS (ES+) 214.1 [M+H]⁺.

### Step c) 1-(cyclopropylsulfonyl)-1H-imidazo[4,5-c]pyridin-2(3H)-one (I-13c)

To the stirred solution of compound I-13b (1.3 g, 6.10 mmol, 1 eq) in DCM (30 mL) was added DIPEA (6.3 mL, 65.4 mmol, 8 eq) followed by addition of CDI (1.98 g, 12.2 mmol, 2.0 eq) under nitrogen atmosphere and the resulting mixture was stirred at ambient temperature for 12 h. The reaction mixture was then diluted with water and the organic components were extracted into DCM. The organic layer was washed with water and brine, dried over anhyd. Na₂SO₄ and concentrated under reduced pressure. The afforded crude material was stirred with acetonitrile for 30 min, then filtered which gave the title compound (800 mg, 55%) as a solid. MS (ES+) 240.0 [M+H]⁺. ¹H NMR DMSO-d₆ δ 1.20-1.21 (m, 2H), 1.27-1.31 (m, 2H), 7.49-7.50 (d, 1H), 8.28-8.33 (m, 2H), 11.8836 (s, 1H).

### Intermediate 14

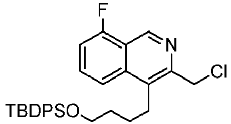

### 4-(4-((Tert-butyldiphenylsilyl)oxy)butyl)-3-(chloromethyl)-8-fluoroisoquinoline (1-14)

The title compound was prepared according to the procedure described for Intermediate 4, but starting from instead of 2-bromobenzaldehyde. Overall yield: 8.5%. MS (ES+) 506.2 [M+H]⁺. ¹H NMR DMSO-d₆ δ0.87-0.96 (s, 9H), 1.75 (m, 4H), 3.16 (m, 2H), 3.73 (m, 2H), 5.02 (m, 2H), 7.40-7.45 (m, 6H), 7.50-7.60 (m, 5H), 7.79-7.83 (m, 1H), 7.99 (m, 1H), 8.00 (m, 1H), 9.35 (s, 1H).

### Intermediate 15

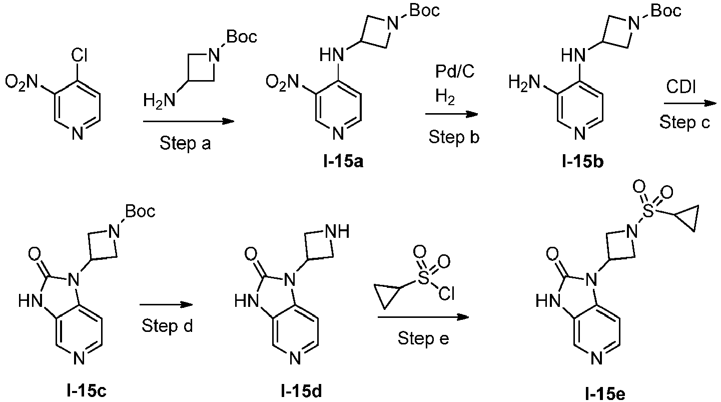

### Step a) tert-butyl 3-((3-nitropyridin-4-yl)amino)azetidine-1-carboxylate (I-15a)

To a stirred solution of 4-chloro-3-nitropyridine (2.5 g, 15.8 mmol, 1 eq) in ethanol was added triethyl amine (4.4 mL, 31.5 mmol, 2 eq) and the reaction mixture was cooled to 0 °C. To the reaction mixture was then added tert-butyl 3-aminoazetidine-1-carboxylate (2.98 g, 17.3 mmol, 1.1 eq) dropwise at 0 °C and the reaction mixture was slowly warmed up to 80 °C stirred at 80 °C for 12 h. The reaction mixture was concentrated under reduced pressure and diluted with water and the organic components were extracted in EtOAc. The organic layer was washed with brine, dried over anhyd. Na₂SO₄ and concentrated *in vacuo.* The crude material was purified by silica gel (100-200 mesh) column chromatography using 50% ethyl acetate/ hexane to obtain the title compound (3.5 g, 75%) as a solid. MS (ES+) 294.8 [M+H]⁺.

### Step b) tert-butyl 3-((3-aminopyridin-4-yl)amino)azetidine-1-carboxylate (I-15b)

To a stirred solution of compound I-15a (13.5 g, 45.9 mmol) in EtOAc (200 mL) was added 10% palladium on charcoal (2.5g, 50% moist) and stirred under hydrogen atmosphere (50 psi) in Parr shaker at ambient temperature for 2 h. The reaction mixture was filtered through Celite and the filtrate was concentrated *in vacuo.* The obtained crude material was triturated with MTBE to obtain the title compound (12.1 g, 99%) as a solid. MS (ES+) 264.9 [M+H]⁺.

### Step c) tert-butyl 3-(2-oxo-2,3-dihydro-1H-imidazo[4,5-c]pyridin-1-yl)azetidine-1-carboxylate (I-15c)

CDI (2.38g, 14.7 mmol, 1.05 eq) was added at 0 °C to a stirred solution of compound I-15b (3.7 g, 14 mmol, 1 eq) in acetonitrile (40 mL). The reaction mixture was stirred at ambient temperature for 1h, then filtered and the solid residue was triturated with acetonitrile and dried under reduced pressure to get the title compound (2.7 g, 66%) as a solid. MS (ES+) 291.2 [M+H]⁺.

### Step d) 1-(azetidin-3-yl)-1H-imidazo[4,5-c]pyridin-2(3H)-one (I-15d)

HCl (4M in dioxane, 20 mL) was added at 0 °C to a stirred solution of compound I-15c (4.2 g, 14.5 mmol, 1 eq) in dioxane (5 mL). The reaction mixture was stirred at ambient temperature for 1h, then filtered and the solid residue was washed with MTBE to get the title compound (3.6 g).

### Step e) 1-(1-(cyclopropylsulfonyl)azetidin-3-yl)-1H-imidazo[4,5-c]pyridin-2(3H)-one (I-15e)

DIPEA (2.5 eq) was added at ambient temperature to a stirred solution of compound I-15d (0.4 g, 0.53 mmol) in DCM. The mixture was cooled to 0 °C, then cyclopropanesulfonyl chloride was slowly added and the mixture was stirred for 1h at 0 °C, then at rt for 12h. The reaction mixture was diluted with water and extracted with EtOAc. The organic layer was dried over anhyd. Na₂SO₄ and concentrated under reduced pressure which gave the title compound (73 mg).

### Intermediate 16

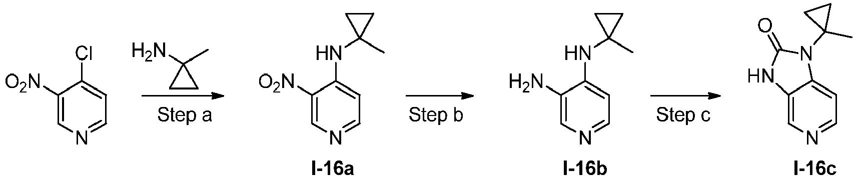

### Step a) N-(1-methylcyclopropyl)-3-nitropyridin-4-amine (I-16a)

TEA (1.8 mL, 12.66 mmol) was added to a stirred solution of 4-chloro-3-nitropyridine (1.0 g, 6.32 mmol) in EtOH (20 mL) followed by addition of the HCl salt of 1-methylcyclopropanamine (0.74 g, 6.96 mmol) under N₂ atmosphere, at an ice cooled condition. The resulting mixture was stirred at 80 °C for 16 h, then concentrated under reduced pressure. The residue was diluted with water and extracted with EtOAc. The organic layer was washed with water and brine, dried (Na₂SO₄) and concentrated under reduced pressure. The afforded crude product was purified by silica gel column chromatography using 30% EtOAc in hexane, which gave the title compound (410 mg, 60%) as a solid. MS (ES+) 193.6 [M+H]⁺.

### Step b) N⁴-(1-methylcyclopropyl)pyridine-3,4-diamine (I-16b)

Pd/C (10%, 50% moist, 50 mg) was added to a stirred, with argon degassed solution of compound I-16a (0.38 g, 1.96 mmol) in EtOH (10 mL). The resulting reaction mixture was shaken in Parr shaker under hydrogen atmosphere (40 psi) at ambient temperature for 16 h, then filtered through a pad of Celite and the filtrate was concentrated under reduced pressure which gave the title compound (290 mg, 90%) as a solid. MS (ES+) 164.0 [M+H]⁺.

### Step c) 1-(1-methylcyclopropyl)-1H-imidazo[4.5-c]pyridin-2(3H)-one (I-16c)

DIPEA (0.9 mL, 5.34 mmol) was added under nitrogen atmosphere to a stirred solution of compound I-16b (0.29 g, 1.8 mmol) in DCM (10 mL) followed by addition of CDI (0.346 g, 2.14 mmol). The reaction mixture was stirred at ambient temperature for 16 h, then diluted with water and the mixture extracted with DCM. The organic layer was washed with water and brine, dried (Na₂SO₄), filtered and concentrated under reduced pressure to afford crude compound as dark brown solid. The crude compound was purified by silica gel (100-200 mesh) column chromatography using 4% MeOH in DCM to obtain compound RSV_BB132 (150 mg, 45%) as off white solid. MS (ES+) 189.09 [M+H]⁺.

### Intermediate 17

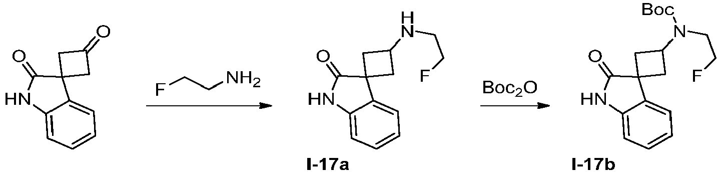

### Step a) 3-((2-Fluoroethyl)amino)spiro[cyclobutane-1,3'-indolin]-2'-one (I-17a)

2-Fluoroethanamine hydrochloride (691 mg, 6.94 mmol) and TEA (1.94 mL, 13.9 mmol) were added under nitrogen at rt to a stirred solution of spiro[cyclobutane-1,3'-indoline]-2',3-dione (1.3 g, 6.94 mmol) in MeOH (20 mL). The reaction mixture was stirred at rt for 1h, then sodium cyanoborohydride (873 mg, 13.9 mmol) was added and the resulting reaction mixture was stirred at rt for 12 h. The reaction mixture was diluted with water (50 mL), extracted with EtOAc (2 x 50 mL) and the combined organic layers were washed with water (20 mL) and brine (10 mL). The organic layer was dried over sodium sulfate, filtered and concentrated under reduced. The crude material was purified by flash chromatography on silica gel eluted with 4% MeOH in DCM which gave the title compound (1.3 g, 60%) as a solid. MS (ES+) 235.13 [M+H]⁺.

### Step b) Tert-butyl (2-fluoroethyl)(2'-oxospiro[cyclobutane-1,3'-indolin]-3-yl)carbamate (I-17b)

Boc anhydride (1.29 mL, 5.64 mmol) was added under nitrogen at 0 °C to a stirred solution of I-17a (1.1 g, 4.70 mmol and TEA (1.31 mL) in DCM (30 mL). The afforded mixture was stirred at rt for 3h, then diluted with water (50 mL) and extracted with DCM (2 x 50 mL). The combined organic layers were washed with water (50 mL) and brine (50 mL), dried over sodium sulfate, filtered and concentrated under reduced. The crude material was purified by flash chromatography eluted with 3% MeOH in DCM, which gave the title compound (650 mg, 41%). MS (ES+) 335.26 [M+H]⁺.

### Intermediate 18

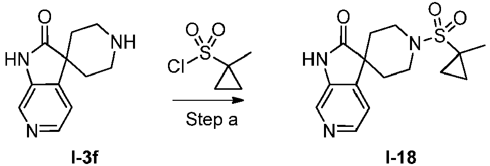

### 1-((1-methylcyclopropyl)sulfonyl)spiro[piperidine-4,3'-pyrrolo[2,3-c]pyridin]-2'(1'H)-one (I-18)

To a stirred solution of compound I-3f (0.50 g, 2.46 mmol) in pyridine (6 mL) was added 1-methylcyclopropane-1-sulfonyl chloride (0.61 g, 3.94 mmol) in pyridine (2 mL) under nitrogen atmosphere at 0 °C. The reaction mixture for 6 h at rt, then diluted with cold water (30 mL) and extracted with EtOAc (2x50 mL). The combined organic layers were washed with brine and cold water (2x30 mL), dried over anhydrous sodium sulphate and concentrated under reduced pressure. The afforded crude was purified by column chromatography on neutral alumina eluted with 2% MeOH:DCM, which gave the title compound (110 mg, 10%). MS (ES+) 322.98 [M+H]⁺.

### Intermediate 19

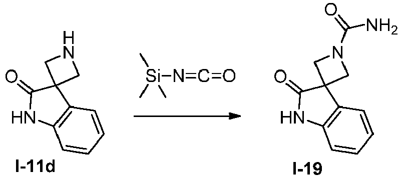

### 2'-oxospiro[azetidine-3,3'-indoline]-1-carboxamide (I-19)

To a stirred solution of the TFA salt of I-11d (1.1 g) in DCM (20 mL) was added triethylamine (1.9 mL) and (trimethylsilyl)isocyanate (0.67 mL) at rt. The resulting mixture was stirred at room temperature for 1 h, whereafter the formed solid was filtered and dried, which gave the title compound (400 mg, 46%). MS (ES+) 218.26 [M+H]⁺.

### Intermediate 20

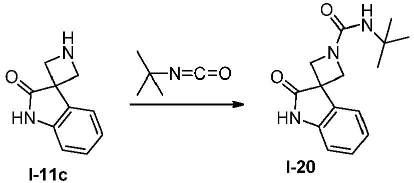

### N-(tert-butyl)-2'-oxospiro[azetidine-3,3'-indoline]-1-carboxamide (I-20)

To a stirred suspension of the TFA salt of I-11d (300 mg, 1.72 mmol) in DCM (10 mL) was added triethyl amine (0.266 mL) at 0 °C. The suspension was stirred for 5 min, then tert-butylisocyanate (256 mg, 2.58 mmol) was added and the stirring was continued at rt for 2 h. The reaction mixture was concentrated under vacuum which gave the crude title compound (300 mg, 73%). MS (ES+) 274.24 [M+H]⁺.

### Intermediate 21

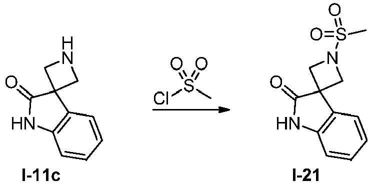

### 1-(Methylsulfonyl)spiro[azetidine-3,3'-indolin]-2'-one (I-21)

To a stirred solution of I-11c (1.8 g) in DMF (15 mL) was added triethylamine (2.6 mL) and methanesulfonyl chloride (0.48 mL) at 0 °C. The resulting mixture was stirred at 0 °C for 1 h, then diluted with ice water and stirred for 10 minutes, solid was formed and filtered and dried which gave the title compound (480 mg, 30%) as a solid. MS (ES+) 253.14 [M+H]⁺.

### Intermediate 22

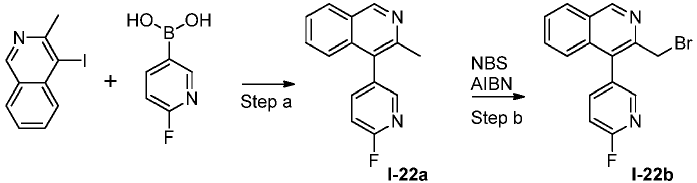

### Step a) 4-(6-fluoropyridin-3-yl)-3-methylisoquinoline (I-22a)

(6-Fluoropyridin-3-yl)boronic acid (1.05 g, 7.43 mmol) and sodium carbonate (1.97 g, 18.6 mmol) were added under argon to a solution of 4-iodo-3-methylisoquinoline (2.00 g, 7.43 mmol) in 1,2-dimethoxyethane (25 mL) and water (10 mL). The reaction mixture was degassed with argon for 15 minutes then 1,1'-bis(diphenylphosphino)ferrocene]-dichloropalladium(II), complex with dichloromethane (1.09 g, 1.49 mmol) was added under argon at rt. The reaction mixture was stirred at 100 °C in a sealed tube. When the reaction was deemed completed as judged by TLC (∼6h), the mixture was cooled to rt, water (100 mL) was added and the mixture was extracted with EtOAc (2 X100 mL). The combined organic phases were dried (Na₂SO₄), filtered and concentrated. The afforded crude compound was purified by column chromatography on silica gel, eluted with 20-30% EtOAc in p. ether, which gave the title compound (1.4 g, 72%) as a solid. MS (ES+) 239.43 [M+H]⁺.

### Step b) 3-(bromomethyl)-4-(6-fluoropyridin-3-yl)isoquinoline (I-22b)

Azobisisobutyronitrile (103 mg) and N-bromosuccinimide (822 mg) were added at 0 °C to a stirred solution of compound I-22a (1.00 g) in carbon tetrachloride (50 mL). The reaction mixture was stirred at 70 °C and the progress was monitored by TLC. After 6 h, the mixture was diluted with saturated sodium bicarbonate solution (100 mL) and extracted with DCM (2X50 mL). The combined organic layers were dried (Na₂SO₄), filtered and concentrated under reduced pressure. The afforded crude was purified by column chromatography on silica gel eluted with 24% EtOAc in p. ether, which gave the title compound (700 mg, 43%) as a solid. MS (ES+) 318.85 [M+H]⁺.

The following isoquinoline derivatives s were prepared using the method described for Intermediate 22, using the appropriate boronic acid, and reaction times as judged by TLC:

| | | |
|---|---|---|
| 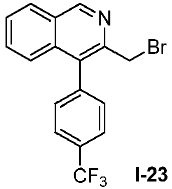 | 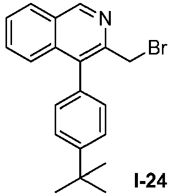 | 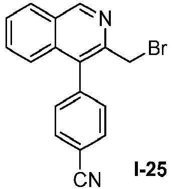 |
| Yield 28%, MS 366.22 [M+H]⁺ | Yield 43%, MS 356.37 [M+H]⁺. | Yield 57%, MS 323.15 [M+H]⁺ |
| 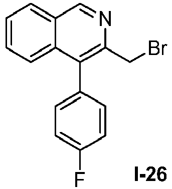 | 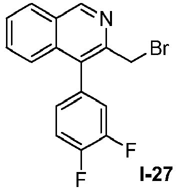 | 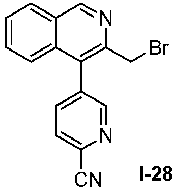 |
| Yield 39%, MS 318.07 [M+H]⁺ | Yield 38%, MS 336.27 [M+H]⁺ | Yield 15%, MS 324.20 [M+H]⁺ |
| 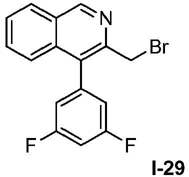 | 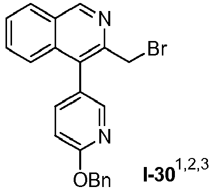 | |
| Yield 36%, MS 336.27 [M+H]⁺ | Yield 4.3 %, MS 406.91 [M+H]⁺. | |

| | | |
|---|---|---|
| ¹Tetrakis(triphenylphosphine)-palladium(0) was used as catalyst in step a. ²1,4-Dioxane/H₂O was uses as solvent in step a. ³The (4,4,5,5-tetramethyl-1,3,2-dioxaborolan-2-yl) derivative was used instead of the corresponding boronic acid derivative in step a. | | |

### Intermediate 31

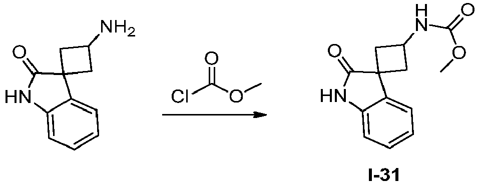

### Spiro[cyclobutane-1,3'-indoline]-2',3-dione (I-31)

To a stirred solution of 3-aminospiro[cyclobutane-1,3'-indolin]-2'-one (1,0 g, 5.3 mmol, prepared as described in WO2014/060411) in THF (30 mL) was added TEA (1.48 mL, 10.6 mol) at rt under nitrogen. The reaction mixture was cooled to 0 °C, then methyl chloroformate (493 µl, 6.38 mmol) was added The resulting reaction mixture was stirred at rt for 1 h, diluted with water (100 mL), extracted with EtOAc (2 x 200 mL). The combined organic layers were washed with water (100 mL) and saturated bicarbonate solution (50 mL), dried over sodium sulfate, filtered and concentrated under reduced pressure. The crude material was purified by silica gel column chromatography (eluent 1% MeOH in DCM), which gave the title compound (700 mg, 49%) as a solid. MS (ES+) 247.20 [M+H]⁺.

### Intermediate 32

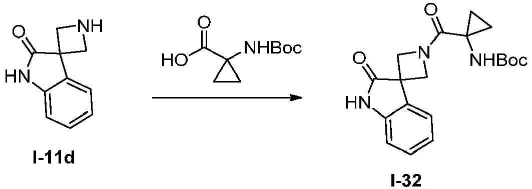

### Tert-butvl (1-(2'-oxospiro[azetidine-3,3'-indolin]-1-ylcarbonyl)cyclopropyl)carbamate (I-32)

To a stirred solution of 1-((tert-butoxycarbonyl)amino)cyclopropanecarboxylic acid (1.36 g, 6.77 mmol) in DMF (20 mL) was added EDAC (1.20 g, 6.24 mmol), HOBt (0.960 g, 6.24 mmol), Et₃N (2.1 mL, 20.8 mmol) and I-11d (1.50 g, 5.20 mmol) at rt. The resulting reaction mixture was stirred at room temperature for 16h, then diluted with water (30 mL) and extracted with EtOAc (2x30 mL). The organic layer was washed with water (3x50 mL), dried (Na₂SO₄), filtered and concentrated under reduced pressure which gave the title compound (800 mg, 41%) as a solid. MS (ES+) 358.26 [M+H]⁺.

### Intermediate 33

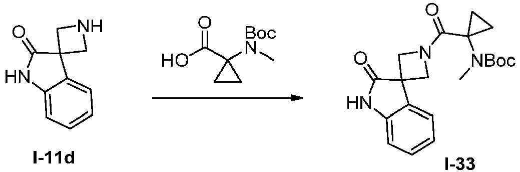

### Tert-butvl methyl(1-(2'-oxospiro[azetidine-3,3'-indolin]-1-ylcarbonyl)cyclopropyl)carbamate (I-33)

The TFA salt of compound I-11d (536 mg, 2.0 mmol) was reacted with 1-((tert-butoxycarbonyl)(methyl)amino)cyclopropanecarboxylic acid according to the method described for Intermediate 32, which gave the title compound (350 mg, 41%). MS (ES+) 372.27 [M+H]⁺.

### Intermediate 34

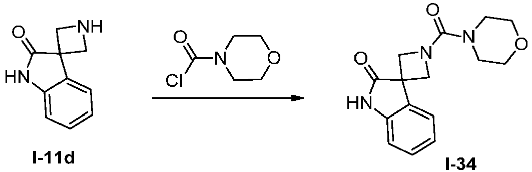

### 1-(morpholine-4-carbonyl)spiro[azetidine-3,3'-indolin]-2'-one (I-34)

A solution of morpholine-4-carbonyl chloride (519 mg) in DCM (10 mL) was added during 5 min to a stirred solution of compound I-11d (500 mg) in a mixture of DCM (10 mL) and 10 % sodium hydroxide (10 mL). The reaction mixture was stirred at rt for 3 h, then the phases were separated and the sodium hydroxide layer was washed with DCM (50 mL). The combined DCM layers were washed with brine (20 mL), dried over sodium sulfate, filter, concentrated and dried under vacuum, The afforded sold was washed with MeCN (4 mL), which gave the title compound (400 mg, 69% yield) as a solid. MS (ES+) 288.42 [M+H]⁺.

### Intermediate 35

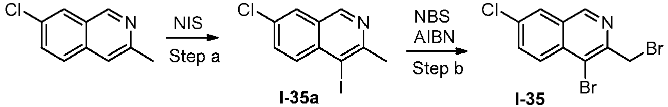

### Step a) 4-iodo-3-methylisoquinoline (I-35a)

To a stirred solution of 7-chloro-3-methylisoquinoline (4.80 g, 20.8 mmol) in acetic acid (50 mL) was added N-lodo succinimide (5.7 g, 25.3 mmol). The mixture was heated at 80 °C for 3 days, then cooled to rt. and the reaction was quenched with saturated sodium hydroxide solution (100 mL) and extracted with ethyl acetate (2 x 300 mL). The combined organic phases were washed with saturated sodium thiosulfate solution (100 mL), dried over sodium sulfate, filtered and concentrated under reduced pressure. The afforded crude compound was purified by column chromatography on silica gel eluted with 10% EtOAc in p. ether. Pure fractions were collected and concentrated under reduced pressure which gave the title compound (4 g, 62%). The structure was confirmed by ¹H NMR.

### Step b) 4-bromo-3-(bromomethyl)-7-chloroisoquinoline (I-35b)

To a stirred solution of compound I-35a (4 g) in CCl₄ (400 mL), were added azobisisobutyronitrile (425 mg) and N-bromosuccinimide (9.3 g) at rt under nitrogen. The resulting reaction mixture was at reflux for 18 h, then cooled to rt. A saturated solution of Na₂S₂O₃ (100 mL) was added and the mixture was extracted with DCM (2x250 mL). The combined organic phases were dried over sodium sulfate, filtered and concentrated under reduced pressure. The crude product was purified by silica gel column chromatography eluting with 15-20% EtOAc in p. ether, which gave the title compound together with an inseparable byproduct (2.1 g). The material was used as such in following steps without further purification.

### Intermediate 36

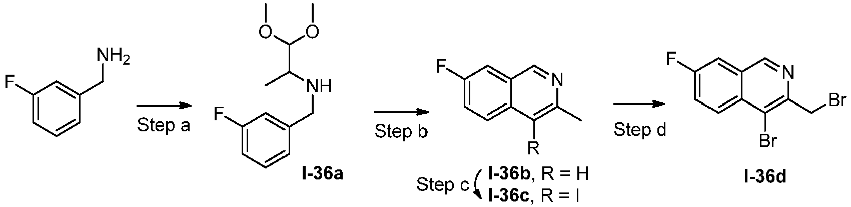

### Step a) N-(3-fluorobenzyl)-1,1-dimethoxypropan-2-amine (I-36a)

To a stirred solution of (3-fluorophenyl)methanamine (20.0 g) in 1,2-dichloroethane (800 mL) was added pyruvic aldehyde dimethyl acetal (28.3 g) at rt followed by addition of sodium triacetoxyborohydride (67.7 g) the mixture was stirred for 16 h, then aqueous 2 N NaOH (100 mL) solution was added and the mixture was stirred until the organic layer was almost clear. The layers were separated, the aqueous layer was extracted with DCM (2x50 mL).The combined organics were dried over Na₂SO₄, filtered and concentrated under reduced pressure to obtain crude title compound (32 g). MS (ES+) 228.17 [M+H]⁺.

### Step b) 7-fluoro-3-methylisoquinoline (I-36b)

Compound 36a (32 g, 141 mmol) was added dropwise at <5 °C to chlorosulfonic acid (96.5 mL, 1.41 mmol). The mixture was heated to 100 °C for 10 min, then cooled and poured into ice (700 g). The mixture was washed with MTBE (700 mL), the aqueous layer was cooled to 5 °C and basified pH14 with 50% aq NaOH solution (400 mL). The aqueous layer was extracted with DCM (2 x 100 mL). The combined organic layers were dried over Na₂SO₄, filtered and concentrated under reduced pressure to obtain crude title compound (11 g, 34%). MS (ES+) 162.09 [M+H]⁺.

### Step c) 3-(bromomethyl)-7-fluoro-4-iodoisoquinoline (I-36c)

N-iodo succinimide (8.38 g) was added to a stirred solution of I-36b (5 g) in acetic acid (50 mL). The reaction mixture was heated at 80 °C for 3 days, then cooled to rt. Sodium hydroxide solution (150 mL) was added and the mixture was extracted with ethyl acetate (2 x 200 mL). The combined organic phases were washed with saturated sodium thiosulfate solution (100 mL), dried over sodium sulfate, filtered and concentrated under reduced pressure. The obtained crude compound was purified by column chromatography on silica gel eluted with 10% EtOAc in p. ether. Pure fractions were pooled and concentrated under reduced pressure which gave the title compound (2, 3 g, 33%). MS (ES+) 288.01 [M+H]⁺.

### Step d) 4-bromo-3-(bromomethyl)-7-fluoroisoquinoline (I-36d)

Azobisisobutyronitrile (343 mg) and N-bromosuccinimide (7.44 g) were added at rt under nitrogen to a stirred solution of compound I-36c (3.0 g, 10.4 mmol) in CCl₄ (300 mL). The resulting mixture was stirred at reflux for 18 h, then cooled to rt. A solution of Na₂S₂O₇ (100 mL) was added and the mixture was extracted with DCM(2 x 250 mL). The combined organic layers were dried over sodium sulfate, filtered and concentrated under reduced pressure. The obtained crude product was combined with a previously prepared batch and purified by column chromatography on silica eluting with 15-20% EtOAc in p. ether. Pure fractions were pooled and concentrated under reduced pressure which gave the title compound.

### Intermediate 37

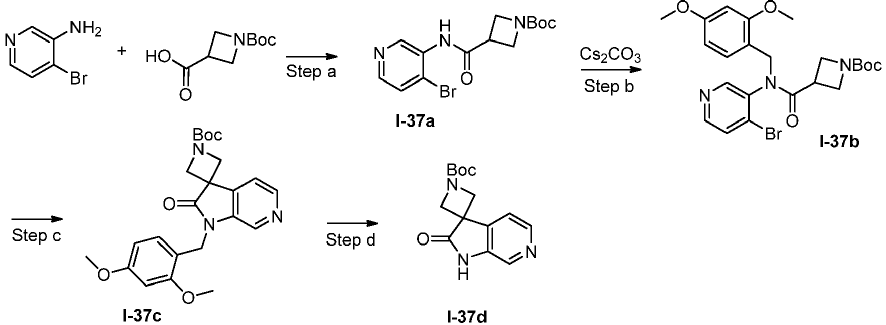

### Step a) tert-butyl 3-((4-bromopyridin-3-yl)carbamoyl)azetidine-1-carboxylate (I-37a)

To a solution of 3-amino-4-bromopyridine (5.05 g, 29.2 mmol) and N -boc-azetidine-3-carboxylic acid (6.17 g, 30.6 mmol) in dry DCM (100 mL) was added DMAP (4.64 g, 38.0 mmol) and EDC-hydrochloride (7.27 g, 38.0 mmol). The mixture was stirred at rt for three days, then diluted with ethyl acetate and washed twice with water and brine. The water phase was extracted once with ethyl acetate and the combined organic phases were dried over sodium sulfate and concentrated under reduced pressure. The product was isolated by silica gel chromatography eluted with DCM and 0 to 3% MeOH, which gave the title compound (9.6 g, 92%). MS (ES+) 356.2 & 358.2 [M+H]⁺.

### Step b) Tert-butyl 3-((4-bromopyridin-3-yl)(2,4-dimethoxybenzyl)carbamoyl)azetidine-1-carboxylate (I-37b)

To a solution of I-37a (7.12 g, 20.0 mmol) in dry DMF (25 mL) was added cesium carbonate (1.63 g, 50.0 mmol) and the mixture was stirred for 30 minutes at rt. A solution of 1-(chloromethyl)-2,4-dimethoxybenzene (8.77 g, 47.0 mmol) in benzene (∼10 mL) was added and the mixture was stirred for two h at rt. Water was added and the mixture extracted three times with ethyl acetate. The organic phase was washed with brine, dried over sodium sulfate and concentrated under reduced pressure. The product was purified by silica gel chromatography eluted with DCM and 10 to 50% EtOAc, which gave the title compound (10.1 g, 79%).

### Step c) Tert-butyl 1'-(2,4-dimethoxybenzyl)-2'-oxo-1',2'-dihydrospiro[azetidine-3,3'-pyrrolo[2,3-c]pyridine]-1-carboxylate (I-37c)

Sodium-tert-butoxide (2.28 g, 23.7 mmol), palladium(II)acetate (355 mg, 1.58 mmol) and tricyclohexylphosphine (443 mg, 1.58 mmol) were added under argon to a solution of I-37b in dry dioxane (85 mL). The mixture was stirred under argon for at 95 °C two hours, then cooled to rt and added to a saturated ammonium chloride solution. The mixture was extracted four times with DCM, the organic phase was dried over sodium sulfate and concentrated under reduced pressure. The product was isolated by silica gel chromatography eluted with DCM and 20 to 60% EtOAc, which gave the title compound (6.72 g, 80%). MS (ES+) 426.4 [M+H]⁺.

### Step d) tert-butyl 2'-oxo-1',2'-dihydrospiro[azetidine-3,3'-pyrrolo[2,3-c]pyridine]-1-carboxylate (I-37d)

A solution of I-37c (3.22 g, 7.58 mmol) in acetonitrile (80 mL) was added to an ice cooled solution of ammonium cerium nitrate (3.29 g) in water (40 mL). The reaction mixture was stirred for two hours at rt, then additional ammonium cerium nitrate (1,64 g) was added and the mixture was stirred for two more hours at rt. 5% potassium carbonate solution (400mL) was added and the mixture was extracted four times with ethyl acetate. The organic phase was dried over sodium sulfate and concentrated under reduced pressure. The product was isolated by silica gel chromatography eluted with DCM and 2 to 8% MeOH, which gave the title compound (1.05 g, 50%). MS (ES+) 276.3 [M+H]⁺.

### Intermediate 38

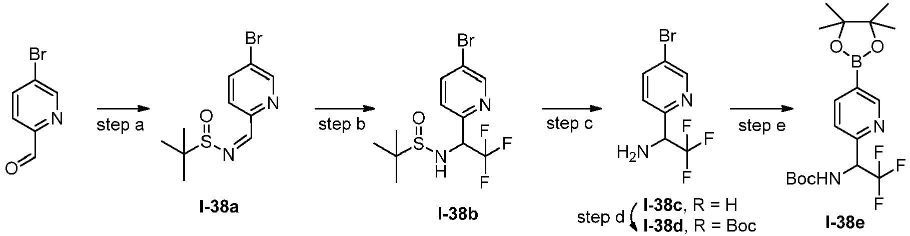

### Step a) N-((5-bromopyridin-2-yl)methylene)-2-methylpropane-2-sulfinamide (I-38a)

Cesium carbonate (2.5 g, 7.68 mmol) was added to a mixture of 5-bromo-2-pyridinecabonaldehyde (1.19 g, 6.4 mmol) and 2-methyl-2-propanesulfinamide (0.78 g, 6.4 mmol) in DCM (6 mL). The mixture was stirred at rt for 20 h, then diluted with DCM, washed with H₂O, dried over Na₂SO₄ and concentrated. Purification by column chromatography on silica gel, gradient elution with EtOAc 0 to 51% in n-heptane yielded the title compound (1.76 g, 95%). MS (ES+) 289.1; 291.1 [M+H]⁺.

### Step b) N-(1-(5-bromopyridin-2-yl)-2,2,2-trifluoroethyl)-2-methylpropane-2-sulfinamide (I-38b)

A solution of N-((5-bromopyridin-2-yl)methylene)-2-methylpropane-2-sulfinamide (1.91 g, 6.6 mmol) and tetramethyl ammoniumfluoride (738 mg, 7.93 mmol) in THF (20 mL) at rt was purged with Ar for 15 min. To this was added trifluromethyltrimethylsilane TMSCF₃ (2.44 mL, 16.5 mmol) at -55 °C /-60 °C. The reaction mixture was stirred at same temperature for 1h, then the reaction was quenched by addition of aq. sat NH₄Cl solution (15 mL) at 0 °C. The organic layer was separated, the water phase was extracted with EtOAc (2x 20 mL) and the combined organic layers were dried over Na₂SO₄ and concentrated at reduced pressure. Purification by column chromatography on silica gel, gradient elution with EtOAc in n-heptane, yielded the title compound (1.80 g, 76%). MS (ES+) 359.1; 361.2 [M+H]⁺.

### Step c) 1-(5-bromopyridin-2-yl)-2,2,2-trifluoroethanamine hydrochloride (I-38c)

4M HCl in 1,4-dioxane (0.52 ml) was added to a solution of N-(1-(5-bromopyridin-2-yl)-2,2,2-trifluoroethyl)-2-methylpropane-2-sulfinamide (148 mg, 0.41 mmol) in MeOH (0.5 mL) and the reaction was stirred for 2 h . The mixture was concentrated at reduced pressure, co-evaporated with toluene and dried at reduced pressure for 18 h to give the title compound (120 mg, 99 %). MS (ES+) 255.1; 257.1 [M+H]⁺.

### Step d) tert-Butyl (1-(5-bromopyridin-2-yl)-2,2,2-trifluoroethyl)carbamate (I-38d)

Di-tert-butyl dicarbonate (180 mg, 0.82 mmol) was added to a solution of 1-(5-bromopyridin-2-yl)-2,2,2-trifluoroethanamine hydrochloride (120 mg, 0.41 mmol), pyridine (0.07 ml, 0.82 mmol) and 4-4-dimethylaminopyridine (10.1 mg, 0.08 mmol) in 1,4-dioxane (3 mL). The obtained reaction mixture was stirred for 24 h. The solids were filtered off and the filtrate was concentrated under reduced pressure. Purification by column chromatography on silica gel, gradient elution with EtOAc in n-heptane, gave the title compound (146 mg, 99%). MS (ES+) 299.1; 301.1 [M+H]+.

### Step e) tert-Butyl (2,2,2-trifluoro-1-(5-(4,4,5,5-tetramethyl-1,3,2-dioxaborolan-2-yl)pyridin-2-yl)ethyl)carbamate (I-38e)

A suspension of *tert*-Butyl (1-(5-bromopyridin-2-yl)-2,2,2-trifluoroethyl)carbamate (146 mg, 0.41 mmol), bis(pinacolato)diboron (113 mg, 0.44 mmol), Pd(dppf)Cl₂ (15,04 mg, 0,02 mmol) and potassium acetate (80,69 mg, 0,82 mmol) in 1,4-dioxane (3 mL) was degassed by passing N₂ gas through for 3-5 min. The reaction was then heated by microwave irradiation at 130 °C for 1 h. The mixture was then partitioned between EtOAc and 5% NaHCO₃. The organic layer was dried over Na₂SO₄ and concentrated at reduced pressure. Purification with silica gel chromatography, gradient elution with EtOAc in n-heptane, gave the title compound (140 mg, 85%). MS (ES+) 403.4 [M+H]+.

### Intermediate 39

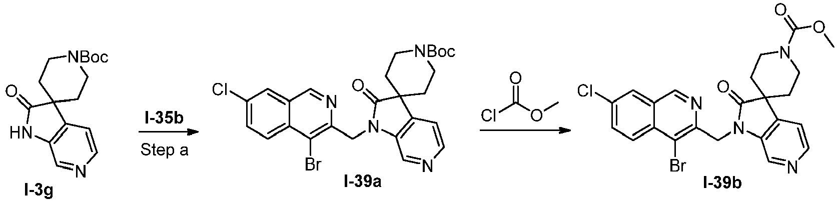

### Step a) Tert-butyl 1'-((4-bromo-7-chloroisoquinolin-3-yl)methyl)-2'-oxo-1',2'-dihydrospiro[piperidine-4,3'-pyrrolo[2,3-c]pyridine]-1-carboxylate (I-39a)

Cesium carbonate (2.1g, 6.45 mmol) was added to a solution of I-3g (633mg, 2.09 mmol) in DMF (20 ml).The slurry was stirred at rt for 1h, then I-35b (700 mg, 2,09 mmol) was added and the mixture was stirred overnight. The reaction mixture was extracted between EtOAc and H₂O. The aq. phase further extracted with EtOAc (x 2). The pooled organic phases were dried over MgSO₄, concentrated and dried in vacuum. The crude material was purified by chromatography on silica gel eluting with 0-5 % MeOH in DCM, which gave the title compound (843 mg, 72 %). MS (ES+) 559.33 [M+H]⁺.

### Step b) Methyl 1'-((4-bromo-7-chloroisoquinolin-3-yl)methyl)-2'-oxo-1',2'-dihydrospiro[piperidine-4,3'-pyrrolo[2,3-c]pyridine]-1-carboxylate (I-39b)

Compound I-39a (501 mg, 0.898 mmol) was sirred with 4M HCI in dioxane (6.75 mL), MeOH (3 mL), and DCM (11 mL) for 1h 15 min at rt. The mixture was concentrated under vacuum, and then coevaporatd 2x from toluene to give pale yellow solids of the HCI salt. DCM (4 mL) was added, followed by DIEA (0.8 mL, 4.6 mmol) and methyl chloroformate. The mixture was stirred for 1h 45 min, then was under vacuum and co-evaporated several times with more DCM. The afforded oil. Purification by silica chromatography (gradient 1% to 7% MeOH in DCM) gave the title compound as solids (410 mg, 88%). MS (ES+) 517.3 [M+H].

### Intermediate 40

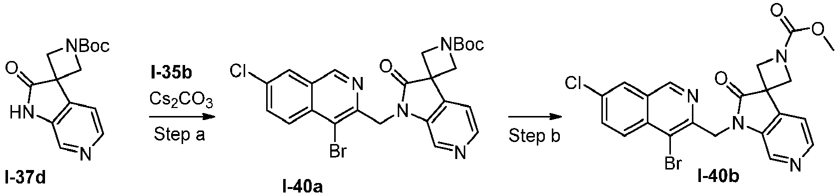

### Step a) tert-butyl 1'-((4-bromo-7-chloroisoquinolin-3-yl)methyl)-2'-oxo-1',2'-dihydrospiro[azetidine-3,3'-pyrrolo[2,3-c]pyridine]-1-carboxylate (I-40a)

Cesium carbonate (7.2 g, 22.1 mmol) was added under nitrogen at rt to a stirred solution of I-37d (2.0 g, 7.26 mmol) in acetonitrile (30 mL). The reaction mixture was stirred for 15 min, then I-35b (2.5 g, 7.45 mmol) was added. The resulting reaction mixture was stirred for 3 h, then concentrated under reduced pressure. The residue was diluted with water (80 mL), extracted with 5% MeOH in DCM (3 x 100 mL). Combined organic layers were washed with water (80 mL), dried over sodium sulfate, filtered and concentrated under reduced pressure. The obtained solid was triturated with diethyl ether (30 mL), stirred for 15 min and filtered which gave the title compound (2.9 g, 68%) as a solid. The compound was used in next step without further purification. MS (ES+) 531.07 [M+H]⁺.

### Step b) methyl 1'-((4-bromo-7-chloroisoquinolin-3-yl)methyl)-2'-oxo-1',2'-dihydrospiro[azetidine-3,3'-pyrrolo[2,3-c]pyridine]-1-carboxylate (I-40b)

TFA (5 mL, 64.9 mmol) was added at rt to a stirred solution of I-40a (1.0 g, 1.88 mmol) in DCM (5 mL) The mixture was stirred at for 2 h, then concentrated under reduced pressure, diluted with DCM (80 mL) and washed with saturated sodium bicarbonate solution (2 x 30 mL). The organic layer was washed with water (15 mL), dried over sodium sulfate, filtered and concentrated under reduced pressure. The residue was dissolved in DCM (20 mL) and ET₃N (2.6 mL, 18.7 mmol) and methyl chloroformate (220 mg, 2.33 mmol) were added at 0 °C under nitrogen. The resulting mixture was stirred at rt for 2 h, then diluted with DCM (50 mL) and washed with water (30 mL). The organic layer was dried over sodium sulfate filtered and concentrated under reduced pressure. The crude material was purified by flash chromatography on silica gel eluted with 5% MeOH in DCM, which gave the title compound (620 mg, 57%) as a solid. MS (ES+) 489.25 [M+H]⁺.

### Example 1

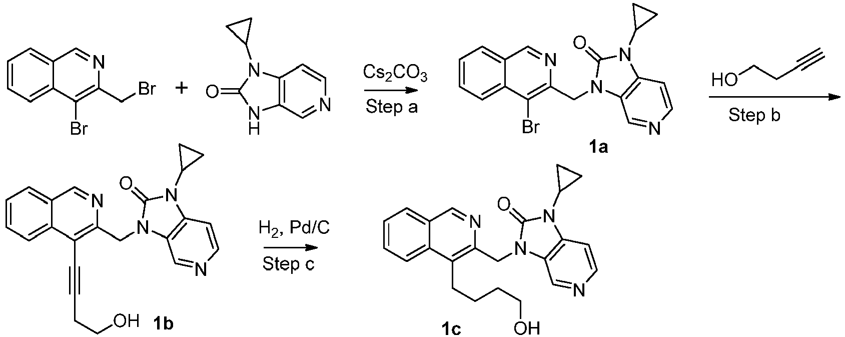

### Step a) 3-((4-Bromoisoquinolin-3-yl)methyl)-1-cyclopropyl-1H-imidazo[4,5-c]pyridin-2(3H)-one (1a)

Cesium carbonate (2.44 g, 7.50 mmol) was added to a solution of 4-bromo-3-(bromomethyl)isoquinoline (755 mg, 2.51 mmol) and 1-cyclopropyl-1H-imidazo[4,5-c]pyridin-2(3H)-one (442 mg, 2.52 mmol) in DMF (24 mL) and the mixture was stirred at RT for 3h. Ice-water (150 mL) was added and the mixture was stirred for 30 min, then filtered and the formed precipitates were washed with water. The precipitates were dissolved in CH₂Cl₂ with some MeOH, solvent was evaporated under vacuum and the afforded crude product was purified on a silica column which gave the title compound as solids (0.542 g, 55%). MS (ES+) 396.95 [M+H]⁺.

### Step b) 1-cyclopropyl-3-((4-(4-hydroxybut-1-yn-1-yl)isoquinolin-3-yl)methyl)-1H-imidazo[4,5-c]pyridin-2(3H)-one (1b)

To a suspension of bromoquinoline 1a (100 mg, 0.254 mmol) in DMF (3 mL) were added in succession PPh₃ (14.2 mg, 0.054 mmol), Cul (7.40 mg, 0.039 mmol), Et₂NH (276 mg, 3.77 mmol), but-3-yn-1-ol (23.3 mg, 0.333 mmol), and lastly Pd(PPh₃)₂Cl₂ (18.7 mg, 0.027 mmol). The suspension was heated in a microwave reactor at 120 °C for 30 min, then allowed to cool to rt. The above procedure was repeated once in double size and the two solutions combined, diluted with water and extracted with EtOAc (3x30 mL). The organic phases were combined, washed with water (2 x 20 mL), dried (Na₂SO₄), and concentrated under vacuum. The afforded crude was purified by flash chromatography on 35 grams silica eluted with a gradient of 2 - 6% MeOH in DCM which gave the title compound (217.6 mg, 74.5%).
LCMS (ES+) 385.06 [M+H]⁺. ¹H NMR in CDCl₃ is consistent with structure.

### Step c) 1-cyclopropyl-3-((4-(4-hydroxybutyl)isoquinolin-3-yl)methyl)-1H-imidazo[4,5-c]pyridin-2(3H)-one (1c)

A solution of the alkyne 1b (108 mg, 0.282 mmol) in 95% EtOH (19 mL) + MeOH (1 mL) was hydrogenated at RT using H₂ (g) in a balloon and 10% Pd on carbon (105 mg). After 18h, additional Pd/C (21 mg) MeOH (1 mL) and 95% EtOH (2 mL) were added and the mixture was stirred for additional 24h.The mixture was filtered through fluted filter paper, and the precipitates were washed several times with 95% EtOH (20 mL) and MeOH (30 mL). The solvents were removed under vacuum which gave the title compound (76.2 mg, 70%) as solids. MS (ES+) 389.1 [M+H]⁺. The structure was confirmed by ¹H and ¹³C NMR.

### Example 2

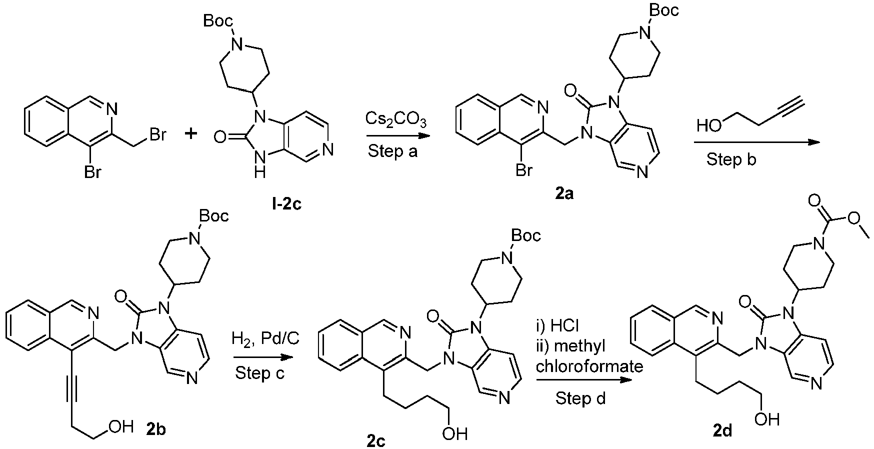

### Step a) Tert-butyl 4-(3-((4-bromoisoquinolin-3-yl)methyl)-2-oxo-2,3-dihydro-1H-imidazo[4,5-c]pyridin-1-yl)piperidine-1-carboxylate (2a)

Cesium carbonate (650 mg, 1.99 mmol) was added to a solution of 4-bromo-3-(bromomethyl)isoquinoline (200 mg, 0.665 mmol) and I-2c (212 mg, 0.665 mmol) in DMF (7.0 mL).The obtained slurry was stirred at rt for 2 h, then water (10 mL) and EtOAc (20 mL) were added. The water phase was extracted twice with EtOAc (2x10 mL), and the organic phase was washed twice with brine, then dried (Na₂SO₄) and concentrated. The obtained crude was purified by chromatography on silica eluting with DCM:MeOH 97:3, which gave the title compound (210 mg, 59%). MS (ES+) 540.15 [M+H]⁺. The structure was confirmed by ¹H NMR.

### Step b) Tert-butyl 4-(3-((4-(4-hydroxybut-1-yn-1-yl)isoquinolin-3-yl)methyl)-2-oxo-2,3-dihydro-1H-imidazo[4,5-c]pyridin-1-yl)piperidine-1-carboxylate (2b)

PdCl₂(PPh₃)₂ (13.7 mg, 0.020 mmol), copper iodide (14.9 mg, 0.078 mmol) and compound 2a (210 mg, 0.390 mmol) were dissolved in DMF (3.0 mL) in a microvial, and stirred until a clear solution. The vial was evacuated using nitrogen gas whereafter 3-butyn-1-ol (54.7 mg, 0.78 mmol) and TEA (98.7 mg, 0.975 mmol) were added. The vial was evacuated again and then heated using microwave irradiation, 110 °C for 75 min, then conventional heating, 80 °C for 16 hours. Another 1eq. of 3-butyn-1-ol, 0.05 eq PdCl₂(PPh₃)₂ and 0.2 eq copper iodide were added and the mixture was irradiated by microwave irradiation at 110 °C for 120 min. The mixture was concentrated to dryness before and the residue purified by chromatography on silica eluting with DCM:MeOH 95:5, which gave the title compound (151 mg, 73%). MS (ES+) 528.21 [M+H]⁺.

### Step c) tert-butyl 4-(3-((4-(4-hydroxybutyl)isoquinolin-3-yl)methyl)-2-oxo-2,3-dihydro-1H-imidazo[4,5-c]pyridin-1-yl)piperidine-1-carboxylate (2c)

A solution of compound 2b (40.0 mg, 0.076 mmol) in MeOH (10 mL) was hydrogenated in the presence of 10% Pd/C two loops in a H-cube (1 mL/min, 30 °C, 30 bar). The mixture was then used directly in next step.

### Step d) Methyl 4-(3-((4-(4-hydroxybutyl)isoquinolin-3-yl)methyl)-2-oxo-2,3-dihydro-1H-imidazo[4,5-c]pyridin-1-yl)piperidine-1-carboxylate (2d)

Compound 2c (28 mg, 0.053 mmol) was dissolved in MeOH 2.0 mL), 4M HCl in dioxane (3 mL) was added and the clear solution was stirred at rt for 30 min, then concentrated. The crude HCl salt was diluted with DMF and DIPEA (68.9 mg, 0.533 mmol) was added. The mixture was stirred at rt for 15 minutes then cooled in an ice-bath and methyl chloroformate (10.1 mg, 0.107 mmol) was added. The mixture was stirred at 0 °C for 90 minutes, then the reaction was quenched with 2 mL 1M NaOH (aq), diluted with EtOAc and washed with NaHCO₃ and brine, dried Na2SO4.filtered and concentrated. The residue was purified by column chromatography on silica and appropriate fractions were freeze dried, which gave the title compound (3 mg, 12%). MS (ES+) 490.2 [M+H]⁺. The structure was confirmed by ¹H and ¹³C NMR.

### Example 3

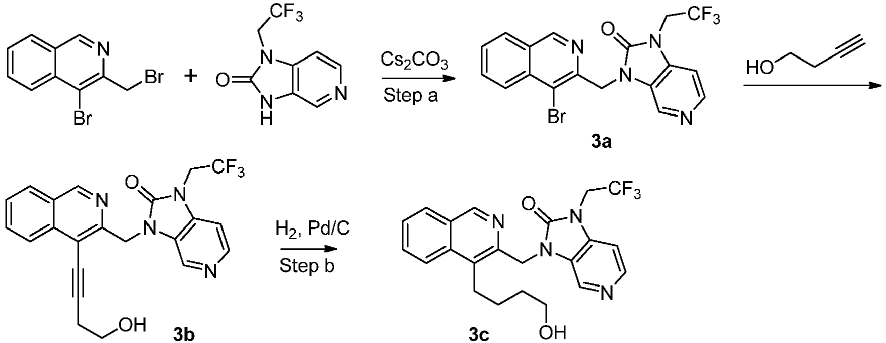

### Step a) 3-((4-bromoisoquinolin-3-yl)methyl)-1-(2,2,2-trifluoroethyl)-1H-imidazo[4,5-c]pyridin-2(3H)-one (3a)

4-bromo-3-(bromomethyl)isoquinoline (300 mg, 0.997 mmol) and 1-(2,2,2-trifluoroethyl)-1H-imidazo[4,5-c]pyridin-2(3H)-one (217 mg, 0.997 mmol) were reacted according to the procedure of Example 1 step a, which gave the title compound (305.7 mg, 70%). MS (ES+) 436.94 & 438.92 [M+H]⁺.

### Step b) 3-((4-(4-hydroxybut-1-yn-1-yl)isoquinolin-3-yl)methyl)-1-(2,2,2-trifluoroethyl)-1H-imidazo[4,5-c]pyridin-2(3H)-one (3b)

Compound 3a (299 mg, 0.684 mmol) and 3-butyn-1-ol (62.3 mg, 0.889 mmol) were reacted according to the procedure of Example 1 step b, which gave the title compound (191 mg, 66%). MS (ES+) 427.09 [M+H]⁺.

### Step c) 3-((4-(4-hydroxybutyl)isoquinolin-3-yl)methyl)-1-(2,2,2-trifluoroethyl)-1H-imidazo[4,5-c]pyridin-2(3H)-one (3c)

Alkyne 3b was hydrogenated according to the procedure of Example 1 step c, which gave the title compound (78 mg, 41%). MS (ES+) 431.1 [M+H]⁺.
¹H NMR (500 MHz, DMSO-*d*₆) δ 9.08 (s, 1H), 8.35 (d, *J* = 0.7 Hz, 1H), 8.26 (d, *J* = 5.3 Hz, 1H), 8.19 - 8.13 (m, 1H), 8.12 - 8.04 (m, 1H), 7.83 (ddd, *J* = 8.4, 6.9, 1.3 Hz, 1H), 7.67 (ddd, *J* = 7.9, 6.9, 0.9 Hz, 1H), 7.40 (d, *J* = 5.3 Hz, 1H), 5.45 (s, 2H), 4.90 (q, *J* = 9.3 Hz, 2H), 4.43 (t, *J* = 5.1 Hz, 1H), 3.47 (q, *J* = 6.1 Hz, 2H), 3.26 - 3.19 (m, 2H), 1.70 - 1.52 (m, 4H).

### Example 4

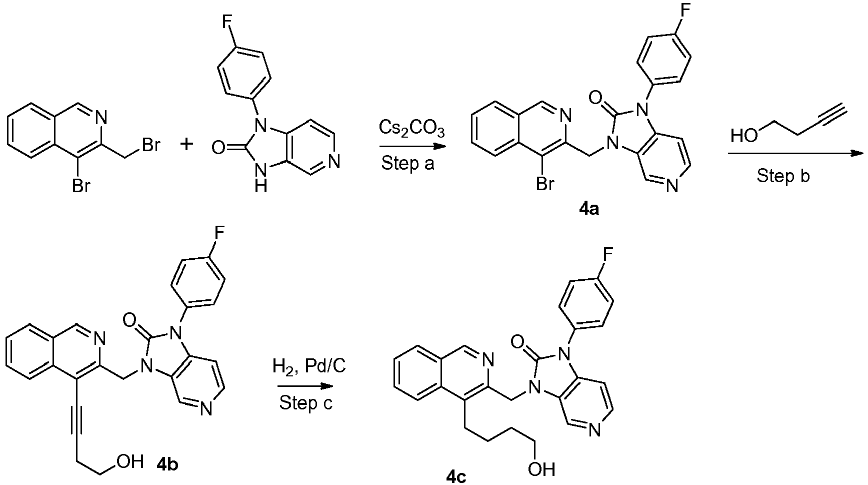

### Step a) 3-((4-bromoisoquinolin-3-yl)methyl)-1-(4-fluorophenyl)-1H-imidazo[4,5-c]pyridin-2(3H)-one (4a)

1-(4-fluorophenyl)-1H-imidazo[4,5-c]pyridin-2(3H)-one (171 mg, 0.757 mmol) and Cs₂CO₃ (728 mg, 2.24 mmol) were suspended in DMF (7.0 mL) and stirred at rt for 30 min. 4-bromo-3-(bromomethyl)isoquinoline (228 mg, 0.757 mmol) was then added as a solid, and the stirring was continued for 3h. Ice-water (50 mL) was poured into the reaction and the mixture was stirred for 30 min, then filtered. The precipitates were washed several times with total 50 mL water, and then dissolved in DCM. The solvent was removed by rotavap, and the solids dried under vacuum. The crude material was dissolved in DCM (5 mL) and purified by column chromatography on silica, eluted with a gradient 0.4% to 8% MeOH in DCM which gave the title compound (256 mg, 75%). MS (ES+) 450.86 [M+H]⁺.

### Step b) 1-(4-fluorophenyl)-3-((4-(4-hydroxybut-1-yn-1-yl)isoquinolin-3-yl)methyl)-1H-imidazo[4,5-c]pyridin-2(3H)-one (4b)

Compound 4a (239 mg, 0.532 mmol) and 3-butyn-1-ol (48.5 mg, 0.692 mmol) were reacted according to the procedure of Example 1 step b, which gave the title compound (128 mg, 55%). MS (ES+) 439.03 [M+H]⁺. The structure was confirmed by NMR.

### Step c) 1-(4-fluorophenyl)-3-((4-(4-hydroxybutyl)isoquinolin-3-yl)methyl)-1H-imidazo[4,5-c]pyridin-2(3H)-one (4c)

Compound 4b was subjected to hydrogenation according to the procedure of Example 1 step c, which gave the title compound (51 mg, 40%). MS (ES+) 443.2 [M+H]⁺.
¹H NMR (500 MHz, DMSO-*d*₆) δ 9.14 (s, 1H), 8.46 - 8.41 (m, 1H), 8.24 - 8.14 (m, 2H), 8.09 (dd, *J* = 8.2, 1.3 Hz, 1H), 7.84 (ddd, *J* = 8.4, 6.8, 1.3 Hz, 1H), 7.73 - 7.59 (m, 3H), 7.49 - 7.40 (m, 2H), 7.09 (dd, *J* = 5.3, 0.8 Hz, 1H), 5.49 (s, 2H), 4.43 (t, *J* = 5.1 Hz, 1H), 3.47 (q, *J* = 5.8 Hz, 2H), 3.25 (dd, *J* = 9.3, 6.4 Hz, 2H), 1.69 - 1.54 (m, 3H).

### Example 5

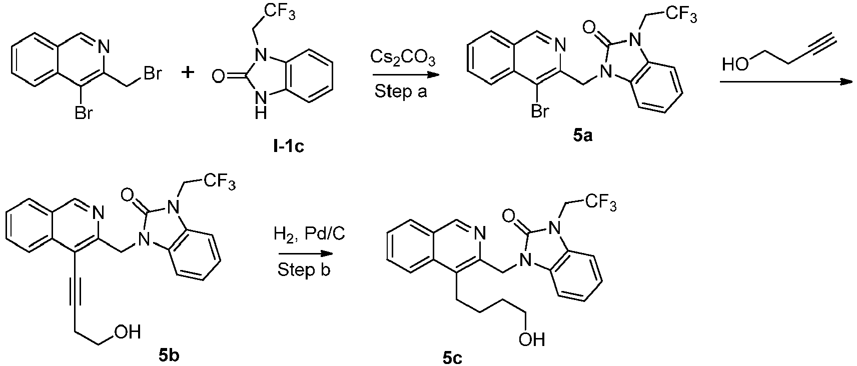

### Step a) 1-((4-bromoisoquinolin-3-yl)methyl)-3-(2,2,2-trifluoroethyl)-1H-benzo[d]imidazol-2(3H)-one (5a)

4-bromo-3-(bromomethyl)isoquinoline (300 mg, 0.997 mmol) and I-1c (216 mg, 1.00 mmol) were reacted according to the procedure of Example 1 step a, which gave the title compound (388 mg, 89%). MS (ES+) 436.01 & 437.93 [M+H]⁺.

### Step b) 1-((4-(4-hydroxybut-1-yn-1-yl)isoquinolin-3-yl)methyl)-3-(2,2,2-trifluoroethyl)-1H-benzo[d]imidazol-2(3H)-one (5b)

Compound 5a and 3-butyn-1-ol were reacted according to the procedure of Example 1 step b, which gave the title compound (69%). MS (ES+) 426.05 [M+H]⁺.

### Step c) 1-((4-(4-hydroxybutyl)isoquinolin-3-yl)methyl)-3-(2,2,2-trifluoroethyl)-1H-benzo[d]imidazol-2(3H)-one (5c)

Alkyne 5b (247 mg, 0.58 mmol) was hydrogenated according to the procedure of Example 1 step c, which gave the title compound (141 mg, 57%).
¹H NMR (500 MHz, DMSO-*d*₆) δ 9.08 (s, 1H), 8.18 - 8.11 (m, 1H), 8.09 - 8.03 (m, 1H), 7.82 (ddd, *J* = 8.4, 6.8, 1.3 Hz, 1H), 7.66 (ddd, *J* = 7.9, 6.8, 0.9 Hz, 1H), 7.30 (d, *J* = 7.7 Hz, 1H), 7.12 (dd, *J* = 7.6, 1.2 Hz, 1H), 7.03 (dtd, *J* = 27.2, 7.7, 1.2 Hz, 2H), 5.40 (s, 2H), 4.84 (q, *J* = 9.3 Hz, 2H), 4.42 (t, *J* = 5.1 Hz, 1H), 3.46 (td, *J* = 6.3, 5.0 Hz, 2H), 3.25 - 3.18 (m, 2H), 1.64 (p, *J* = 6.7 Hz, 2H), 1.53 (ddt, *J* = 9.4, 6.1, 3.2 Hz, 2H).

### Example 6

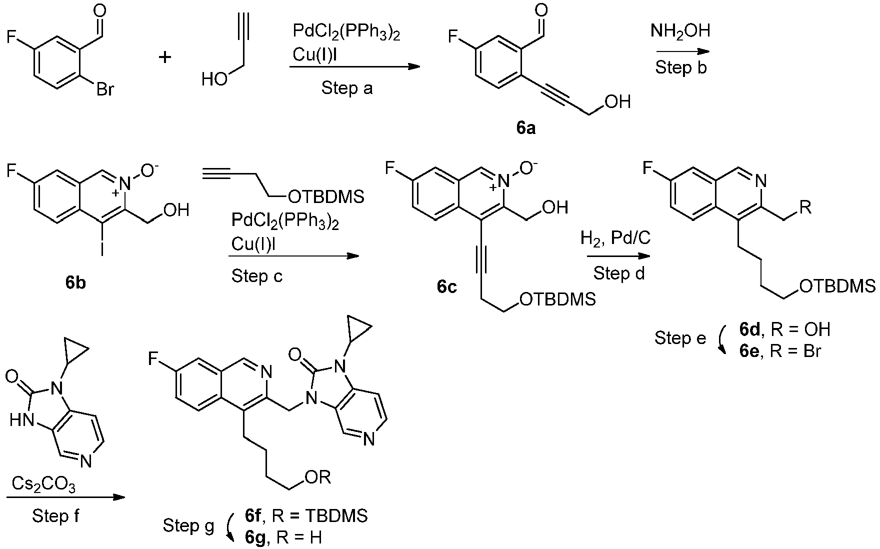

### Step a) 5-Fluoro-2-(3-hydroxyprop-1-yn-1-yl)benzaldehyde (6a)

3-Butyn-1-ol (331 mg, 5.91 mmol) was added under nitrogen to a solution of PdCl₂(PPh₃)₂ (86.4 mg, 0.123 mmol), copper iodide (37.5 mg, 0.197 mmol) and 2-bromo-5-fluorobenzaldehyde (1.00 g, 4.93 mmol) in TEA (5 mL) and DMF (5 mL). The solution was heated by microwave irradiation at 40 °C over the weekend, then filtered through a pad of Celite. The filtrate was diluted with EtOAc to about 20 mL, then washed with NaHCO₃ and brine. The organic layer was dried Na₂SO₄ and concentrated and the afforded crude compound was purified on silica eluting with heptane:EtOAC (4:1), which gave the title compound as a solid (570 mg, 65%).

### Step b) (4-Iodoisoquinolin-3-yl)methanol N-oxide (6b)

A solution of 6a (550 mg, 3.09 mmol) in ethanol (10 mL) was added dropwise at rt to a stirred solution of hydroxylamine (322 mg, 4.63 mmol) in ethanol (10 mL) and pyridine (0.5 mL). The solution was stirred at rt for 30 min, then iodine (3.13 g, 12.4 mmol) was added and the stirring was continued for 15 minutes. Sodium thiosulfate (sat. aq., 10 mL) was added and the mixture was stirred until it became a clear solution. The solution was extracted with DCM, (3x20 mL), and the combined organic phases were washed with brine, dried (Na₂SO₄) and concentrated. A small amount of DCM was added followed by addition of Et₂O (20 mL). The title compound crashed out immediately and was filtered off. (467 mg, 47%).
LC-MS (ES+) 319.82 [M+H]⁺.

### Step c) (4-(4-((tert-butyldimethylsilyl)oxy)but-1-yn-1-yl)-7-fluoroisoquinolin-3-yl)methanol N-oxide (6c)

4-TBDMS-butyne (260 mg, 1.41 mmol) was added under nitrogen to a mixture of PdCl₂(PPh₃)₂ (19.8 mg, 0.028 mmol), copper iodide (8.95 mg, 0.047 mmol) and 6b (300 mg, 0.94 mmol) in DMF/TEA (3+3 mL). The mixture was heated at 50 °C under nitrogen for 24 h. The mixture was concentrated, diluted with EtOAc (10 mL), washed with NaHCO₃ and brine, dried (Na₂SO₄) and concentrated. The afforded crude compound was purified by column chromatography on silica eluting with EtOAc:heptane 1:1, which gave the title compound as a solid (252 mg, 71%). LC-MS (ES+) 377.01 [M+H]⁺.

### Step d) (4-(4-((tert-butyldimethylsilyl)oxy)butyl)-7-fluoroisoquinolin-3-yl)methanol (6d)

Compound 6c (252 mg, 0.671 mmol) was dissolved in EtOH (15 mL), 10% palladium on carbon (250 mg, 0.211 mmol) was added and the mixture was subjected to hydrogenation at rt for 3 h,then additional Pd/C (50 mg) was added and the hydrogenation was continued for another 2 h. The mixture was filtered through Celite, the filtrate was concentrated and the residue purified by column chromatography on silica eluting with EtOAC:heptane1:1,which gave the title compound (113 mg, 46%).

### Step e) 3-(Bromomethyl)-4-(4-((tert-butyldimethylsilyl)oxy)butyl)-7-fluoroisoquinoline (6e)

Triphenylphosphine (111 mg, 0.424 mmol) was added at 0 °C to a solution of compound 6d (110 mg, 0.303 mmol) in dry DCM (16 mL). After 5 min, CBr₄ (151 mg, 0.454 mmol) was added and the solution was stirred at 0 °C for 10 min then at RT for 1.5 h. The reaction mixture was concentrated and the afforded crude was purified by column chromatography on silica eluting with EtOAc:hexane 1:9, which gave the title compound (98 mg, 76%).

### Step f) 3-((4-(4-((tert-butyldimethylsilyl)oxy)butyl)-7-fluoroisoquinolin-3-yl)methyl)-1-cyclopropyl-1H-imidazo[4,5-c]pyridin-2(3H)-one (6f)

Cesium carbonate (103 mg, 0.317 mmol) was added to a solution 1-cyclopropyl-1H-imidazo[4,5-c]pyridin-2(3H)-one (18.5 mg, 0.106 mmol) in DMF. The slurry was stirred at rt for 15min, then cooled to 0 °C and compound 6e (45 mg, 0.106 mmol) dissolved in DMF was added slowly. The mixture was allowed to attain rt and was stirred for 16 h. Water (5 mL) and EtOAc (10 mL) were added. The water phase was extracted twice with EtOAc (5 x 2 mL). The combined organic phases were washed twice with brine, then dried (Na₂SO₄) and concentrated. The afforded crude was purified by column chromatography on silica eluting with EtOAC:MeOH 98:2, which gave the title compound (43 mg, 78%).

### Step g) 1-cyclopropyl-3-((7-fluoro-4-(4-hydroxybutyl)isoquinolin-3-yl)methyl)-1H-imidazo[4,5-c]pyridin-2(3H)-one (6g)

4M HCl in dioxane (2 mL) was added to a solution of compound 6e (43 mg, 0.083 mmol) in MeOH (2 mL). The solution was stirred for 30 min, then the pH was adjusted to ∼7 by addition of a few drops of 1M NaOH. The solution was diluted with EtOAc and the organic phase was washed with NaHCO₃ and brine, dried (Na₂SO₄) and concentrated. The afforded crude was purified by column chromatography on_silica eluting with DCM:MeOH 96:4. Appropriate fractions were combined and freeze dried which gave the title compound as a white powder (26 mg, 78%). MS (ES+) 407.1 [M+H]⁺.
¹H NMR (500 MHz, DMSO-*d*₆) δ 9.09 (s, 1H), 8.25 (s, 1H), 8.28 - 8.21 (m, 1H), 8.20 (d, *J* = 5.2 Hz, 1H), 7.88 (dd, *J* = 9.1, 2.7 Hz, 1H), 7.74 (td, *J* = 9.0, 2.8 Hz, 1H), 7.25 (dd, *J* = 5.2, 0.8 Hz, 1H), 5.35 (s, 2H), 4.43 (t, *J* = 5.0 Hz, 1H), 3.49 - 3.40 (m, 2H), 3.25 - 3.17 (m, 2H), 2.98 (tt, *J* = 7.1, 3.7 Hz, 1H), 1.62 (p, *J* = 6.7 Hz, 2H), 1.48 (tt, *J* = 9.7, 6.1 Hz, 2H), 1.10 - 1.00 (m, 2H), 0.94 - 0.87 (m, 2H).

### Example 7

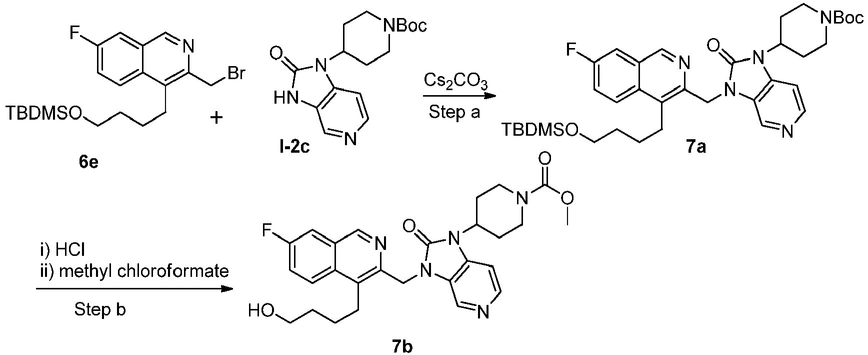

### Step a) tert-butvl 4-(3-((4-(4-((tert-butyldimethylsilyl)oxy)butyl)-7-fluoroisoquinolin-3-yl)methyl)-2-oxo-2,3-dihydro-1H-imidazo[4,5-c]pyridin-1-yl)piperidine-1-carboxylate (7a)

Compound 6e (45 mg, 0.106 mmol) was reacted with I-2c (34 mg, 0.106 mmol) according to the method described in Ex. 6 step f, which gave the title compound (49 mg, 70%). MS (ES+) 664.37 [M+H]⁺.

### Step b) Methyl 4-(3-((7-fluoro-4-(4-hydroxybutyl)isoquinolin-3-yl)methyl)-2-oxo-2,3-dihydro-1H-imidazo[4,5-c]pyridin-1-yl)piperidine-1-carboxylate (7b)

4M HCl in dioxane was added to a solution of compound 7a (35 mg, 0.053 mmol) in MeOH (2 mL) and the clear solution was stirred at rt for 90 min, then concentrated. The residue dissolved in DMF (3 mL), DIPEA (57.5 mg, 0.445 mmol) was added and the mixture was stirred at rt for 15 minutes then cooled in a ice-bath. Methyl chloroformate (8.41 mmol, 0.089 mmol) was added and the stirring was continued for 90 min at 0 °C. 1M NaOH (aq, 2 mL) was added and the mixture was diluted with EtOAc, washed with NaHCO₃ and brine, dried (Na₂SO₄), filtered and concentrated. The afforded crude product was purified by column chromatography on silica gel eluted with DCM:MeOH 96:4. Appropriate fractions were combined and freeze dried which gave the title compound as a solid (14 mg, 62%). MS (ES+) 508.1 [M+H]⁺.
¹H NMR (500 MHz, DMSO-*d*₆) δ 9.09 (s, 1H), 8.31 (s, 1H), 8.25 (dd, *J* = 9.4, 5.1 Hz, 1H), 8.17 (d, *J* = 4.9 Hz, 1H), 7.88 (dd, *J* = 9.1, 2.7 Hz, 1H), 7.74 (td, *J* = 9.0, 2.8 Hz, 1H), 7.41 (d, *J* = 5.3 Hz, 1H), 5.38 (s, 2H), 4.52 - 4.40 (m, 2H), 4.14 (s, 2H), 3.63 (s, 3H), 3.49 - 3.41 (m, 2H), 3.30 (s, 1H), 3.26 - 3.18 (m, 2H), 2.21 (qd, *J* = 12.5, 4.5 Hz, 2H), 1.79 - 1.72 (m, 2H), 1.62 (p, *J* = 6.7 Hz, 2H), 1.51 (ddt, *J* = 15.7, 9.8, 5.7 Hz, 2H).

### Example 8

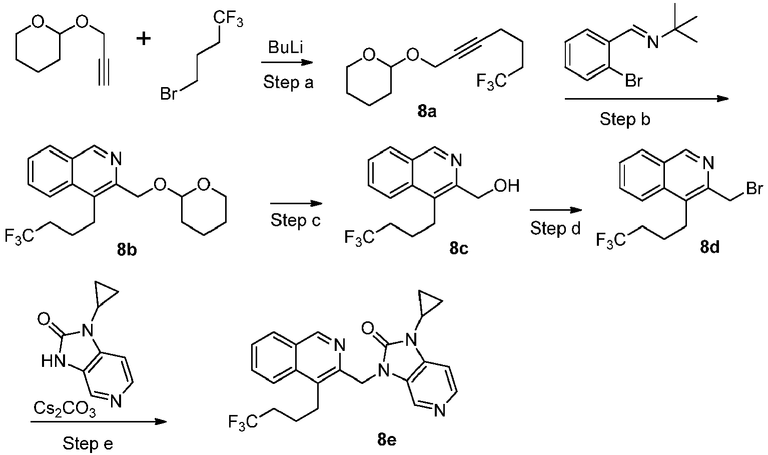

### Step a) 2-((7,7,7-Trifluorohept-2-yn-1-yl)oxy)tetrahydro-2H-pyran (8a)

DMPU (1.10 g, 8.56 mmol) followed by n-BuLi (548 mg, 8.56 mmol) was added dropwise at -78 °C to a stirred solution of 2-(prop-2-yn-1-yloxy)tetrahydro-2H-pyran (1.00 g, 7.13 mmol) in THF (20 mL). The solution was stirred for 30 minutes at -78 °C, then 4-bromo-1,1,1-trifluorobutane (1.43 g, 7.49 mmol) was added dropwise. The cold bath was removed and the reaction mixture was allowed to attain rt. After 18 NaHCO₃ was added and the mixture was diluted with EtOAc (30 mL). The organic phase was separated and washed with water and brine, dried (Na₂SO₄), filtered and concentrated. The afforded crude was filtered through a silica plug eluting with 4:1 Heptane EtOAc, which gave the title compound (1.29 g 72%).

### Step b) 3-(((Tetrahydro-2H-pyran-2-yl)oxy)methyl)-4-(4,4,4-trifluorobutyl)isoquinoline (8b)

To a solution of (E)-N-(2-bromobenzylidene)-2-methylpropan-2-amine and compound 8a (1.25 g, 5.00 mmol) in acetonitrile (100 mL) under argon was added zinc powder (817 mg, 12.5 mmol) followed by dibromobis(triphenylphosphine)nickel(II) (309 mg, 0.416 mmol). The reaction mixture was heated with a condenser at 80 °C under argon. After 3 h the catalyst was filtered off through a small pad of Celite. The filtrate was concentrated and the residue purified with silica eluting with heptane:EtOAc 9:1, which gave the title compound (505 mg, 34%).

### Step c) (4-(4,4,4-Trifluorobutyl)isoquinolin-3-yl)methanol (8c)

A mixture of compound 8b (550 mg, 1.56 mmol) in acetic acid/water (8:2, 10 mL) was heated to 60 °C for 16 h, then concentrated and diluted with EtOAc. Washed 3 times with 1M NaOH and brine. The water phase was extracted with EtOAc until not UV-active components were detected. The combined organic layers were dried using (Na2SO4), filtered and concentrated. The afforded crude compound was purified by column chromatography on silica gel eluting with EtOAc:heptane 2:1, which gave the title compound (200 mg, 48%). MS (ES+) 270.42 [M+H]+.

### Step d) 3-(bromomethyl)-4-(4,4,4-trifluorobutyl)isoquinoline (8d)

Compound 8c (200 mg, 0.743 mmol) was converted to the corresponding bromo derivative according to the method described in Ex. 6 step e. Yield 204 mg, 83%.

### Step e) 1-cyclopropyl-3-((4-(4,4,4-trifluorobutyl)isoquinolin-3-yl)methyl)-1H-imidazo[4,5-c]pyridin-2(3H)-one (8e)

Compound 8d (30 mg, 0.090 mmol) was reacted with 1-cyclopropyl-1H-imidazo[4,5-c]pyridin-2(3H)-one (15.8 mg, 0.090 mmol) according to the method described in Ex. 6 step f, which gave the title compound (21 mg, 55%). MS (ES+) 427.09 [M+H]⁺.
¹H NMR (500 MHz, DMSO-*d*₆) δ 9.14 (s, 1H), 8.32 (s, 1H), 8.23 - 8.17 (m, 2H), 8.09 (dd, *J* = 8.2, 1.2 Hz, 1H), 7.86 (ddd, *J* = 8.4, 6.8, 1.3 Hz, 1H), 7.69 (ddd, *J* = 8.0, 6.9, 0.9 Hz, 1H), 7.25 (dd, *J* = 5.2, 0.8 Hz, 1H), 5.38 (s, 2H), 3.32 (s, 2H), 2.96 (tt, *J* = 7.1, 3.7 Hz, 1H), 2.54 (dd, *J* = 8.2, 3.5 Hz, 1H), 1.74 - 1.63 (m, 2H), 1.12 - 1.00 (m, 2H), 0.93 - 0.86 (m, 2H).

### Example 9

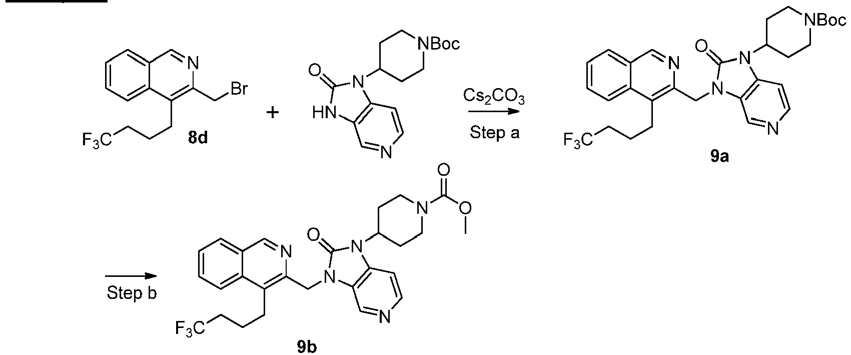

### Step a) tert-butyl 4-(2-oxo-3-((4-(4,4,4-trifluorobutyl)isoquinolin-3-yl)methyl)-2,3-dihydro-1H-imidazo[4,5-c]pyridin-1-yl)piperidine-1-carboxylate (9a)

Compound 8d (35.0 mg, 0.105 mmol) was reacted with I-2c (40.3 mg, 0.126 mmol) according to the method described in Ex. 6 step f, which gave the title compound (42 mg, 70%). MS (ES+) 570.18 [M+H]⁺.

### Step b) methyl 4-(2-oxo-3-((4-(4,4,4-trifluorobutyl)isoquinolin-3-yl)methyl)-2,3-dihydro-1H-imidazo[4,5-c]pyridin-1-yl)piperidine-1-carboxylate (9b)

Compound 9a (42 mg, 0.074 mmol) was reacted according to the procedure described in Ex. 7 step b, which gave the title compound (18 mg, 46%). MS (ES+) 528.2 [M+H]⁺.
¹H NMR (500 MHz, DMSO-*d*₆) δ 9.12 (s, 1H), 8.40 (s, 1H), 8.24 - 8.15 (m, 2H), 8.09 (dd, *J* = 8.2, 1.2 Hz, 1H), 7.86 (ddd, *J* = 8.4, 6.8, 1.3 Hz, 1H), 7.69 (ddd, *J* = 8.0, 6.9, 0.9 Hz, 1H), 7.45-7.39 (m, 1H), 5.41 (s, 2H), 4.46 (tt, *J* = 12.3, 4.0 Hz, 1H), 3.63 (s, 3H), 3.31 (d, *J* = 11.8 Hz, 6H), 2.96 (s, 2H), 2.60 - 2.51 (m, 1H), 2.21 (qd, *J* = 12.5, 4.5 Hz, 2H), 1.74 (dq, *J* = 11.7, 4.0 Hz, 4H).

### Example 10

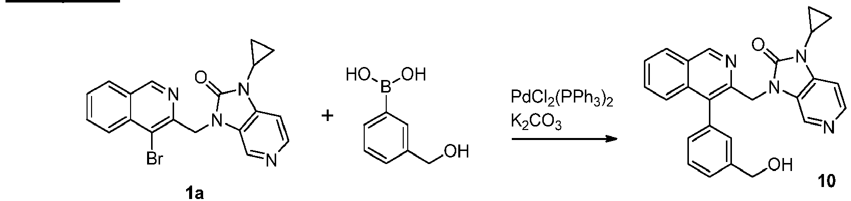

### 1-Cyclopropyl-3-((4-(3-(hydroxymethyl)phenyl)isoquinolin-3-yl)methyl)-1H-imidazo[4,5-c]pyridin-2(3H)-one (10)

A mixture of compound 1a (110 mg, 0.278 mmol), (3-(hydroxymethyl)phenyl)boronic acid (63.4 mg, 0.417 mmol), potassium carbonate (154 mg, 1.11 mmol) and PdCl₂(PPh₃)₂ (9.77 mg, 0.014 mmol) in DMF:water 4:1 (2.5 mL) was heated to 80 °C for 18 h, then solids were filtered of and EtOAc 10 mL and water were added. The phases were separated and the water phase extracted once with EtOAc (5 mL). The combined organic phases were washed with saturated NaHCO₃ and brine, dried (Na₂SO₄) filtered and concentrated. The afforded crude was purified by column chromatography on silica gel eluting with DCM:MeOH (98:2). Appropriate fractions were combined and freeze dried which gave the title compound (25 mg, 21%). MS (ES+) 423.2
[M+H]⁺.
¹H NMR (500 MHz, DMSO-*d*₆) δ 9.25 (s, 1H), 8.21 - 8.12 (m, 2H), 7.96 (s, 1H), 7.70 (dddd, *J* = 16.5, 8.0, 6.9, 1.4 Hz, 2H), 7.51 (t, *J* = 7.5 Hz, 1H), 7.44 (dt, *J* = 7.7, 1.5 Hz, 1H), 7.36 (dd, *J* = 8.3, 1.4 Hz, 1H), 7.31 - 7.23 (m, 2H), 7.23 - 7.17 (m, 1H), 5.27 (t, *J* = 5.7 Hz, 1H), 5.09 (d, *J* = 2.5 Hz, 2H), 4.58 (d, *J* = 5.6 Hz, 2H), 2.88 (tt, *J* = 7.0, 3.6 Hz, 1H), 1.01 (dt, *J* = 7.1, 3.4 Hz, 2H), 0.87 - 0.80 (m, 2H).

### Example 11

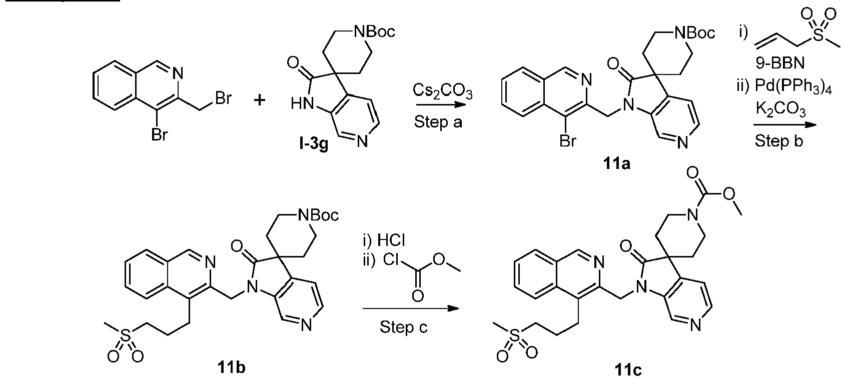

### Step a) Tert-butyl 1'-((4-bromoisoquinolin-3-yl)methyl)-2'-oxo-1',2'-dihydrospiro[piperidine-4,3'-pyrrolo[2,3-c]pyridine]-1-carboxylate (11a)

Cesium carbonate (1.62 g, 4.98 mmol) was heated under vacuum for 5 min, then allowed to cool to rt and the flask was flushed with nitrogen. MeCN (10 mL) and I-3g (504 mg, 1.66 mmol) were added and the solution was stirred at rt for 30 minutes, then 4-bromo-3-(bromomethyl)isoquinoline (500 mg, 1.66 mmol) was added dropwise. The reaction mixture was stirred for 18 h, then water (5 mL) was added and the MeCN was removed by evaporation and the resulting slurry was diluted with EtOAc. The phases were separated and the organic phase was washed with brine and (Na₂SO₄), filtered and concentrated. The afforded crude was purified by column chromatography on silica gel eluting with EtOAc, which gave the title compound (759 mg, 87%). MS (ES+) 523.10 & 525.09 [M+H]⁺.

### Step b) Tert-butyl 1'-((4-(3-(methylsulfonyl)propyl)isoquinolin-3-yl)methyl)-2'-oxo-1',2'-dihydrospiro[piperidine-4,3'-pyrrolo[2,3-c]pyridine]-1-carboxylate (11b)

A solution of 3-(methylsulfonyl)prop-1-ene (33.5 mg, 0.279 mmol) and 0,5 M 9-borabicyclo[3.3.1]nonane (0,56 mL, 0.279 mmol) in tetrahydrofuran (2 mL) were stirred at room temperature under nitrogen for 60 minutes. Water (100 µl) was added and stirring was continued for 10 minutes, then an aqueous solution of 2M potassium carbonate was added. The solution was stirred another 30 minutes before compound 11a (112 mg, 0.214 mmol) and Pd(PPh3)4 (24.8 mg, 0.021 mmol) were added. Nitrogen was bubbled through the solution for 15 minutes, then the vial was capped and heated at 120 °C in a microwave reactor for 30 minutes.

Water (5 mL) and EtOAc (10 mL) were added, the phases were separated and the organic phase was washed with brine, dried and concentrated. The afforded crude was purified by prep HPLC on a C18, 2cm, Gemini column eluting with NH₄OAc at pH 7. Pure fractions were pooled and concentrated, dissolved in water:MeCN 1:1 and freeze dried to give the title compound as a solid, (29 mg, 24%). MS (ES+) 566.23 [M+H]⁺.

### Step c) Methyl 1'-((4-(3-(methylsulfonyl)propyl)isoquinolin-3-yl)methyl)-2'-oxo-1',2'-dihydrospiro[piperidine-4,3'-pyrrolo[2,3-c]pyridine]-1-carboxylate (11c)

4M HCl in dioxane was added to a solution of compound 11b in MeOH. The solution was stirred at rt for 90 min, then concentrated. The afforded residue was dissolved in DMF, DIPEA (55.6 mg, 0.43 mmol) was added and the reaction mixture was stirred at rt for 15 minutes then cooled in a ice-bath and methyl chloroformate (4.07 mg, 0.043 mmol) was added and the stirring was continued for 90 minutes at 0 °C. The reaction was quenched with 1M NaOH (aq, 2 mL), EtOAc was added and the mixture was washed with NaHCO₃ and brine, dried (Na₂SO₄), filtered and concentrated. The afforded crude was purified by column chromatography on silica gel eluting with DCM:MeOH 96:4. Pure fractions were pooled and freeze dried, which gave the title compound (8 mg, 36%).
¹H NMR (500 MHz, DMSO-*d*₆) δ 9.08 (s, 1H), 8.38 - 8.34 (m, 1H), 8.30 (s, 1H), 8.24 (d, *J* = 8.6 Hz, 1H), 8.07 (dd, *J* = 8.3, 1.3 Hz, 1H), 7.86 (ddd, *J* = 8.4, 6.8, 1.3 Hz, 1H), 7.73 - 7.64 (m, 2H), 5.29 (s, 2H), 3.74 (tt, *J* = 13.5, 6.2 Hz, 4H), 3.65 (s, 3H), 3.38 (dt, *J* = 19.1, 7.9 Hz, 4H), 3.02 (s, 3H), 2.10 - 1.99 (m, 2H), 1.80 (tp, *J* = 13.5, 4.9 Hz, 4H).

### Example 12

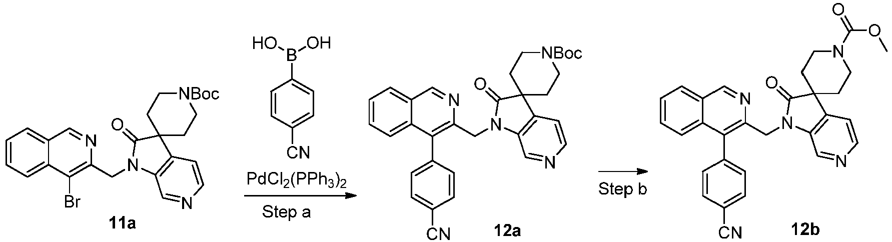

### Step a) Tert-butyl 1'-((4-(4-cyanophenyl)isoquinolin-3-yl)methyl)-2'-oxo-1',2'-dihydrospiro[piperidine-4,3'-pyrrolo[2,3-c]pyridine]-1-carboxylate (12a)

Compound 11a (100 mg, 0.191 mmol), (4-cyanophenyl)boronic acid (42.1 mg, 0.287 mmol), potassium carbonate (106 mg, 0.764 mmol) and PdCl₂(PPh₃)₂ (13.4 mg, 0.019 mmol) were dissolved in MeCN (2 mL), the reaction vessel was sealed and the mixture was heated to 80 °C. When the reaction was deemed completed as judged by TLC (after -18 h), solids were filtered of, EtOAc (10 mL) was added and the solution was washed with water. The water phase was extracted once with EtOAc (5 mL) and the combined organic layers were washed with saturated NaHCO₃ and brine, dried (Na₂SO₄), filtered and concentrated. The afforded crude was purified by column chromatography on silica gel eluting with EtOAc:heptane 1:1 then EtOAc. Pure fractions were pooled and freeze dried, which gave the title compound (78 mg, 74%). MS (ES+) 546.25 [M+H]⁺.

### Step b) Methyl 1'-((4-(4-cyanophenyl)isoquinolin-3-yl)methyl)-2'-oxo-1',2'-dihydrospiro[piperidine-4,3'-pyrrolo[2,3-c]pyridine]-1-carboxylate (12b)

4M HCl in dioxane (3 mL) was added to a solution of compound 12a (78.0 mg, 0.143 mmol) in MeOH (2 mL). The solution was stirred at rt for 90 min, then concentrated. The afforded residue was dissolved in DMF (5 mL), DIPEA (186 mg, 1.46 mmol) was added and the reaction mixture was stirred at rt for 15 minutes then cooled in a ice-bath and methyl chloroformate (13.8 mg, 0.146 mmol) was added and the stirring was continued for 90 minutes at 0 °C. The reaction was quenched with 1M NaOH (aq, 2 mL), EtOAc was added and the mixture was washed with NaHCO₃ and brine, dried (Na₂SO₄), filtered and concentrated. The afforded crude was purified by prep. HPLC C18, 2 cm, Gemini gel eluting with NH₄OAc at pH 7. Pure fractions were pooled and freeze dried, which gave the title compound (32 mg, 44%). MS (ES+) 504.3 [M+H]⁺.
¹H NMR (500 MHz, DMSO-*d*₆) δ 9.31 (d, *J* = 0.9 Hz, 1H), 8.27 (s, 1H), 8.21 - 8.15 (m, 1H), 8.11 - 8.06 (m, 2H), 7.79 - 7.68 (m, 5H), 7.61 (d, *J* = 4.3 Hz, 1H), 7.31 (dd, *J* = 8.3, 1.4 Hz, 1H), 4.95 (s, 2H), 3.76 - 3.65 (m, 1H), 3.70 (s, 1H), 3.64 (s, 4H), 1.76 (ddd, *J* = 13.2, 8.1, 4.9 Hz, 2H), 1.65 (dt, *J* = 13.4, 5.0 Hz, 2H).

### Example 13

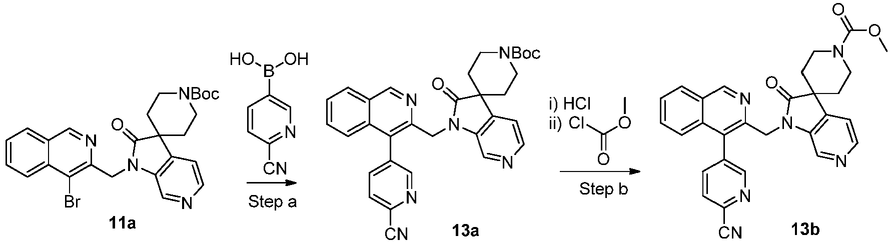

### Methyl 1'-((4-(6-cyanopyridin-3-yl)isoquinolin-3-yl)methyl)-2'-oxo-1',2'-dihydrospiro[piperidine-4,3'-pyrrolo[2,3-c]pyridine]-1-carboxylate (13b)

The title compound was prepared as described in Example 12 but using (6-cyanopyridin-3-yl)boronic acid in step a. MS (ES+) 505 [M+H]⁺. The structure was confirmed by ¹H and ¹³C
NMR.

### Example 14

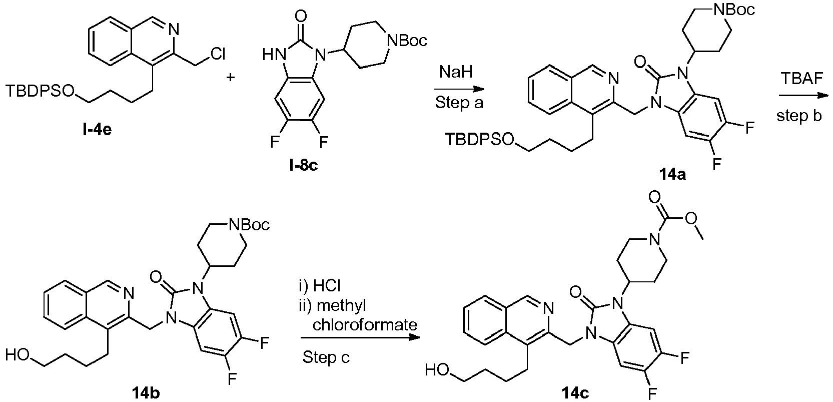

### Step a) tert-butyl 4-(3-((4-(4-((tert-butyldiphenylsilyl)oxy)butyl)isoquinolin-3-yl)methyl)-5,6-difluoro-2-oxo-2,3-dihydro-1H-benzo[d]imidazol-1-yl)piperidine-1-carboxylate (14a)

Sodium hydride (60% dispersed in mineral oil, 9 mg, 0,23 mmol) was added under nitrogen to a stirred solution of compound I-8c (66 mg, 0,19 mmol) in DMF (1 mL). The resulting mixture was stirred at room temperature for 30 min, then the temperature was lowered to 0 °C and compound I-4e (100 mg, 0,2 mmol) was added. The reaction mixture was stirred at room temperature for 22 h, then partitioned between water and DCM. The layers were separated and the aqueous phase was extracted twice with fresh dichloromethane and three times with ethyl acetate. The combined organic extracts were dried over Na₂SO₄ and concentrated under reduced pressure to give crude title compound 136 mg (90,4%) which was used in next step without further purification. MS (ES+) 805.37 & 806.30 [M+H]⁺.

### Step b) tert-butvl 4-(5,6-difluoro-3-((4-(4-hydroxybutyl)isoquinolin-3-yl)methyl)-2-oxo-2,3-dihydro-1H-benzo[d]imidazol-1-yl)piperidine-1-carboxylate (14b)

To compound 14a (136 mg, 0,17 mmol) in THF (1 mL) at 0 °C was 1M tetrabutylammonium fluoride in THF (253 µl) added. The resulting mixture was stirred at room temperature over night, then the reaction was quenched by addition of saturated aqueous NH₄Cl. The organic component was extracted into ethyl acetate and washed with brine. The organic extract was dried over Na₂SO₄ and concentrated under reduced pressure. The residue was purified by flash chromatography using a gradient of 0-100 % ethyl acetate in heptane. The desired fractions were pooled and concentrated under reduced pressure which gave the title compound (65 mg, 62%). MS (ES+) 567.21 [M+H]⁺.

### Step c) methyl 4-(5,6-difluoro-3-((4-(4-hydroxybutyl)isoquinolin-3-yl)methyl)-2-oxo-2,3-dihydro-1H-benzo[d]imidazol-1-yl)piperidine-1-carboxylate (14c)

Methanol (0,5 mL) was added to a solution of compound 14b (119 mg, 0,19 mmol) in 4M hydrochloric acid in dioxane (1,8 mL). The resulting mixture was stirred at room temperature for 1 h, then toluene was added and the mixture was concentrated under reduced pressure. Methanol and toluene was added to the residue and the resulting mixture was concentrated under reduced pressure. Repeated twice. The residue was dissolved in DCM (1 mL) and N,N-diisopropylethylamine (187 µl, 1,07 mmol) and methyl chloroformate (9 µl, 0,12 mmol) were successively added under a nitrogen atmosphere. The resulting mixture was stirred at room temperature over night and was then concentrated under reduced pressure. The afforded residue was purified by flash chromatography eluted with methanol in dichloromethane. The desired fractions were pooled and concentrated under reduced pressure. and further purified by preparative HPLC, on a C18 Phenomenex Kinetex 5µ XB- 100 A, 150x21.20 mm column eluted with water/MeCN. The desired fractions were pooled and concentrated under reduced pressure. The residue was dissolved in acetonitrile and transferred to a 4 mL vial. Water was added and the resulting mixture was freeze-dried to give the title compound (22 mg, 37,6%). MS (ES+) m/z 525 [M+H]⁺; MS (ES-) m/z 583 [M+OAc]⁻.
¹H NMR (500 MHz, DMSO-*d*₆) δ 9.10 (s, 1H), 8.17 - 8.11 (m, 1H), 8.10 - 8.04 (m, 1H), 7.82 (ddd, *J* = 8.4, 6.8, 1.3 Hz, 1H), 7.67 (ddd, *J* = 8.0, 6.9, 0.9 Hz, 1H), 7.61 (dd, *J* = 10.8, 7.3 Hz, 1H), 7.31 (dd, *J* = 10.5, 7.4 Hz, 1H), 5.32 (s, 2H), 4.46 - 4.35 (m, 2H), 3.63 (s, 3H), 3.46 (q, *J* = 6.2 Hz, 2H), 3.33 (s, 3H), 3.20 (d, *J* = 16.3 Hz, 1H), 3.20 (s, 1H), 2.93 (s, 2H), 2.25 (qd, *J* = 12.5, 4.5 Hz, 2H), 1.71 (d, *J* = 11.5 Hz, 2H), 1.62 (p, *J* = 6.7 Hz, 2H), 1.52 (ddt, *J* = 15.4, 9.6, 5.7 Hz, 2H).

### Example 15

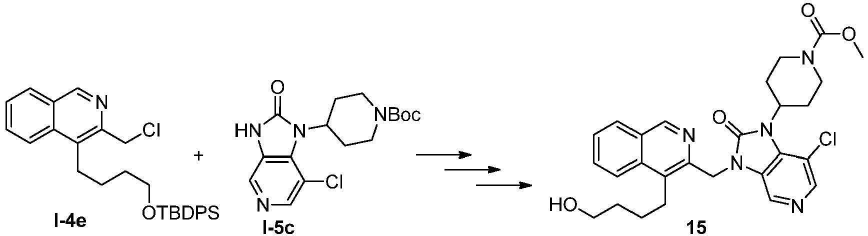

### Methyl 4-(7-chloro-3-((4-(4-hydroxybutyl)isoquinolin-3-yl)methyl)-2-oxo-2,3-dihydro-1H-imidazo[4,5-c]pyridin-1-yl)piperidine-1-carboxylate(15)

The title compound was prepared as described in Example 14 but using I-5c instead of I-8c. Overall yield 40%. MS (ES+) m/z 524 [M+H]⁺; MS (ES-) m/z 582 [M+OAc]⁻.
¹H NMR (500 MHz, DMSO-*d*₆) δ 9.07 (s, 1H), 8.33 (s, 1H), 8.21 - 8.13 (m, 2H), 8.07 (dt, *J* = 8.2, 0.9 Hz, 1H), 7.83 (ddd, *J* = 8.4, 6.8, 1.3 Hz, 1H), 7.67 (ddd, *J* = 7.9, 6.8, 0.9 Hz, 1H), 5.40 (s, 2H), 5.08 (s, 1H), 4.44 (t, *J* = 5.0 Hz, 1H), 4.11 (d, *J* = 12.8 Hz, 2H), 3.62 (s, 3H), 3.48 (q, *J* = 5.8 Hz, 2H), 3.24 - 3.14 (m, 1H), 2.92 (s, 3H), 2.41 (qd, *J* = 12.6, 4.7 Hz, 2H), 1.85 (d, *J* = 11.4 Hz, 2H), 1.69 - 1.51 (m, 4H).

### Example 16

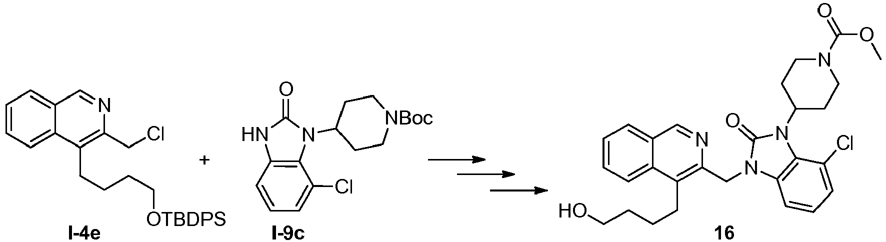

### Methyl 4-(7-chloro-3-((4-(4-hydroxybutyl)isoquinolin-3-yl)methyl)-2-oxo-2,3-dihydro-1H-benzo[d]imidazol-1-yl)piperidine-1-carboxylate (16)

The title compound was prepared as described in Example 14 but using I-9c instead of I-8c. Overall yield 42%. MS (ES+) m/z 523 [M+H]⁺, MS (ES-) m/z 581 [M+OAc]⁻.
¹H NMR (500 MHz, DMSO-*d*₆) δ 9.07 (s, 1H), 8.17 - 8.11 (m, 1H), 8.09 - 8.03 (m, 1H), 7.82 (ddd, *J* = 8.4, 6.8, 1.3 Hz, 1H), 7.66 (ddd, *J* = 7.9, 6.8, 0.9 Hz, 1H), 7.14 - 7.08 (m, 1H), 7.04 (dd, *J* = 8.2, 1.1 Hz, 1H), 6.97 (t, *J* = 8.0 Hz, 1H), 5.34 (s, 2H), 5.17 (s, 1H), 4.43 (t, *J* = 5.1 Hz, 1H), 3.62 (s, 3H), 3.46 (td, *J* = 6.3, 5.0 Hz, 2H), 3.33 (s, 6H), 3.23 - 3.16 (m, 2H), 2.91 (s, 2H), 2.45 (td, *J* = 12.4, 4.4 Hz, 1H), 1.82 (d, *J* = 11.9 Hz, 2H), 1.63 (p, *J* = 6.7 Hz, 2H), 1.57 - 1.46 (m, 1H), 1.52 (s, 1H).

### Example 17

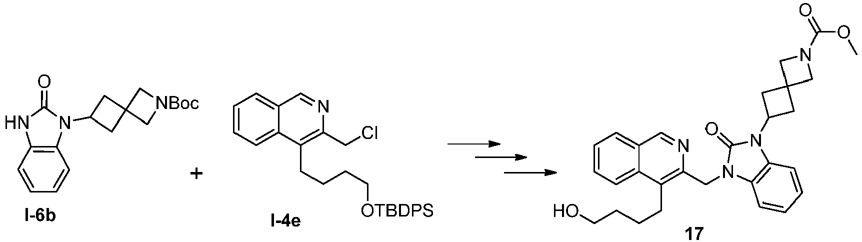

### Methyl 6-(3-((4-(4-hydroxybutyl)isoquinolin-3-yl)methyl)-2-oxo-2,3-dihydro-1H-benzo[d]imidazol-1-yl)-2-azaspiro[3.3]heptane-2-carboxylate (17)

The title compound was prepared essentially as described in Example 14 but using I-6b instead of I-8c. Overall yield 1.5%. MS (ES+) m/z 501 [M+H]⁺; MS (ES-) m/z 559 [M+OAc]⁻.
¹H NMR (500 MHz, DMSO-*d*₆) δ 9.10 (s, 1H), 8.15 - 8.09 (m, 1H), 8.07 (dd, *J* = 8.3, 1.3 Hz, 1H), 7.81 (ddd, *J* = 8.5, 6.8, 1.3 Hz, 1H), 7.66 (ddd, *J* = 7.9, 6.8, 0.9 Hz, 1H), 7.26 (d, *J* = 7.8 Hz, 1H), 7.07 - 6.96 (m, 2H), 6.93 (td, *J* = 7.7, 1.1 Hz, 1H), 5.33 (s, 2H), 4.78 (p, *J* = 8.8 Hz, 1H), 4.41 (t, *J* = 4.9 Hz, 1H), 4.08 (s, 2H), 3.56 (s, 3H), 3.42 (q, *J* = 6.3 Hz, 2H), 3.23 - 3.14 (m, 2H), 3.02 (td, *J* = 9.4, 2.9 Hz, 2H), 2.61 (ddd, *J* = 9.8, 8.3, 3.1 Hz, 2H), 1.61 (p, *J* = 6.8 Hz, 2H), 1.47 - 1.33 (m, 2H).

### Example 18

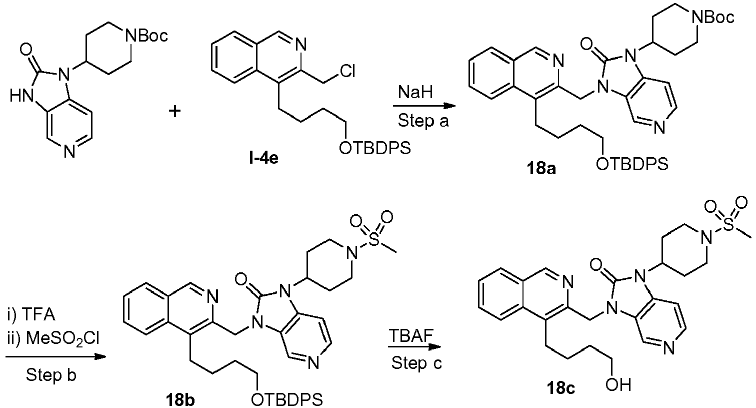

### Step a) Tert-butyl 4-(3-((4-(4-((tert-butyldiphenylsilyl)oxy)butyl)isoquinolin-3-yl)methyl)-2-oxo-2,3-dihydro-1H-imidazo[4,5-c]pyridin-1-yl)piperidine-1-carboxylate (18a)

Tert-butyl 4-(2-oxo-2,3-dihydro-1H-imidazo[4,5-c]pyridin-1-yl)piperidine-1-carboxylate (161 mg, 0.506 mmol) and I-4e (184 mg, 0.377 mmol) were reacted as described in Example 14 step a which gave the title compound (162 mg, 56%). MS (ES+) 770.44 [M+H]⁺.

### Step b) 3-((4-(4-((Tert-butyldiphenylsilyl)oxy)butyl)isoquinolin-3-yl)methyl)-1-(1-(methylsulfonyl)piperidin-4-yl)-1H-imidazo[4,5-c]pyridin-2(3H)-one (18b)

TFA was added dropwise to a solution of compound 18a (158 mg, 0.205 mmol) in DCM (2 mL). The solution was stirred at room temperature for 75 min, then concentrated under vacuum, diluted with DCM (5 mL) and basified with saturated NaHCO₃ (5 mL). The aqueous phase was extracted with 2 x 5 mL DCM. Organic phases were combined and concentrated under vacuum. The afforded residue was dissolved in DCM (5 mL) and MsCl (35.1 mg, 0.307 mmol) and DIEA (134 mg, 1.04 mmol) were added. The reaction mixture was stirred at room temperature for 18h, then diluted with DCM (10 mL) and washed with 2 x 15 mL saturated NaCl. The organic phase was concentrated under vacuum. The crude material was suspended in DCM (6 mL) + MeOH (0.8 mL) and purified by column chromatography on silica gel eluted with a gradient of MeOH in DCM, which gave the title compound (79 mg, 52%) not completely pure. MS (ES+) 748.37 [M+H]⁺.

### Step c) 3-((4-(4-hydroxybutyl)isoquinolin-3-yl)methyl)-1-(1-(methylsulfonyl)piperidin-4-yl)-1H-imidazo[4,5-c]pyridin-2(3H)-one (18c)

TBAF (1M in THF) was added to a solution of compound 18b (79 mg, 0.106 mmol) in THF (2 mL), cooled in an ice bath. The mixture was stirred at rt overnight, then concentrated, quenched with saturated NH₄Cl (5 mL), and extracted with EtOAc (3 x 5 mL). The organic phases were combined, washed with saturated NaCl (5 mL), dried (Na₂SO₄), and concentrated in vacuo. The afforded crude material was dissolved in MeCN (2.2 mL) + water (0.4 mL) an d purified by prep HPLC on a Phenomenex Gemini-NX 5 µ C18, 110 A, AX, 100 x 30 mm column

eluted with a gradient of Solvent A: 10 mM NH₄OAc in 95/5 H₂O - MeCN and Solvent B: 10 mM NH₄OAc in 90/10 MeCN - H₂O. Appropriate fractions were combined and freeze-dried which gave the title compound (17.5 mg, 32%). MS (ES+) 510.2 [M+H]⁺.
¹H NMR (500 MHz, DMSO-*d*₆) δ 9.11 (s, 1H), 8.32 (s, 1H), 8.20 - 8.12 (m, 2H), 8.08 (dd, *J* = 8.3, 1.2 Hz, 1H), 7.83 (ddd, *J* = 8.4, 6.8, 1.3 Hz, 1H), 7.67 (ddd, *J* = 8.0, 6.8, 0.9 Hz, 1H), 7.39 (d, *J* = 5.2 Hz, 1H), 5.40 (s, 2H), 4.42 (tt, *J* = 12.3, 4.2 Hz, 2H), 3.78 - 3.69 (m, 2H), 3.46 (dt, *J* = 10.3, 5.0 Hz, 2H), 3.26 - 3.18 (m, 2H), 2.95 (s, 3H), 2.95 (t, *J* = 11.2 Hz, 1H), 2.38 (qd, *J* = 12.5, 4.3 Hz, 2H), 1.87 (dt, *J* = 12.9, 2.8 Hz, 2H), 1.63 (p, *J* = 6.7 Hz, 2H), 1.53 (ddt, *J* = 15.4, 9.7, 5.7 Hz, 2H).

### Example 19

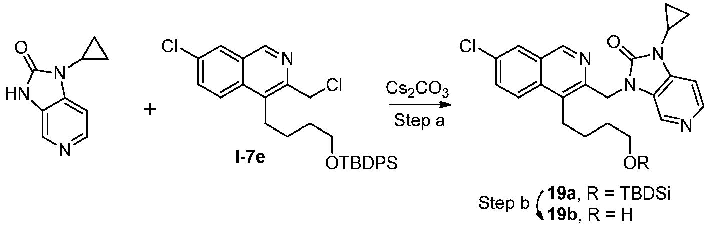

### Step a) 3-((4-(4-((tert-butyldiphenylsilyl)oxy)butyl)-7-chloroisoquinolin-3-yl)methyl)-1-cyclopropyl-1H-imidazo[4,5-c]pyridin-2(3H)-one (19a)

To a stirred solution of 1-cyclopropyl-1H-imidazo[4,5-c]pyridin-2(3H)-one (65 mg, 0.37 mmol, 1 eq) in DMF (7 mL) was added Cs₂CO₃ (362 mg, 1.11 mmol, 3 eq) followed by compound I-7e (194 mg, 0.37 mmol, 1 eq). The reaction mixture was stirred at ambient temperature for 5 h, then diluted with ethyl acetate and washed with water and brine. The combined organic layers were dried (Na₂SO₄) and concentrated under reduced pressure which gave the crude title compound (240 mg), which was used for the next step without further purification. MS (ES+) 661.0 [M+H]⁺.

### Step b) 3-((7-chloro-4-(4-hydroxybutyl)isoquinolin-3-yl)methyl)-1-cyclopropyl-1H-imidazo[4,5-c]pyridin-2(3H)-one (19b)

To a stirred solution of compound 19a (450 mg, 0.68 mmol, 1 eq) in THF (3.0 mL) at 0 °C was added TBAF (1 M in THF, 1.02 mL, 1.02 mmol, 1.5 eq) drop wise and the reaction mixture was stirred at ambient temperature for 5 h, then the reaction was quenched with saturated aq. NH₄Cl solution and the organic components were extracted into ethyl acetate and washed thoroughly with water and brine. The combined organic layers were dried (Na₂SO₄) and concentrated. The afforded crude was purified by Prep HPLC which gave the title compound (50 mg, 17 %). MS (ES+) 423.1 [M+H]⁺.
¹H NMR (500 MHz, DMSO-*d*₆) δ 9.09 (s, 1H), 8.25 (s, 1H), 8.23 - 8.16 (m, 3H), 7.82 (dd, *J* = 9.1, 2.3 Hz, 1H), 7.25 (d, *J* = 5.2 Hz, 1H), 5.36 (s, 2H), 4.44 (t, *J* = 5.1 Hz, 1H), 3.45 (q, *J* = 6.0 Hz, 2H), 3.19 (s, 0H), 2.98 (tt, *J* = 7.1, 3.6 Hz, 1H), 1.62 (p, *J* = 6.8 Hz, 2H), 1.55 - 1.44 (m, 2H), 1.24 (d, *J* = 4.3 Hz, 0H), 1.06 (dt, *J* = 7.1, 3.4 Hz, 2H), 0.91 (p, *J* = 5.3, 4.8 Hz, 2H).

### Example 20

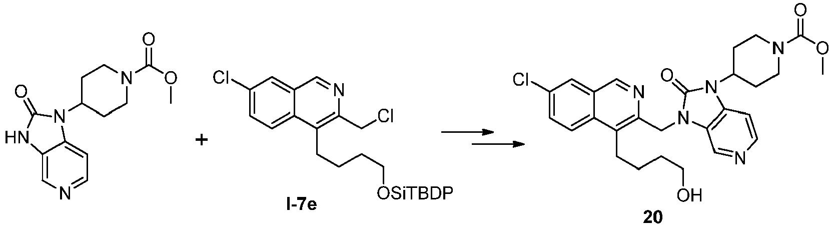

### Methyl 4-(3-((7-chloro-4-(4-hydroxybutyl)isoquinolin-3-yl)methyl)-2-oxo-2,3-dihydro-1H-imidazo[4,5-c]pyridin-1-yl)piperidine-1-carboxylate (20)

Methyl 4-(2-oxo-2,3-dihydro-1H-imidazo[4,5-c]pyridin-1-yl)piperidine-1-carboxylate was reacted with I-7e according to the method described in Example 19 step a, whereafter the SiTBDP-group was removed according to the method described in Example 19 step b. Yield 51%. MS (ES+) 524.2 [M+H]⁺.
¹H NMR (500 MHz, DMSO-*d*₆) δ 9.08 (s, 1H), 8.31 (s, 1H), 8.23 - 8.14 (m, 3H), 7.83 (dd, *J* = 9.1, 2.3 Hz, 1H), 7.42 (d, *J* = 5.4 Hz, 1H), 5.39 (s, 2H), 4.52 - 4.40 (m, 2H), 4.14 (s, 2H), 3.63 (s, 3H), 3.45 (td, *J* = 6.3, 5.0 Hz, 2H), 3.21 (s, 1H), 3.25 - 3.12 (m, 1H), 2.96 (s, 2H), 2.21 (qd, J = 12.5, 4.5 Hz, 2H), 1.76 (dd, J= 12.9, 3.6 Hz, 2H), 1.62 (p, J= 6.6 Hz, 2H), 1.52 (ddt, J= 15.4, 9.8, 5.7 Hz, 2H), 1.23 (s, 0H).

### Example 21

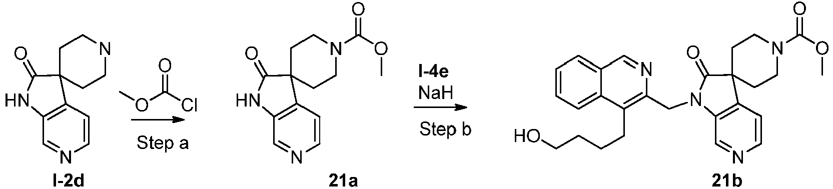

### Step a) methyl 2'-oxo-1',2'-dihydrospiro[piperidine-4,3'-pyrrolo[2,3-c]pyridine]-1-carboxylate (21a)

To a stirred solution of compound I-2d (0.5 g, 2.46 mmol, 1 eq) in THF (3 mL) was added TEA (1 mL, 7.38 mmol, 3 eq) followed by addition of methyl chloroformate (0.2 mL, 2.46 mmol, 1 eq). The mixture was stirred at ambient temperature for 16 h, then diluted with water and the organic components were extracted into ethyl acetate and washed with water and brine. The organic layer was dried over anhyd. Na₂SO₄ and concentrated under reduced pressure. The afforded crude product was purified by silica gel (100-200 mesh) column chromatography using 5% MeOH in DCM to afford the title compound (250 mg, 39%) as a solid. MS (ES+) 262.1 [M+H]⁺.

### Step b) Methyl 1'-((4-(4-hydroxybutyl)isoquinolin-3-yl)methyl)-2'-oxo-1',2'-dihydrospiro[piperidine-4,3'-pyrrolo[2,3-c]pyridine]-1-carboxylate (21b)

NaH (60% dispersed in mineral oil, 13.8 mg, 0.345 mmol, 1.5 eq) was added at 0 °C to a stirred solution of compound 21a (60 mg, 0.23 mmol, 1 eq) in DMF (4 mL). The mixture was stirred for 15 min at 0 °C whereafter compound I-4e (123 mg, 0.25 mmol, 1.1 eq) was added. The reaction mixture was allowed to attain ambient temperature and was stirred for 17 h, then cooled to 0 °C and chilled water was added. The organic components were extracted into EtOAc. The organic part was washed with water and brine, dried over anhyd. Na₂SO₄ and concentrated under reduced pressure. The afforded crude compound was purified by reverse phase prep HPLC which gave the title compound (0.06 g, 55%) as a solid. MS (ES+) 475.2 [M+H]⁺.
¹H NMR (500 MHz, DMSO-*d*₆) δ 9.06 (s, 1H), 8.32 - 8.24 (m, 2H), 8.16 (d, *J* = 8.6 Hz, 1H), 8.05 (d, *J* = 8.1 Hz, 1H), 7.83 (ddd, *J* = 8.4, 6.7, 1.3 Hz, 1H), 7.66 (dd, *J* = 11.2, 6.0 Hz, 2H), 5.26 (s, 2H), 4.46 (t, *J* = 5.1 Hz, 1H), 3.75 (qd, *J* = 16.6, 13.5, 8.7 Hz, 4H), 3.66 (s, 3H), 3.50 (q, *J* = 5.6 Hz, 2H), 3.20 (d, *J* = 15.2 Hz, 1H), 3.20 (s, 1H), 1.88 - 1.74 (m, 4H), 1.65 (dq, *J* = 10.2, 6.1 Hz, 4H).

### Example 22

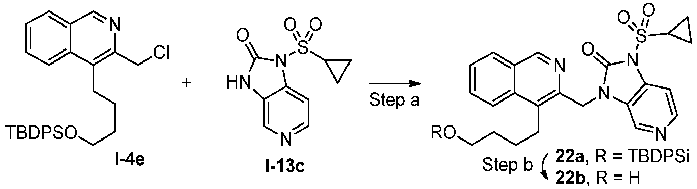

### Step a) 3-((4-(4-((tert-butyldiphenylsilyl)oxy)butyl)isoquinolin-3-yl)methyl)-1-(cyclopropylsulfonyl)-1H-imidazo[4,5-c]pyridin-2(3H)-one (22a)

To a stirred solution of compound 1-(cyclopropylsulfonyl)-1H-imidazo[4,5-c]pyridin-2(3H)-one I-13c (0.2 g, 0.84 mmol, 1 eq) in DMF (8 mL) were added K₂CO₃ (0.173 g, 1.2 mmol, 1.4 eq) and KI (1.6 mg, 0.01 mmol, 0.01 eq) followed by addition of compound I-4e (0.41 g, 0.84 mmol, 1 eq). The mixture was stirred at ambient temperature for 12 h, then diluted with water and extracted with ethyl acetate. The organic layer was washed with water and brine, dried over anhyd. Na₂SO₄ and concentrate under reduced pressure. The crude product was purified by silica gel (100-200 mesh) column chromatography using 2% MeOH in DCM which gave the title compound (150 mg, 40%) as a solid. MS (ES+) 691.3 [M+H]⁺.

### Step b) 1-(cyclopropylsulfonyl)-3-((4-(4-hydroxybutyl)isoquinolin-3-yl)methyl)-1H-imidazo[4,5-c]pyridin-2(3H)-one (22b)

To a stirred solution of compound 22a (200 mg, 0.3 mmol) in MeOH (2 mL) was added HCl (0.5 mL) in water (2 mL) at 0 °C. The solution was stirred at ambient temperature for 12 h, then concentrated in vacuo. The residue was diluted with water, the pH was adjusted to 8 by addition of aq. NaHCO₃ and the solution was extracted with EtOAc. The organic layer was dried over anhyd. Na₂SO₄ and concentrated under reduced pressure. The crude compound was purified by reverse phase prep HPLC to obtain the title compound (29 mg, 21%) as a solid. MS (ES+) 453.1 [M+H]⁺.
¹H NMR (500 MHz, DMSO-*d*₆) δ 9.07 (s, 1H), 8.47 (s, 1H), 8.33 (d, *J* = 5.4 Hz, 1H), 8.18 (d, *J* = 8.6 Hz, 1H), 8.06 (d, *J* = 8.0 Hz, 1H), 7.84 (ddd, *J* = 8.4, 6.8, 1.3 Hz, 1H), 7.68 (t, *J* = 7.5 Hz, 1H), 7.58 (d, *J* = 5.4 Hz, 1H), 5.46 (s, 2H), 4.45 (t, *J* = 5.1 Hz, 1H), 3.50 (q, *J* = 5.7 Hz, 2H), 3.40 (tt, *J* = 7.9, 4.7 Hz, 1H), 3.22 (d, *J* = 15.2 Hz, 1H), 3.22 (s, 1H), 1.65 (dp, *J* = 14.1, 7.3, 6.8 Hz, 4H), 1.36 (dt, *J* = 6.9, 3.5 Hz, 2H), 1.24 (qd, *J* = 6.1, 5.7, 1.3 Hz, 2H).

### Example 23

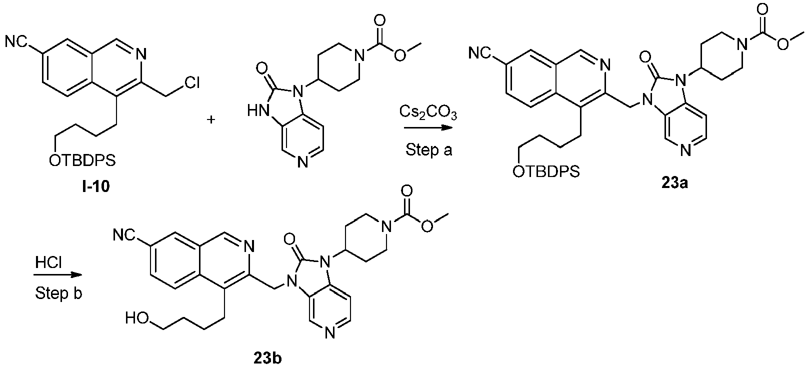

### Step a) methyl 4-(3-((4-(4-((tert-butyldiphenylsilyl)oxy)butyl)-7-cyanoisoquinolin-3-yl)methyl)-2-oxo-2,3-dihydro-1H-imidazo[4,5-c]pyridin-1-yl)piperidine-1-carboxylate (23a)

To a stirred solution of methyl 4-(2-oxo-2,3-dihydro-1H-imidazo[4,5-c]pyridin-1-yl)piperidine-1-carboxylate (175 mg, 0.63 mmol, 1 eq) in DMF (5.0 mL) was added Cs₂CO₃ (619 mg, 1.9 mmol, 3.0 eq) followed by addition of compound I-10 (325 mg, 0.63 mmol, 1 eq). The reaction mixture was stirred at 80 °C for 90 min, then diluted with ethyl acetate and washed with water and brine. The combined organic layers were dried over anhyd. sodium sulphate and concentrated under reduced pressure to get the crude title compound (475 mg, 99 %). MS (ES+) 753.5 [M+H]⁺.

### Step b) methyl 4-(3-((7-cyano-4-(4-hydroxybutyl)isoquinolin-3-yl)methyl)-2-oxo-2,3-dihydro-1H-imidazo[4,5-c]pyridin-1-yl)piperidine-1-carboxylate (23b)

To a stirred solution of compound 23a (475 mg, 0.63 mmol, 1 eq) in MeOH (10.0 mL) under ice cooled condition was added 1:1 aqueous HCl solution (4.0 mL). The reaction mixture was stirred at ambient temperature for 5 h, then concentrated under reduced pressure and distilled azeotropically with toluene. The sticky solid obtained was dissolved in water and extracted with ethyl acetate. The organic layer was washed with water and brine, dried over anhyd. Na₂SO₄ and concentrated to get the title compound (320 mg, 98 %) as a solid. MS (ES+) 515.2 [M+H]⁺.
¹H NMR (500 MHz, DMSO-*d*₆) δ 9.19 (s, 1H), 8.74 (d, *J* = 1.7 Hz, 1H), 8.36 - 8.29 (m, 2H), 8.18 (d, *J* = 5.4 Hz, 1H), 8.09 (dd, *J* = 8.9, 1.8 Hz, 1H), 7.42 (d, *J* = 5.4 Hz, 1H), 5.43 (s, 2H), 4.46 (dt, *J* = 10.1, 4.5 Hz, 2H), 4.14 (s, 2H), 3.63 (s, 3H), 3.46 (q, *J* = 5.9 Hz, 2H), 3.33 (s, 5H), 3.24 (d, *J* = 16.3 Hz, 1H), 2.21 (qd, *J* = 12.5, 4.6 Hz, 2H), 1.76 (dd, *J* = 12.6, 3.8 Hz, 2H), 1.63 (p, *J* = 6.6 Hz, 2H), 1.55 (tq, *J* = 9.8, 6.5, 5.4 Hz, 2H).

### Example 24

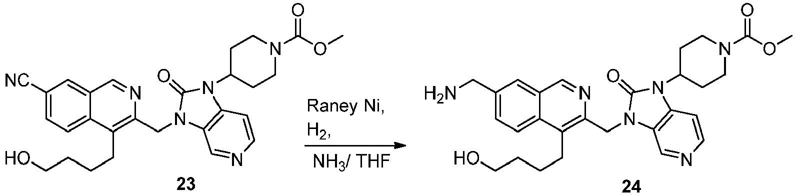

### Methyl 4-(3-((7-(aminomethyl)-4-(4-hydroxybutyl)isoquinolin-3-yl)methyl)-2-oxo-2,3-dihydro-1H-imidazo[4,5-c]pyridin-1-yl)piperidine-1-carboxylate (24)

To a stirred solution of compound 23b (700 mg, 1.36 mmol, 1 eq) in ethanol (2.0 mL) was added Raney nickel (2.0 g) followed by addition of ethanolic NH₃ (20 mL) and the mixture was stirred at ambient temperature under hydrogen atmosphere (balloon pressure) for 17 h, then filtered through celite and washed thoroughly with 10% MeOH in DCM and concentrated *in vacuo.* The afforded crude product was purified by reverse phase prep HPLC and the pure fractions were pooled and concentrated. The afforded compound was dissolved in a minimum volume of MeOH, HCl in dry ether was added and the solution was lyophilized which gave the HCl salt of title compound (40 mg). MS (ES+) 519.34 [M+H]⁺.
¹H NMR (500 MHz, DMSO-*d*₆) δ 9.08 (s, 1H), 8.86 (s, 1H), 8.63 (d, *J* = 5.8 Hz, 2H), 8.27 (d, *J*= 8.9 Hz, 1H), 8.17 (d, *J* = 1.8 Hz, 1H), 8.08 (d, *J* = 6.5 Hz, 1H), 8.02 (dd, *J* = 8.9, 1.8 Hz, 1H), 5.55 (s, 2H), 4.67 (tt, *J* = 12.2, 4.0 Hz, 1H), 4.23 (q, *J* = 5.8 Hz, 2H), 4.15 (s, 2H), 3.62 (s, 3H), 3.49 (s, 2H), 3.49 (d, *J* = 11.7 Hz, 0H), 3.24 (dt, *J* = 8.1, 4.2 Hz, 2H), 2.97 (s, 3H), 2.22 (qd, *J* = 12.4, 4.5 Hz, 2H), 1.83 (dd, *J* = 12.6, 3.6 Hz, 2H), 1.64 (dq, *J* = 6.6, 3.3 Hz, 4H), 1.23 (s, 1H).

### Example 25

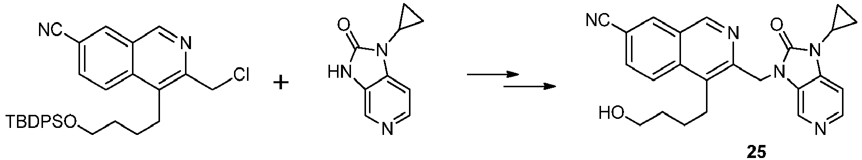

### 3-((1-cyclopropyl-2-oxo-1H-imidazo[4,5-c]pyridin-3(2H)-yl)methyl)-4-(4-hydroxybutyl)isoquinoline-7-carbonitrile (25)

The title compound was prepared according to the method described in Example 23, but using 1-cyclopropyl-1H-imidazo[4,5-c]pyridin-2(3H)-one instead of methyl 4-(2-oxo-2,3-dihydro-1H-imidazo[4,5-c]pyridin-1-yl)piperidine-1-carboxylate. Yield 98%. MS (ES+) 414.1 [M+H]⁺.
¹H NMR (500 MHz, DMSO-*d*₆) δ 9.20 (s, 1H), 8.74 (d, *J* = 1.7 Hz, 1H), 8.33 (d, *J* = 8.9 Hz, 1H), 8.25 (s, 1H), 8.21 (d, *J* = 5.2 Hz, 1H), 8.09 (dd, *J* = 8.9, 1.7 Hz, 1H), 7.26 (d, *J* = 5.2 Hz, 1H), 5.41 (s, 2H), 4.44 (t, *J* = 5.1 Hz, 1H), 3.46 (q, *J* = 6.0 Hz, 2H), 3.25 (s, 0H), 3.24 (s, 1H), 3.22 (s, 0H), 2.98 (tt, *J* = 7.1, 3.7 Hz, 1H), 1.63 (p, *J* = 6.7 Hz, 2H), 1.57 - 1.47 (m, 2H), 1.06 (dt, *J* = 7.1, 3.5 Hz, 2H), 0.91 (p, *J* = 5.3, 4.9 Hz, 2H).

### Example 26

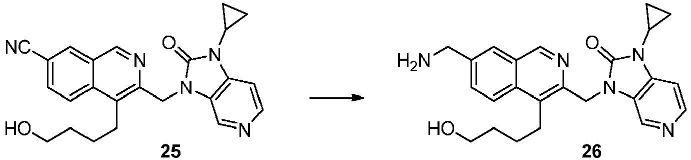

### 3-((7-(Aminomethyl)-4-(4-hydroxybutyl)isoquinolin-3-yl)methyl)-1-cyclopropyl-1H-imidazo[4,5-c]pyridin-2(3H)-one (26)

Compound 25 (170 mg, 0.41 mmol) was reduced according to the procedure described in Example 24, which gave the title compound (40 mg) as a solid. MS (ES+) 418.2 [M+H]⁺.
¹H NMR (500 MHz, DMSO-*d*₆) δ 9.12 (s, 1H), 8.82 (s, 1H), 8.66 (d, *J* = 5.9 Hz, 2H), 8.58 (d, *J*= 6.4 Hz, 1H), 8.29 (d, *J* = 8.9 Hz, 1H), 8.20 (d, *J* = 1.8 Hz, 1H), 8.04 (dd, *J* = 8.9, 1.8 Hz, 1H), 7.81 (d, *J* = 6.3 Hz, 1H), 5.54 (s, 2H), 4.23 (q, *J* = 5.8 Hz, 2H), 3.49 (d, *J* = 5.8 Hz, 2H), 3.25 (s, 2H), 3.15 (tt, *J* = 7.1, 3.6 Hz, 1H), 1.70 - 1.58 (m, 4H), 1.25 (d, *J* = 11.0 Hz, 1H), 1.13 (dt, *J* = 7.2, 3.5 Hz, 2H), 1.03 - 0.96 (m, 2H).

### Example 27

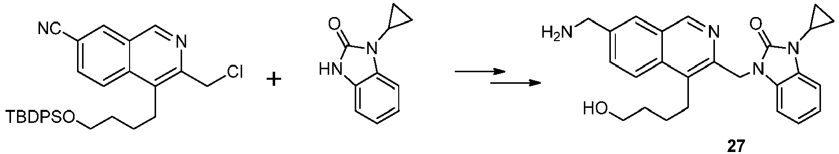

### 3-((7-(Aminomethyl)-4-(4-hydroxybutyl)isoquinolin-3-yl)methyl)-1-cyclopropyl-1H-imidazo[4,5-c]pyridin-2(3H)-one (27)

The title compound (51 mg) was prepared according to the method described in Examples 23 and 24, but using 1-cyclopropyl-1H-benzo[d]imidazol-2(3H)-one instead of methyl 4-(2-oxo-2,3-dihydro-1H-imidazo[4,5-c]pyridin-1-yl)piperidine-1-carboxylate. MS (ES+) 417.2 [M+H]⁺.
¹H NMR (500 MHz, DMSO-*d*₆) δ 9.25 (s, 1H), 8.61 (s, 1H), 8.25 (d, *J* = 16.3 Hz, 2H), 8.03 (dd, *J* = 8.7, 1.8 Hz, 1H), 7.25 - 7.19 (m, 1H), 7.04 (ddd, *J* = 7.2, 3.8, 2.7 Hz, 2H), 6.99 - 6.92 (m, 1H), 5.39 (s, 2H), 4.25 (q, *J* = 5.8 Hz, 2H), 3.40 (t, *J* = 6.4 Hz, 2H), 3.24 - 3.17 (m, 2H), 2.92 (tt, *J* = 7.0, 3.6 Hz, 1H), 1.57 (p, *J* = 6.7 Hz, 2H), 1.45 - 1.33 (m, 2H), 1.23 (d, *J* = 4.6 Hz, 1H), 1.05 (td, *J* = 7.2, 5.1 Hz, 2H), 0.90 (p, *J* = 5.3, 4.8 Hz, 2H).

### Example 28

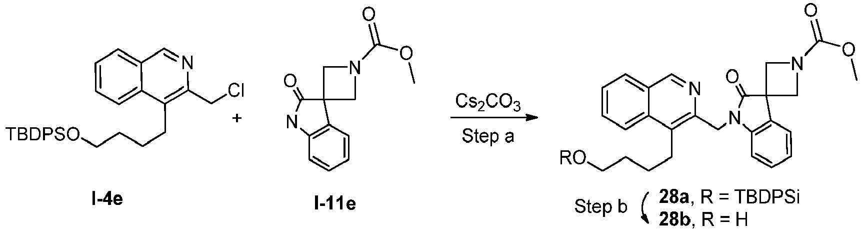

### Step a) Methyl 1'-((4-(4-((tert-butyldiphenylsilyl)oxy)butyl)isoquinolin-3-yl)methyl)-2'-oxospiro[azetidine-3,3'-indoline]-1-carboxylate (28a)

To a stirred solution of compound I-11e (0.222 g, 0.96mmol, 1 eq) in DMF were added Cs₂CO₃ (0.936 g, 2.88 mmol, 3 eq) at 0 °C and compound I-4e (0.467g, 0.96mmol, 1eq). The mixture was stirred at room temperature for 12 h, then diluted with water and extracted with ethyl acetate. The organic layer was washed with brine, dried (Na₂SO₄) and concentrated under reduced pressure. The crude compound was used for the next step without further purification.

### Step b) Methyl 1'-((4-(4-hydroxybutyl)isoquinolin-3-yl)methyl)-2'-oxospiro[azetidine-3,3'-indoline]-1-carboxylate (28b)

To the stirred solution of compound 28a (0.3 g, 0.44 mmol, 1 eq) in MeOH (2mL) was added aq. HCl (2 mL, 3N) at 0 °C. The resulting reaction mixture was stirred at room temperature for 12 h, then concentrated *in vacuo* and the residue dissolved in water. The pH of the solution was adjusted to 8 by addition of aq. NaHCO₃ solution and the organic components were extracted into ethyl acetate. The organic layer was dried (Na₂SO₄) and concentrated under reduced pressure. The obtained crude compound was purified by reverse phase preparative HPLC, which gave the title compound (70 mg). MS (ES+) 446.2 [M+H]⁺.
¹H NMR (500 MHz, DMSO-*d*₆) δ 9.08 (s, 1H), 8.17 - 8.11 (m, 1H), 8.06 (dd, *J* = 8.3, 1.3 Hz, 1H), 7.82 (ddd, *J* = 8.4, 6.8, 1.3 Hz, 1H), 7.72 - 7.62 (m, 2H), 7.21 (td, *J* = 7.8, 1.2 Hz, 1H), 7.07 (td, *J* = 7.5, 1.0 Hz, 1H), 7.01 (d, *J* = 7.8 Hz, 1H), 5.21 (s, 2H), 4.44 (t, *J* = 5.1 Hz, 1H), 4.20 (s, 4H), 3.65 (s, 3H), 3.51 - 3.44 (m, 2H), 3.22 - 3.15 (m, 2H), 1.69 - 1.51 (m, 4H).

### Example 29

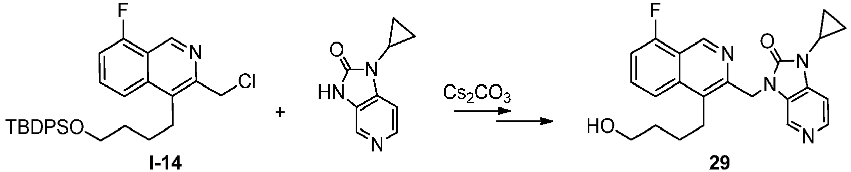

### Step a) 1-Cyclopropyl-3-((8-fluoro-4-(4-hydroxybutyl)isoquinolin-3-yl)methyl)-1H-imidazo[4,5-c]pyridin-2(3H)-one (29)

The title compound was prepared from compound I-14 and 1-cyclopropyl-1H-imidazo[4,5-c]pyridin-2(3H)-one according to the procedure described in Example 28. Yield 20%. MS (ES+) 407.2 [M+H]⁺. The structure was confirmed by ¹H NMR.

### Example 30

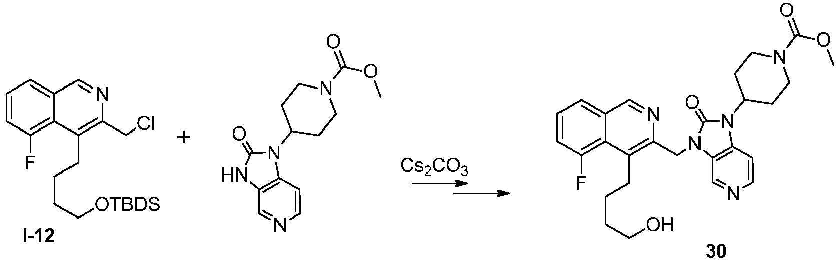

### Methyl 4-(3-((5-fluoro-4-(4-hydroxybutyl)isoquinolin-3-yl)methyl)-2-oxo-2,3-dihydro-1H-imidazo[4,5-c]pyridin-1-yl)piperidine-1-carboxylate (30)

The title compound was prepared from I-12 and methyl 4-(2-oxo-2,3-dihydro-1H-imidazo[4,5-c]pyridin-1-yl)piperidine-1-carboxylate according to the procedure described in Example 28. Yield 21%. MS (ES+) 508.2 [M+H]⁺.
¹H NMR (500 MHz, DMSO-*d*₆) δ 9.14 (d, *J* = 2.4 Hz, 1H), 8.31 (s, 1H), 8.17 (d, *J* = 5.4 Hz, 1H), 7.93 (dd, *J* = 8.0, 1.3 Hz, 1H), 7.66 (td, *J* = 7.8, 4.8 Hz, 1H), 7.62 (ddd, *J* = 13.9, 7.7, 1.3 Hz, 1H), 7.45 - 7.39 (m, 1H), 5.42 (s, 2H), 4.47 (tt, *J* = 12.2, 4.0 Hz, 1H), 4.41 (t, *J* = 5.1 Hz, 1H), 4.15 (s, 2H), 3.63 (s, 3H), 3.44 (q, *J* = 6.0 Hz, 2H), 3.26 (dd, *J* = 15.6, 2.4 Hz, 1H), 3.25 (s, 1H), 2.97 (s, 2H), 2.22 (qd, *J* = 12.5, 4.5 Hz, 2H), 1.80 - 1.73 (m, 2H), 1.66 - 1.50 (m, 4H).

### Example 31

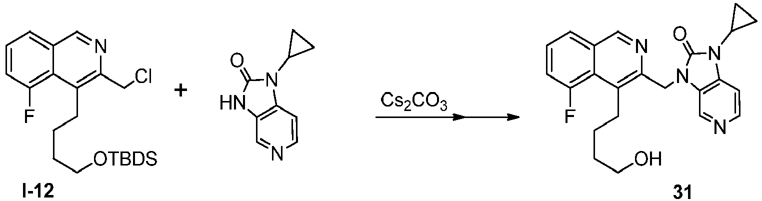

### 1-Cyclopropyl-3-((5-fluoro-4-(4-hydroxybutyl)isoquinolin-3-yl)methyl)-1H-imidazo[4,5-c]pyridin-2(3H)-one (31)

The title compound was prepared from I-12 and 1-cyclopropyl-1H-imidazo[4,5-c]pyridin-2(3H)-one according to the procedure described in Example 28. Yield 21%. MS (ES+) 407.2 [M+H]⁺. ¹H NMR (500 MHz, DMSO-*d*₆) δ 9.15 (d, *J* = 2.4 Hz, 1H), 8.24 (s, 1H), 8.21 (d, *J* = 5.2 Hz, 1H), 7.94 (dd, *J* = 8.0, 1.3 Hz, 1H), 7.66 (td, *J* = 7.8, 4.7 Hz, 1H), 7.61 (ddd, *J* = 13.9, 7.7, 1.3 Hz, 1H), 7.26 (dd, *J* = 5.2, 0.8 Hz, 1H), 5.39 (s, 2H), 4.41 (t, *J* = 5.1 Hz, 1H), 3.44 (td, *J* = 6.4, 5.0 Hz, 2H), 3.25 (dd, *J* = 16.1, 2.4 Hz, 1H), 3.25 (s, 1H), 2.99 (tt, *J* = 7.0, 3.6 Hz, 1H), 1.61 (p, *J* = 6.7 Hz, 2H), 1.57 - 1.47 (m, 2H), 1.07 (td, *J* = 7.3, 5.2 Hz, 2H), 0.91 (qd, *J* = 5.1, 3.7 Hz, 2H).

### Example 32

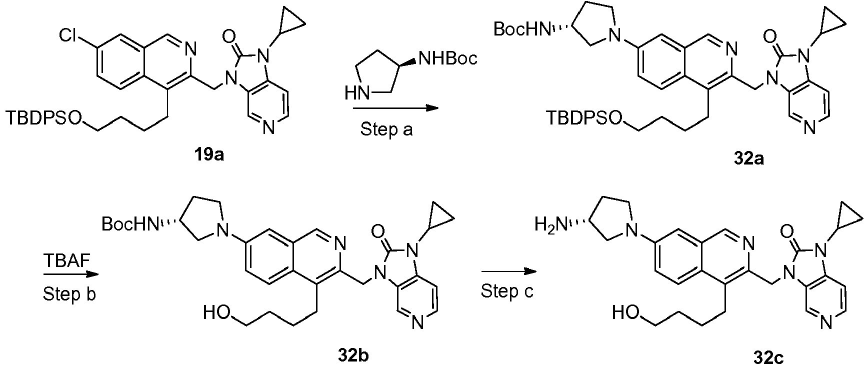

### Step a) (R)-tert-butyl (1-(4-(4-((tert-butyldiphenylsilyl)oxy)butyl)-3-((1-cyclopropyl-2-oxo-1H-imidazo[4,5-c]pyridin-3(2H)-yl)methyl)isoquinolin-7-yl)pyrrolidin-3-yl)carbamate (32a)

A solution of compound 19a (200 mg, 0.302 mmol), (R)-(+)-3-(Boc-amino)pyrrolidine (113 mg, 0.605 mmol) cesium carbonate (148 mg, 0.454 mmol), palladium acetate (6.79 mg, 0.030 mmol) and 2,2'-bis(diphenylphosphino)-1,1'-binaphthyl (37.7 mg, 0.060 mmol) in 1,4-dioxane (3 mL)) in a microwave tube was ultra sonicated for a short while, then degassed with Ar-gas (x3) and heated at 200 °C for 50 min at a pressure of ∼7 bar. The reaction slurry was filtered through Celite, washed with DCM and the filtrate was concentrated in vacuo. The residue was dissolved in DCM and extracted with sat. aq. NaHCO₃, the DCM phase was dried (Na₂SO₄) and concentrated. The afforded residue was purified by silica gel column chromatography eluted with gradient EtOH/DCM which gave the title compound (17.4 mg, 6.6%). MS (ES+) 811.42
[M+H]⁺.

### Step b) (R)-tert-butyl (1-(3-((1-cyclopropyl-2-oxo-1H-imidazo[4,5-c]pyridin-3(2H)-yl)methyl)-4-(4-hydroxybutyl)isoquinolin-7-yl)pyrrolidin-3-yl)carbamate (32b)

1M TBAF-hydrate in THF was added (44 µL) was added to a solution of compound 32a (32.5 mg, 0.040 mmol) in THF (0.40 mL). The solution was stirred for 4 h at rt, then concentrated. The residue was dissolved in a few mL of CHCl₃ and purified by prep-TLC eluted with 10%MeOH/CHCl₃, which gave the title compound (18 mg, 78%). MS (ES+) 573.26 [M+H]⁺.

### Step c) (R)-3-((7-(3-aminopyrrolidin-1-yl)-4-(4-hydroxybutyl)isoquinolin-3-yl)methyl)-1-cyclopropyl-1H-imidazo[4,5-c]pyridin-2(3H)-one (32c)

4M HCl in dioxane (393 µl) was added dropwise to a solution of compound 32b (18 mg, 0.031 mmol) in MeOH (4 mL). The reaction mixture was stirred at rt for 3h, then additional 4M HCl in dioxane (80 µL) was added and the solution was stirred for 18h. The reaction mixture was concentrated and co-evaporated from MeOH (x3). The resulting solid was dissolved in water:MeCN 1:2, insolubles were filtered off through cotton and Celite plugs and the filtrate was freeze dried. The residue was dissolved in 50% H₂O/MeCN (1200 µL) and purified by prep HPLC on a Gemini C18 20 mm column eluted with a gradient of 30-40%B; Buffer A = 0.1% NH₄OH/water; Buffer B: 0.1% NH₄OH/MeCN. Appropriate fractions were combined and concentrated and then freeze dried from 30% H₂O/MeCN which gave the title compound (9.34 mg, 63%). MS (ES+) 473.25 [M+H]⁺.
¹H NMR (500 MHz, DMSO-*d*₆) δ 8.84 (s, 1H), 8.25 (s, 1H), 8.17 (d, *J* = 5.2 Hz, 1H), 7.93 (d, *J* = 9.3 Hz, 1H), 7.30 - 7.20 (m, 2H), 6.81 (d, *J* = 2.5 Hz, 1H), 5.25 (s, 2H), 4.40 (s, 1H), 3.62 (p, *J* = 5.8 Hz, 1H), 3.55 - 3.33 (m, 4H), 3.42 (s, 2H), 3.09 (s, 1H), 3.09 (d, *J* = 16.5 Hz, 1H), 3.05 - 2.92 (m, 2H), 2.16 - 2.06 (m, 1H), 1.76 (dq, *J* = 12.8, 6.2 Hz, 1H), 1.58 (p, *J* = 6.7 Hz, 2H), 1.41 (dq, *J* = 9.4, 6.1, 5.6 Hz, 2H), 1.24 (s, 1H), 1.06 (td, *J* = 7.3, 5.2 Hz, 2H), 0.94 - 0.82 (m, 2H).

### Example 33

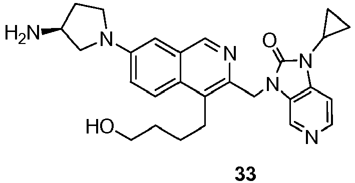

### (S)-3-((7-(3-aminopyrrolidin-1-yl)-4-(4-hydroxybutyl)isoquinolin-3-yl)methyl)-1-cyclopropyl-1H-imidazo[4,5-c]pyridin-2(3H)-one (33)

The title compound was prepared according to the procedure described in Example 32, using (S)-(-)-3-(Boc-amino)pyrrolidine instead of (R)-(+)-3-(Boc-amino)pyrrolidine.
¹H NMR (500 MHz, DMSO-*d*₆) δ 8.84 (s, 1H), 8.25 (s, 1H), 8.17 (d, *J* = 5.2 Hz, 1H), 7.92 (d, *J* = 9.3 Hz, 1H), 7.30 - 7.20 (m, 2H), 6.81 (d, *J* = 2.5 Hz, 1H), 5.24 (s, 2H), 4.40 (t, *J* = 5.0 Hz, 1H), 3.63 - 3.59 (m, 1H), 3.55 - 3.33 (m, 5H), 3.13 - 3.05 (m, 2H), 3.04 - 2.92 (m, 2H), 2.11 (dq, *J* = 12.8, 6.4 Hz, 1H), 1.75 (dq, *J* = 12.6, 6.4 Hz, 1H), 1.58 (p, *J* = 6.7 Hz, 2H), 1.49 - 1.33 (m, 2H), 1.24 (s, 1H), 1.06 (dt, *J* = 7.1, 3.5 Hz, 2H), 0.94 - 0.82 (m, 2H).

### Example 34

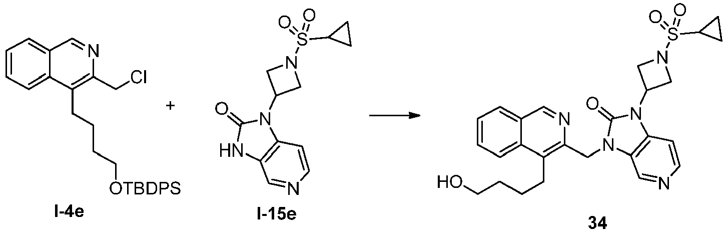

### 1-(1-(cyclopropylsulfonyl)azetidin-3-yl)-3-((4-(4-hydroxybutyl)isoquinolin-3-yl)methyl)-1H-imidazo[4,5-c]pyridin-2(3H)-one (34)

Compound I-4e (255 mg, 0.52 mmol) and I-15e (0.14 g, 0.48 mmol) were reacted according to the method described in Ex. 21 step b, which gave the title compound (50 g, 29%) as a solid. MS (ES+) 508.0 [M+H]⁺.
¹H NMR (500 MHz, DMSO-*d*₆) δ 9.11 (s, 1H), 8.35 (s, 1H), 8.26 (d, *J* = 5.3 Hz, 1H), 8.16 (d, *J*= 8.6 Hz, 1H), 8.08 (d, *J* = 8.1 Hz, 1H), 7.83 (ddd, *J* = 8.4, 6.8, 1.3 Hz, 1H), 7.68 (t, *J* = 7.5 Hz, 1H), 7.50 (d, *J* = 5.4 Hz, 1H), 5.43 - 5.31 (m, 3H), 4.58 (dd, *J* = 8.5, 6.8 Hz, 2H), 4.42 (t, *J* = 5.1 Hz, 1H), 4.30 (t, *J* = 8.5 Hz, 2H), 3.45 (q, *J* = 6.0 Hz, 2H), 3.26 - 3.18 (m, 2H), 2.89 (tt, *J* = 7.9, 4.8 Hz, 1H), 1.63 (p, *J* = 6.7 Hz, 2H), 1.58 - 1.47 (m, 2H), 1.10 (dt, *J* = 7.2, 3.5 Hz, 2H), 1.06 - 0.96 (m, 2H).

### Example 35

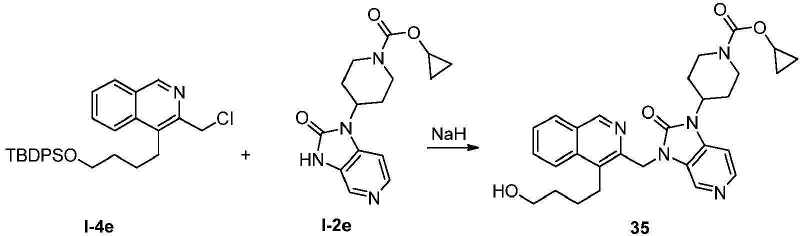

### cyclopropyl 4-(3-((4-(4-hydroxybutyl)isoquinolin-3-yl)methyl)-2-oxo-2,3-dihvdro-1H-imidazo[4,5-c]pyridin-1-yl)piperidine-1-carboxylate (35)

Compound I-4e (248 mg, 0.51 mmol) and I-2e (140 mg, 0.46 mmol) were reacted according to the method described in Ex. 21 step b, which gave the title compound (40 g, 16%) as a solid. MS (ES+) 515.25 [M+H]⁺.
¹H NMR (500 MHz, DMSO-*d*₆) δ 9.10 (s, 1H), 8.31 (s, 1H), 8.19 - 8.10 (m, 2H), 8.07 (dd, *J* = 8.2, 1.2 Hz, 1H), 7.83 (ddd, *J* = 8.4, 6.8, 1.3 Hz, 1H), 7.71 - 7.64 (m, 1H), 7.40 (d, *J* = 5.4 Hz, 1H), 5.39 (s, 2H), 4.51 - 4.40 (m, 2H), 4.16 (s, 1H), 4.02 (ddd, *J* = 9.3, 6.3, 4.2 Hz, 1H), 3.46 (q, *J* = 6.1 Hz, 2H), 3.21 (s, 1H), 3.21 (d, *J* = 16.4 Hz, 1H), 2.94 (s, 2H), 2.26 - 2.14 (m, 2H), 1.75 (d, *J=* 12.1 Hz, 2H), 1.62 (p, *J=* 6.7 Hz, 2H), 1.52 (ddt, *J* = 9.3, 6.7, 4.1 Hz, 2H), 0.66 (s, 1H), 0.65 (s, 3H).

### Example 36

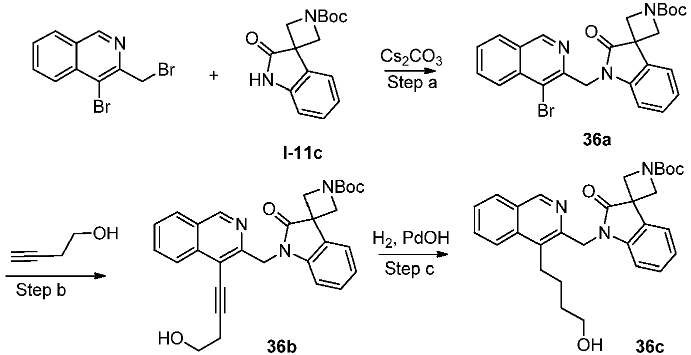

### Step a) tert-butyl 1'-((4-bromoisoquinolin-3-yl)methyl)-2'-oxospiro[azetidine-3,3'-indoline]-1-carboxylate (36a)

A solution of compound I-11 c (449 mg, 1.64 mmol) and cesium carbonate (1.6 g, 4.91 mol) in acetonitrile (10 mL) was stirred for 15 minutes, then 4-bromo-3-(bromomethyl)isoquinoline (493 mg, 1.64 mol) was added in portions. The solution was stirred at rt for16h, then concentrated and the afforded crude product was purified by column chromatography on silica gel eluted with a gradient of heptane-EtOAC, which gave the title compound (750 mg, 93%). MS (ES+) 494.1 & 496.1 [M+H]⁺.

### Step b) Tert-butyl 1'-((4-(4-hydroxybut-1 -yn-1 -yl)isoquinolin-3-yl)methyl)-2'-oxospiro[azetidine-3,3'-indoline]-1-carboxylate (36b)

compound 36a (365 mg, 0.738 mmol), Cul (21.1 mg, 0.111 mmol) and Pd(PPh₃)₂Cl₂ (51.8 mg, 0.074 mmol) were dissolved in DMF (5 mL). The flask was evacuated and put under N2, whereafter Et₃N (1.03 mL, 7.38 mmol) and 3-butyn-1-ol (0.073 mL, 0.960 mmol) were added. The flask was evacuated and flushed with nitrogen again. The reaction was heated at 70 °C over night, then heated in a microwave reactor for 1h at 120 °C. The reaction mixture was filtered through a pad of Celite, rinsed with EtOAc and concentrated. The crude product was purified by column chromatography on silica gel eluted with MeOH in DCM, which gave the title compound (199 mg, 55%). MS (ES+) 484.3 [M+H]⁺.

### Step c) tert-butyl 1'-((4-(4-hydroxybutyl)isoquinolin-3-yl)methyl)-2'-oxospiro[azetidine-3.3'-indoline]-1-carboxylate (36c)

A solution of compound 36b in MeOH (20 mL), EtOAc (5 mL) and AcOH (0.5 mL) was subjected to hydrogenation using a H-cube instrument and 20% palladium hydroxide as catalyst cartridge at rt and ambient pressure. A second run through the H-cube was performed at 40 °C and 10 atm pressure. The solution was concentrated and lyophilized which gave the title compound (89 mg, 44%). MS (ES+) 488 [M+H]⁺.
¹H NMR (500 MHz, DMSO-*d*₆) δ 9.08 (s, 0H), 8.17 - 8.11 (m, 0H), 8.09 - 8.03 (m, 0H), 7.82 (ddd, *J* = 8.4, 6.8, 1.3 Hz, 1H), 7.70 - 7.61 (m, 1H), 7.20 (td, *J* = 7.8, 1.2 Hz, 0H), 7.07 (td, *J* = 7.5, 1.0 Hz, 1H), 7.01 (d, *J* = 7.9 Hz, 0H), 5.21 (s, 1H), 4.44 (t, *J* = 5.1 Hz, 1H), 4.32 (t, *J* = 5.2 Hz, 2H), 4.15 (s, 1H), 4.10 (s, 1H), 3.48 (q, *J* = 6.0 Hz, 1H), 3.37 (td, *J* = 6.6, 5.1 Hz, 4H), 3.18 (s, 1H), 1.62 (dt, *J* = 15.6, 8.3 Hz, 1H), 1.60 - 1.52 (m, 1H), 1.44 (s, 4H), 1.39 (q, *J* = 6.6 Hz, 4H), 1.26 (d, *J* = 10.0 Hz, 2H), 1.25 (s, 5H).

### Example 37

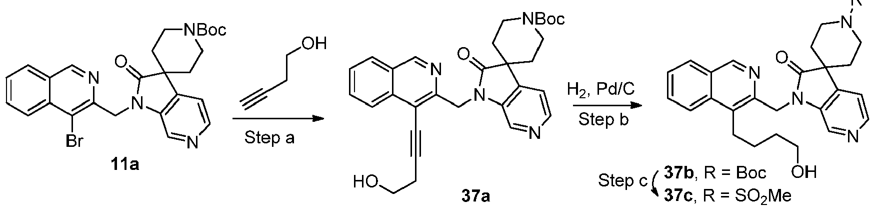

### Step a) Tert-butyl 1'-((4-(4-hydroxybut-1-yn-1-yl)isoquinolin-3-yl)methyl)-2'-oxo-1',2'-dihydrospiro[piperidine-4,3'-pyrrolo[2,3-c]pyridinel-1-carboxylate (37a)

Compound 11a (500 mg, 0.956 mmol) was reacted with 3-butyn-1-ol (87.1 mg, 1.24 mol) according to the method described in Example 36 step b, which gave the title compound (490 mg, 84%). MS (ES+) 513.22 [M+H]⁺.

### Step b) Tert-butvl 1'-((4-(4-hydroxybutyl)isoquinolin-3-yl)methyl)-2'-oxo-1',2'-dihydrospiro[piperidine-4,3'-pyrrolo[2,3-c]pyridine]-1-carboxylate (37b)

Compound 37a (409 mg, 0.798 mmol) was dissolved in EtOH (19 mL) and MeOH (1 mL). The flask was evacuated followed by addition of nitrogen gas, repeated twice, then 10% Pd/C (150 mg, 1.27 mmol) was added. The flask was evacuated and filled with hydrogen gas (from balloon) and the reaction was stirred at ambient temperature over night. The reaction catalyst was filtered off and the filtrate was concentrated. The remains were dissolved in EtOH and MeOH and the reaction was started again as described vide supra and left to proceed for 72h. The reaction mixture was filtered through a pad of Celite and concentrated in vacuo, which gave the title compound in quantitative yield. The afforded compound was used in the next step without further purification. MS (ES+) 517.21 [M+H]⁺.

### Step c) 1'-((4-(4-Hydroxybutyl)isoquinolin-3-yl)methyl)-1-(methylsulfonyl)spiro[piperidine-4,3'-pyrrolo[2,3-c]pyridin]-2'(1'H)-one (37c)

TFA (1 mL, 13.1 mol) was added to a solution of compound 37b in DCM (3 mL), and the solution was stirred at room temperature until complete deprotection. The mixture was concentrated in vacuo, diluted with toluene and concentrated twice. The afforded residue and DIPEA (47 µl, 0.27 mmol) were dissolved in MeCN (4 mL), the solution was stirred for 15 min, then a solution of methanesulfonyl chloride (10 µl, 0.129 mmol) in MeCN (1mL) was added slowly. When the reaction was deemed complete as judged by LCMS, the mixture was concentrated in vacuo. The crude product was purified by preparative HPLC on a Gemini C18 100x30mm using a gradient of 95% water, 5% acetonitrile (10mM in ammonium acetate)and 10% water, 90% acetonitrile (10mM in ammonium acetate). Appropriate fractions were concentrated which gave the title compound (14.5 mg, 38%). MS (ES+) 495.2 [M+H]⁺.
¹H NMR (500 MHz, DMSO-*d*₆) δ 9.05 (s, 1H), 8.33 - 8.26 (m, 2H), 8.16 (d, *J* = 8.6 Hz, 1H), 8.05 (dd, *J* = 8.2, 1.2 Hz, 1H), 7.83 (ddd, *J* = 8.4, 6.8, 1.3 Hz, 1H), 7.70 - 7.62 (m, 2H), 5.27 (s, 2H), 3.61 - 3.41 (m, 5H), 3.20 (d, *J* = 15.2 Hz, 0H), 3.20 (s, 1H), 2.99 (s, 3H), 1.96 (dddd, *J* = 35.2, 13.7, 7.3, 4.0 Hz, 4H), 1.72 (d, *J* = 3.9 Hz, 3H), 1.70 - 1.59 (m, 4H), 1.29 - 1.21 (m, 1H).

### Example 38

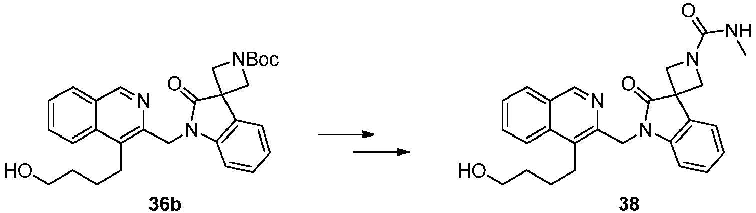

### 1'-((4-(4-hydroxybutyl)isoquinolin-3-yl)methyl)-N-methyl-2'-oxospiro[azetidine-3,3'-indoline]-1-carboxamide (38)

4M HCl in dioxane (2 mL) was added to a solution of compound 36b (35 mg, 0.072 mmol) in DCM. The reaction was stirred for 1h, then concentrated in vacuo and co-evaporated with toluene. The afforded HCl salt was dissolved in MeCN (4 mL), sodium bicarbonate (12.1 mg, 0.144 mmol) and DIEA 43.8 µl, 0.251 mmol) were added and the mixture was stirred for 15 min at 0 °C. A solution of 4-nitrophenyl chloroformate in MeCN (1 mL) was added slowly. After 2h, 40% methylamine in water (100 µl, 1.00 mmol) was added and the mixture was stirred at rt, then concentrated in vacuo. The crude product was purified by preparative HPLC on a Gemini C18 100x30mm using a gradient from 25% to 35%B over 12 min and a flow of 40mL/min. Solvent A: 95% water, 5% acetonitrile (10 mM in ammonium acetate); Solvent B: 10% water, 90% acetonitrile (10 mM in ammonium acetate). The fractions corresponding to the product were pooled together and freeze-dried. The afforded product was further purified by silica gel chromatography eluted with DCM-MeOH, appropriate fractions were pooled and lyophilized, which gave the title compound. (3.7 mg, 12%). MS (ES+) 445.3 [M+H]⁺.
¹H NMR (500 MHz, DMSO-*d*₆) δ 9.08 (s, 1H), 8.17 - 8.11 (m, 1H), 8.06 (dd, *J* = 8.2, 1.3 Hz, 1H), 7.82 (ddd, *J* = 8.4, 6.8, 1.3 Hz, 1H), 7.66 (ddd, *J* = 7.9, 6.8, 0.9 Hz, 1H), 7.58 (dd, *J* = 7.4, 1.2 Hz, 1H), 7.19 (td, *J* = 7.8, 1.3 Hz, 1H), 7.07 (td, *J* = 7.5, 1.0 Hz, 1H), 6.98 (d, *J* = 7.8 Hz, 1H), 6.54 (q, *J*= 4.5 Hz, 1H), 5.21 (s, 2H), 4.44 (t, *J* = 5.1 Hz, 1H), 4.13 (d, *J* = 7.8 Hz, 2H), 3.98 (d, *J* = 7.7 Hz, 2H), 3.48 (td, *J* = 6.2, 5.0 Hz, 2H), 3.18 (s, 1H), 3.16 (s, 0H), 2.61 (d, *J* = 4.5 Hz, 3H), 1.68 - 1.50 (m, 4H), 1.24 (s, 1H).

### Example 39

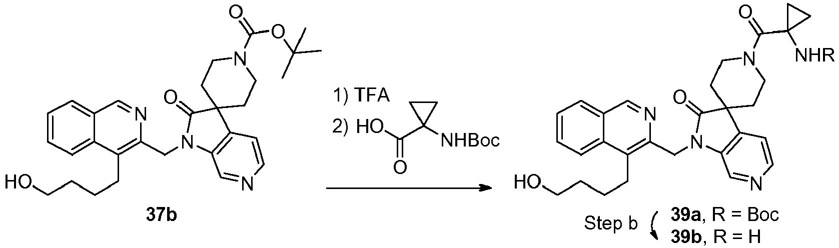

### Step a) Tert-butyl (1-(1'-((4-(4-hydroxybutyl)isoquinolin-3-yl)methyl)-2'-oxo-1',2'-dihydrospiro[piperidine-4,3'-pyrrolo[2,3-c]pyridin]-1-ylcarbonyl)cyclopropyl)carbamate (39a)

TFA (1.0 mL, 13.1 mmol) was added to a solution of compound 37b (120 mg, 0.232 mmol) in DCM (3.0 mL). The reaction was stirred at room temperature until complete deprotection, then concentrated in vacuo, diluted with toluene and concentrated (x2). A part of the residue (64 mg, 0.15 mmol) and 1-((tert-butoxycarbonyl)amino)cyclopropanecarboxylic acid (31 mg, 0.15 mmol) were dissolved in DMF (5.0 mL), DIPEA (99 mg, 0.77 mmol) and HATU (76 mg, 0.20 mol) were added at 0°C and the mixture was stirred at 0 °C for 5 minutes and then left to attain room temperature and stirred for 30 minutes. The crude product was purified by preparative HPLC on a Gemini C18 100x30mm using a gradient from 28- 38%B over 12 min and a flow of 40mL/min. Solvent A: 95% water, 5% acetonitrile (10 mM in ammonium acetate); Solvent B: 10% water, 90% acetonitrile (10 mM in ammonium acetate). Appropriate fractions were pooled and concentrated, which gave the title compound (14 mg, 15%). MS (ES+) 600.4 [M+H]⁺.

### Step b) 1-(1-Aminocyclopropanecarbonyl)-1'-((4-(4-hydroxybutyl)isoquinolin-3-yl)methyl)spiro[piperidine-4,3'-pyrrolo[2,3-c]pyridin]-2'(1'H)-one (39b)

A 4M solution of HCl in dioxane (2.5 mL) was added to a solution of compound 39a (10 mg) in in DCM (4 mL) and MeOH (2 mL). The reaction was stirred at rt for 1h, then concentrated in vacuo.

The residue was dissolved in MeOH and purified by Prep LCMS at pH 10 on a Gemini-NX Prep C18 5 mm OBD 21 x 100 mm, eluted with water/ acetonitrile, using 0.1%NH4OH in both eluents. Appropriate fractions were pooled and freeze-dried which gave the title compound (5.4 mg, 65%). MS (ES+) 500.3 [M+H]⁺.
¹H NMR (500 MHz, DMSO-*d*₆) δ 9.06 (s, 1H), 8.33 - 8.25 (m, 2H), 8.16 (d, *J* = 8.6 Hz, 1H), 8.06 (dd, *J* = 8.2, 1.2 Hz, 1H), 7.83 (ddd, *J* = 8.4, 6.8, 1.3 Hz, 1H), 7.70 - 7.61 (m, 2H), 5.27 (s, 2H), 4.46 (t, *J* = 5.1 Hz, 1H), 3.50 (q, *J* = 5.4 Hz, 2H), 3.33 (s, 11H), 3.21 (d, *J* = 15.2 Hz, 0H), 3.21 (s, 1H), 1.84 (s, 3H), 1.65 (dd, *J* = 7.7, 4.4 Hz, 3H), 0.90 (q, *J* = 4.4 Hz, 2H), 0.68 (t, *J* = 3.3 Hz, 2H).

### Example 40

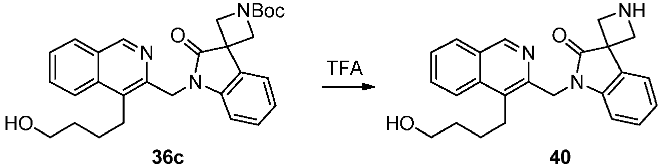

### 1'-((4-(4-Hydroxybutyl)isoquinolin-3-yl)methyl)spiro[azetidine-3,3'-indolin]-2'-one (40)

TFA (0.5 mL) was added to a solution of compound 36c (22 mg, 0.045 mmol) in DCM (3 mL). The solution was stirred at rt for 30 min, then concentrated. The residue was purified by reverse phase prep LCMS, using a gradient of water (+0.1 %NH₄OH) and MeCN (+0.1 %NH₄OH). Appropriate fractions were pooled and freeze dried, which gave the title compound (11.4 mg, 65%). MS (ES+) 388.3 [M+H]⁺.
¹H NMR (500 MHz, DMSO-*d*₆) δ 9.09 (s, 1H), 8.12 (d, *J* = 8.6 Hz, 1H), 8.09 - 8.03 (m, 1H), 7.81 (ddd, *J* = 8.4, 6.8, 1.3 Hz, 1H), 7.74 (s, 1H), 7.66 (t, *J* = 7.4 Hz, 1H), 7.15 (t, *J* = 7.7 Hz, 1H), 7.06 (t, *J* = 6.6 Hz, 1H), 6.95 (d, *J* = 7.8 Hz, 1H), 5.19 (s, 2H), 4.43 (s, 1H), 4.03 (s, 1H), 3.45 (s, 2H), 3.45 (d, *J* = 14.9 Hz, 1H), 3.38 (d, *J* = 7.3 Hz, 1H), 3.16 (d, *J* = 16.4 Hz, 1H), 3.16 (s, 1H), 1.62 (p, *J* = 6.7 Hz, 2H), 1.51 (d, *J* = 7.5 Hz, 1H), 1.49 (s, 2H), 1.39 (q, *J* = 6.7 Hz, 0H), 1.25 (q, *J* = 7.6, 6.4 Hz, 2H).

### Example 41

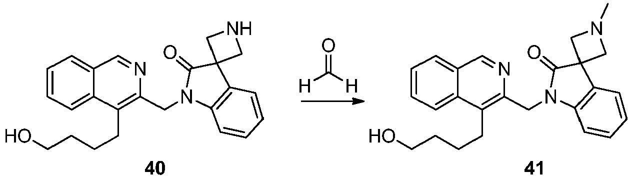

### 1'-((4-(4-Hydroxybutyl)isoquinolin-3-yl)methyl)-1-methylspiro[azetidine-3,3'-indolin]-2'-one (41)

A 37% water solution of formaldehyde (0.041 mmol) was added to a solution of compound 40 (8.0 mg, 0.021 mmol) in DCM (5 mL) followed by addition of acetic acid (7.0 µl, 0.126 mol) and sodium triacetoxyborohydride (13 mg, 0.062 mol). The reaction mixture was stirred at rt for 15 min, then MeOH was added and then the solution was concentrated in vacuo. The residue was purified by Prep LCMS using a Prep Gemini-NX C18 5 mm OBD 21 x 100 mm column, eluted at pH 10 with a gradient of water (+0.1 %NH₄OH) and MeCN (+0.1 %NH₄OH). Appropriate fractions were pooled and freeze dried, which gave the title compound (5.0 mg, 60%). MS (ES+) 402.3 [M+H]⁺.
¹H NMR (500 MHz, DMSO-*d*₆) δ 9.09 (s, 1H), 8.12 (d, *J* = 8.5 Hz, 1H), 8.06 (dd, *J* = 8.3, 1.2 Hz, 1H), 7.81 (ddd, *J* = 8.4, 6.8, 1.3 Hz, 1H), 7.66 (td, *J* = 7.3, 1.0 Hz, 2H), 7.16 (td, *J* = 7.7, 1.3 Hz, 1H), 7.05 (td, *J* = 7.5, 1.0 Hz, 1H), 6.95 (d, *J* = 7.8 Hz, 1H), 5.19 (s, 2H), 4.44 (s, 1H), 3.16 (dd, *J* = 7.1, 4.2 Hz, 2H), 2.36 (s, 4H), 1.61 (p, *J* = 6.7 Hz, 2H), 1.50 (ddd, *J* = 13.6, 10.4, 5.4 Hz, 3H), 1.26 - 1.21 (m, 1H).

### Example 42

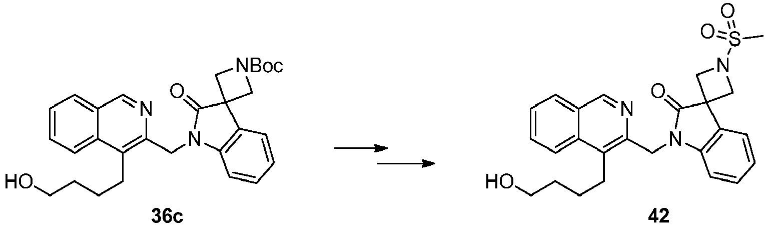

### 1'-((4-(4-Hydroxybutyl)isoquinolin-3-yl)methyl)-1-(methylsulfonyl)spiro[azetidine-3,3'-indolin]-2'-one (42)

TFA (0.5 mL) was added to a solution of compound 36c in DCM (3 mL). The solution was stirred at rt for 30min, then concentrated and co-evaporated with toluene.

The residue and DIEA (28 µl, 0.16 mol) were dissolved in MeCN (4 mL), the solution was stirred for 15 min at 0 °C, then a mixture of methanesulfonyl chloride (40 µl, 0.054 mmol) in MeCN (1 mL) was added slowly. When no starting material was left, the solution was concentrated in vacuo and the crude product was purified further by preparative HPLC on a Gemini C18 100x30mm using a gradient of water and acetonitrile containing 10 mM in ammonium acetate. Appropriate fractions were pooled and freeze-dried and further purified by Prep LCMS eluted with water/ acetonitrile containing 0.1%NH₄OH at pH 10, which gave the title compound (2.1mg, 10%). MS (ES+) 466.3 [M+H]⁺.
¹H NMR (500 MHz, DMSO-*d*₆) δ 9.08 (s, 1H), 8.15 (d, *J* = 8.6 Hz, 1H), 8.06 (d, *J* = 8.2 Hz, 1H), 7.83 (ddd, *J* = 8.3, 6.8, 1.3 Hz, 1H), 7.70 - 7.62 (m, 1H), 7.24 (td, *J* = 7.8, 1.2 Hz, 1H), 7.12 (t, *J* = 7.5 Hz, 1H), 7.03 (d, *J* = 7.8 Hz, 1H), 5.22 (s, 1H), 4.45 (s, 0H), 4.24 (d, *J* = 8.3 Hz, 1H), 4.18 (d, *J* = 8.3 Hz, 1H), 3.33 (s, 11H), 3.20 (s, 1H), 1.70 - 1.53 (m, 3H), 1.24 (s, 1H).

### Example 43

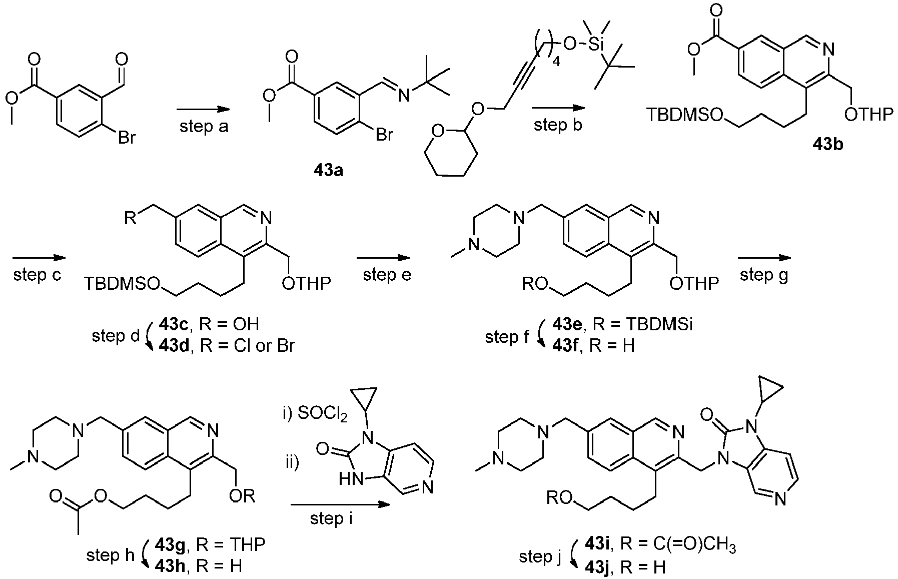

### Step a) (E)-Methyl 4-bromo-3-((tert-butylimino)methyl)benzoate (43a)

To a solution of methyl 4-bromo-3-formylbenzoate (1.22 g, 5.00 mmol) in dry diethyl ether (7.5 mL) under argon was added tert. butylamine (1.10 g, 15.0 mmol) and the mixture was stirred for four hours at RT. The suspension was diluted with DCM (25 mL), magnesium sulfate (9 g) was added and the mixture was stirred at RT overnight. The salts were filtered of and washed with DCM. The solution was concentrated in vacuo and co-evaporated with benzene. The product was dried in vacuo. Yield 1.48 g, 99%. MS (ES+) 299.52 [M+H]⁺.

### Step b) Methyl 4-(4-((tert-butyldimethylsilyl)oxy)butyl)-3-(((tetrahydro-2H-pyran-2-yl)oxy)methyl)isoquinoline-7-carboxylate (43b)

To a solution of compound 43a (1.38g, 4.62 mol) and tert-butyldimethyl((7-((tetrahydro-2H-pyran-2-yl)oxy)hept-5-yn-1-yl)oxy)silane (1.81 g, 5.55 mmol) in acetonitrile (120 mL) under argon was added zinc powder (907 mg, 13.9 mmol) and the NiBr₂(PPh₃)₂ (343 g, 0.462 mmol) and the reaction was refluxed for 3 hours. Additional Zn (450 mg) and NiBr₂(PPh₃)₂ (175 mg) were added and the mixture was refluxed for one more hour. The mixture was cooled and diluted with acetonitrile. The catalyst was filtered of and washed with acetonitrile. The solution was concentrated and the products isolated by silica gel chromatography eluted with isohexane and 10 to 30 % ethyl acetate, which gave the title compound (735 mg, 33%).

### Step c) (4-(4-((Tert-butyldimethylsilyl)oxy)butyl)-3-(((tetrahydro-2H-pyran-2-yl)oxy)methyl)isoquinolin-7-yl)methanol (43c)

To a solution of compound 43b 8700 mg, 1.44 mmol) in dry DCM (25 mL) under argon at -70° C was added drop wise 1M DIBAL solution (3.16 mmol) in toluene. The mixture was stirred for one hour at -70 °C and allowed to come to -50 °C. The reaction was quenched with 10% sodium chloride solution and extracted three times with ethyl acetate. The organic phase was washed with brine, dried over sodium sulfate and concentrated under reduced pressure. The products were isolated by silica gel column chromatography eluted with DCM and 0 to 5% methanol, which gave the title compound (470 mg, 71%).

### Step d) 4-(4-((Tert-butyldimethylsilyl)oxy)butyl)-7-(chloromethyl)-3-(((tetrahydro-2H-pyran-2-yl)oxy)methyl)isoquinoline (43d)

Carbon tetrachloride(108 mg, 0.700 mmol) was added under argon to an ice cooled solution of compound 43c (230 mg, 0.500 mol) and triphenylphosphine (171 mg, 0.650 mmol) in DCM (10 mL). The mixture was stirred at RT for two hours. Only about 10% conversion. Carbon tetrabromide (232 mg, 0.700 mmol) was added and the mixture was stirred for 30 minutes at RT. The solution was added to a silica gel column packed with DCM and the product was eluted with 2 to 6% methanol, which gave a mixture of the chloro and bromo compound which was used directly in the next step as. The yield was estimated to 50%.

### Step e) 4-(4-((Tert-butyldimethylsilyl)oxy)butyl)-7-((4-methylpiperazin-1-yl)methyl)-3-(((tetrahydro-2H-pyran-2-yl)oxy)methyl)isoquinoline (43e)

N-methylpiperazine (136 mg, 1.36 mmol) was added to a solution of compound 43d (130 mg, 0.272 mmol). The mixture was stirred for 72h, then concentrated and the product was isolated by silica gel column chromatography eluted with DCM and 5 to 20% methanol, which gave the title compound (160 mg, 109%). MS (ES+) 542.35 [M+H]⁺.

### Step f) 4-(7-((4-Methylpiperazin-1-yl)methyl)-3-(((tetrahydro-2H-pyran-2-yl)oxy)methyl)isoquinolin-4-yl)butan-1-ol (43f)

To a solution of compound 43e (150 mg, 0.277 mmol) in THF (5 mL) was added 1M solution of tetrabutylammonium fluoride (0.831 mol) and the mixture was stirred at RT overnight. The mixture was diluted with methanol and evaporated onto silica. The product was isolated by silica gel column chromatography eluted with DCM and 10 to 30% methanol, which gave the title compound contaminated with tetrabutylammonium salts (120 mg, 101%).

### Step q) 4-(7-((4-Methylpiperazin-1-yl)methyl)-3-(((tetrahydro-2H-pyran-2-yl)oxy)methyl)isoquinolin-4-yl)butyl acetate (43g)

To a solution of compound 43f ()120 mg, 0.281 mmol) in dry DCM (5 mL) was added TEA (199 mg, 1.96 mmol) and acetic anhydride (142 mg, 1.403 mmol). The solution was stirred for 18h, then concentrated and co-evaporated with toluene. The product was isolated by silica gel column chromatography eluted with DCM and 5 to 20% methanol. Yield125 mg, 95%.
MS (ES+) 470.33 [M+H]⁺.

### Step h) 4-(3-(Hydroxymethyl)-7-((4-methylpiperazin-1-yl)methyl)isoquinolin-4-yl)butyl acetate (43h)

A solution of compound 43g (120 mg, 0.256 mmol) in 80% acetic acid (16 mL) was stirred for two hours at 80 °C, then concentrated under reduced pressure and the product was purified by HPLC (Gemini-NX 100x30mm 20 to 40% acetonitrile flow 40mL per minute). Appropriate fractions were pooled and freeze dried, which gave the title compound (95 mg, 96%). MS (ES+) 386.2 [M+H]⁺.

### Step i) 4-(3-((1-Cyclopropyl-2-oxo-1H-imidazo[4,5-c]pyridin-3(2H)-yl)methyl)-7-((4-methylpiperazin-1-yl)methyl)isoquinolin-4-yl)butyl acetate (43i)

Compound 43h (90 mg) was co-evaporated three times from dioxane toluene which gave 70 mg dry product. The dry product was dissolved in dry DCM under argon and cooled to 0 °C. Thionyl chloride 54.0 mg, 0.454 mmol) was added and the mixture was stirred for two hours in an ice bath. The suspension was concentrated and co-evaporated with toluene. The solid was suspended in about DMF (3 mL) and added to a pre stirred (30 min)suspension of 1-cyclopropyl-1H-imidazo[4,5-c]pyridin-2(3H)-one (31.8 mg, 0.182 mmol) and cesium carbonate (177 mg, 0.454 mmol) in DMF (3 mL).The suspension was stirred at RT for 18h, then additional cesium carbonate (177 mg, 0.454 mmol) was added and the stirring was continued for 72h. The reaction was quenched with water and extracted three times with DCM. The organic phase was washed with brine, dried over sodium sulfate and concentrated under reduced pressure. The product was used in the next step without further purification.

### Step i) 1-Cyclopropyl-3-((4-(4-hydroxybutyl)-7-((4-methylpiperazin-1-yl)methyl)isoquinolin-3-yl)methyl)-1H-imidazo[4,5-c]pyridin-2(3H)-one (43j)

1M sodium methylate solution in methanol was added to a solution of compound 43i (90 mg, 0.166 mmol) in THF (1 mL) and methanol (2 mL). The mixture was stirred for one hour at RT, then diluted with methanol and acidified with acetic acid. The solution was concentrated under reduced pressure and the product was isolated by HPLC.

(Gemini-NX, 100x20mm, 20 to 40% acetonitrile in water). Appropriate fractions were pooled and freeze dried, which gave the title compound (55 mg, 66%). MS (ES+) 501.23 [M+H]⁺.
¹H NMR (500 MHz, DMSO-*d*₆) δ 9.07 (s, 1H), 8.23 (s, 1H), 8.19 (d, *J* = 5.3 Hz, 1H), 8.10 (d, *J* = 8.8 Hz, 1H), 7.93 (d, *J* = 1.7 Hz, 1H), 7.76 (dd, *J* = 8.8, 1.7 Hz, 1H), 7.24 (d, *J* = 5.2 Hz, 1H), 5.35 (s, 2H), 3.63 (s, 2H), 3.44 (t, *J* = 6.4 Hz, 2H), 3.22 - 3.15 (m, 2H), 2.98 (tt, *J* = 7.1, 3.7 Hz, 1H), 2.39 (s, 3H), 2.31 (s, 4H), 2.14 (s, 3H), 1.86 (s, 2H), 1.62 (p, *J* = 6.7 Hz, 2H), 1.53 - 1.42 (m, 2H), 1.06 (dt, *J* = 7.1, 3.5 Hz, 2H), 0.94 - 0.88 (m, 1H).

### Example 44

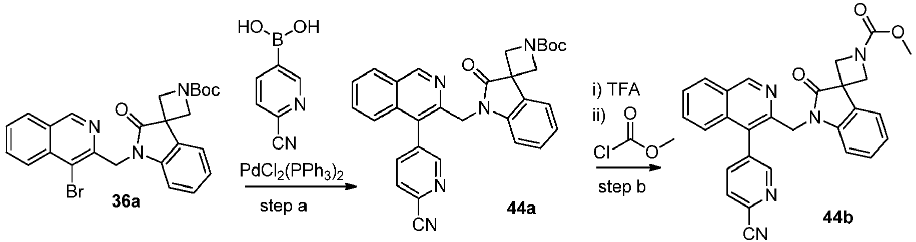

### Step a) Tert-butyl 1'-((4-(6-cyanopyridin-3-yl)isoquinolin-3-yl)methyl)-2'-oxospiro[azetidine-3,3'-indoline]-1-carboxylate (44a)

Compound 36a (200 mg, 0.405 mmol) and (6-cyanopyridin-3-yl)boronic acid (89.8 mg, 0.607 mmol) were coupled together according to the method described in Example 12 step a, which gave the title compound (130 mg, 62%).

### Step c) Methyl 1'-((4-(6-cyanopyridin-3-yl)isoquinolin-3-yl)methyl)-2'-oxospiro[azetidine-3,3'-indoline]-1-carboxylate (44b)

TFA (1 mL) was added to a solution compound 44a in DCM (6 mL). The mixture was stirred for one hour at rt, then concentrated in vacuo, co-evaporated twice with toluene, dried under vacuum. The residue was dissolved in DMF (20 mL) and 10 mL of the afforded solution was cooled to 0 °C, DIEA (151 mg, 1.17 mmol) was added followed by slow addition of methyl chloroformate (16.5 mg, 0.175 mmol). The mixture was stirred for 15 minutes at 0 °C, then ethanol and water was added. The mixture was extracted three times with ethyl acetate. The organic phase was washed with brine, dried over sodium sulfate and concentrated under reduced pressure. The product was purified by column chromatography on silica gel eluted with DCM and 3% methanol. The afforded product was further purified by HPLC on a Gemini-NX 100x30mm column eluted with a gradient of water and 40 to 60% acetonitrile. Appropriate fractions were pooled, concentrated and freeze dried from acetonitrile-water which gave the title compound (35 mg, 63%). MS (ES+) 476.13 [M+H]⁺.
¹H NMR (500 MHz, DMSO-*d*₆) δ 9.40 (d, *J* = 0.8 Hz, 1H), 8.74 (dd, *J* = 2.2, 0.9 Hz, 1H), 8.26 - 8.18 (m, 2H), 8.14 (dd, *J* = 7.9, 2.2 Hz, 1H), 7.79 - 7.70 (m, 2H), 7.63 (dd, *J* = 7.4, 1.2 Hz, 1H), 7.29 - 7.23 (m, 1H), 7.19 (td, *J* = 7.8, 1.3 Hz, 1H), 7.06 (td, *J* = 7.6, 1.0 Hz, 1H), 6.81 (d, *J* = 7.8 Hz, 1H), 5.03 - 4.91 (m, 2H), 3.64 (s, 3H), 3.32 (s, 3H).
¹³C NMR (126 MHz, DMSO) δ 41.88, 44.21, 51.99, 57.36, 109.33, 117.42, 122.55, 123.18, 123.78, 126.06, 126.93, 127.68, 127.87, 128.60, 128.91, 129.12, 131.70, 132.08, 134.48, 135.32, 139.19, 142.88, 145.93, 151.52, 152.83, 156.09, 175.99.

### Example 45

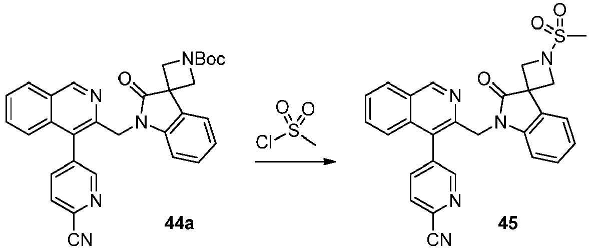

### Step a) Tert-butyl 1'-((4-bromoisoquinolin-3-yl)methyl)-2'-oxospiro[azetidine-3,3'-indoline]-1-carboxylate (45)

10 mL of the DMF-solution from Example 44 step b was put under argon and cooled to 0 °C, DIEA (151 mg, 1.17 mmol) was added followed by slow addition of methane sulfonylchloride (20.5 mg, 0.175 mmol). The mixture was stirred for 10 minutes at 0 °C, then ethanol and water was added. The mixture was extracted three times with ethyl acetate. The organic phase was washed with brine, dried over sodium sulfate and concentrated under reduced pressure. The product was purified by column chromatography on silica gel eluted with DCM and 3% methanol. The afforded product was further purified by HPLC on a Gemini-NX 100x30mm column eluted with a gradient of water and 40 to 60% acetonitrile. Appropriate fractions were pooled, concentrated and freeze dried from acetonitrile-water, which gave the title compound (25 mg, 43%). MS (ES+) 496.12 [M+H]⁺.
¹H NMR (500 MHz, DMSO-*d*₆) δ 9.39 (d, *J* = 0.8 Hz, 1H), 8.77 (dd, *J* = 2.2, 0.9 Hz, 1H), 8.28 - 8.19 (m, 2H), 8.17 (dd, *J* = 7.9, 2.1 Hz, 1H), 7.79 - 7.70 (m, 2H), 7.59 (dd, *J* = 7.5, 1.2 Hz, 1H), 7.32 - 7.24 (m, 1H), 7.22 (td, *J* = 7.8, 1.3 Hz, 1H), 7.10 (td, *J* = 7.6, 1.0 Hz, 1H), 6.87 (d, *J* = 7.8 Hz, 1H), 4.98 (s, 1H), 4.94 (d, *J* = 15.9 Hz, 1H), 4.07 (s, 4H), 3.17 (s, 3H), 1.91 (s, 0H), 1.23 (d, *J* = 6.5 Hz, 0H).

### Example 46

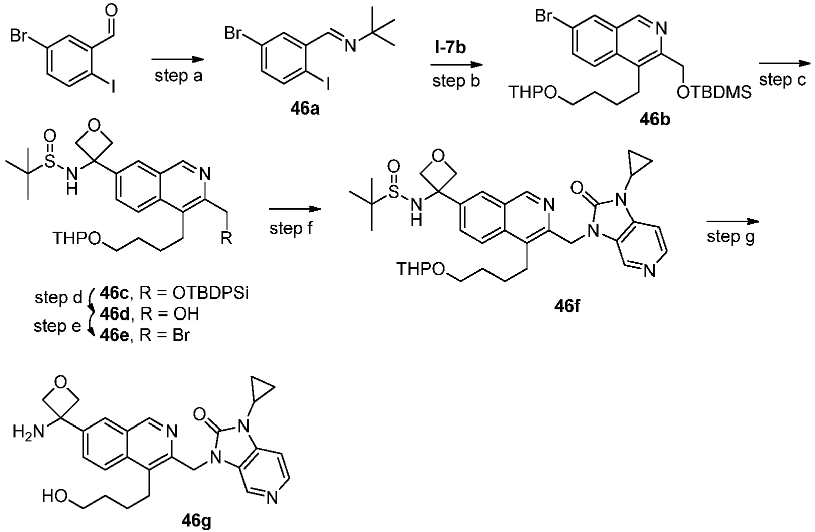

### Step a) (E)-N-(5-Bromo-2-iodobenzylidene)-2-methylpropan-2-amine (46a)

To a solution of 5-bromo-1-iodobenzaldehyde (1.00 g, 3.22 mmol) in dry diethyl ether (5 mL) under argon was added t-butylamine (1.01 mL, 9.65 mmol) and the mixture was stirred for 4 hours at rt. The suspension was diluted with DCM (16 mL), magnesium sulfate (4.5 g) was added and the mixture was stirred at rt overnight, then salts were filtered of and washed with DCM. The solution was concentrated and co-evaporated with toluene. The product was dried in vacuo to which gave the title compound (1.14 g, 97%). MS (ES+) 367.84 [M+H]⁺.

### Step b) 7-Bromo-3-(((tert-butyldiphenylsilyl)oxy)methyl)-4-(4-((tetrahydro-2H-pyran-2-yl)oxy)butyl)isoquinoline (46b)

A solution of compound 46a (2.17 g, 4.81 mmol) in DMF (40 mL) was added to Pd(OAc)₂ (29.2 mg, 0.130 mmol), Ph₃P (63.1 mg, 0.240 mmol) and Na₂CO₃ (255 mg, 2.40 mmol). The mixture was stirred a couple of min then a solution of I-7b (880 mg, 2.40 mmol) in DMF (8 mL) was added. The mixture was put under nitrogen, and stirred at 100 °C for 18h. The mixture was filtered, the filtrate was concentrated and partitioned between conc. NH₄Cl (∼50 mL) and diethyl ether (50-60 mL). The ether phase was dried (Na₂SO₄), filtered and concentrated. The afforded residue was purified by column chromatography on silica gel eluted with EtOAc:heptane. The afforded product was purified again by a second silica gel chromatography eluted with EtOAc:heptane. Total amount obtained: 378 mg, 25%. MS (ES+) 634.11 [M+H]⁺.

### Step c) N-(3-(3-(((Tert-butyldiphenylsilyl)oxy)methyl)-4-(4-((tetrahydro-2H-pyran-2-yl)oxy)butyl)isoquinolin-7-yl)oxetan-3-yl)-2-methylpropane-2-sulfinamide (46c)

Then BuLi 1.5 M solution in hexane (0.565 mmol) was added dropwise at -78 °C to a solution of compound 46b (376 mg, 0.594 mmol) in dry THF (1.4 mL). The mixture was stirred for ∼30 min at -78 °C, then a solution of 3 in THF (0.24 mL) was added dropwise and the mixture was stirred at -78 °C for ∼20 min then left to attain temp rt. After 30 min rt, conc. NH₄Cl (∼0.5 mL) was added and most of the THF was evaporated. Water was added and the mixture was extracted with DCM (x3). The org. phase was extracted with brine (x1) and dried (Na₂SO₄). The residue was purified by column chromatography on silica gel eluted with a gradient of EtOH:DCM, which gave the title compound (46 mg, 11%). MS (ES+) 729.4 [M+H]⁺.

### Step d) N-(3-(3-(Hydroxymethyl)-4-(4-((tetrahydro-2H-pyran-2-yl)oxy)butyl)isoquinolin-7-yl)oxetan-3-yl)-2-methylpropane-2-sulfinamide (46d)

TBAF hydrate (73 µL, 0.073 mmol) was added to a solution of compound 46c (46.0 mg, 0.063 mmol) in THF (0.63 mL). The solution was shaken for 8h at rt, then concentrated to dryness. Molecular sieves (0.5 mL) and dry THF were added and the mixture was shaken for 3h, then the solution was concentrated and the afforded residue was purified by prep-TLC eluted with 10% MeOH in CHCl₃, which gave the title compound (26 mg, 84%). MS (ES+) 491.2 [M+H]⁺.

### Step e N-(3-(3-(bromomethyl)-4-(4-((tetrahydro-2H-pyran-2-yl)oxy)butyl)isoquinolin-7-yl)oxetan-3-yl)-2-methylpropane-2-sulfinamide (46e)

Triphenylphosphine (19.5 mg, 0.074 mmol) was added at 0 °C to a solution of compound 46d (26.0 mg, 0.053 mmol) in DCM (1.3 mL). The mixture was stirred at 0 °C for 15 min, then carbontetrabromide (26.4 mg, 0.079 mmol) was added, the solution was allowed to attain rt and the stirring was continued for 60 min. The solution was concentrated and the residue was purified by prep-TLC (1 mm) eluted with 10% MeOH/CHCl₃. The appropriate band was scraped off and the gel was washed extensively with 10% MeOH/CHCl₃ and the filtrate was concentrated and co-evaporated with DCM/heptane, which gave the title compound (11 mg, 38%). MS (ES+) 555.1 [M+H]⁺.

### Step f N-(3-(3-((1-Cyclopropyl-2-oxo-1H-imidazo[4,5-c]pyridin-3(2H)-yl)methyl)-4-(4-((tetrahydro-2H-pyran-2-yl)oxy)butyl)isoquinolin-7-yl)oxetan-3-yl)-2-methylpropane-2-sulfinamide (46f)

Compound 46e (11.0 mg, 0.020.mmol) was coupled with 1-cyclopropyl-1H-imidazo[4,5-c]pyridin-2(3H)-one (3.66 mg, 0.021 mmol) according to the procedure described in example 4 step a. Purification of the crude compound was performed by prep TLC eluted with 10% MeOH in DCM, which gave the title compound (8.5 mg, 66%). MS (ES+) 648.4 [M+H]⁺.

### Step g) 3-((7-(3-Aminooxetan-3-yl)-4-(4-hydroxybutyl)isoquinolin-3-yl)methyl)-1-cyclopropyl-1H-imidazo[4,5-c]pyridin-2(3H)-one (46g)

A solution of compound 46f (8.5 mg, 0.013 mmol) in AcOH:THF:H₂O (4:2:1 v/v/v) (∼0.7 mL) was warmed at 40 °C for 16 h then at 50 °C for another 16h. The solution was concentrated and co-evaporated with THF (x3). The afforded residue was dissolved in MeOH (1mL) and cooled to 0°C. HCl (4M in dioxane, 250 µL) was added and the reaction mixture was stirred for approximately one minute, then concentrated under cold conditions until no starting material was detected. The afforded crude was dissolved in MeOH and purified by prep. LCMS at pH 10 eluted with a gradient of water and acetonitrile both containing 0.1 % NH₄OH. Appropriate fractions were collected and freeze dried which gave the title compound (4.5 mg, 75%). LCMS: ES(+): 460.3 [M+H]⁺.
¹H NMR (500 MHz, DMSO-*d*₆) δ 9.11 (s, 1H), 8.25 - 8.15 (m, 4H), 8.08 (dd, *J* = 8.9, 2.0 Hz, 1H), 7.27 - 7.21 (m, 1H), 5.36 (s, 2H), 4.79 (d, *J* = 6.0 Hz, 2H), 4.73 (d, *J* = 6.1 Hz, 2H), 4.43 (t, *J* = 5.1 Hz, 1H), 3.45 (q, *J* = 6.0 Hz, 2H), 3.21 (s, 1H), 2.98 (tt, *J* = 7.1, 3.7 Hz, 1H), 2.70 (s, 2H), 1.62 (p, *J* = 6.7 Hz, 2H), 1.58 - 1.44 (m, 2H), 1.24 (s, 1H), 1.07 (td, *J* = 7.3, 5.2 Hz, 2H), 0.95 - 0.82 (m, 2H).

### Example 47

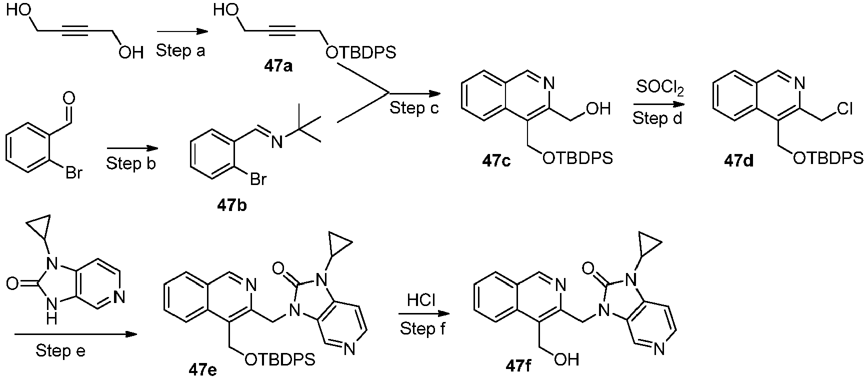

### Step a) 4-((tert-butyldiphenylsilyl)oxy)but-2-yn-1-ol (47a)

To a stirred solution of but-2-yne-1,4-diol (20 g, 233 mmol) in CH₂Cl₂ (250 mL), was added imidazole (9.5 g, 140 mmol, 0.6 eq) followed by a slow addition of a solution of TBDPSCI (30.2 mL, 116 mmol) in DCM, (50 mL) at ambient temperature. The reaction mixture was stirred for 16 h, then diluted with CH₂Cl₂, washed with water and brine, dried over anhyd. Na₂SO₄ and concentrated under reduced pressure. The afforded crude material was purified by silica gel (100-200 mesh) column chromatography using 10% EtOAc in hexane which gave the title compound (75.5 g, 16%)

### Step b) (E)-N-(2-bromobenzylidene)-2-methylpropan-2-amine (47b)

2-Bromobenzaldehyde (5.0 g, 27.0 mmol) and tert. butylamine (25 mL) were taken in a sealed tube and heated at 80 °C for 17 h. The reaction mixture was then concentrated *in vacuo* which gave the title compound (5.5 g, 85%) as a liquid. MS (ES+) 241.7 [M+H]⁺.

### Step c) (4-(((tert-butyldiphenylsilyl)oxy)methyl)isoquinolin-3-yl)methanol (47c)

In a round bottom flask were taken Zn (8.17 g, 125 mmol, 3 eq) and NiBr₂(PPh₃)₂ (1.55 g, 2.1 mmol, 0.05 eq), evacuated and back filled with argon. This process was repeated thrice and then a degassed solution of compound 47a (13.5 g, 41.7 mmol, 1 eq) and 47b (10 g, 41.7 mmol, 1 eq) I acetonitrile (200 mL) was added. The reaction mixture was heated at 80 °C under argon for 4 h, then filtered through Celite, the filtrate was concentrated under reduced pressure and the residue purified by silica gel (100-200 mesh) column chromatography using 0-20% EtOAc in hexane providing two isolated isomers. The required isomer was isolated as a solid (31%). MS (ES+) 428.1 [M+H]⁺.

### Step d) 4-(((tert-butyldiphenylsilyl)oxy)methyl)-3-(chloromethyl)isoquinoline (47d):

SOCl₂ (0.42 mL, 5.85 mmol, 5 eq) was added drop wise to an ice-cooled solution of compound 47c (0.5 g, 1.17 mmol, 1 eq) in DCM (20 mL). The reaction mixture was stirred at ambient temperature for of 5 h, then concentrated under reduced pressure and diluted with DCM and washed with saturated aq. sodium bicarbonate solution, water and brine. The combined organic layer was dried over anhyd. Na₂SO₄ and concentrated under reduced pressure to afford the title compound (0.5 g, 98%). MS (ES+) 445.9 [M+H]⁺.

### Step e) 3-((4-(((Tert-butyldiphenylsilyl)oxy)methyl)isoquinolin-3-yl)methyl)-1-cyclopropyl-1H-imidazo[4,5-c][pyridin-2(3H)-one (47e)

Cs₂CO₃ (2.19 g, 6.72 mmol, 3 eq) was added to a stirred solution of compound 47d (1.0 g, 2.24 mmol, 1 eq) in DMF (15 mL) followed by addition of 1-cyclopropyl-1H-imidazo[4,5-c]pyridin-2(3H)-one (0.39 g, 2.24 mmol, 1 eq). The mixture was stirred at 80 °C for 90 min, then diluted with ethyl acetate and washed with water and brine. The combined organic layer was dried over anhyd. sodium sulfate and concentrated under reduced pressure to give the crude title compound (1.3 g) as red sticky solid, which was used for the next step without further purification.

### Step f) 1-Cyclopropyl-3-((4-(hydroxymethyl)isoquinolin-3-yl)methyl)-1H-imidazo[4,5-c]pyridin-2(3H)-one (47f)

Aqueous HCl (6M, 2 mL) was added to a stirred ice cooled solution of compound 47e (3.5 g, 5.98 mmol, 1 eq) in MeOH (10 mL). The solution was stirred at ambient temperature for 4 h, then concentrated *in vacuo* and dried completely by azeotropic distillation with toluene. The afforded solid was dissolved in water and extracted with ethyl acetate. NaHCO₃ was added to the aqueous layer which then was extracted with ethyl acetate. The combined organic layers were washed with water and brine, dried over anhyd. Na₂SO₄ and concentrated. The afforded crude material was triturated with pentane, diethyl ether and acetone which gave the pure title compound (0.68 g, 33%) as a solid. MS (ES+) 347.2 [M+H]⁺. The structure was confirmed by ¹H NMR.

### Example 48

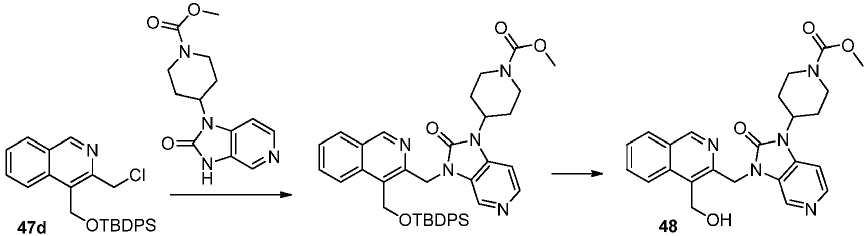

### Methyl 4-(3-((4-(hydroxymethyl)isoquinolin-3-yl)methyl)-2-oxo-2.3-dihydro-1H-imidazo[4,5-c]pyridin-1-yl)piperidine-1-carboxylate (48)

The title compound was prepared from compound 47d (2.6 g, 5.8 mmol) and methyl 4-(2-oxo-2,3-dihydro-1H-imidazo[4,5-c]pyridin-1-yl)piperidine-1-carboxylate (1.6 g, 5.8 mmol) according to the procedure described in Example 47 steps e and f. Yield: 1.5 g, 59%. MS (ES+) 448.1 [M+H]⁺. The structure was confirmed by ¹H NMR.

### Example 49

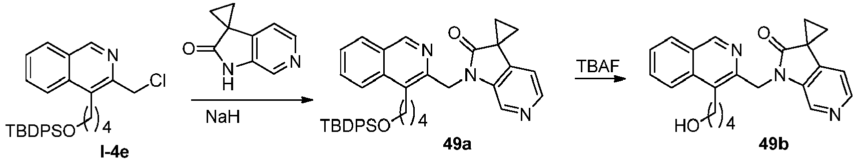

### Step a) 1'-((4-(4-((tert-butyldiphenylsilyl)oxy)butyl)isoquinolin-3-yl)methyl)spiro[cyclopropane-1,3'-pyrrolo[2,3-c]pyridin]-2'(1'H)-one (49a)

NaH (26 mg, 60% dispersed in mineral oil, 0.65 mmol) was added at 0 °C under inert gas to a solution of spiro[cyclopropane-1,3'-pyrrolo[2,3-c]pyridin]-2'(1'H)-one (81.6 mg, 0.509 mmol) in DMF (3.5 mL). After stirring for 15 min at rt, the chloride I-4e (250 mg, 0.512 mmol) was added, while cooling in an ice bath. DMF (1 mL) was added to rinse the sides of the tube. The solution was stirred at rt overnight, then saturated aqueous NaCl (30 mL) + water (15 mL) were added and the mixture was extracted with EtOAc (3 x 40 mL, & 1 x 20 mL). The organic phases were combined, dried (Na₂SO₄), and concentrated under vacuum. The afforded crude product was purified by column chromatography on silica gel eluted with MeOH-DCM, which gave the title compound (168 mg, 54%). MS (ES+) 612.4 [MH]⁺.

### Step b) 1'-((4-(4-hydroxybutyl)isoquinolin-3-yl)methyl)spiro[cyclopropane-1,3'-pyrrolo[2,3-c]pyridin]-2'(1'H)-one (49b)

Compound 49a was reacted as described in Example 14 step b, which gave the title compound (56.8 mg, 55%).
¹H NMR (500 MHz, DMSO-*d*₆) δ 9.17 (s, 1H), 9.14 (s, 1H), 8.35 (s, 2H), 8.26 (s, 1H), 8.21 (s, 1H), 8.15 (d, *J* = 8.6 Hz, 1H), 8.08 (d, *J* = 8.0 Hz, 1H), 7.87 - 7.81 (m, 1H), 7.72 - 7.66 (m, 1H), 7.19 (s, 1H), 7.15 (s, 1H), 5.42 (s, 0H), 5.38 (s, 1H), 5.35 (s, 1H), 4.44 (s, 1H), 3.20 (s, 1H), 1.82 (s, 2H), 1.70 (s, 2H), 1.63 (s, 2H), 1.53 (s, 2H).

### Example 50

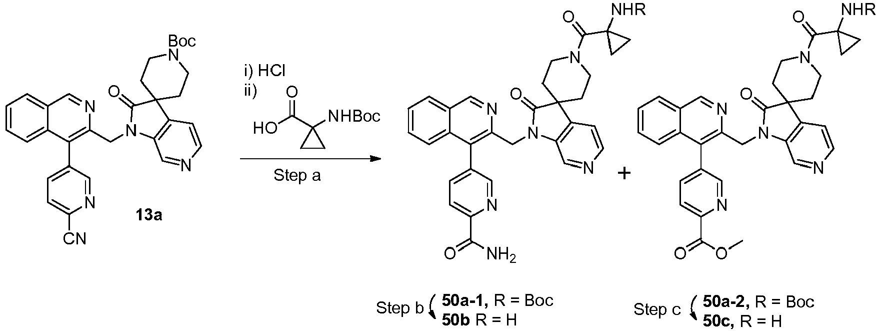

### Step a) tert-butvl (1-(1'-((4-(6-carbamoylpyridin-3-yl)isoquinolin-3-yl)methyl)-2'-oxo-1',2'-dihydrospiro[piperidine-4,3'-pyrrolo[2,3-c]pyridin]-1-ylcarbonyl)cyclopropyl)carbamate(50a-1) & Methyl 5-(3-((1-(1-((tert-butoxycarbonyl)amino)cyclopropanecarbonyl)-2'-oxospiro[piperidine-4,3'-pyrrolo[2,3-c]pyridin]-1'(2'H)-yl)methyl)isoquinolin-4-yl)picolinate (50a-2)

MeOH (3.0 mL) was added to a suspension of compound 13a (160 mg, 0.293 mmol) in 4M HCl (3 mL) The mixture was stirred for 90 min at rt, then cooled on an ice bath and 1-bocamino 1-cyclopopanecarboxylic acid, DIEA and HATU were added and the mixture was stirred for two hours on the ice bath. The reaction mixture was added to a saturated solution of sodium hydrogen carbonate and extracted three times with EtOAc. The organic phase was washed with brine, dried over sodium sulfate and concentrated under reduced pressure. The afforded crude was purified by silica gel chromatography eluted with DCM and 2 to 6% MeOH, which gave the compound 50a-1 (100 mg, 53%). MS (ES+) 648.45 [MH]⁺ and 50a-2 (45 mg, 23%). MS (ES+) 663.44 [MH]⁺.

### Step b) 5-(3-((1-(1-aminocyclopropanecarbonyl)-2'-oxospiro[piperidine-4,3'-pyrrolo[2,3-c]pyridin]-1'(2'H)-yl)methyl)isoquinolin-4-yl)picolinic acid (50b)

TFA (864 mg, 7.58 mmol) was added to a solution of compound 50a-1 (70 mg, 0.108 mmol) in DCM (8 mL). The mixture was stirred at RT for 1 h, then concentrate under reduced pressure and co-evaporated with toluene. The residue was dissolved in MeOH (1.5 mL) and 1M NaOMe in MeOH (1 mL) and purified by HPLC (Gemini NX100x30mm 15 to 35% acetonitrile) which gave the title compound (59 mg, 62%). MS (ES+) 548.3 [MH]⁺.
¹H NMR (500 MHz, DMSO-*d*₆) δ 9.38 (s, 1H), 8.71 (d, *J* = 2.1 Hz, 1H), 8.30 - 8.12 (m, 7H), 7.75 (dddd, *J* = 13.8, 7.9, 5.4, 3.6 Hz, 3H), 7.57 (d, *J* = 4.7 Hz, 1H), 7.34 (dd, *J* = 7.9, 1.6 Hz, 1H), 5.05 (d, *J* = 15.9 Hz, 1H), 4.98 (d, *J* = 15.9 Hz, 1H), 3.93 (s, 2H), 3.76 (s, 2H), 1.88 (s, 2H), 1.76 (s, 2H), 1.67 (s, 2H), 0.88 (q, *J* = 4.0 Hz, 2H), 0.66 (q, *J* = 3.7 Hz, 2H).

### Step c) Methyl 5-(3-((1-(1-aminocyclopropanecarbonyl)-2'-oxospiro[piperidine-4,3'-pyrrolo[2,3-c]pyridin]-1'(2'H)-yl)methyl)isoquinolin-4-yl)picolinate (50c)

Compound 50a-2 was deprotected as described in Ex. 50 step b, which gave the title compound (28 mg, 66%). MS (ES+) 563.6 [MH]⁺.
¹H NMR (500 MHz, DMSO-*d*₆) δ 9.38 (d, *J* = 0.9 Hz, 1H), 8.79 (dd, *J* = 2.2, 0.9 Hz, 1H), 8.30 - 8.24 (m, 2H), 8.25 - 8.16 (m, 2H), 8.18 (s, 1H), 7.75 (tt, *J* = 6.9, 5.2 Hz, 2H), 7.57 (d, *J* = 4.7 Hz, 1H), 7.35 - 7.29 (m, 1H), 5.05 (d, *J* = 15.9 Hz, 1H), 4.96 (d, *J* = 15.9 Hz, 1H), 3.96 (s, 3H), 3.92 (s, 2H), 3.76 (s, 2H), 1.79 (d, *J* = 8.1 Hz, 1H), 1.70 - 1.65 (m, 2H), 0.88 (q, *J* = 4.4 Hz, 2H), 0.66 (t, *J* = 3.3 Hz, 2H).

### Example 51

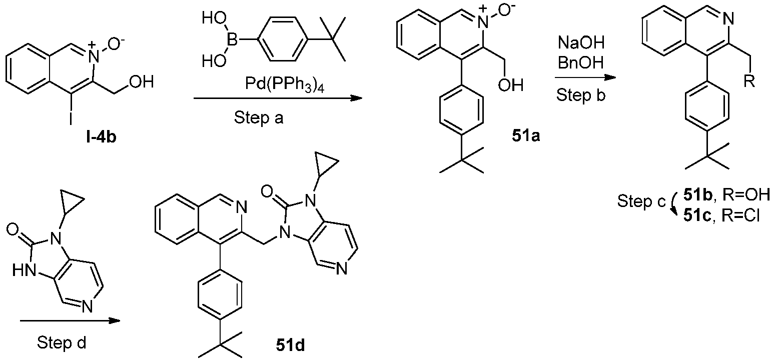

### Step a) (4-(4-(tert-butyl)phenyl)isoquinolin-3-yl)methanol N-oxide (51a)

Potassium carbonate (1.38 g, 9.96 mmol) and (4-(tert-butyl)phenyl)boronic acid (0.887 g, 4.98 mmol) were added to a stirred solution of compound I-4b (1.0 g, 3.32 mmol) in 1,4-dioxane (16 mL) and water (4.0 mL). The mixture was degassed for 15 min with N₂, then tetrakis(triphenylphosphine)palladium(0) (0.192 g, 0.166 mmol) was added and the mixture was again degassed for 10 min with N₂. The reaction mixture was stirred under N₂ atmosphere at 100 °C. The progress of the reaction was monitored by TLC and when deemed complete (∼16 h) the mixture was cooled to rt, concentrated under reduced pressure. Water (15 mL) was added and the mixture was extracted with DCM (2 x 25 mL). The organic layer was washed with brine, dried over Na₂SO₄ and concentrated. The afforded crude was
precipitated with 10% EtOAc/p.ether, the afforded slurry was stirred for 20 min, filtered and dried, which gave the title compound (900 mg, 83 %) as a solid. MS (ES+) 308.25 [MH]⁺.

### Step b) (4-(4-(tert-butyl)phenyl)isoquinolin-3-yl)methanol (51b)

Sodium hydroxide (0.439 g, 11.0 mmol) was added to a stirred solution of compound 51a (0.900 g, 2.20 mmol) in benzyl alcohol (16.0 mL, 0.154 mol) and the resulting mixture was stirred at 120 °C for 16 h, then cooled. Water (20 mL) was added and the mixture was extracted with DCM (2x25 mL), dried (Na₂SO₄) and concentrated. The afforded crude was purified by column chromatography on silica gel (100-200 mesh) eluted with EtOAc/p. ether, which gave the title compound (0.60 g, 85% yield) as a solid. MS (ES+) 292.22 [MH]⁺.

### Step c) 4-(4-(tert-butyl)phenyl)-3-(chloromethyl)isoquinoline (51c)

Thionyl chloride (0.3 mL, 4.12 mmol) was added at 0 °C to a stirred solution of a compound 51b (0.60 g, 2.06 mmol) in DCM (10 mL). The resulting mixture was stirred at reflux for 2 h, then cooled to rt and concentrated under reduced pressure. Cold water (5 mL) was added, the pH was adjusted to 7-7.5 with saturated NaHCO₃ solution, and the mixture was extracted with DCM (2x 25 mL). The organic layer was dried (Na₂SO₄) and concentrated under vacuum. The afforded residue was precipitated with n-pentane (10 mL) and the formed solids were collected by filtration, which gave the title compound (580 mg, 91%) as a solid. MS (ES+) 310.19 [MH]⁺.

### Step d) 3-((4-(4-(tert-butyl)phenyl)isoquinolin-3-yl)methyl)-1-cyclopropyl-1H-imidazof4,5-c]pyridin-2(3H)-one (51d)

Cesium carbonate (1.42 g, 4.36 mmol) and compound 51c (0.450 g, 1.45 mmol) were added at rt under nitrogen to a stirred solution of 1-cyclopropyl-1H-imidazo[4,5-c]pyridin-2(3H)-one (0.280 g, 1.60 mmol) in acetonitrile (15 mL). The resulting mixture was stirred for 16 h at rt, then concentrated under reduced pressure, slowly diluted by ice cold water (15 mL), extracted with DCM (2x25 mL). The organic layer was washed with water (15 mL), brine (15 mL), dried (Na₂SO₄) and concentrated. The resulting residue was triturated with diethyl ether (15 mL). The crude was purified by column chromatography using silica gel (100-200 mesh) eluted with 1-2% MeOH/DCM, which gave the title compound (0.19, 29 %) as a solid. MS (ES+) 449.41 [MH]⁺. ¹H NMR (500 MHz, DMSO-*d*₆) δ 9.25 - 9.21 (m, 1H), 8.20 - 8.10 (m, 2H), 8.05 - 8.00 (m, 1H), 7.70 (dddd, *J* = 22.6, 8.0, 6.8, 1.3 Hz, 2H), 7.61 - 7.54 (m, 2H), 7.35 (dq, *J* = 8.7, 2.4, 1.8 Hz, 3H), 7.20 (dd, *J* = 5.2, 0.8 Hz, 1H), 5.08 (s, 2H), 2.89 (tt, *J* = 7.0, 3.6 Hz, 1H), 1.37 (s, 9H), 1.01 (td, *J* = 7.3, 5.2 Hz, 2H), 0.87 - 0.80 (m, 2H).

### Example 52

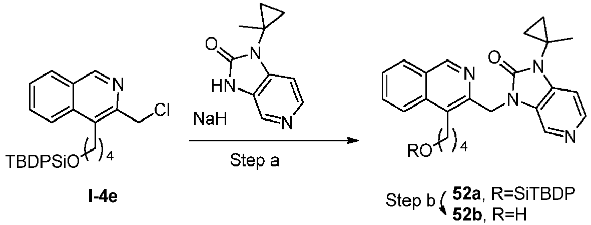

### Steps a & b) 3-((4-(4-hvdroxybutyl)isoquinolin-3-yl)methyl)-1-(1-methylcyclopropyl)-1H-imidazo[4,5-c]pyridin-2(3H)-one (52b)

I-4e (201 mg, 0.413 mmol) was reacted with I-16c (81.3 mg, 0.430 mmol) as described in Example 14 step a, whereafter the TBDSi group was removed as described in Example 14 step b, which gave the title compound (28.7 mg, 13%). MS (ES+) 403.2 [M+H]⁺.
¹H NMR (500 MHz, DMSO-*d*₆) δ 9.12 (s, 1H), 8.29 (s, 1H), 8.21 (d, *J* = 5.2 Hz, 1H), 8.15 (d, *J* = 8.6 Hz, 1H), 8.12 - 8.06 (m, 1H), 7.83 (ddd, *J* = 8.4, 6.8, 1.3 Hz, 1H), 7.68 (t, *J* = 7.5 Hz, 1H), 7.28 (d, *J* = 5.2 Hz, 1H), 5.37 (s, 2H), 4.44 (s, 1H), 3.46 (t, *J* = 6.5 Hz, 2H), 3.25 - 3.18 (m, 2H), 1.63 (p, *J* = 6.8 Hz, 2H), 1.50 (ddt, *J* = 15.9, 10.2, 5.9 Hz, 2H), 1.42 (s, 3H), 1.09-0.97 (m, 4H).

### Example 53

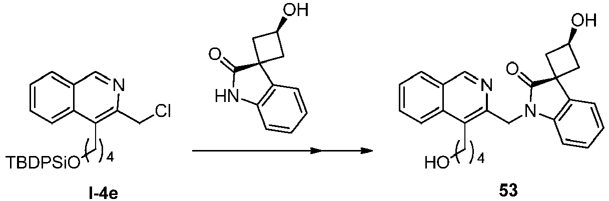

### 3-((4-(4-hydroxybutyl)isoquinolin-3-yl)methyl)-1-(1-methylcyclopropyl)-1H-imidazo[4,5-c]pyridin-2(3H)-one (53)

I-4e (1.1 g, 2.0 mmol) was reacted with A-14 (469 mg, 2.0 mmol) as described in Example 11 step f, whereafter the TBDSi group was removed as described in Example 14 step b, which gave the title compound (8.2%). MS (ES+) 641.52 [M+H]⁺.
¹H NMR (500 MHz, DMSO-*d*₆) δ 9.10 (s, 1H), 8.15 - 8.09 (m, 1H), 8.07 (dd, *J* = 8.2, 1.2 Hz, 1H), 7.81 (ddd, *J* = 8.4, 6.8, 1.3 Hz, 1H), 7.66 (ddd, *J* = 7.9, 6.8, 0.9 Hz, 1H), 7.46 (dd, *J* = 7.4, 1.2 Hz, 1H), 7.11 (td, *J* = 7.7, 1.2 Hz, 1H), 7.05 - 6.93 (m, 2H), 5.57 (d, *J* = 6.8 Hz, 1H), 5.21 (s, 2H), 4.77 - 4.65 (m, 1H), 4.44 (t, *J* = 5.0 Hz, 1H), 3.46 (td, *J* = 6.4, 5.0 Hz, 2H), 3.20 - 3.12 (m, 2H), 2.45 - 2.37 (m, 2H), 1.62 (p, *J* = 6.7 Hz, 2H), 1.53- 1.42 (m, 2H).

### Example 54

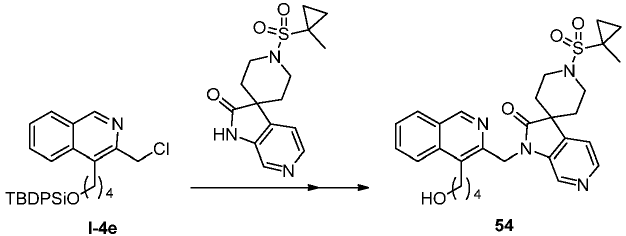

### 1'-((4-(4-hydroxybutyl)isoquinolin-3-yl)methyl)-1-((1-methylcyclopropyl)sulfonyl)spiro[piperidine-4,3'-pyrrolo[2,3-c]pyridin]-2'(1'H)-one (54)

I-4e (304 mg, 0.622 mmol) was reacted with I-18 (100 mg, 0.311 mmol) as described in Example 11 step f, whereafter the TBDSi group was removed as described in Example 14 step b, which gave the title compound (69 mg, 30%). MS (ES+) 641.52 [M+H]⁺.
¹H NMR (500 MHz, DMSO-*d*₆) δ 9.05 (s, 1H), 8.32 - 8.25 (m, 2H), 8.16 (d, *J* = 8.6 Hz, 1H), 8.05 (dd, *J* = 8.3, 1.2 Hz, 1H), 7.83 (ddd, *J* = 8.4, 6.8, 1.3 Hz, 1H), 7.70-7.63 (m, 1H), 7.62 (d, *J* = 4.8 Hz, 1H), 5.76 (s, 1H), 5.27 (s, 2H), 4.46 (s, 1H), 3.76 (ddd, *J* = 12.8, 9.3, 3.5 Hz, 2H), 3.61 (dt, *J* = 12.6, 4.8 Hz, 2H), 3.50 (s, 2H), 3.50 (d, *J* = 11.5 Hz, 1H), 3.20 (d, *J* = 15.1 Hz, 1H), 3.20 (s, 1H), 1.96 (ddd, *J* = 13.5, 9.3, 4.2 Hz, 2H), 1.88 (dt, *J* = 13.7, 4.4 Hz, 2H), 1.65 (dt, *J* = 9.9, 4.3 Hz, 4H), 1.49 (s, 3H), 1.20 (q, *J* = 4.5 Hz, 2H), 0.91 - 0.83 (m, 2H).

### Example 55

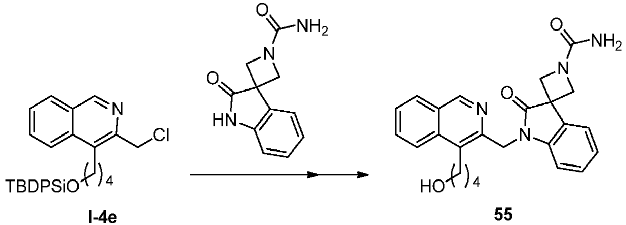

### 1'-((4-(4-hydroxybutyl)isoquinolin-3-yl)methyl)-2'-oxospiro[azetidine-3,3'-indoline]-1-carboxamide (55)

To a stirred solution of I-19 (200 mg, 0.921 mmol) in acetonitrile (15 mL) was added iron (II) trifluoromethanesulfonate (65.2 mg, 0.184 mmol) and cesium carbonate (600 mg, 1.84 mmol) at room temperature. The reaction mixture was stirred at 70 °C for 15 minutes, then I-43 (674 mg, 1.38 mmol) was added and the resulting reaction mixture was stirred at 70 °C for 6 h, then concentrated under reduced pressure. The afforded crude was diluted with water (25 mL) and stirred for 10 minutes, a solid was formed which was collected by filtration, then purified by column chromatography on silica gel eluted with 5% MeOH in DCM. The afforded compound was treated as described in Example 14 step b, which gave the title compound (220 mg, 51%) as a solid. MS (ES+) 431.20 [M+H]⁺.
¹H NMR (500 MHz, DMSO-*d*₆) δ 9.08 (s, 1H), 8.14 (d, *J* = 8.6 Hz, 1H), 8.06 (d, *J* = 8.1 Hz, 1H), 7.82 (t, *J* = 7.7 Hz, 1H), 7.66 (t, *J* = 7.5 Hz, 1H), 7.59 (d, *J* = 7.3 Hz, 1H), 7.19 (t, *J* = 7.7 Hz, 1H), 7.07 (t, *J* = 7.5 Hz, 1H), 6.98 (d, *J* = 7.8 Hz, 1H), 6.15 (s, 2H), 5.21 (s, 2H), 4.45 (t, *J* = 5.1 Hz, 1H), 4.12 (dd, *J* = 18.9, 6.5 Hz, 2H), 3.99 (d, *J=* 7.9 Hz, 2H), 3.48 (q, *J=* 5.9 Hz, 2H), 3.16 (s, 3H), 1.63 (t, *J* = 7.1 Hz, 1H), 1.63 - 1.55 (m, 0H), 1.55 (s, 1H).

### Example 56

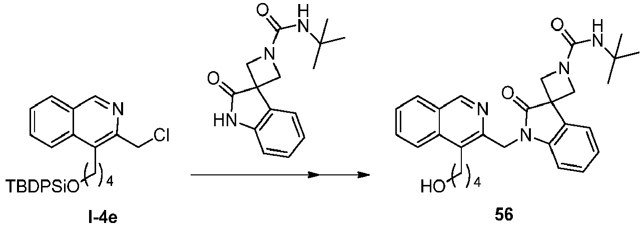

### N-(tert-butyl)-1'-((4-(4-hydroxybutyl)isoquinolin-3-yl)methyl)-2'-oxospiro[azetidine-3,3'-indoline]-1-carboxamide (56)

I-4e (400 mg, 0.819 mmol) was reacted with I-19 (246 mg, 0.901 mmol) as described in Example 11 step f, whereafter the TBDSi-group was removed as described in Example 14 step b, which gave the title compound (55 mg, 20%). MS (ES+) 487.26 [M+H]⁺.
¹H NMR (500 MHz, DMSO-*d*₆) δ 9.08 (s, 1H), 8.14 (d, *J* = 8.6 Hz, 1H), 8.06 (d, *J* = 8.1 Hz, 1H), 7.82 (t, *J* = 7.6 Hz, 1H), 7.66 (t, *J* = 7.4 Hz, 1H), 7.55 (d, *J* = 7.2 Hz, 1H), 7.19 (t, *J* = 7.6 Hz, 1H), 7.07 (t, *J* = 7.5 Hz, 1H), 6.99 (d, *J* = 7.8 Hz, 1H), 5.99 (s, 1H), 5.21 (s, 2H), 4.45 (t, *J* = 5.2 Hz, 1H), 4.13 (d, *J* = 7.5 Hz, 2H), 3.96 (d, *J* = 7.9 Hz, 2H), 3.48 (q, *J* = 6.2, 5.7 Hz, 2H), 3.41 (s, 1H), 3.18 (t, *J* = 7.9 Hz, 2H), 1.64 (p, *J* = 6.8, 6.3 Hz, 2H), 1.57 (q, *J* = 7.8 Hz, 2H), 1.29 (s, 9H).

### Example 57

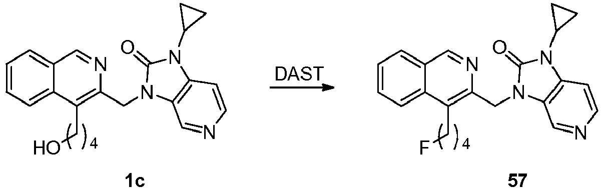

### 1-Cyclopropyl-3-((4-(4-fluorobutyl)isoquinolin-3-yl)methyl)-1H-imidazo[4,5-c]pyridin-2(3H)-one (57)

To stirred solution of compound 1c (0.35 g, 0.90 mmol) in DCM (10 mL) was added a solution of diethylaminosulfur trifluoride(0.73 g, 4.5 mmol) in DCM at -78 ° C. The reaction mixture was stirred at rt for 3 h, then concentrated under reduced pressure, diluted with water(10 mL) and extracted with DCM (3 x 20 mL). The combined the organic layers were dried (Na₂SO₄) and concentrated and the afford crude was purified by silica gel column chromatography, eluted with 3.5% MeOH in DCM, followed by further purification by Prep-HPLC which gave the title compound (50 mg, 14%) as a solid. MS (ES+) 391.26 [M+H]⁺.
¹H NMR (500 MHz, DMSO-*d*₆) δ 9.12 (s, 1H), 8.34 - 8.21 (m, 1H), 8.20 (d, *J* = 5.2 Hz, 1H), 8.23 - 8.08 (m, 1H), 8.09 (dd, *J* = 8.2, 1.2 Hz, 1H), 7.84 (ddd, *J* = 8.4, 6.8, 1.3 Hz, 1H), 7.68 (ddd, *J* = 7.9, 6.8, 0.9 Hz, 1H), 7.25 (dd, *J* = 5.2, 0.8 Hz, 1H), 5.37 (s, 2H), 4.51 (dt, *J* = 47.5, 6.0 Hz, 2H), 3.43 - 3.16 (m, 3H), 2.97 (tt, *J* = 7.1, 3.6 Hz, 1H), 1.99 - 1.75 (m, 2H), 1.65 - 1.45 (m, 2H), 1.06 (td, *J* = 7.3, 5.1 Hz, 2H), 1.02 - 0.79 (m, 2H).

### Example 58 & 59

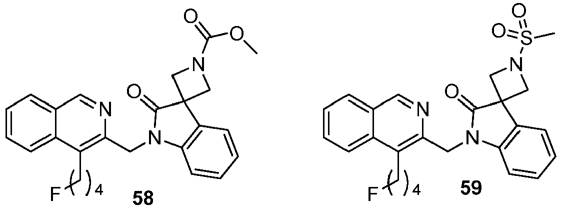

Compound 58 and 59 were prepared from the alcohols 28b and 42 respectively using the procedure of Example 57.

Compound 58: Yield 15%, MS (ES+) 448.31 [M+H]⁺.

Compound 59: Yield 8.7%, MS (ES+) 468.59 [M+H]⁺.

### Example 60

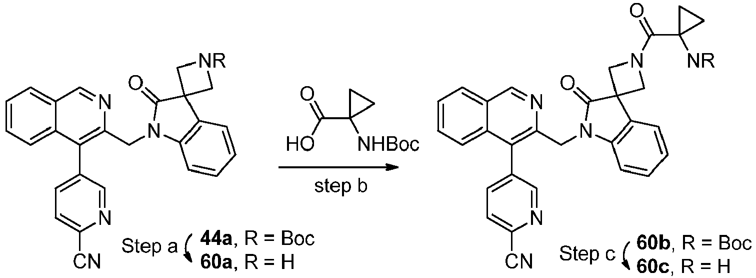

### Step a) 5-(3-((2'-oxospiro[azetidine-3,3'-indolin]-1'-yl)methyl)isoquinolin-4-yl)picolinonitrile (60a)

The Boc group was removed from compound 44a (210 mg, 0.406 mmol) using the method described in Example 40, which gave the title compound (170 mg, 100%).

### Step b) Tert-butyl (1-(1'-((4-(6-cyanopyridin-3-yl)isoquinolin-3-yl)methyl)-2'-oxospiro[azetidine-3,3'-indolin]-1-ylcarbonyl)cyclopropyl)carbamate (60b)

HATU (201 mg, 0.529 mmol) and DIEA (316 mg, 2.44 mmol) were added under argon to a solution of compound 60a (170 mg, 0.406 mmol) and 1-((tert-butoxycarbonyl)amino)-cyclopropanecarboxylic acid (90 mg, 0.448 mmol) in dry DMF (8 mL). The mixture was stirred for 90 min on an ice bath, then added to a 5% solution of sodium hydrogen carbonate and extracted three times with EtOAc. The organic phase was washed with brine, dried over sodium sulfate and concentrated under reduced pressure. The crude was purified by silica gel chromatography eluted with DCM and 0 to 4% MeOH, which gave the title compound (58 mg, 28%). MS (ES+) 601.37 [M+H]⁺.

### Step c) 5-(3-((1-(1-Aminocyclopropanecarbonyl)-2'-oxospiro[azetidine-3,3'-indolin]-1'-yl)methyl)isoquinolin-4-yl)picolinonitrile (60c)

The Boc group was removed from compound 44a (210 mg, 0.406 mmol) using the method described in Example 40. The product was purified by HPLC (Gemini NX, 100x30mm, gradient 30 to 50% acetonitrile) and then freeze dried from acetonitrile water. Yield 20 mg, 24%. MS (ES+) 501.5 [M+H]⁺.
¹H NMR (500 MHz, DMSO-*d*₆) δ 9.39 (s, 1H), 8.75 (dd, *J* = 2.1, 0.9 Hz, 1H), 8.26 - 8.18 (m, 2H), 8.15 (d, *J* = 8.0 Hz, 1H), 7.79 - 7.70 (m, 2H), 7.58 (dd, *J* = 7.4, 1.2 Hz, 1H), 7.30 - 7.24 (m, 1H), 7.19 (td, *J* = 7.8, 1.3 Hz, 1H), 7.08 (td, *J* = 7.5, 1.0 Hz, 1H), 6.79 (d, *J* = 7.8 Hz, 1H), 5.04 - 4.93 (m, 2H), 4.70 (s, 2H), 3.97 (s, 2H), 1.14 - 1.06 (m, 2H), 0.74 - 0.70 (m, 2H).

### Example 61

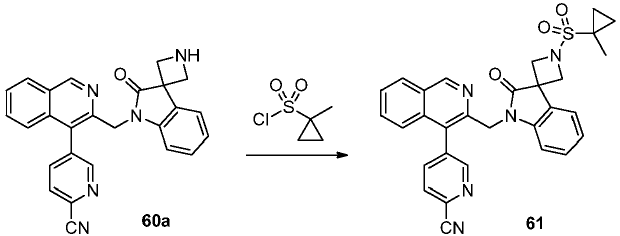

Step a) Tert-butyl (1-(1'-((4-(6-cyanopyridin-3-yl)isoquinolin-3-yl)methyl)-2'-oxospiro[azetidine-Et₃N (0.158 mL) and 1-methylcyclopropane-1-sulfonyl chloride (0.039 mL) were added at 0 °C to a stirred solution of compound 60a (200 mg, 0.376 mmol) in DMF (5 mL). The resulting reaction mixture was stirred at 0 °C for 1 h, then diluted with ice water and stirred for 10 minutes. A solid was formed which was collected by filtration, then purified by preparative HPLC on a C18 column. Appropriate fractions were collected, concentrated and lyophilized which gave the title compound (58 mg, 28%). (MS ES+) 536.17 [M+H]⁺.
¹H NMR (500 MHz, DMSO-*d*₆) δ 9.44 - 9.38 (m, 1H), 8.69 (dd, *J* = 2.1, 0.9 Hz, 1H), 8.27 - 8.19 (m, 2H), 8.11 (dd, *J* = 7.9, 2.2 Hz, 1H), 7.79 - 7.70 (m, 2H), 7.57 (dd, *J* = 7.4, 1.2 Hz, 1H), 7.29 - 7.17 (m, 2H), 7.11 (td, *J* = 7.6, 1.0 Hz, 1H), 6.80 (d, *J* = 7.8 Hz, 1H), 5.76 (s, 1H), 5.01 (s, 1H), 4.97 (d, *J* = 15.8 Hz, 1H), 4.12 (dd, *J* = 7.7, 3.1 Hz, 2H), 3.92 (t, *J* = 7.1 Hz, 2H), 2.47 (s, 1H), 1.55 (s, 3H), 1.26- 1.15 (m, 2H), 0.96-0.89 (m, 2H).

### Example 62

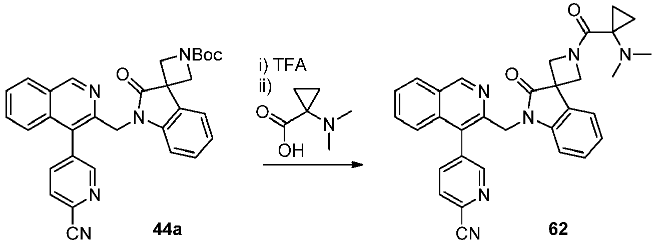

### 5-(3-((1-(1-(dimethylamino)cyclopropanecarbonyl)-2'-oxospiro[azetidine-3,3'-indolin]-1'-yl)methyl)isoquinolin-4-yl)picolinonitrile (62)

TFA was added to a solution of compound 44a (170 mg, 0.263 mmol) in dry DCM (8 mL), the solution was stirred at rt for 90 min then concentrated and co-evaporated twice with toluene. The residue was dissolved in dry DMF (10 mL) and 1-(dimethylamino)cyclopropanecarboxylic acid (50.9 mg, 0.394 mmol) and DIEA (340 mg, 2.63 mmol) were added. The solution was cooled in an ice bath and HATU (130 mg, 0.342 mmol) was added. The mixture was stirred for one hour in the ice bath, then water (40 mL) and saturated sodium hydrogen carbonate solution (40 mL) were added. The mixture was extracted three times with EtOAc. The organic phase was washed with brine, dried over sodium sulfate and concentrated under reduced pressure. The product was purified by silica gel chromatography eluted with DCM and 2 to 4% methanol. The afforded product was further purified by HPLC (Gemini NX 100x30, 25-50% acetonitrile in water 10 mmol ammonium acetate buffer, gradient 14 minutes, flow 40 mL per minute). Appropriate fractions were pooled, concentrated and freeze dried from acetonitrile water, which gave the title compound (55 mg, 40%).
¹H NMR (500 MHz, DMSO-*d*₆) δ 9.42 (d, *J* = 0.8 Hz, 1H), 8.27 - 8.19 (m, 2H), 8.12 (s, 1H), 7.78 - 7.70 (m, 2H), 7.59 (dd, *J* = 7.4, 1.2 Hz, 1H), 7.27 - 7.21 (m, 1H), 7.18 (td, *J* = 7.8, 1.2 Hz, 1H), 7.07 (td, *J* = 7.5, 1.0 Hz, 1H), 6.76 (d, *J* = 7.8 Hz, 1H), 4.40 (s, 2H), 3.99 (s, 1H), 2.21 (s, 6H), 2.08 (s, 3H), 1.24 (s, 1H), 0.94 (s, 4H).

### Example 63

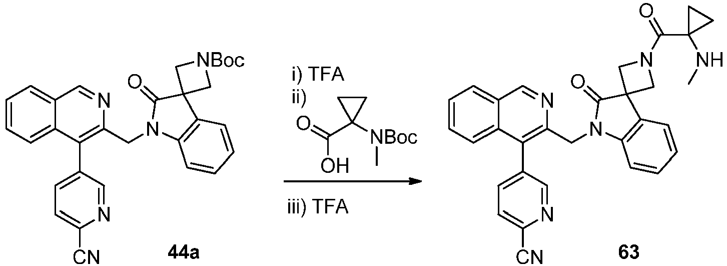

### 5-(3-((1-(1-(Methylamino)cyclopropanecarbonyl)-2'-oxospiro[azetidine-3,3'-indolin]-1'-yl)methyl)isoquinolin-4-yl)picolinonitrile (63)

Compound 44a (160 mg, 0.247 mmol) was N-deprotected and then reacted with 1-((tert-butoxycarbonyl)(methyl)amino)cyclopropanecarboxylic acid (63.9 mg, 0.297 mmol) as described in Example 62. The residue was dissolved in dry DCM (8 mL), TFA was added and the solution was stirred for 90 min at rt, then concentrated and co-evaporated twice with toluene. The residue was dissolved in DCM (5 mL) and TEA (0,5 mL) was added. The solution was concentrated under reduced pressure and the residue purified by HPLC. (Gemini NX 100x30mm; 30-50% acetonitrile in water 10 mmol ammonium acetate buffer). Appropriate fractions were pooled, concentrated and freeze dried from acetonitrile water, which gave the title compound (56 mg, 60%).
¹H NMR (500 MHz, DMSO-*d*₆) δ 9.41 (s, 1H), 8.75 (s, 1H), 8.71 (s, 1H), 8.23 (dt, *J* = 7.5, 2.6 Hz, 2H), 8.14 (s, 1H), 7.79 - 7.70 (m, 2H), 7.59 (dd, *J* = 7.4, 1.2 Hz, 1H), 7.29 - 7.23 (m, 1H), 7.18 (td, *J* = 7.8, 1.3 Hz, 1H), 7.07 (td, *J* = 7.5, 1.0 Hz, 1H), 6.79 (d, *J* = 7.8 Hz, 1H), 4.54 (s, 2H), 4.51 (s, 1H), 3.98 (s, 2H), 2.28 (s, 3H), 2.00 (d, *J* = 7.4 Hz, 1H), 1.24 (s, 0H), 1.04 (d, *J* = 14.6 Hz, 2H), 0.79 (s, 2H).

### Example 64

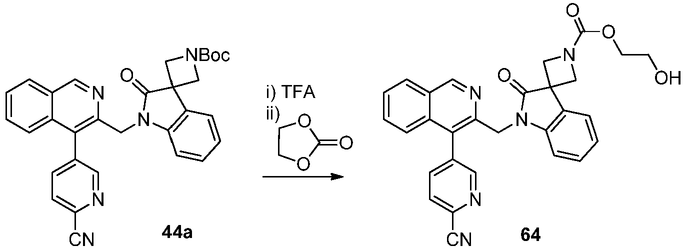

### 2-Hydroxyethyl 1'-((4-(6-cyanopyridin-3-yl)isoquinolin-3-yl)methyl)-2'-oxospiro[azetidine-3,3'-indolinel-1-carboxylate (64)

TFA was added to a solution of compound 44a (360 mg, 0.696 mmol) in dry DCM (8 mL), the solution was stirred at rt for 90 min then concentrated and co-evaporated twice with toluene. The residue, cesium carbonate (552 mg, 1.69 mmol) and ethylene carbonate (149 mg, 1.69 mmol) was stirred in a microwave oven at 70 °C for 4 h. The reaction was quenched with water (10 mL) and extracted with ethyl acetate (2x50 mL), the organic layer was washed with brine, dried over anhydrous sodium sulphate, filtered and concentrated under reduced pressure. The afforded crude was purified by prep. HPLC on a C18 column using 10 mM ammonium bicarbonate in H₂O: acetonitrile as mobile phase. Pure fractions were pooled, concentrated and lyophilized which gave the title compound (60 mg, 21%). (MS ES+) 506.21 [M+H]⁺.
¹H NMR (500 MHz, DMSO-*d*₆) δ 9.40 (s, 1H), 8.75 (d, *J* = 2.1 Hz, 1H), 8.27 - 8.18 (m, 2H), 8.15 (dd, *J* = 7.9, 2.2 Hz, 1H), 7.79 - 7.70 (m, 3H), 7.62 (dd, *J* = 7.4, 1.2 Hz, 1H), 7.30 - 7.23 (m, 1H), 7.19 (td, *J* = 7.8, 1.3 Hz, 1H), 7.07 (td, *J* = 7.6, 1.0 Hz, 1H), 6.82 (d, *J* = 7.8 Hz, 1H), 5.04 - 4.91 (m, 3H), 4.84 (s, 1H), 4.07 (s, 1H), 3.57 (t, *J* = 5.1 Hz, 2H), 3.39 (s, 1H).

### Example 65

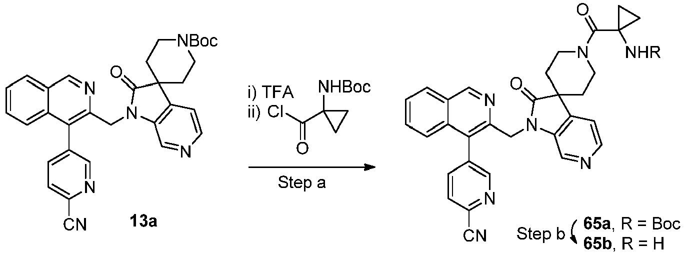

### Step a) tert-butyl (1-(1'-((4-(6-cyanopyridin-3-yl)isoquinolin-3-yl)methyl)-2'-oxo-1',2'-dihydrospiro[piperidine-4,3'-pyrrolo[2,3-c]pyridin]-1-ylcarbonyl)cyclopropyl)carbamate (65a)

TFA (17.15 mmol) was added to a solution of compound 13a (125 mg, 0.229 mmol) in dry DCM (10 mL). The solution was stirred at rt for 90 minutes, then concentrated under reduced pressure and co-evaporated twice with toluene.

The residue and 1-(boc amino)cyclopropane carboxylic acid (55.1 mg, 0.274 mmol) were dissolved in DMF (10 mL). The solution was put under argon and cooled on an ice bath and DIEA (295 mg, 2.28 mmol) and HATU (113 mg, 0.297 mmol) were added. The mixture was stirred for 90 min on ice bath, then a saturated solution of sodium hydrogen carbonate was added and the mixture was extracted three times with EtOAc. The organic phase was washed with brine, dried over sodium sulfate and concentrated under reduced pressure. The product was isolated by silica gel chromatography eluted with DCM and 0 to 4% MeOH. Yield 140 mg, 97%.(MS ES+) 630.6 [M+H]⁺.

### Step b) 5-(3-((1-(1-aminocyclopropanecarbonyl)-2'-oxospiro[piperidine-4,3'-pyrrolo[2,3-c]pyridin]-1'(2'H)-yl)methyl)isoquinolin-4-yl)picolinonitrile (65b)

TFA (23.5 mg, 0.206 mmol) was added to a solution of compound 65a (130 mg, 0.206 mmol) in dry DCM (8 mL). The solution was stirred at rt for 90 min, then concentrated under reduced pressure and co-evaporated twice with toluene. The residue was dissolved in DCM (5 mL), TEA (0,5mL) was added and the solution was concentrated. The product was purified by HPLC (Gemini NX 100x30, 15 to 45% acetonitrile in water 10 mM ammonium acetate) and freeze dried from acetonitrile water. Yield 65 mg, 59%. MS (ES+) 530.4 [M+H]⁺.
¹H NMR (500 MHz, DMSO-*d*₆) δ 9.40 (d, *J* = 0.8 Hz, 1H), 8.89 (dd, *J* = 1.9, 1.0 Hz, 1H), 8.34 - 8.24 (m, 3H), 8.22 (d, *J* = 7.2 Hz, 2H), 7.81 - 7.71 (m, 2H), 7.60 - 7.54 (m, 1H), 7.36 - 7.30 (m, 1H), 5.00 (d, *J* = 11.2 Hz, 2H), 3.94 (s, 2H), 3.32 (s, 5H), 1.88 (s, 1H), 1.76 (d, *J* = 9.8 Hz, 2H), 1.69 (s, 2H), 0.89 (q, *J* = 4.2 Hz, 2H), 0.67 (t, *J* = 3.2 Hz, 2H).

### Example 66

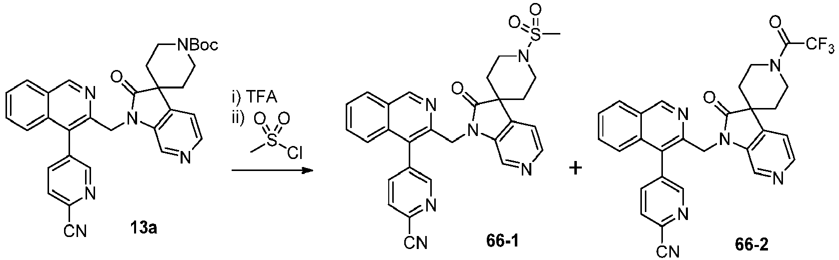

### 5-(3-((1-(Methylsulfonyl)-2'-oxospiro[piperidine-4,3'-pyrrolo[2,3-c]pyridin]-1'(2'H)-yl)methyl)isoquinolin-4-yl)picolinonitrile (66-1) & 5-(3-((2'-Oxo-1-(2,2,2-trifluoroacetyl)spiro[piperidine-4,3'-pyrrolo[2,3-c]pyridin]-1'(2'H)-yl)methyl)isoquinolin-4-yl)picolinonitrile (66-2)

TFA (207 µl, 2.71 mmol) was added dropwise at rt to a solution of compound 13a (75.0 mg, 0.137 mmol) in DCM (2 mL). The solution was stirred for 2h, then concentrated and co-evaporated with toluene. The residue and DIEA (62.1 mg, 0.480 mmol) were dissolved at rt in MeCN (5 mL), methanesulfonyl chloride (18.9 mg, 0.165 mmol) was added. The solution was stirred for 18h, then additional DIEA and sulfonyl chloride were added. After 4h the starting material was consumed and the reaction mixture was concentrated. The afforded crude was dissolved in MeCN/MeOH and purified by Prep LCMS at pH7 on a Gemini-NX Prep C18 5 mm column eluted with 10 nM ammonium acetate in water/10 nM ammonium acetate in acetonitrile. The two title compounds were separately collected and freeze-dried. The second compound of the second peak was further purified by column chromatography on silica gel eluted with DCM/MeOH and freeze dried.
66-1: Yield 16.8 mg, 23%, MS (ES+) 525.1 [M+H]⁺.
¹H NMR (500 MHz, DMSO) δ 9.40 (s, 1H, 10), 8.89 (m, 1H, 35), 8.32 (m, 1H, 32), 8.27 (dd, *J* = 7.9, 2.1 Hz, 3H, 18, 20, 31), 8.22 (dd, *J* = 6.7, 2.4 Hz, 1H, 6), 7.76 (m, 2H, 1, 2), 7.60 (dd, *J* = 10.8, 5.8 Hz, 1H, 21), 7.34 (m, 1H, 3), 4.99 (d, *J* = 3.3 Hz, 2H, 12), 3.41 (m, 4H, 23, 25), 2.97 (s, 3H, 30), 1.92 (m, 2H, 22", 26"), 1.77 (m, 2H, 22', 26').
¹³C NMR (126 MHz, DMSO) δ 177.00, 17, 152.80, 10, 151.86, 35, 145.78, 8, 143.91 , 20, 141.09 , 15, 139.50 , 31, 139.28 , 14, 135.29 , 11, 134.44 , 4, 132.20 , 33, 131.73 , 2, 130.46 , 18, 129.13 , 32, 127.88 , 6, 127.75 , 1, 127.01 , 5, 126.53 , 7, 123.87 , 3, 118.37 , 21, 117.53 , 37, 43.52 , 12, 16, 40.53 , 23, 25, 34.11 , 30, 31.16, 31.05, 31.04 , 22, 26.
66-2: Yield 31.8 mg, 43%, MS (ES+) 543.1 [M+H]⁺.
¹H NMR (500 MHz, DMSO) δ 9.39 (d, *J=* 1.6 Hz, 1H, 11), 8.89 (m, 1H, 35), 8.29 (m, 3H, 21, 31, 32), 8.25 (d, *J* = 6.3 Hz, 1H, 19), 8.22 (m, 1H, 7), 7.76 (ddt, *J* = 10.1, 6.9, 3.6, 3.6 Hz, 2H, 2, 3), 7.64 (d, *J=* 4.7 Hz, 1H, 22), 7.33 (d, *J=* 7.9 Hz, 1H, 4), 5.00 (m, 2H, 13), 4.04 (m, 1H, 26"), 3.89 (m, 1H, 24"), 3.82 (dt, *J* = 14.7, 7.2, 7.2 Hz, 1H, 24'), 3.74 (t, *J* = 11.1, 11.1 Hz, 1H, 26'), 1.94 (ddd, *J* = 13.6, 9.7, 4.0 Hz, 2H, 23", 27"), 1.76 (m, 2H, 23', 27').
¹³C NMR (126 MHz, DMSO) δ 177.07 (d, *J* = 4.9 Hz, 18), 154.22 (d, *J* = 34.7 Hz, 29), 152.86 , 11, 151.89 , 35, 145.70 , 9, 144.04 , 21, 140.76 , 16, 139.53 , 31, 139.18 , 15, 135.28 , 33, 134.44 , 5, 132.20 , 12, 131.74 , 3, 130.55 , 19, 129.09, 127.89 , 7, 127.76 , 2, 127.00 , 6, 126.50, 126.46 , 8, 123.87 , 4, 118.31 , 22, 117.48, 115.17 , 36, 44.05 , 17, 43.52 (d, *J* = 3.3 Hz, 13), 40.66 , 24, 38.37 , 26, 31.45 , 27, 30.52 , 23.

### Example 67

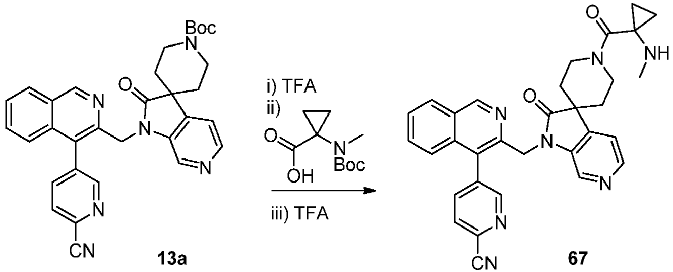

### 5-(3-((1-(1-(Methylamino)cyclopropanecarbonyl)-2'-oxospiro[piperidine-4,3'-pyrrolo[2,3-c]pyridin]-1'(2'H)-yl)methyl)isoquinolin-4-yl)picolinonitrile (67)

The title compound was obtained from compound 13a (100 mg, 0.183 mmol) and 1-((tert-butoxycarbonyl)(methyl)amino)cyclopropanecarboxylic acid (47.2 mg, 0.220 mmol) using the procedure of in Example 63. Yield 45 mg, 46%. MS (ES+) 544.5 [M+H]⁺.
¹H NMR (500 MHz, DMSO-*d*₆) δ 9.40 (d, *J* = 0.9 Hz, 1H), 8.88 (dd, *J* = 2.0, 1.0 Hz, 1H), 8.34 - 8.18 (m, 6H), 7.76 (tt, *J* = 7.0, 5.3 Hz, 2H), 7.58 (d, *J* = 4.8 Hz, 1H), 7.36 - 7.30 (m, 1H), 5.00 (d, *J* = 12.2 Hz, 2H), 2.26 (s, 3H), 2.21 (s, 1H), 1.77 - 1.66 (m, 5H), 0.88 (s, 2H), 0.68 (s, 2H).

### Example 68

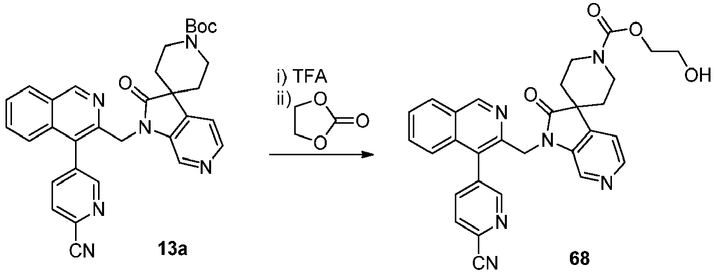

### 2-hydroxyethyl 1'-((4-(6-cyanopyridin-3-yl)isoquinolin-3-yl)methyl)-2'-oxo-1',2'-dihydrospiro[piperidine-4,3'-pyrrolo[2,3-c]pyridine]-1-carboxylate (68)

The title compound was obtained from compound 13a (236 mg, 0.432 mmol) and ethylene carbonate (189 mg, 2.14 mmol) using the method described in Example 64, but the microwave reaction was performed at 130 °C for 1h. Yield: 25 mg, 13%. MS (ES+) 535.18 [M+H]⁺.
¹H NMR (500 MHz, DMSO-*d*₆) δ 9.39 (s, 1H), 8.88 (s, 1H), 8.34 - 8.22 (m, 2H), 8.22 (d, *J* = 7.3 Hz, 1H), 7.76 (ddd, *J* = 7.3, 5.2, 1.7 Hz, 1H), 7.62 - 7.56 (m, 1H), 7.37 - 7.30 (m, 1H), 4.99 (d, *J* = 10.7 Hz, 1H), 4.80 (t, *J=* 5.4 Hz, 1H), 4.07 (dq, *J* = 23.7, 4.6, 4.0 Hz, 2H), 3.59 (q, *J=* 5.0 Hz, 1H), 3.36 (d, *J* = 10.1 Hz, 1H), 3.33 (s, 9H), 3.17 (d, *J* = 5.0 Hz, 1H), 1.75 (ddd, *J* = 13.2, 8.6, 4.5 Hz, 1H), 1.65 (dd, *J* = 12.1, 6.7 Hz, 1H).

### Example 69

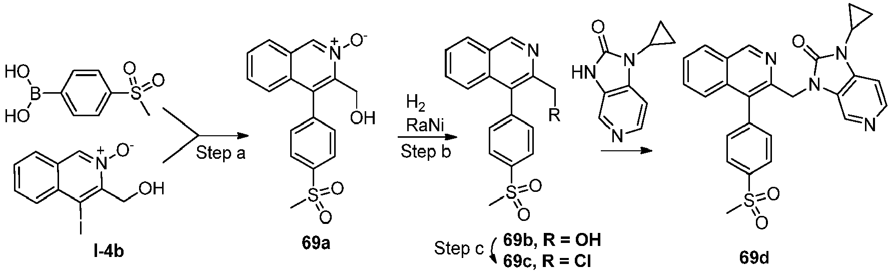

### Step a) (4-(4-(methylsulfonyl)phenyl)isoquinolin-3-yl)methanol N-oxide (69a)

I-1b (500 mg, 1.66 mmol) in dioxane-water (10 mL-2 mL) was reacted with 4-(methylsulfonyl)phenylboronic acid (498 mg, 20 mmol) as described in Example 51 step a, which gave the title compound. MS (ES+) 330.22 [M+H]⁺.

### Step b) (4-(4-(methylsulfonyl)phenyl)isoquinolin-3-yl)methanol (69b)

Raney Nickel(156 mg, 1.0 eq) was added at rt to a solution of compound I-22a (600 mg, 1.0 eq) in MeOH (100 mL). The reaction mixture was stirred at rt under hydrogen atmosphere for 1 h, then distilled out. The afforded crude product was purified by column chromatography on silica gel eluted with 30-100% EtOAc in p. ether, which gave the title compound (400 mg, 64%) as a solid. MS (ES+) 314.23 [M+H]⁺.

### Step c) 3-(chloromethyl)-4-(4-(methylsulfonyl)phenyl)isoquinoline (69c)

Compound I-22b (400 mg, 1.28 mmol) was reacted with thionyl chloride as described in Example 51 step c, which gave the title compound (360 mg, 81%). MS (ES+) 332.20 [M+H]⁺.

### Step d) 1-cyclopropyl-3-((4-(4-(methylsulfonyl)phenyl)isoquinolin-3-yl)methyl)-1H-imidazo[4,5-c]pyridin-2(3H)-one (69d)

Compound 69c (350 mg, 1.06 mmol) was reacted with 1-cyclopropyl-1H-imidazo[4,5-c]pyridin-2(3H)-one (222 mg, 1.27 mmol) as described in Example 11 step a, which gave the title compound (160 mg, 32%). MS (ES+) 471.24 [M+H]⁺.
¹H NMR (500 MHz, DMSO-*d*₆) δ 9.33 (s, 1H), 8.23 - 8.14 (m, 2H), 8.10 - 8.04 (m, 3H), 7.78 - 7.68 (m, 2H), 7.70 - 7.63 (m, 2H), 7.30 - 7.24 (m, 1H), 7.20 (d, *J* = 5.2 Hz, 1H), 5.10 (s, 2H), 3.33 (s, 3H), 2.87 (tt, *J* = 7.1, 3.7 Hz, 1H), 1.00 (td, *J* = 7.3, 5.2 Hz, 2H), 0.86 - 0.79 (m, 2H).

### Example 70

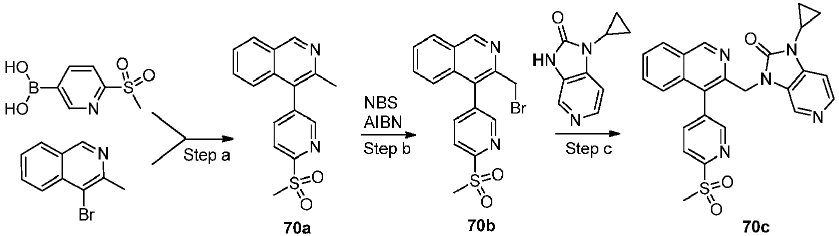

### Step a) 3-Methyl-4-(6-(methylsulfonyl)pyridin-3-yl)isoquinoline (70a)

4-bromo-3-methylisoquinoline (600 mg, 2.70 mmol) was reacted with (6-(methylsulfonyl)pyridin-3-yl)boronic acid (597 mg, 2.97 mmol) as described in Example 51 step a, which gave the title compound (560 mg, 61%). MS (ES+) 299.21 [M+H]⁺.

### Step b) 3-(Bromomethyl)-4-(6-(methylsulfonyl)pyridin-3-yl)isoquinoline (70b)

To stirred solution of compound 70a (550 mg, 1.84 mmol) in carbon tetrachloride (10 mL) was added N-bromosuccinimide (361 mg) and azobisisobutyronitrile (30 mg, 0.184 mmol). The resulting mixture was stirred for 16 h at reflux, then cooled to rt and concentrated under reduced pressure. Water (10 mL) was added and the mixture was extracted with DCM (2 x 25 mL). The combined organic layers were washed with brine, dried (Na₂SO₄) and concentrated. The afforded crude was purified by column chromatography on silica gel eluted with 1-2% MeOH in DCM, which gave the title compound (230 mg, 19%). 379.2 [M+H]⁺.

### Step c) 1-Cyclopropyl-3-((4-(6-(methylsulfonyl)pyridin-3-yl)isoquinolin-3-yl)methyl)-1H-imidazo[4,5-c]pyridin-2(3H)-one (70c)

Compound 70b (220 mg, 0.583 mmol) was reacted with 1-cyclopropyl-1H-imidazo[4,5-c]pyridin-2(3H)-one (112 mg, 0.641 mmol) as described in Example 11 step a, which gave the title compound (58 mg, 21%). MS (ES+) 472.36 [M+H]⁺.
¹H NMR (500 MHz, DMSO-*d*₆) δ 9.40 (s, 1H), 8.80 (s, 1H), 8.23 (d, *J* = 7.3 Hz, 1H), 8.18 (d, *J* = 7.3 Hz, 3H), 8.12 (s, 1H), 7.76 (p, *J* = 6.8 Hz, 2H), 7.28 (d, *J* = 7.8 Hz, 1H), 7.20 (d, *J* = 5.2 Hz, 1H), 5.13 (s, 2H), 3.38 (s, 4H), 2.86 (tt, *J* = 6.8, 3.3 Hz, 1H), 1.00 (d, *J* = 7.1 Hz, 2H), 0.85 - 0.80 (m, 2H).

### Example 71

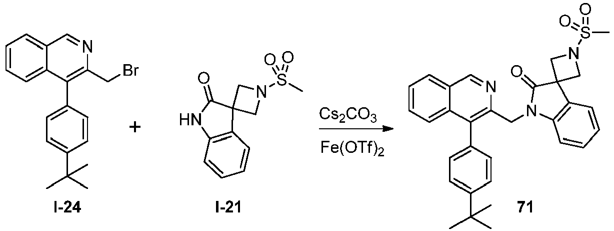

### 1'-((4-(4-(Tert-butyl)phenyl)isoquinolin-3-yl)methyl)-1-(methylsulfonyl)spiro[azetidine-3,3'-indolin]-2'-one (71)

To a stirred solution of I-21 (400 mg) in acetonitrile (30 mL) was added Iron(II) trifluoromethanesulfonate (561 mg) and cesium carbonate (1.03 g) at rt. The reaction mixture was stirred at 70 °C for 15 min, then I-24 (677 mg) was added and the resulting mixture was stirred at 70 ° for 6 h. The reaction mixture was concentrated under reduced pressure and the residue was diluted with water (20 mL) and stirred for 20 min. A solid was formed which was collected by filtration and purified by column chromatography on silica gel eluted with 40% EtOAc in p.ether. Appropriate fractions were pooled and concentrated under reduced pressure and the afforded solid was triturated in diethyl ether and pentane (3:1, 5mL), filtered and dried which gave the title compound (40 mg) as a solid. MS (ES+) 526.64 [M+H]⁺.

The following compounds s were prepared using the method described in Example 71, using the indicated intermediates, reaction times were judged by TLC:

| | | |
|---|---|---|
| Example 72 | 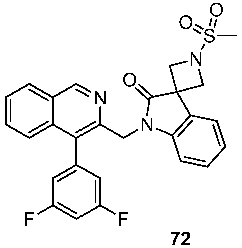 | ¹H NMR (500 MHz, DMSO-*d*₆) δ 9.32 (s, 1H), 8.18 (d, *J* = 8.0 Hz, 1H), 7.73 (dt, *J* = 21.4, 7.2 Hz, 2H), 7.60 (d, *J* = 7.4 Hz, 1H), 7.38 (dd, *J* = 14.5, 8.6 Hz, 2H), 7.20 (d, *J* = 7.3 Hz, 3H), 7.09 (t, *J* = 7.5 Hz, 1H), 6.80 (d, *J* = 7.9 Hz, 1H), 4.99 (s, 2H), 4.09 (q, *J* = 8.3 Hz, 4H), 3.47 - 3.41 (m, 1H), 3.18 (s, 3H). |
| I-29 + I-21 Yield 13%, MS 506.29 [M+H]⁺ | | |
| Example 73 | 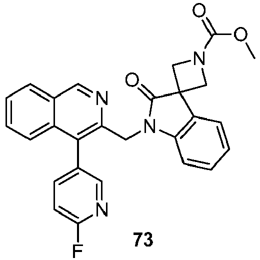 | ¹H NMR (500 MHz, DMSO-*d*₆) δ 9.36 (s, 1H), 8.25 - 8.17 (m, 2H), 8.01 (td, *J* = 8.1, 2.4 Hz, 1H), 7.73 (dq, *J* = 13.1, 6.6 Hz, 2H), 7.64 (d, *J* = 7.4 Hz, 1H), 7.38 (dd, *J* = 8.4, 2.5 Hz, 1H), 7.31 (d, *J* = 8.0 Hz, 1H), 7.18 (t, *J =* 7.7 Hz, 1H), 7.06 (t, *J* = 7.5 Hz, 1H), 6.76 (d, *J* = 7.8 Hz, 1H), 4.98 (s, 2H), 4.06 (s, 4H), 3.64 (s, 3H). |
| I-22b + I-11e Yield 31%, MS 469.29 [M+H]⁺ | | |
| Example 74 | 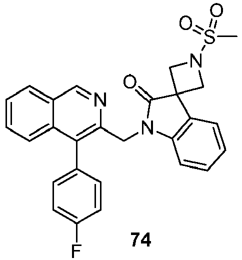 | ¹H NMR (500 MHz, DMSO-*d*₆) δ 9.27 (s, 1H), 8.15 (d, *J* = 7.8 Hz, 1H), 7.90 (s, 0H), 7.76 - 7.65 (m, 2H), 7.60 (d, *J* = 7.3 Hz, 1H), 7.48 - 7.36 (m, 4H), 7.32 (d, *J* = 8.3 Hz, 1H), 7.19 (t, *J* = 7.7 Hz, 1H), 7.08 (t, *J* = 7.5 Hz, 1H), 6.68 (d, *J* = 7.8 Hz, 1H), 4.94 (s, 2H), 4.09 (s, 3H), 3.37 (s, 1H), 3.18 (s, 3H). |
| I-26 + I-21 Yield 39%, MS 488.25 [M+H]⁺ | | |
| Example 75 | 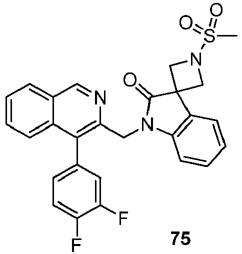 | ¹H NMR (500 MHz, DMSO-*d*₆) δ 9.30 (s, 1H), 8.17 (d, *J* = 7.9 Hz, 1H), 7.72 (dt, *J* = 19.3, 7.1 Hz, 1H), 7.60 (q, *J* = 6.6, 5.9 Hz, 2H), 7.36 (d, *J* = 8.2 Hz, 1H), 7.20 (t, *J* = 7.7 Hz, 2H), 7.09 (t, *J* = 7.5 Hz, 1H), 6.76 (d, *J* = 7.8 Hz, 1H), 4.97 (q, *J* = 16.0 Hz, 2H), 4.10 (d, *J* = 9.6 Hz, 3H), 3.42 - 3.36 (m, 2H), 3.18 (s, 2H), 2.50 (s, 3H). |
| I-27 + I-21 Yield 38%, MS 336.27 [M+H]⁺ | | |
| Example 76 | 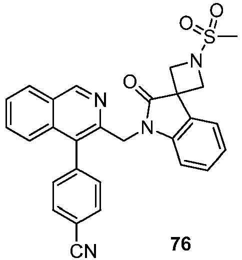 | |
| I-25 + I-21 Yield 6.2% MS 495.13 [M+H]⁺ | | |
| Example 77 | 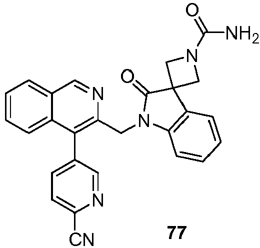 | ¹H NMR (500 MHz, DMSO-*d*₆) δ 9.39 (s, 1H), 8.78 (d, *J* = 2.0 Hz, 1H), 8.26 - 8.18 (m, 2H), 8.15 (dd, *J* = 7.9, 2.2 Hz, 1H), 7.74 (hept, *J* = 5.0 Hz, 2H), 7.56 - 7.50 (m, 1H), 7.31 - 7.24 (m, 1H), 7.18 (td, *J* = 7.7, 1.2 Hz, 1H), 7.07 (t, *J* = 7.5 Hz, 1H), 6.80 (d, *J* = 7.8 Hz, 1H), 6.11 (s, 2H), 4.96 (s, 2H), 3.97 (dd, *J* = 7.9, 3.7 Hz, 2H), 3.89 (dd, *J* = 7.9, 2.1 Hz, 2H), 3.35 (s, 1H), 3.17 (d, *J* = 5.2 Hz, 1H), 2.55 (s, 0H). |
| I-28 + I-19 Yield 22%, MS 461.16 [M+H]⁺ | | |
| Example 78 | 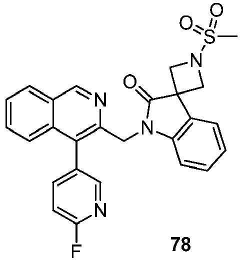 | ¹H NMR (500 MHz, DMSO-*d*₆) δ 9.35 (s, 0H), 8.26 (d, *J* = 2.4 Hz, 0H), 8.19 (d, *J* = 7.6 Hz, 0H), 8.05 (td, *J* = 8.2, 2.4 Hz, 0H), 7.74 (p, *J* = 6.9 Hz, 1H), 7.60 (d, *J* = 7.3 Hz, 0H), 7.40 (dd, *J* = 8.4, 2.5 Hz, 0H), 7.32 (d, *J* = 8.1 Hz, 0H), 7.21 (t, *J* = 7.7 Hz, 0H), 7.09 (t, *J* = 7.5 Hz, 0H), 6.82 (d, *J* = 7.8 Hz, 0H), 4.97 (d, *J* = 3.8 Hz, 1H), 4.20 (s, 1H), 4.13 - 4.05 (m, 1H), 4.08 (s, 1H), 3.35 (d, *J* = 5.9 Hz, 15H), 3.17 (s, 1H), 2.61 (s, 1H). |
| I-22 + I-21 Yield 19%, MS 489.13 [M+H]⁺ | | |

### Example 79

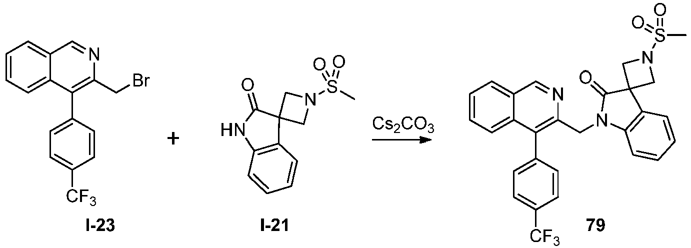

### 1-(Methylsulfonyl)-1'-((4-(4-(trifluoromethyl)phenyl)isoquinolin-3-yl)methyl)spiro[azetidine-3,3'-indolin]-2'-one (79)

Cesium carbonate (1.55 g) was added at rt to a stirred mixture of I-21 (400 mg, 1.58 mmol) and I-23 (813 mg, 2.22 mmol) in acetonitrile (30 mL). The mixture was stirred at 70-80 °C until the reaction was deemed completed according to TLC (∼4 h), then concentrated under reduced pressure. The afforded crude was diluted with water (20 mL) and stirred for 20 minutes. The formed solid was collected and triturated with acetonitrile (20 mm), filtered, washed with acetonitrile (10 mL) and dried, which gave the title compound (170 mg, 20%) as a solid. MS (ES+) 538.57 [M+H]⁺.
¹H NMR (500 MHz, DMSO-*d*₆) δ 9.33 (s, 1H), 8.19 (dd, *J* = 6.8, 2.5 Hz, 1H), 7.91 (d, *J* = 8.0 Hz, 2H), 7.72 (tt, *J* = 7.0, 5.2 Hz, 2H), 7.60 (dd, *J=* 12.0, 7.4 Hz, 3H), 7.29 - 7.22 (m, 1H), 7.19 (td, *J* = 7.8, 1.2 Hz, 1H), 7.08 (t, *J* = 7.5 Hz, 1H), 6.72 (d, *J* = 7.8 Hz, 1H), 4.96 (s, 2H), 4.05 (s, 4H), 3.15 (s, 3H).

The following compounds s were prepared using the method described in Example 79, using the indicated intermediates, reaction times were judged by TLC:

| Example | Structure | NMR data |
|---|---|---|
| Example 80 | 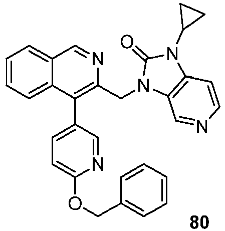 | |
| I-30b + A-9 Yield 51%, MS 500.33 [M+H]⁺ As Ex. 79 but at rt | | |
| Example 81 | 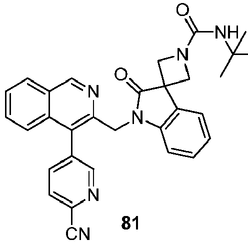 - | ¹H NMR (500 MHz, DMSO-*d*₆) δ 9.39 (s, 1H), 8.74 (s, 1H), 8.22 (d, *J* = 7.7 Hz, 2H), 8.15 (d, *J* = 8.0 Hz, 1H), 7.79 - 7.70 (m, 2H), 7.50 (d, *J* = 7.3 Hz, 1H), 7.27 (d, *J* = 7.7 Hz, 1H), 7.18 (t, *J* = 7.8 Hz, 1H), 7.06 (t, *J* = 7.5 Hz, 1H), 6.79 (d, *J* = 7.9 Hz, 1H), 5.94 (s, 1H), 4.98 (t, *J* = 11.8 Hz, 2H), 3.95 (d, *J* = 7.9 Hz, 2H), 3.85 (d, *J* = 8.0 Hz, 2H), 3.40 (s, 1H), 2.25 (s, 1H), 1.28 (s, 8H), 1.17 (s, 1H). |
| I-28 + I-20 Yield 22%, MS 517.25 [M+H]⁺ As Ex. 79 but at rt | | |
| Example 82 | 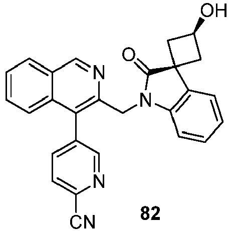 | ¹H NMR (500 MHz, DMSO-*d*₆) δ 9.42 (s, 1H), 8.64 (dd, *J* = 2.0, 0.9 Hz, 1H), 8.21 (ddd, *J* = 19.0, 7.1, 2.1 Hz, 2H), 8.03 (dd, *J* = 7.9, 2.2 Hz, 1H), 7.74 (dt, *J* = 6.4, 3.4 Hz, 2H), 7.41 (dd, *J* = 7.4, 1.2 Hz, 1H), 7.23 (dt, *J* = 6.3, 3.5 Hz, 1H), 7.08 (td, *J* = 7.7, 1.2 Hz, 1H), 6.98 (td, *J* = 7.5, 1.0 Hz, 1H), 6.66 (d, *J* = 7.8 Hz, 1H), 5.53 (d, *J* = 6.5 Hz, 1H), |
| I-28 + A-14 Yield 7.5%, MS 433.51 [M+H]⁺ | | |
| | | 5.01 (d, *J* = 1.3 Hz, 2H), 4.63 (q, *J* = 7.5 Hz, 1H), 2.28 (td, *J* = 8.8, 7.8, 3.7 Hz, 4H). |
| Example 83 | 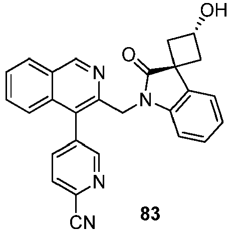 | ¹H NMR (500 MHz, DMSO-*d*₆) δ 9.38 (s, 1H), 8.75 (d, *J* = 2.1 Hz, 1H), 8.27 - 8.17 (m, 2H), 8.16 (dd, *J* = 7.9, 2.1 Hz, 1H), 7.73 (td, *J* = 8.0, 7.2, 4.2 Hz, 2H), 7.43 (d, *J* = 7.3 Hz, 1H), 7.31 -7.24 (m, 1H), 7.10 (t, *J* = 7.6 Hz, 1H), 7.02 (t, *J* = 7.4 Hz, 1H), 6.71 (d, *J* = 7.7 Hz, 1H), 5.41 (d, *J* = 7.2 Hz, 1H), 4.93 (d, *J* = 11.1 Hz, 2H), 4.44 (p, *J* = 7.3 Hz, 1H), 3.36 (s, 1H), 2.57 (s, OH), 2.48 (s, OH), 2.23 (dd, *J* = 11.8, 7.5 Hz, 2H). |
| I-28 + A-15 Yield 6.7%, MS 433.15 [M+H]⁺ | | |
| Example 84 | 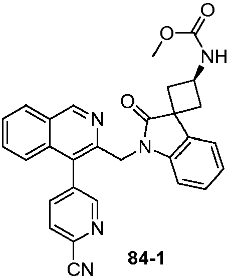 | ¹H NMR (500 MHz, DMSO-*d*₆) δ 9.41 (s, 1H), 8.70 (s, 1H), 8.30 (s, OH), 8.21 (dd, *J* = 15.9, 7.2 Hz, 2H), 8.05 (d, *J* = 8.0 Hz, 1H), 7.81 - 7.75 (m, 1H), 7.73 (s, OH), 7.50 (d, *J* = 7.1 Hz, 1H), 7.24 (d, *J* = 7.1 Hz, 1H), 7.11 (t, *J* = 7.6 Hz, 1H), 7.02 (t, *J* = 7.5 Hz, 1H), 6.71 (d, *J* = 7.8 Hz, 1H), 4.99 (d, *J* = 6.2 Hz, 1H), 4.52 (dt, *J* = 17.6, 9.4 Hz, 1H), 3.60 (s, OH), 3.55 (s, 2H), 3.42 (s, 1H), 2.69 (s, 1H), 2.35 (dd, *J* = 18.2, 9.4 Hz, 4H). |
| I-28 + I-31 A diastereomeric mixture was obtained which was separated by SFC. MS 490.2 [M+H]⁺ Yield 84-1: 1.8%, 84-2: 1.4% | | |
| | 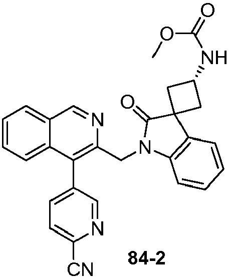 | ¹H NMR (500 MHz, DMSO-*d*₆) δ 9.39 - 9.35 (m, 1H), 8.80 - 8.75 (m, 1H), 8.27 - 8.15 (m, 3H), 7.78 - 7.69 (m, 1H), 7.63 (d, *J* = 8.7 Hz, 1H), 7.47 (d, *J* = 7.3 Hz, 1H), 7.31 - 7.24 (m, 1H), 7.12 (td, *J* = 7.7, 1.3 Hz, 1H), 7.04 (td, *J* = 7.5, 1.0 Hz, 1H), 6.73 (d, *J* = 7.7 Hz, 1H), 5.01 - 4.90 (m, 2H), 4.36 (q, *J* = 8.5 Hz, 1H), 3.56 (s, 3H), 3.38 - 3.32 (m, 1H), 2.51 - 2.35 (m, 3H). |

### Example 85

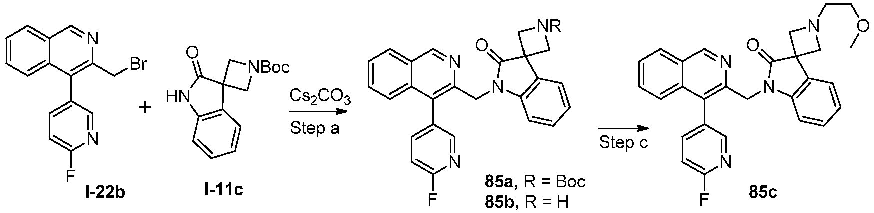

### Step a) tert-butyl 1'-((4-(6-Fluoropyridin-3-yl)isoquinolin-3-yl)methyl)-2'-oxospiro[azetidine-3,3'-indoline]-1-carboxylate (85a)

Compounds I-22b (2.00 g, 6.31 mmol) and I-11c (1.73 g, 6.31 mmol) were reacted according to the method described in Example 79, which gave the title compound (1.20 g, 27%). MS (ES+) 511.36 [M+H]⁺.

### Step b) 1'-((4-(6-Fluoropyridin-3-yl)isoquinolin-3-yl)methyl)spiro[azetidine-3,3'-indolin]-2'-one (85b)

TFA (2.2 mL, 29 mmol) was added at 0 °C to a stirred suspension of compound 85a (1.0 g, 2.0 mmol) in DCM (20 mL). The mixture was stirred at rt for 4 h, then concentrated under reduced pressure. NaHCO₃ was added and the mixture was extracted with DCM (2x15 mL). The combined organic layers were dried over anhydrous Na₂SO₄ and concentrated, which gave the title compound (700 mg, 73%). MS (ES+) 411.25 [M+H]⁺.

### Step c) 1'-((4-(6-Fluoropyridin-3-yl)isoquinolin-3-yl)methyl)-1-(2-methoxyethyl)spiro[azetidine-3,3'-indolin]-2'-one (85c)

To a stirred solution of compound 85b (300 mg, 0.731 mmol) in acetonitrile (30 mL) were added 2-methoxyethyl 4-methylbenzenesulfonate (0.167 mL, 0.877 mmol) and cesium carbonate (714 mg, 2.19 mmol) at rt. The resulting reaction mixture was heated at 100 °C for 48 h, then concentrated under reduced pressure, diluted with water (30 mL) and extracted with DCM (2x50 mL). The combined the organic layers were dried over anhydrous Na₂SO₄ and concentrated. The afforded crude was purified by column chromatography on silica gel eluted with 2.5% MeOH in DCM. Appropriate fractions were pooled and concentrated and further purified by Prep-HPLC on an X- Bridge C18, 25*150, 10µ column, mobile phase: 10 mM ammonium bicarbonate in H₂O:acetonitrile, which gave the title compound (45 mg, 13%) as a solid. MS (ES+) 469.70 [M+H].
¹H NMR (500 MHz, DMSO-*d*₆) δ 9.35 (s, 1H), 8.19 (dt, *J* = 4.4, 2.2 Hz, 2H), 7.96 (td, *J* = 8.1, 2.5 Hz, 1H), 7.79 - 7.68 (m, 2H), 7.61 (dd, *J* = 7.4, 1.2 Hz, 1H), 7.35 (dd, *J* = 8.4, 2.6 Hz, 1H), 7.29 (dd, *J* = 7.9, 1.6 Hz, 1H), 7.12 (td, *J* = 7.7, 1.3 Hz, 1H), 7.04 (td, *J* = 7.5, 1.0 Hz, 1H), 6.67 (d, *J* = 7.7 Hz, 1H), 4.97 (d, *J* = 2.4 Hz, 2H), 3.44 - 3.33 (m, 5H), 3.25 (s, 3H), 2.65 (t, *J* = 5.7 Hz, 2H), 2.55 (d, *J* = 16.0 Hz, 1H).

### Example 86

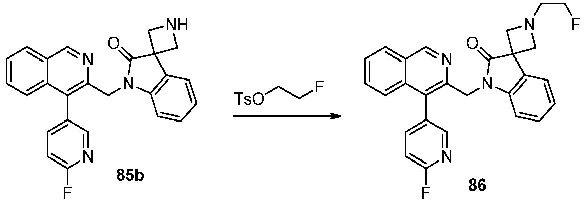

### 1-(2-fluoroethyl)-1'-((4-(6-fluoropyridin-3-yl)isoquinolin-3-yl)methyl)spiro[azetidine-3,3'-indolin]-2'-one (86)

Compound 85b (300 mg, 731 mmol) was reacted with 2-fluoroethyl 4-methylbenzenesulfonate (191 mg, 877 mmol) according to the method described in Example 85 step c, which gave the title compound (30 mg, 8.8%) MS (ES+) 457.18 [M+H]⁺.
¹H NMR (500 MHz, DMSO-*d*₆) δ 9.35 (s, 1H), 8.19 (dd, *J* = 7.3, 2.1 Hz, 2H), 7.97 (td, *J* = 8.1, 2.5 Hz, 1H), 7.82 - 7.63 (m, 2H), 7.64 (dd, *J* = 7.3, 1.2 Hz, 1H), 7.35 (dd, *J* = 8.4, 2.6 Hz, 1H), 7.29 (dd, *J* = 7.9, 1.5 Hz, 1H), 7.13 (td, *J* = 7.8, 1.3 Hz, 1H), 7.05 (td, *J* = 7.5, 1.0 Hz, 1H), 6.69 (d, *J* = 7.7 Hz, 1H), 4.97 (d, *J* = 2.9 Hz, 2H), 4.48 (dt, *J* = 47.7, 4.8 Hz, 2H), 3.52 - 3.24 (m, 4H), 2.80 (dt, *J* = 29.3, 4.8 Hz, 2H), 2.55 (s, 1H).

### Example 87

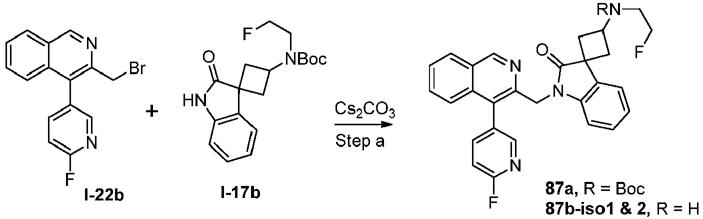

### Step a) Tert-butyl (2-fluoroethyl)(1'-((4-(6-fluoropyridin-3-yl)isoquinolin-3-yl)methyl)-2'-oxospiro[cyclobutane-1,3'-indolin]-3-ul)carbamate (87a)

Compounds I-22b (600 mg, 1.89 mmol) and I-17b (633 mg, 1.89 mmol) were reacted according to the method described in Example 79, which gave the title compound (850 mg, 64%). MS (ES+) 571.39 [M+H]⁺.

### Step b) 3-((2-fluoroethyl)amino)-1'-((4-(6-fluoropyridin-3-yl)isoquinolin-3-yl)methyl)spiro[cyclobutane-1,3'-indolin]-2'-one (87b, iso-1 & iso-2)

TFA (2.21 mL) was added at rt under nitrogen to a stirred solution of compound 87a (825 mg, 1.45 mmol) in DCM (20 mL). The reaction mixture was stirred at rt for 2h, then concentrated under reduced pressure. The resulting crude was diluted with DCM (100 mL), washed with saturated sodium bicarbonate solution (50 mL), dried over sodium sulphate, filtered and concentrated under reduced pressure. The crude was purified by prep HPLC, mobile phase: 10 mM ammonium bicarbonate in H₂O:acetonitrile, column: X- Bridge C18, 30*150, 5µ. The two isomers of the afforded solid were separated by SFC (stationary phase: Chiralpak IC 250 x30mm, 5µ, Mobile phase: CO₂, 30 mM NH₄OMe in MeOH), and subsequently purified by flash chromatography on silica gel (12 g, 3% MeOH in DCM). Pure fractions were combined, concentrated and triturated with pentane and lyophilized which gave the title compound as Isomer 1: 151 mg, 22%.
¹H NMR (500 MHz, DMSO-*d*₆) δ 9.40 - 9.26 (m, 1H), 8.28 (d, *J* = 2.5 Hz, 1H), 8.26 - 8.10 (m, 1H), 8.07 (td, *J* = 8.1, 2.5 Hz, 1H), 7.83 - 7.63 (m, 2H), 7.56 (dd, *J* = 7.4, 1.3 Hz, 1H), 7.40 (dd, *J* = 8.4, 2.6 Hz, 1H), 7.40 - 7.25 (m, 1H), 7.09 (td, *J* = 7.7, 1.3 Hz, 1H), 7.00 (td, *J* = 7.5, 1.0 Hz, 1H), 6.68 (d, *J* = 7.7 Hz, 1H), 4.92 (d, *J* = 2.6 Hz, 2H), 4.45 (dt, *J* = 47.8, 5.1 Hz, 2H), 3.56 (p, *J* = 7.9 Hz, 1H), 2.80 (dt, *J* = 27.5, 5.1 Hz, 2H), 2.61 - 2.34 (m, 2H), 2.15 (dd, *J* = 10.9, 8.2 Hz, 2H).
Isomer 2: 50 mg, 7.1%. MS (ES+) 471.84 [M+H]⁺.
¹H NMR (500 MHz, DMSO-*d*₆) δ 9.36 (s, 1H), 8.19 (h, *J* = 5.8, 5.3 Hz, 2H), 7.96 (td, *J* = 8.1, 2.5 Hz, 1H), 7.83 - 7.63 (m, 2H), 7.50 (dd, *J* = 7.4, 1.2 Hz, 1H), 7.48 - 7.22 (m, 2H), 7.09 (td, *J* = 7.7, 1.2 Hz, 1H), 6.99 (td, *J* = 7.5, 1.0 Hz, 1H), 6.68 (d, *J* = 7.7 Hz, 1H), 5.09 - 4.85 (m, 2H), 4.47 (dt, *J* = 47.6, 5.2 Hz, 2H), 3.75 (p, *J* = 8.2 Hz, 1H), 2.83 (dt, *J* = 26.5, 5.2 Hz, 2H), 2.32 (qd, *J* = 6.8, 3.3 Hz, 2H), 2.19 (dt, *J* = 11.5, 8.3 Hz, 2H).

### Example 88

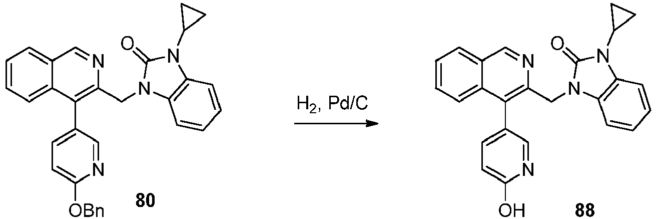

### 1-Cyclopropyl-3-((4-(6-hydroxypyridin-3-yl)isoquinolin-3-yl)methyl)-1H-benzo[d]imidazol-2(3H)-one (88)

Compound 80 (140 mg, 0.28 mmol), was dissolved in MeOH, 10% palladium on carbon (15 mg) was added and the mixture was put under H₂ and hydrogenated at balloon pressure for 16h. The reaction mixture was filtered through Celite, the filter cake was washed with MeOH (2x10 mL). The solution was concentrated under vacuum and the afforded crude was purified by prep HPLC which gave the title compound (26 mg, 23%) as a solid. MS (ES+) 410.14 [M+H]⁺.
¹H NMR (500 MHz, DMSO-*d*₆) δ 11.91 (d, *J* = 0.7 Hz, 1H), 9.27 (s, 1H), 8.18 (d, *J* = 5.2 Hz, 1H), 8.15 (d, *J* = 8.1 Hz, 1H), 7.99 (s, 1H), 7.78 (t, *J* = 7.5 Hz, 1H), 7.71 (t, *J* = 7.4 Hz, 1H), 7.57 (d, *J* = 8.4 Hz, 1H), 7.55 - 7.51 (m, 1H), 7.25 (dd, *J* = 9.3, 2.2 Hz, 1H), 7.22 (d, *J* = 5.2 Hz, 1H), 6.42 (d, *J* = 9.3 Hz, 1H), 5.29 (d, *J* = 15.9 Hz, 1H), 5.15 (d, *J* = 15.9 Hz, 1H), 2.90 (tt, *J* = 6.8, 3.5 Hz, 1H), 1.02 (d, *J* = 6.8 Hz, 2H), 0.87 (q, *J* = 10.2, 9.7 Hz, 2H).

### Example 89

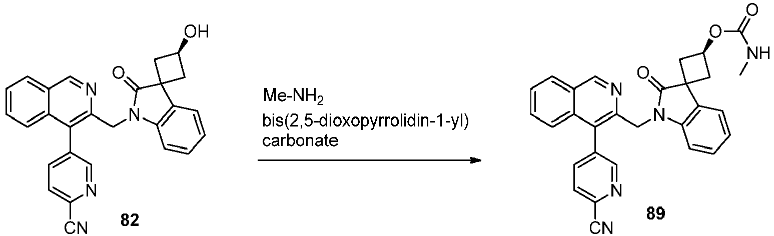

### 1'-((4-(6-Cyanopyridin-3-yl)isoquinolin-3-yl)methyl)-2'-oxospro[cyclobutane-1,3'-indolin]-3-yl methylcarbamate (89)

were added at rt to A solution of compound 82 (140 mg, 0.324 mmol), Et₃N (0.226 mL, 1.62 mmol) and bis(2,5-dioxopyrrolidin-1-yl) carbonate (415 mg, 1.62 mmol) in DCM (20 mL) was stirred temperature for 2 h, then a 1.0 M solution of methylamine in THF (1.62 mmol) was added and the reaction mixture was stirred for 16 h. The reaction mixture was concentrated under reduced pressure and the afforded crude product was purified by column chromatography on silica gel eluted with 50% EtOAc in p. ether followed by prep HPLC on an XSELECT column using 10 mM ammonium bicarbonate: acetonitrile as mobile phase, which gave the title compound (30 mg, 19%) as a solid. MS (ES+) 490.16 [M+H]⁺.
¹H NMR (500 MHz, DMSO-*d*₆) δ 9.42 (s, 1H), 8.66 (d, *J* = 2.1 Hz, 1H), 8.26 - 8.17 (m, 2H), 8.06 (dd, *J* = 7.9, 2.2 Hz, 1H), 7.74 (dt, *J* = 6.3, 3.4 Hz, 2H), 7.48 (dd, *J* = 7.4, 1.2 Hz, 1H), 7.24 (dt, *J* = 6.2, 3.6 Hz, 1H), 7.23 - 7.08 (m, 2H), 7.03 (td, *J* = 7.6, 1.0 Hz, 1H), 6.71 (d, *J* = 7.8 Hz, 1H), 5.36 (p, *J* = 7.9 Hz, 1H), 5.06 - 4.98 (m, 2H), 2.58 (d, *J* = 4.6 Hz, 3H), 2.49 - 2.36 (m, 3H).

### Example 90

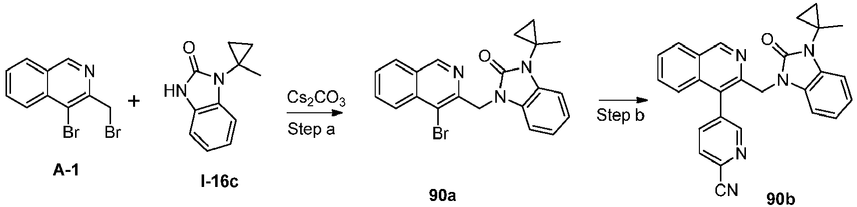

### Step a 1-((4-bromoisoquinolin-3-yl)methyl)-3-(1-methylcyclopyl)-1H-benzo[d]imidazol-2(3H)-one (90a)

Compounds A-1 (120 mg, 0.399 mmol) and I-16c (75.4 mg, 0.399 mmol) were reacted according to the method described in Example 79, which gave the title compound (98 mg, 60%). MS (ES+) 409.3 [M+H]⁺.

### Step b) 5-(3-((3-(1-methylcyclopropyl)-2-oxo-2,3-dihydro-1H-benzo[d]imidazol-1-yl)methyl)isoquinolin-4-yl)picolinonitrile (90b)

Compound 90a (70 mg, 0.17 mmol) was reacted with (6-cyanopyridin-3-yl)boronic acid (38 mg, 0.26 mmol) acid using the method described in Example 12 step a, which gave the title compound (43 mg, 58%). MS (ES+) 433.3 [M+H]⁺.
¹H NMR (500 MHz, DMSO-*d*₆) δ 9.43 (d, *J* = 0.8 Hz, 1H), 8.64 (dd, *J* = 2.2, 0.9 Hz, 1H), 8.27 - 8.20 (m, 1H), 8.22 - 8.15 (m, 2H), 8.08 (dd, *J* = 7.9, 2.2 Hz, 1H), 8.05 (s, 1H), 7.74 (dt, *J* = 6.9, 3.4 Hz, 2H), 7.29 - 7.18 (m, 2H), 5.17 (s, 1H), 5.14 (s, 1H), 1.34 (s, 3H), 0.94 (s, 3H).

### Example 91

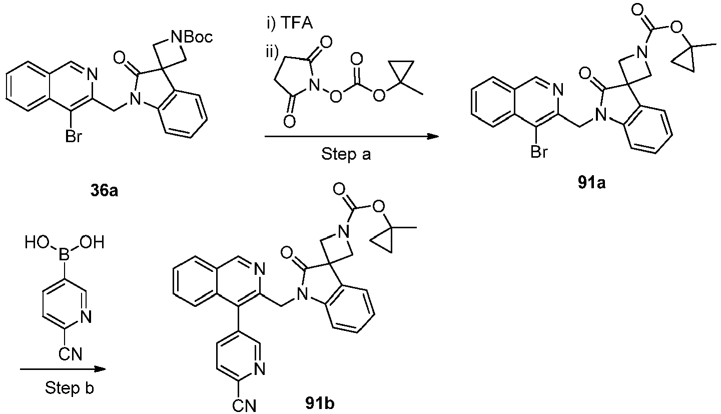

### Step a) 1-Methylcyclopropyl 1'-((4-bromoisoquinolin-3-yl)methyl)-2'-oxospiro[azetidine-3,3'-indoline]-1-carboxylate (91a)

TFA (397 µl, 5.19 mmol) was added dropwise at rt to a solution of the compound 36a (130 mg, 0.263 mmol) in DCM (2 mL). The reaction was stirred for 2 h, then concentrated and co-evaporated with toluene. The residue and DIEA (119 mg, 0.920 mmol) were dissolved in DCM (3 mL) at rt. To this 2,5-dioxopyrrolidin-1-yl (1-methylcyclopropyl) carbonate (56.0 mg, 0.263 mmol) was added and the reaction was stirred for 16h, then concentrated under vacuum. The crude was dissolved in DCM and purified by silica column chromatography eluted with DCM:MeOH which gave the title compound (118 mg, 91%). MS (ES+) 492.3 [M+H]⁺.

### Step b) 1-Methylcyclopropyl 1'-((4-(6-cyanopyridin-3-yl)isoquinolin-3-yl)methyl)-2'-oxospiro[azetidine-3,3'-indoline]-1-carboxylate (91b)

Compound 91a (75 mg, 0.15 mmol) was reacted with (6-cyanopyridin-3-yl)boronic acid (34 mg, 0.28 mmol) using the method described in Example 12 step a, which gave the title compound (34 mg, 44%). MS (ES+) 516.4 [M+H]⁺.
¹H NMR (500 MHz, DMSO-*d*₆) δ 9.40 (s, 1H), 8.72 (s, 1H), 8.22 (dt, *J* = 7.6, 2.3 Hz, 2H), 8.13 (d, *J* = 7.9 Hz, 1H), 7.79 - 7.70 (m, 2H), 7.60 (dd, *J* = 7.4, 1.2 Hz, 1H), 7.29 - 7.22 (m, 1H), 7.18 (td, *J* = 7.8, 1.3 Hz, 1H), 7.06 (td, *J* = 7.6, 1.0 Hz, 1H), 6.80 (d, *J* = 7.8 Hz, 1H), 4.99 (t, *J* = 14.4 Hz, 2H), 4.04 - 3.94 (m, 4H), 1.50 (s, 3H), 0.83 (s, 2H), 0.64 (q, *J* = 2.9 Hz, 2H).

### Example 92

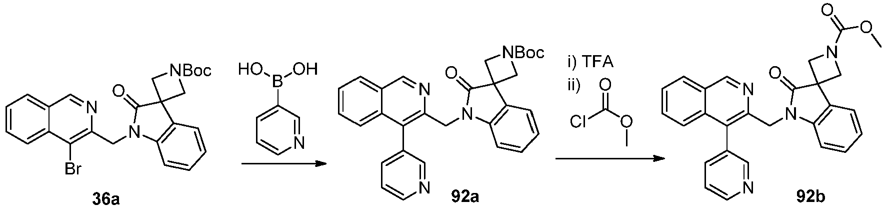

### Step a) tert-butyl 2'-oxo-1'-((4-(pyridin-3-yl)isoquinolin-3-yl)methyl)spiro[azetidine-3,3'-indoline]-1-carboxylate (92a)

The title compound was prepared according to the procedure described in Example 51 step a, but using 3-pyridine boronic acid and as base, sodium carbonate was used. Yield 97%. MS (ES+) 493.5 [M+H]⁺.

### Step b) Methyl 2'-oxo-1'-((4-(pyridin-3-yl)isoquinolin-3-yl)methyl)spiro[azetidine-3,3'-indoline]-1-carboxylate (92b)

Compound 92a was N-deprotected and acylated as described in Example 44 using DCM as solvent in the acylation step, which gave the title compound (76%). MS (ES+) 451.4 [M+H]⁺.
¹H NMR (500 MHz, DMSO-*d*₆) δ 9.34 (d, *J* = 0.8 Hz, 1H), 8.71 (dd, *J* = 4.9, 1.7 Hz, 1H), 8.53 (dd, *J* = 2.3, 0.9 Hz, 1H), 8.22 - 8.16 (m, 1H), 7.84 (dt, *J* = 7.8, 2.0 Hz, 1H), 7.80 - 7.66 (m, 2H), 7.66 - 7.55 (m, 2H), 7.29 - 7.23 (m, 1H), 7.17 (td, *J* = 7.8, 1.2 Hz, 1H), 7.05 (td, *J* = 7.6, 1.0 Hz, 1H), 6.72 (d, *J* = 7.8 Hz, 1H), 5.03 - 4.90 (m, 2H), 4.07 - 3.99 (m, 3H), 3.63 (s, 3H).

### Example 93

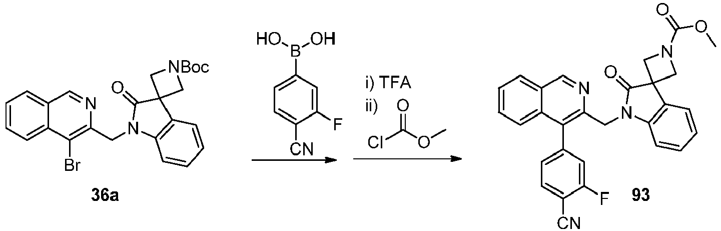

### Methyl 1'-((4-(4-cyano-3-fluorophenyl)isoquinolin-3-yl)methyl)-2'-oxospiro[azetidine-3,3'-indoline]-1-carboxylate (93)

The title compound was prepared according to the procedure described in Example 51 step a, but using (4-cyano-3-fluorophenyl)boronic acid, followed by N-deprotection and acylation as described in Example 44. Yield 27%. MS (ES+) 493.4 [M+H]⁺.
¹H NMR (500 MHz, DMSO-*d*₆) δ 9.36 (d, *J* = 0.8 Hz, 1H), 8.24 - 8.16 (m, 1H), 8.07 (dd, *J* = 7.9, 6.9 Hz, 1H), 7.78 - 7.67 (m, 3H), 7.63 (dd, *J* = 7.4, 1.2 Hz, 1H), 7.37 (dd, *J* = 7.9, 1.4 Hz, 1H), 7.34 - 7.27 (m, 1H), 7.18 (td, *J* = 7.8, 1.2 Hz, 1H), 7.06 (td, *J* = 7.5, 1.0 Hz, 1H), 6.76 (d, *J* = 7.8 Hz, 1H), 5.03 (d, *J* = 16.0 Hz, 1H), 4.94 (d, *J* = 16.0 Hz, 1H), 3.64 (s, 3H), 3.32 (s, 5H).

### Example 94

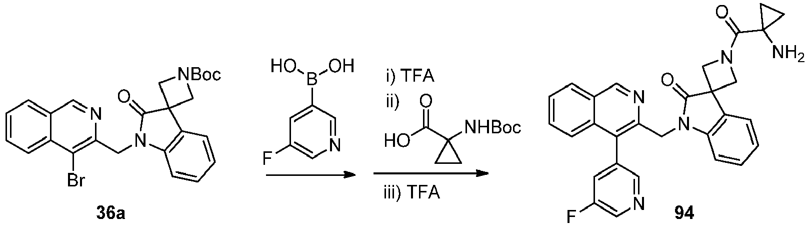

### 1-(1-aminocyclopropanecarbonyl)-1'-((4-(5-fluoropyridin-3-yl)isoquinolin-3-yl)methyl)spiro[azetidine-3,3'-indolin]-2'-one (94)

The title compound was prepared according to the procedure described in Example 12 step a, but using (5-fluoropyridin-3-yl)boronic acid, followed by N-deprotection, acylation and N-deprotection as described in Example 60 steps a, b and c. Yield 85%. MS (ES+) 494.3 [M+H]⁺. ¹H NMR (500 MHz, DMSO-*d*₆) δ 9.37 (d, *J* = 0.8 Hz, 1H), 8.72 (d, *J* = 2.8 Hz, 1H), 8.39 (s, 1H), 8.23 - 8.17 (m, 1H), 7.94 (dt, *J* = 9.4, 2.1 Hz, 1H), 7.79 - 7.69 (m, 2H), 7.59 (dd, *J* = 7.4, 1.3 Hz, 1H), 7.31 (dd, *J* = 8.1, 1.5 Hz, 1H), 7.18 (td, *J* = 7.7, 1.2 Hz, 1H), 7.07 (td, *J* = 7.5, 1.0 Hz, 1H), 6.78 (d, *J* = 7.8 Hz, 1H), 5.05 (d, *J* = 16.1 Hz, 1H), 4.95 (d, *J* = 16.0 Hz, 1H), 3.96 (s, 1H), 1.24 (s, 0H), 1.14 (s, 2H), 0.76 (s, 2H).

### Example 95

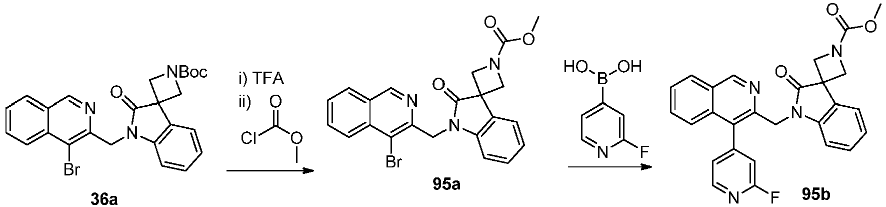

### Step a) Methyl 1'-((4-bromoisoquinolin-3-yl)methyl)-2'-oxospiro[azetidine-3,3'-indoline]-1-carboxylate (95a)

Compound 36a (450 mg, 0.910 mmol) was N-deprotected and acylated according to the procedure described in Example 44 step b, which gave the title compound (405 mg, 98%). MS (ES+) 452.2 & 454.2 [M+H]⁺.

### Step b) Methyl 1'-((4-(2-fluoropyridin-4-yl)isoquinolin-3-yl)methyl)-2'-oxospiro[azetidine-3,3'-indoline]-1-carboxylate (95b)

Compound 95a (67 mg, 0.148 mmol) was reacted with 2-fluoropyridin-4-yl)boronic acid (31.3 mg, 0.222 mmol) according to the procedure described in Example 12 step a which gave the title compound (26.7 mg, 38%). MS (ES+) 469.2 [M+H]⁺.
¹H NMR (500 MHz, DMSO-*d*₆) δ 9.38 (s, 1H), 8.36 (d, *J* = 5.0 Hz, 1H), 8.25 - 8.17 (m, 1H), 7.74 (tt, *J* = 6.9, 5.2 Hz, 2H), 7.63 (dd, *J* = 7.4, 1.2 Hz, 1H), 7.36 - 7.25 (m, 3H), 7.17 (td, *J* = 7.8, 1.3 Hz, 1H), 7.05 (td, *J* = 7.5, 1.0 Hz, 1H), 6.72 (d, *J* = 7.8 Hz, 1H), 5.06 (s, 1H), 4.96 (s, 1H), 4.05 (s, 3H), 3.98 (s, 1H), 3.64 (s, 3H), 2.07 (s, 1H), 1.26 - 1.21 (m, 1H).

### Example 96

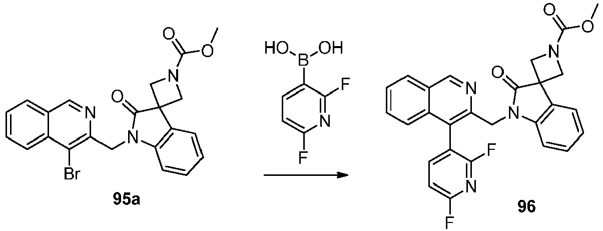

### Methyl 1'-((4-(2,6-difluoropyridin-3-yl)isoquinolin-3-yl)methyl)-2'-oxospiro[azetidine-3,3'-indoline]-1-carboxylate (96)

Compound 95a (72 mg, 0.159 mmol) was reacted with (2,6-difluoropyridin-3-yl)boronic acid according to the procedure described in Example 12 step a, with the exception that after 18 h reaction time, additional catalyst (0.1 eq), boronic acid (1.5 eq) and potassium carbonate (318 µL) were added and the reaction was heated at 90 °C for another 4h to get complete conversion. Yield: 19.8 mg, 26%. MS (ES+) 487.1 [M+H]⁺.
¹H NMR (500 MHz, DMSO-*d*₆) δ 9.42 (d, *J* = 0.8 Hz, 1H), 8.31 - 8.19 (m, 2H), 7.75 (tt, *J* = 7.0, 5.3 Hz, 2H), 7.64 (dd, *J* = 7.4, 1.2 Hz, 1H), 7.42 (dd, *J* = 8.1, 2.2 Hz, 1H), 7.39 - 7.32 (m, 1H), 7.18 (td, *J* = 7.8, 1.3 Hz, 1H), 7.06 (td, *J* = 7.6, 1.0 Hz, 1H), 6.86 (d, *J* = 7.8 Hz, 1H), 5.04 (d, *J* = 15.7 Hz, 1H), 4.93 (d, *J* = 15.7 Hz, 1H), 4.08 (s, 3H), 4.03 (s, 1H), 3.64 (s, 3H).

### Example 97

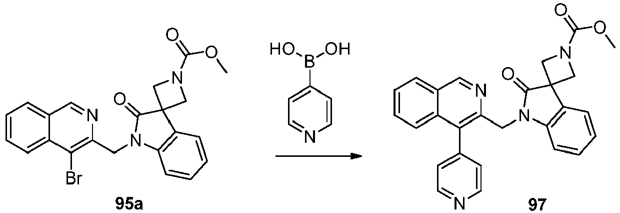

### Methyl 2'-oxo-1'-((4-(pyridin-4-yl)isoquinolin-3-yl)methyl)spiro[azetidine-3,3'-indoline]-1-carboxylate (97)

Compound 95a (115 mg, 0.254 mmol) was reacted with pyridin-4-ylboronic acid (46.9 mg, 0.381 mmol) according to the procedure described in Example 12 step a, which gave the title compound (37.5 mg, 33%). MS (ES+)451.3 [M+H]⁺.
¹H NMR (500 MHz, DMSO-*d*₆) δ 9.35 (d, *J* = 0.8 Hz, 1H), 8.75 - 8.70 (m, 2H), 8.23 - 8.15 (m, 1H), 7.78 - 7.68 (m, 2H), 7.63 (dd, *J* = 7.4, 1.2 Hz, 1H), 7.41 - 7.36 (m, 2H), 7.27 (dt, *J* = 7.4, 1.2 Hz, 1H), 7.16 (td, *J* = 7.8, 1.3 Hz, 1H), 7.05 (td, *J* = 7.6, 1.0 Hz, 1H), 6.64 (d, *J* = 7.8 Hz, 1H), 4.98 (s, 2H), 4.06 (s, 2H), 4.00 (s, 2H), 3.63 (s, 3H), 1.25 (d, *J* = 11.0 Hz, 1H).

### Example 98

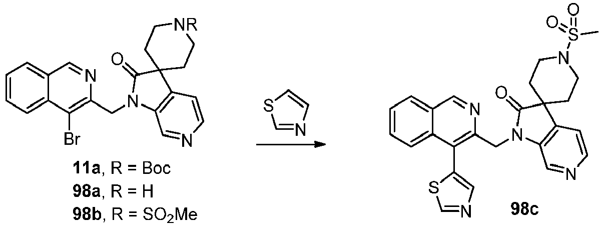

### Step a) 1'-((4-Bromoisoquinolin-3-yl)methyl)spiro[piperidine-4,3'-pyrrolo[2,3-c]pyridin]-2'(1'H)-one (98a)

TFA (5 g) was added dropwise over a period of 5 min at 0°C under nitrogen to a stirred solution of 11a (5 g) in DCM (150 mL). The mixture was stirred at rt for 3 h, then concentrated, diluted with water (50 mL), neutralised with saturated bicarbonate solution (50 mL) and extracted with 5 % MeOH in DCM (2 x200 mL). The combined organic layers were washed with water(50 mL), dried over sodium sulfate, filtered and concentrated under reduced pressure. The crude compound was triturated with diethyl ether (50 mL), stirred for 15 min, filtered and dried to give the title compound (3.4 g, 94%) as a solid. MS (ES+) 423.4 [M+H]⁺.

### Step b) 1'-((4-bromoisoquinolin-3-yl)methyl)-1-(methylsulfonyl)spiro[piperidine-4,3'-pyrrolo[2,3-c]pyridin]-2'(1'H)-one (98b)

To solution of 98a (2 g) in DCM (25 mL) was added triethylamine (2 mL) followed by methanesulfonyl chloride (0.5 mL) at 0 °C, the resulting mixture was stirred at rt for 3 h, then diluted with water (50 mL). The aqueous layer was extracted with DCM (2 x 50 mL), the combined organic layer was dried over sodium sulfate, filtered and concentrated. The obtained solid was triturated with diethyl ether (20 mL) and dried in vacuo which gave the title compound as a solid (1.8 g, 64%). MS (ES+) 503.06 [M+H]⁺.

### Step c) 1-(Methylsulfonyl)-1'-((4-(thiazol-5-yl)isoquinolin-3-yl)methyl)spiro[piperidine-4,3'-pyrrolo[2,3-c]pyridin]-2'(1'H)-one (98c)

To the stirred solution of 98b (300 mg) and thiazole (76 mg) in N,N-dimethylacetamide (10 mL) was added potassium acetate (176 mg) and palladium acetate (7 mg) at rt. The reaction mixture was stirred at 130 °C for 36 h, then diluted with ice water (20 mL) and extracted with ethyl acetate (2X30 mL). The combined organic layers were dried over Na₂SO₄, filtered and concentrated under reduced pressure. The afforded crude was purified by prep C18 HPLC, fractions containing the desired product was pooled, concentrated and purified again by prep C18 HPLC, which gave the title compound (28 mg) as off-white solid. MS (ES+) 506.33 [M+H]⁺. ¹H NMR (500 MHz, DMSO-*d*₆) δ 9.45 (s, 1H), 9.31 (s, 1H), 8.29 (d, *J* = 4.8 Hz, 1H), 8.20 - 8.11 (m, 3H), 7.82 (ddd, *J* = 8.3, 6.9, 1.3 Hz, 1H), 7.74 (ddd, *J* = 8.0, 6.9, 1.1 Hz, 1H), 7.62 (d, *J* = 4.8 Hz, 1H), 7.56 - 7.50 (m, 1H), 5.04 (s, 2H), 3.54 - 3.38 (m, 4H), 2.97 (s, 3H), 1.96 (ddd, *J* = 13.3, 8.9, 4.2 Hz, 2H), 1.86 (ddd, *J* = 13.7, 6.0, 3.7 Hz, 2H).

### Example 99

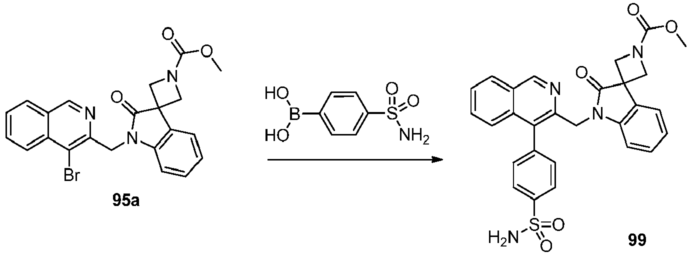

### Methyl 2'-oxo-1'-((4-(4-sulfamoylphenyl)isoquinolin-3-yl)methyl)spiro[azetidine-3,3'-indoline]-1-carboxylate (99)

Compound 95a (300 mg, 0.663 mmol) was reacted with (4-sulfamoylphenyl)boronic acid (146 mg, 0.730 mmol) according to the method described for Intermediate 22, step a, with the exception that the heating was effected by microwave irradiation at 100 °C for 1 h. The crude compound was purified by column chromatography on silica eluted with 2% MeOH in EtOAc, followed by prep HPLC using on an XSELECT column and mobile phase 10 mM ammonium bicarbonate : MeCN, which gave the title compound (135 mg, 38%) as a solid. MS (ES+) 529.18 [M+H]⁺.

### Example 100

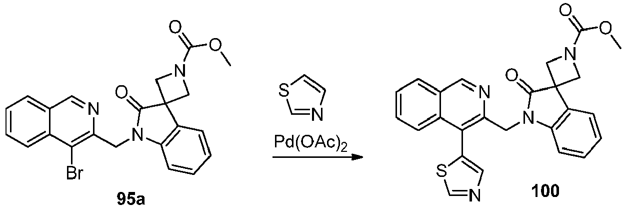

### Methyl 2'-oxo-1'-((4-(thiazol-5-yl)isoquinolin-3-yl)methyl)spiro[azetidine-3,3'-indoline]-1-carboxylate (100)

To the stirred solution of compound 95a (240 mg, 0.531 mol) and thiazole (67.8 mg, 0.796 mmol) in N,N-dimethylacetamide (8 mL) was added potassium acetate (156 mg, 1.59 mmol) and palladium acetate (5.95 mg, 0.027 mmol) at rt. The reaction mixture was stirred at 130 °C for 16 hours, then filtered through Celite. The filtrate was concentrated under reduced pressure and the obtained crude was purified by column chromatography on silica gel eluted with 85% EtOAc:p. ether. Fractions containing the desired product were pooled and concentrated and further purified by Prep HPLC using 10 mM ammonium bicarbonate in H₂O:acetonitrile on an X-Bridge C18 column. The residue was further purified by SFC using methanol as solvent, which gave the title compound (43 mg 17%) as a solid.
MS (ES+) 457.59 [M+H]⁺.
¹H NMR (500 MHz, DMSO-*d*₆) δ 9.40 (s, 1H), 9.36 (s, 1H), 8.19 (d, *J* = 8.1 Hz, 1H), 8.02 (s, 1H), 7.79 (t, *J* = 7.6 Hz, 1H), 7.73 (t, *J* = 7.5 Hz, 1H), 7.66 (d, *J* = 7.3 Hz, 1H), 7.45 (d, *J* = 8.4 Hz, 1H), 7.17 (t, *J* = 7.7 Hz, 1H), 7.06 (t, *J* = 7.5 Hz, 1H), 6.72 (d, *J* = 7.8 Hz, 1H), 5.02 (s, 2H), 4.10 (s, 4H), 3.64 (s, 3H), 3.41 - 3.36 (m, 1H), 2.45 (s, OH).

### Example 101

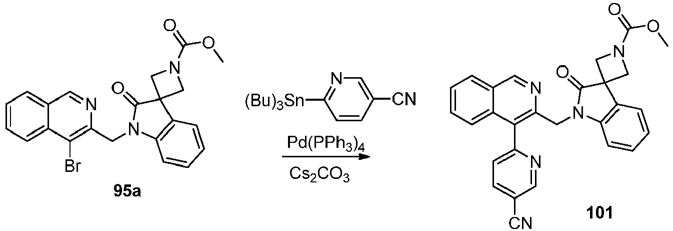

### Methyl 1'-((4-(5-cyanopyridin-2-yl)isoquinolin-3-yl)methyl)-2'-oxospiro[azetidine-3,3'-indoline]-1-carboxylate (101)

6-(tributylstannyl)nicotinonitrile (469 mg, 1.19 mmol) was added under argon at rt to a stirred and degassed solution of compound 95a (450 mg, 0.995 mmol) and cesium carbonate (972 mg, 2.98 mmol) in 1,4-dioxane. The mixture was degassed for 10 min at rt then heated at 100 °C for 16 h. The reaction mixture was concentrated under reduced pressure, slowly diluted with ice cold water (25 mL) and extracted with DCM (2x 25 mL). The organic layer was washed with water (25 mL), brine (25 mL), dried over Na₂SO₄ and concentrated. The obtained crude compound was purified by column chromatography on silica gel eluted with 30% EtOAc / p. ether. Pure fractions were collected, concentrated and further purified by prep HPLC on a Kromasil C18 column. Pure fractions were pooled and concentrated, water was added, pH adjusted to 7.5 with saturated NaHCO₃ solution. The mixture was stirred for 15 min, filtered and dried which gave the title compound (51 mg, 11%) as a solid. MS (ES+) 476.18 [M+H]⁺.
¹H NMR (500 MHz, DMSO-*d*₆) δ 9.38 (s, 1H), 9.20 (dd, *J* = 2.1, 0.9 Hz, 1H), 8.51 (dd, *J* = 8.1, 2.2 Hz, 1H), 8.25 - 8.17 (m, 1H), 7.86 (dd, *J* = 8.1, 0.9 Hz, 1H), 7.77 - 7.68 (m, 2H), 7.62 (dd, *J* = 7.4, 1.2 Hz, 1H), 7.33 - 7.26 (m, 1H), 7.16 (td, *J* = 7.8, 1.2 Hz, 1H), 7.04 (td, *J* = 7.5, 1.0 Hz, 1H), 6.80 (d, *J* = 7.8 Hz, 1H), 4.98 (s, 2H), 4.09 (s, 2H), 4.04 (d, *J* = 8.0 Hz, 2H), 3.64 (s, 3H), 3.35 (s, 1H).

### Example 102

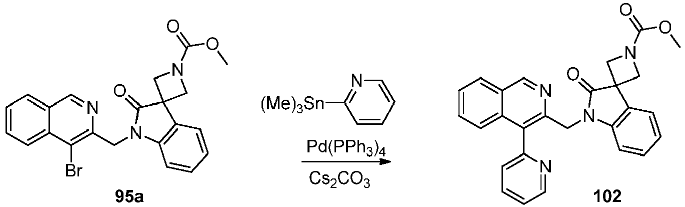

### Methyl 2'-oxo-1'-((4-(pyridin-2-yl)isoquinolin-3-yl)methyl)spiro[azetidine-3,3'-indoline1-1-carboxylate (102)

Compound 95a (220 mg, 0.486 mmol) was reacted with 2-(trimethylstannyl)pyridine (141 mg, 0.584 mmol) according to the procedure described in Example 101 with the exception that the reaction mixture was subjected to microwave irradiation, which gave the title compound (30 mg, 13%). MS (ES+) 451.70 [M+H]⁺.
¹H NMR (500 MHz, DMSO) δ 9.32 (s, 1H), 8.78 (m, 1H), 8.17 (m, 1H), 8.00 (td, *J* = 7.7, 7.7, 1.7 Hz, 1H), 7.71 (pd, *J* = 6.8, 6.8, 6.8, 6.8, 1.3 Hz, 2H), 7.62 (d, *J* = 7.2 Hz, 1H), 7.60 (d, *J* = 7.7 Hz, 1H), 7.52 (m, 1H), 7.31 (d, *J* = 8.0 Hz, 1H), 7.14 (m, 1H), 7.03 (t, *J* = 7.5, 7.5 Hz, 1H), 6.75 (d, *J* = 7.8 Hz, 1H), 4.94 (s, 2H), 4.08 (s, 4H), 3.64 (s, 3H).
¹³C NMR (126 MHz, DMSO) δ 176.13, 156.10, 154.39, 152.21, 149.85, 145.26, 143.09, 136.90, 134.31, 131.19, 129.90, 129.15, 128.68, 128.56, 127.72, 127.31, 127.06, 125.61, 123.94, 123.13, 123.01, 122.35, 109.10, 57.48, 51.99, 43.50, 41.91.

### Example 103

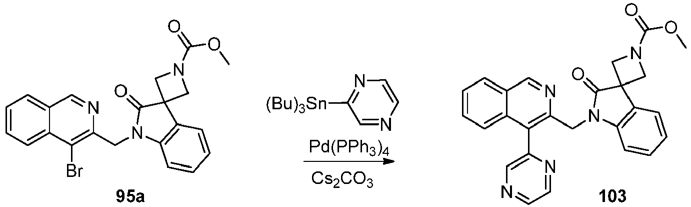

### Methyl 2'-oxo-1'-((4-(pyrazin-2-yl)isoquinolin-3-yl)methyl)spiro[azetidine-3,3'-indoline]-1-carboxylate (103)

Compound 95a (200 mg, 0.442 mmol) was reacted with 2-(tributylstannyl)pyrazine (196 mg, 0.531 mmol) according to the procedure described in Example 101 with the exception that the reaction mixture was subjected to microwave irradiation, which gave the title compound (25 mg, 12%). MS (ES+) 452.10 [M+H]⁺.
¹H NMR (500 MHz, DMSO-*d*₆) δ 9.41 (s, 1H), 8.85 (s, 1H), 8.78 (d, *J* = 2.4 Hz, 1H), 8.25 - 8.18 (m, 1H), 7.78 - 7.69 (m, 2H), 7.62 (dd, *J* = 7.4, 1.2 Hz, 1H), 7.29 (dd, *J* = 7.9, 1.9 Hz, 1H), 7.17 (td, *J* = 7.8, 1.2 Hz, 1H), 7.04 (td, *J* = 7.6, 1.0 Hz, 1H), 6.85 (d, *J* = 7.8 Hz, 1H), 5.00 (s, 2H), 4.06 (d, *J* = 16.0 Hz, 4H), 3.64 (s, 3H), 2.54 (s, OH).

### Example 104

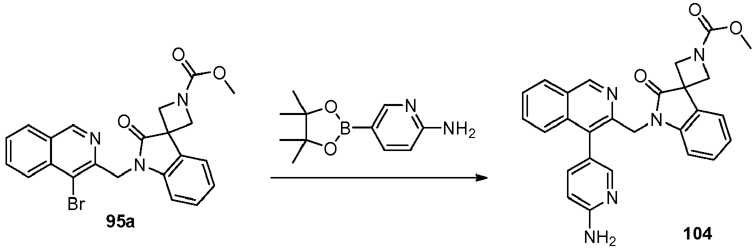

### methyl 1'-((4-(6-aminopyridin-3-yl)isoquinolin-3-yl)methyl)-2'-oxospiro[azetidine-3,3'-indoline]-1-carboxylate (104)

A stirred solution of 95a (200 mg), 5-(4,4,5,5-tetramethyl-1,3,2-dioxaborolan-2-yl)pyridin-2-amine (194 mg) and potassium carbonate (183 mg) in acetonitrile (16 mL) and water (4 mL) was purged with argon for 5 minutes, bis(triphenylphosphine)palladium(II) dichloride (62 mg) was then added. The reaction mixture was purged again with argon for 10 minutes, then stirred in a sealed tube at 100 °C for 7 h. The reaction mixture was filtered through Celite, the filtrate was diluted with water (15 mL) and extracted with EtOAc (2 x15 mL), the combined organic layers were dried over sodium sulfate, filtered and concentrated under reduced pressure. The obtained crude was purified by column chromatography on silica eluted with 5-6% MeOH in DCM. Fractions containing desired product were concentrated under reduced pressure and the afforded solid was grinded with mortar and pestle for 30 minutes which gave the title compound (73 mg, 35%) as a solid. MS (ES+) 466.13 [M+H]⁺.
¹H NMR (500 MHz, DMSO-*d*₆) δ 9.22 (s, 1H), 8.12 (d, *J* = 8.0 Hz, 1H), 7.86 (d, *J* = 2.4 Hz, 1H), 7.76 - 7.61 (m, 3H), 7.48 (d, *J* = 8.4 Hz, 1H), 7.37 (dd, *J* = 8.4, 2.4 Hz, 1H), 7.18 - 7.11 (m, 1H), 7.04 (t, *J* = 7.5 Hz, 1H), 6.60 (d, *J* = 8.1 Hz, 2H), 6.19 (s, 2H), 4.99 (s, 2H), 4.11 (s, 4H), 3.64 (s, 3H), 3.40 - 3.34 (m, 1H).

### Example 105

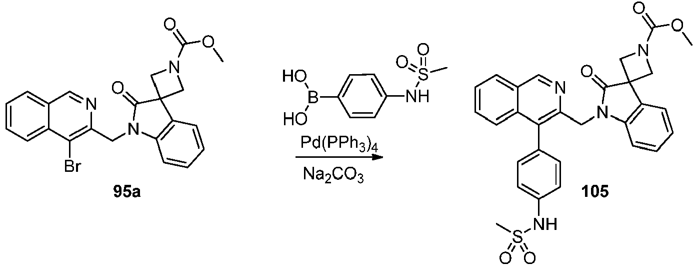

### methyl 1'-((4-(4-(methylsulfonamido)phenyl)isoquinolin-3-yl)methyl)-2'-oxospiro[azetidine-3,3'-indoline]-1-carboxylate (105)

A stirred solution of compound 95a (250 mg, 0.553 mmol), (4-(methylsulfonamido)phenyl)-boronic acid (155 mg, 0.719 mmol) and sodium carbonate (146 mg, 1.38 mmol) in 1,4-dioxane (10 mL) and water (1 mL) was purged with argon for 5 minutes, then tetrakis(triphenylphosphine)palladium (63.9 mg, 0.055 mmol) was added and the mixture was purged again with argon for 10 minutes, stirred under microwave irradiation at 110 °C for 1 hour. The reaction mixture was filtered through Celite, the filtrate was diluted with water (15 mL) and extracted with EtOAc (3x15 mL). The combined organic layers were dried over sodium sulphate, filtered and concentrated under reduced pressure. The afforded crude was purified by column chromatography on silica gel, eluted with 5% MeOH in DCM, which gave the title compound (192 mg, 63%) as a solid. MS (ES+) 543.20 [M+H]⁺.
¹H NMR (500 MHz, DMSO-*d*₆) δ 10.00 (s, 1H), 9.27 (s, 1H), 8.14 (dd, *J* = 7.8, 1.6 Hz, 1H), 7.70 (dddd, *J* = 17.6, 8.0, 6.9, 1.4 Hz, 2H), 7.63 (dd, *J* = 7.4, 1.2 Hz, 1H), 7.40 - 7.30 (m, 5H), 7.15 (td, *J* = 7.8, 1.2 Hz, 1H), 7.04 (td, *J* = 7.5, 0.9 Hz, 1H), 6.64 (d, *J* = 7.8 Hz, 1H), 4.95 (s, 2H), 4.08 (d, *J* = 14.4 Hz, 4H), 3.64 (s, 3H), 3.39 - 3.32 (m, 1H), 3.11 (s, 3H).

### Example 106

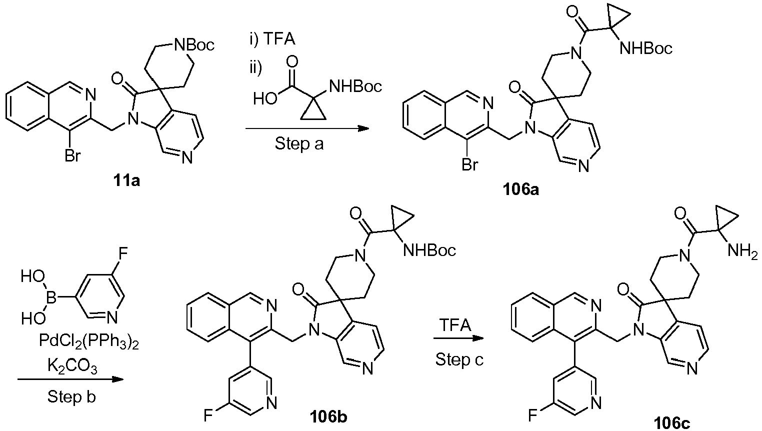

### Step a) tert-butyl (1-(1'-((4-bromoisoquinolin-3-yl)methyl)-2'-oxo-1',2'-dihydrospiro[piperidine-4,3'-pyrrolo[2,3-c]pyridin]-1-ylcarbonyl)cyclopropyl)carbamate (106a)

To a stirred solution of compound 11a in DCM (2 mL) was added TFA (1 mL). The solution was stirred at rt for 90 min, then concentrated at reduced pressure and co-evaporated with toluene. The residue was dissolved in DMF (2 mL), HATU and DIEA were added followed by addition of the Boc protected cyclopropyl amino acid. After 16 h stirring at rt, the reaction mixture was concentrated at vacuo and the residue was purified by column chromatography on silica gel eluted with MeOH- DCM, which gave the title compound (506 mg). MS (ES+) 606.3 & 608.3
[M+H]+.

### Step b) tert-butyl (1-(1'-((4-(5-fluoropyridin-3-yl)isoquinolin-3-yl)methyl)-2'-oxo-1',2'-dihydrospiro[piperidine-4,3'-pyrrolo[2,3-c]pyridin]-1-ylcarbonyl)cyclopropyl)carbamate (106b)

Compound 106a (173 mg, 0.285 mmol) was reacted with boronic acid (60 mg, 0.428 mmol) as described in Example 12 step a, which gave the title compound (158 mg, 89%). MS (ES+) 623.4
[M+H]+.

### Step c) 1-(1-aminocyclopropanecarbonyl)-1'-((4-(5-fluoropyridin-3-yl)isoquinolin-3-yl)methyl)spiro[piperidine-4,3'-pyrrolo[2,3-c]pyridin1-2'(1'H)-one (106c)

To a stirred solution of compound 106b (158 mg, 0.254 mmol) in DCM (2 mL) was added TFA (571 m, 5.01 mmol) dropwise. The solution was stirred at rt for 2h, then concentrated under reduced pressure and co-evaporated with toluene. The residue was purified by prep LCMS on a Gemini-NX Prep C18 column, mobile phase 0.1%NH₄OH in water - 0.1%NH₄OH in acetonitrile (pH 10) and Pure fractions were pooled and freeze-dried, which gave the title compound (72 mg, 54%). MS (ES+) 523.22 [M+H]⁺.
¹H NMR (500 MHz, DMSO-*d*₆) δ 9.37 (d, *J* = 0.8 Hz, 1H), 8.77 (d, *J* = 2.8 Hz, 1H), 8.54 (d, *J* = 3.4 Hz, OH), 8.54 (s, 1H), 8.29 - 8.17 (m, 3H), 8.06 (ddd, *J* = 9.5, 2.8, 1.7 Hz, 1H), 7.76 (dddd, *J* = 18.2, 8.0, 6.9, 1.4 Hz, 2H), 7.57 (dd, *J* = 4.8, 0.8 Hz, 1H), 7.37 (dd, *J* = 8.3, 1.3 Hz, 1H), 5.02 (d, *J* = 12.0 Hz, 2H), 2.30 (s, 2H), 1.77 (s, 2H), 1.70 (s, 2H), 0.89 (q, *J* = 4.3 Hz, 2H), 0.67 (t, *J* = 3.3 Hz, 2H).

### Example 107

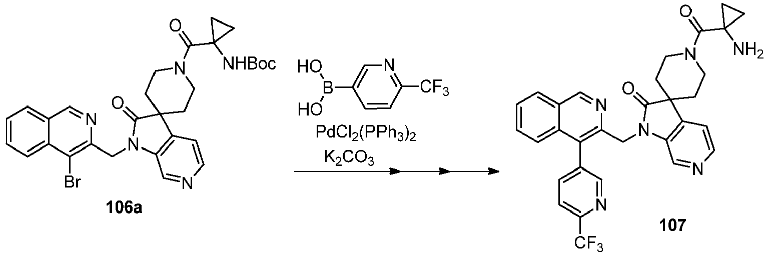

### 1-(1-aminocyclopropanecarbonyl)-1'-((4-(4-(trifluoromethyl)phenyl)isoquinolin-3-yl)methyl)spiro[piperidine-4,3'-pyrrolo[2,3-c]pyridin]-2'(1'H)-one (107)

The title compound was prepared according to the procedure described in Example 106, but using the boronic acid (6-(trifluoromethyl)pyridin-3-yl)boronic acid. Yield 50%, MS (ES+) 573.3
[M+H]+.
¹H NMR (500 MHz, DMSO-*d*₆) δ 9.42 - 9.37 (m, 1H), 8.88 (d, *J* = 2.1 Hz, 1H), 8.33 - 8.24 (m, 2H), 8.26 - 8.19 (m, 1H), 8.21 (s, 1H), 8.15 (dd, *J* = 8.0, 0.9 Hz, 1H), 7.81 - 7.71 (m, 2H), 7.60-7.54 (m, 1H), 7.35 - 7.29 (m, 1H), 5.04 (s, 1H), 4.99 (s, 1H), 3.92 (s, 2H), 3.32 (s, 3H), 2.28 (s, 2H), 1.75 (s, 2H), 1.67 (s, 2H), 0.88 (q, *J* = 4.2 Hz, 2H), 0.66 (t, *J* = 3.0 Hz, 2H).

### Example 108

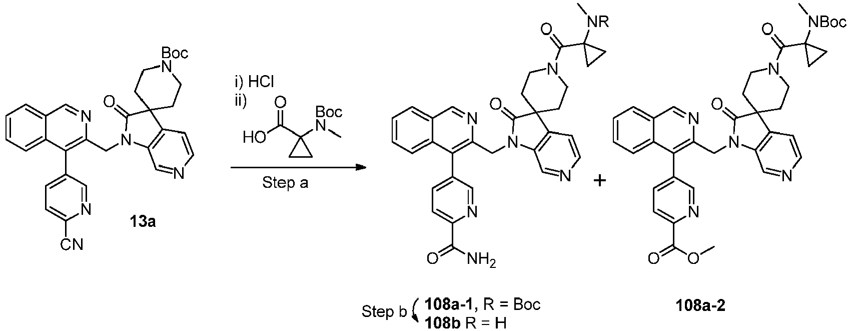

### Step a) Tert-butyl (1-(1'-((4-(6-carbamoylpyridin-3-yl)isoquinolin-3-yl)methyl)-2'-oxo-1',2'-dihydrospiro[piperidine-4,3'-pyrrolo[2,3-c]pyridin]-1-ylcarbonyl)cyclopropyl)(methyl)carbamate (108a-1)

### & Methyl 5-(3-((1-(1-((tert-butoxycarbonyl)(methyl)amino)cyclopropanecarbonyl)-2'-oxospiro[piperidine-4,3'-pyrrolo[2,3-c]pyridin]-1'(2'H)-yl)methyl)isoquinolin-4-yl)picolinate (108a-2)

Compound 13a was reacted with 1-((tert-butoxycarbonyl)(methyl)amino)cyclopropanecarboxylic acid according to the procedure described in Ex. 50, step a, which gave the two title compounds 108a-1, 152 mg, 35%, MS (ES+) 662.5 [M+H]⁺.
108a -2 160 mg, 36%, MS (ES+) 677.6 [M+H]⁺.

### Step b) 5-(3-((1-(1-(Methylamino)cyclopropanecarbonyl)-2'-oxospiro[piperidine-4,3'-pyrrolo[2,3-c]pyridin1-1'(2'H)-yl)methyl)isoquinolin-4-yl)picolinamide (108b)

Compound 108a-1 was deprotected as described in Example 50 step b, which gave the title compound (85 mg, 69.07%), MS (ES+) 562.4 [M+H]⁺.
¹H NMR (500 MHz, DMSO-*d*₆) δ 9.37 (d, *J* = 0.8 Hz, 1H), 8.74 - 8.69 (m, 1H), 8.21 (dd, *J* = 6.7, 1.7 Hz, 1H), 8.15 (dd, *J* = 7.9, 2.2 Hz, 1H), 7.80 - 7.70 (m, 4H), 7.57 (dd, *J* = 4.8, 0.9 Hz, 1H), 7.37 - 7.31 (m, 1H), 5.01 (q, *J* = 15.9 Hz, 3H), 2.26 (s, 4H), 1.73 (d, *J* = 10.3 Hz, 1H), 1.68 (s, 3H), 0.87 (s, 2H).

### Example 109

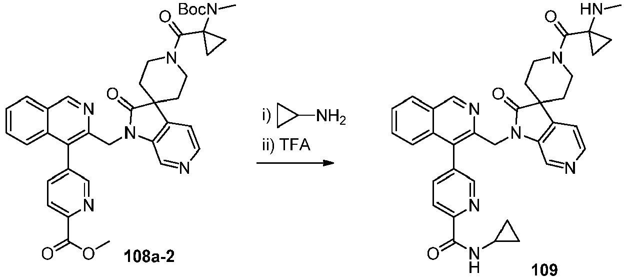

### N-cyclopropyl-5-(3-((1-(1-(methylamino)cyclopropanecarbonyl)-2'-oxospiro[piperidine-4,3'-pyrrolo[2,3-c]pyridin]-1'(2'H)-yl)methyl)isoquinolin-4-yl)picolinamide (109)

A solution of compound 108a-2 (150 mg, 0.222 mmol) and cyclopropylamine (253 mg, 4.43 mmol) in THF:EtOH 1:1 (5 mL) was stirred in a sealed tube for 2 days at 100 °C. The mixture was concentrated under reduced pressed and dried in vacuo. The residue was dissolved in DCM (10 mL), TFA (2.01 g, 17.7 mmol) was added and the mixture was stirred 90 min at rt, then concentrated under reduced pressure and co-evaporated twice with toluene. The residue was dissolved in THF:MeOH 1:1 (2 mL), 1M LiOH in water (1 mL) was added and the mixture was stirred for 1h at rt, then concentrated with acetic acid and reduced pressure. The product was isolated by HPLC (Gemini NX 100x30, gradient 14 minutes, 15-45% acetonitrile in water with 10 mM ammonium acetate buffer) Appropriate fractions were pooled and freeze dried which gave the title compound_(35 mg, 26%), MS (ES+) 602.5 [M+H]⁺.
¹H NMR (500 MHz, DMSO-*d*₆) δ 9.37 (d, *J* = 0.8 Hz, 1H), 8.91 (d, *J* = 5.0 Hz, 1H), 8.68 (d, *J* = 1.9 Hz, 1H), 8.28 - 8.17 (m, 3H), 8.16 (s, 1H), 8.16 (dd, *J* = 7.9, 2.2 Hz, 1H), 7.80 - 7.70 (m, 2H), 7.57 (dd, *J* = 4.8, 0.8 Hz, 1H), 7.37 - 7.30 (m, 1H), 5.04 (d, *J* = 15.9 Hz, 1H), 4.96 (d, *J* = 15.9 Hz, 1H), 3.32 (s, 8H), 3.02 - 2.93 (m, 1H), 2.26 (d, *J* = 3.4 Hz, 3H), 2.16 (s, 1H), 1.70 (s, 1H), 1.68 (s, 2H), 1.65 (s, 1H), 1.24 (s, 1H), 0.87 (s, 2H), 0.74 (dt, *J* = 3.0, 1.6 Hz, 1H), 0.72 (s, 2H), 0.67 (s, 2H).

### Example 110

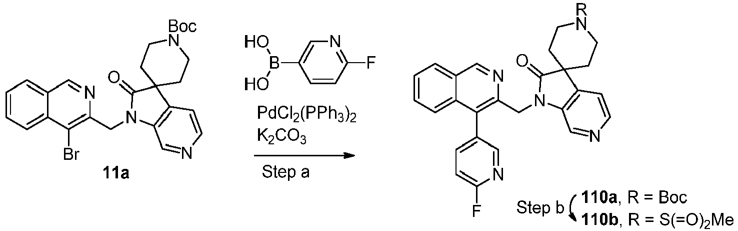

### Step a) tert-butyl 1'-((4-(6-fluoropyridin-3-yl)isoquinolin-3-yl)methyl)-2'-oxo-1',2'-dihydrospiro[piperidine-4,3'-pyrrolo[2,3-c]pyridine]-1-carboxylate (110a)

Compound 11a (147 mg, 0.281 mmol) was coupled with (6-fluoropyridin-3-yl)boronic acid (59.4 mg, 0.421 mmol) according to the procedure described in Example 12 step a, which gave the title compound (150 mg, 99%), MS (ES+) 540.3 [M+H]⁺.

### Step b) 1'-((4-(6-fluoropyridin-3-yl)isoquinolin-3-yl)methyl)-1-(methylsulfonyl)spiro[piperidine-4,3'-pyrrolo[2,3-c]pyridin1-2'(1'H)-one (110b)

4M HCl in dioxane (2 mL) was added to a solution of compound 110a (108 mg, 0.200 mmol). The solution was stirred at rt for 30 min, then concentrated under reduced pressure and co-evaporated with toluene. The residue was dissolved in MeCN (4 mL) and stirred for 10-15 min at 0 °C, then a mixture of methanesulfonyl chloride in MeCN (1 mL) was added slowly. The progress of the reaction was monitored by LCMS and when deemed complete the reaction mixture was concentrated and the crude product was purified by preparative HPLC on a Gemini C18 column using a gradient of 95% water: 5% acetonitrile (10 mM in ammonium acetate) and 10% water: 90% acetonitrile (10 mM in ammonium acetate). Appropriate fractions were pooled, freeze-dried and further purified by column chromatography on silica eluted with DCM MeOH , which gave the title compound (103 mg, 99%). MS (ES+) 518.3 [M+H]⁺.
¹H NMR (500 MHz, DMSO-*d*₆) δ 9.33 (d, *J* = 0.8 Hz, 1H), 8.38 (d, *J* = 2.4 Hz, 1H), 8.29 (d, *J* = 4.7 Hz, 1H), 8.23 (s, 1H), 8.22 - 8.13 (m, 2H), 7.75 (dddd, *J* = 20.2, 8.0, 6.9, 1.3 Hz, 2H), 7.60 (d, *J* = 4.7 Hz, 1H), 7.46 (dd, *J* = 8.3, 2.5 Hz, 1H), 7.38 (dd, *J* = 8.4, 1.2 Hz, 1H), 4.99 (d, *J* = 6.4 Hz, 2H), 3.52 - 3.32 (m, 2H), 3.42 (s, 3H), 2.97 (s, 3H), 2.55 (s, 1H), 1.93 (ddd, *J* = 13.2, 8.8, 4.3 Hz, 2H), 1.81 (dq, *J* = 14.0, 5.1 Hz, 2H).

### Example 111

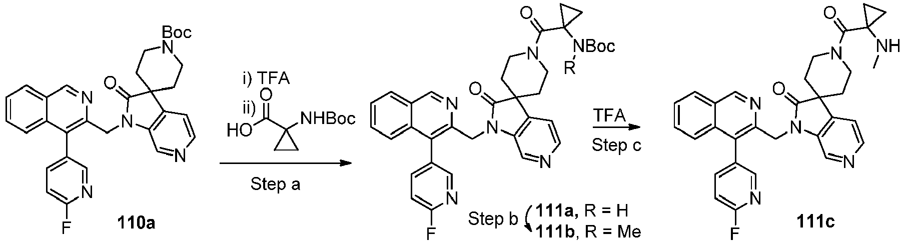

### Step a) tert-butyl (1-(1'-((4-(6-fluoropyridin-3-yl)isoquinolin-3-yl)methyl)-2'-oxo-1',2'-dihydrospiro[piperidine-4,3'-pyrrolo[2,3-c]pyridin]-1-ylcarbonyl)cyclopropyl)carbamate (111a)

Compound 110a (750 mg, 1.39 mmol) was N-deprotected using TFA in DCM as described in Example 60 step a, then coupled with 1-(tert-butoxycarbonylamino)cyclopropanecarboxylic acid (382 mg, 1.90 mmol) according to the procedure described for Intermediate 32, which gave the title compound (500 mg, 59%)

### Step b) tert-butyl (1-(1'-((4-(6-fluoropyridin-3-yl)isoquinolin-3-yl)methyl)-2'-oxo-1',2'-dihydrospiro[piperidine-4,3'-pyrrolo[2,3-c]pyridin]-1-ylcarbonyl)cyclopropyl)(methyl)carbamate (111b)

NaH (96 mg, 2.41mmol) was added at 0 °C to a stirred solution of compound 111a (500 mg, 0.803 mmol) in DMF (10 mL), the reaction mixture was stirred for 10 min, then methyl iodide (0.333 mL, 3.21 mmol) was added under nitrogen and the stirring was continued at rt for 2 h. The reaction was quenched with water (40 mL) and the mixture was extracted with ethyl acetate (2x60 mL), the combined organic layers were washed with brine, dried over sodium sulfate, filtered and concentrated under reduced pressure. The crude was purified by column chromatography on neutral alumina eluted with a gradient of MeOH in DCM, which gave the title compound (350 mg, 60%). MS (ES+) 637.39 [M+H]⁺.

### Step c) 1'-((4-(6-fluoropyridin-3-yl)isoquinolin-3-yl)methyl)-1-(1-(methylamino)cyclopropanecarbonyl)spiro[piperidine-4,3'-pyrrolo[2,3-c]pyridin]-2'(1'H)-one (111c)

Compound 111b (330 mg, 0.518 mmol) N-deprotected using in TFA in DCM (10 mL), as described in Example 60 step a. The afforded crude compound was purified by Prep-HPLC using an X-Bridge C18 column and 10 mM NH₄HCO₃ in MeCN as mobile phase. Pure fractions were pooled and lyophilized, which gave the title compound (100 mg, 35%) as a solid. MS (ES+) 537.24 [M+H]+.
¹H NMR (500 MHz, DMSO-*d*₆) δ 9.34 (d, *J* = 0.8 Hz, 1H), 8.37 (d, *J* = 2.4 Hz, 1H), 8.26 (d, *J* = 4.7 Hz, 1H), 8.26 - 8.13 (m, 2H), 7.75 (dddd, *J* = 19.3, 8.0, 6.9, 1.3 Hz, 1H), 7.58 (dd, *J* = 4.8, 0.8 Hz, 1H), 7.45 (dd, *J* = 8.3, 2.6 Hz, 1H), 7.40 - 7.34 (m, 1H), 5.00 (d, *J* = 6.2 Hz, 1H), 3.39 - 3.30 (m, 1H), 3.33 (s, 12H), 2.26 (s, 2H), 2.18 (s, 1H), 1.72 (s, 3H), 0.88 (s, 1H), 0.68 (s, 1H).

### Example 112

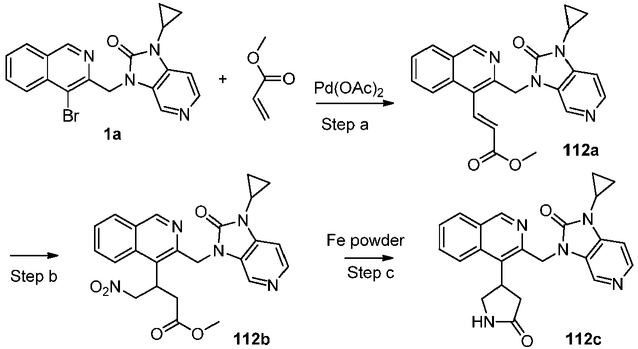

### Step a) (E)-methyl 3-(3-((1-cyclopropyl-2-oxo-1 H-imidazo[4,5-c]pyridin-3(2H)-yl)methyl)isoquinolin-4-yl)acrylate (112a)

Methyl acrylate (26 mg, 0.304 mmol) and Et₃N (0.142 mL, 1.01 mmol) were added to a solution of compound 1a (100 mg, 0.253 mmol) in DMF (5 mL). The solution was degassed with argon for 10 minutes in sealed tube, then palladium acetate (6 mg, 0.025 mmol) and tris(o-tolyl)phosphine (39 mg, 0.127 mmol) was added. The mixture was de-gassed for 10 min, then heated at 100 °C for 24 h. The reaction mixture was cooled to rt and diluted with EtOAc. The organic layer was washed with water (10 mL), brine (10 mL), dried over anhydrous Na₂SO₄, filtered and concentrated. The crude was purified by column chromatography (eluent: 10% MeOH-CHCl₃) which gave the title compound (80 mg, 7.4%). MS (ES+) 401.29 [M+H]⁺.

### Step b) methyl 3-(3-((1-cyclopropyl-2-oxo-1H-imidazo[4,5-c]pyridin-3(2H)-yl)methyl)isoquinolin-4-vl)-4-nitrobutanoate (112b)

A solution of compound 112a (500 mg, 1.25 mmol) in nitromethane (5 mL) was added dropwise at -20 °C to a solution of 1,8-diazabicycloundec-7-ene (0.285 g, 1.87 mmol) in nitromethane (5 mL). The mixture was stirred at -20 °C for 4 h, then left to attain rt and diluted with DCM. The organic layer was washed with water(10 mL), brine (10 mL), dried over anhydrous Na₂SO₄, filtered and concentrated under reduced pressure. The afforded crude was purified by prep HPLC which gave the title compound (180 mg, 31%). MS (ES+) 462.29 [M+H]⁺.

### Step c) 1-cyclopropyl-3-((4-(5-oxopyrrolidin-3-yl)isoquinolin-3-yl)methyl)-1H-imidazo[4,5-c]pyridin-2(3H)-one (112c)

Iron powder (85 mg) was added to a solution of compound 112b (140 mg, 0.303 mmol) in acetic acid (5 ml). The mixture was heated at 80°C for 8h, then filtered through Celite and the filter cake was washed with DCM. The organic layer was concentrated under reduced pressure and the afforded crude was dissolve in 10% MeOH-CHCl₃ and washed with water (5 mL), brine (5 mL), dried over anhydrous Na₂SO₄, filtered and concentrated under reduced pressure. The crude was mixed with another batch (50 mg) and purified by prep HPLC C18 eluted with 10mM ABC in H₂O:MeCN, which gave the title compound (40 mg).
¹H NMR (500 MHz, DMSO-*d*₆) δ 9.15 (s, 1H), 8.33 (s, 1H), 8.20 (d, *J* = 5.2 Hz, 1H), 8.14 (d, *J* = 8.1 Hz, 1H), 8.06 (s, 1H), 7.94 -7.80 (m, 2H), 7.70 (t, *J* = 7.4 Hz, 1H), 7.25 (d, *J* = 5.2 Hz, 1H), 5.47 (s, 1H), 4.75 (qd, *J* = 10.2, 7.5 Hz, 1H), 3.73 (t, *J* = 10.1 Hz, 1H), 3.60 (dd, *J* = 10.0, 7.5 Hz, 1H), 2.99 (tt, *J* = 7.0, 3.6 Hz, 1H), 2.70 - 2.55 (m, 2H), 1.06 (dt, *J* = 7.1, 3.5 Hz, 2H), 0.89 (p, *J* = 4.8 Hz, 2H).

### Example 113

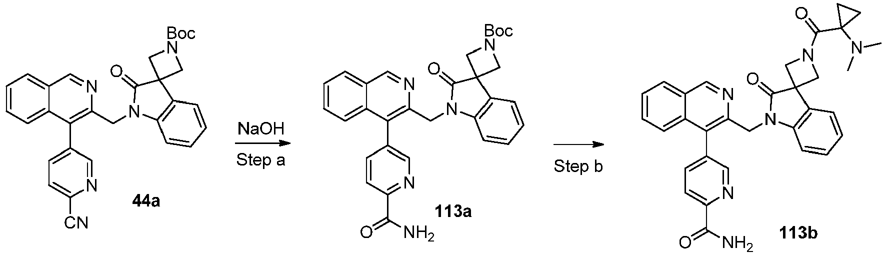

### Step a) tert-butyl 1'-((4-(6-carbamoylpyridin-3-yl)isoquinolin-3-yl)methyl)-2'-oxospiro[azetidine-3,3'-indoline1-1-carboxylate (113a)

To a solution of compound 44a (400 mg, 0.618 mmol) in EtOH was added 1M NaOH solution (3.1mL). The mixture was stirred at rt for 72h, then at 60 °C for 2.5 h, cooled to rt, acidified with acetic acid and concentrated onto silica. The product was purified by silica gel chromatography eluted with DCM and 2 to 10% MeOH, which gave the title compound (142 mg, 43%). MS (ES+) 536.5 [M+H]⁺.

### Step b) 5-(3-((1-(1-(Dimethylamino)cyclopropanecarbonyl)-2'-oxospiro[azetidine-3,3'-indolin]-1'-yl)methyl)isoquinolin-4-yl)picolinamide (113b)

Compound 113a (70 mg, 0.131 mmol) was reacted as described in Example 62, which gave the title compound (71 mg, 42%) MS (ES+) 547.5 [M+H]⁺.
¹H NMR (500 MHz, DMSO-*d*₆) δ 9.40 (d, *J* = 0.8 Hz, 1H), 8.22 (dt, *J* = 7.4, 2.7 Hz, 2H), 8.16 (d, *J* = 7.9 Hz, 1H), 7.81 - 7.69 (m, 2H), 7.57 (dd, *J* = 7.4, 1.2 Hz, 1H), 7.27 - 7.21 (m, 1H), 7.16 (td, *J* = 7.7, 1.3 Hz, 1H), 7.06 (td, *J* = 7.5, 1.0 Hz, 1H), 6.68 (d, *J* = 7.8 Hz, 1H), 4.36 (s, 2H), 2.20 (s, 6H), 0.92 (s, 4H).

### Example 114

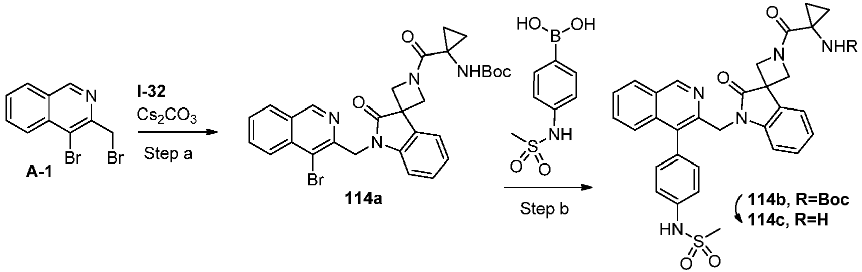

### Step a) tert-butyl (1-(1'-((4-bromoisoquinolin-3-yl)methyl)-2'-oxospiro[azetidine-3,3'-indolin]-1-ylcarbonyl)cyclopropyl)carbamate (114a)

A-1 (960 mg, 2.91 mmol) was reacted with I-32 (800 mg, 2.24 mmol) according to the procedure described in Ex. 79, which gave the title compound (1.29 g, 51%). MS (ES+) 579.23 [M+H]⁺.

### Step b) tert-butyl (1-(1'-((4-(4-(methylsulfonamido)phenyl)isoquinolin-3-yl)methyl)-2'-oxospiro[azetidine-3,3'-indolin]-1-ylcarbonyl)cyclopropyl)carbamate (114b)

Compound 114a (300 mg, 0.52 mmol) was reacted with (4-(methylsulfonamido)phenyl)boronic acid (145 mg, 0.675 mmol) according to the procedure described in Ex. 105, which gave the title compound (280 mg, 77%). MS (ES+) 668.53 [M+H]⁺.

### Step c) N-(4-(3-((1-(1-aminocyclopropanecarbonyl)-2'-oxospiro[azetidine-3,3'-indolin]-1'-yl)methyl)isoquinolin-4-yl)phenyl)methanesulfonamide (114c)

TFA (0.64 mL) was added at 0 °C to a stirred suspension of compound 114b (280 mg) in DCM (10 mL) The resulting reaction mixture was stirred at rt for 8 h, then concentrated under reduced pressure. The obtained residue was basified with saturated sodium bicarbonate solution and extracted with EtOAc (3X20 mL). The organic layer was dried over Na₂SO₄, filtered and concentrated under reduced pressure and the afforded residue was triturated with water: acetonitrile (1:1, 30 mL), stirred for 1 h, filtered and dried which gave the title compound as a solid (115 mg, 46%). MS (ES+) 568.23 [M+H]⁺.
¹H NMR (500 MHz, DMSO-*d*₆) δ 9.26 (s, 1H), 8.14 (d, *J* = 8.0 Hz, 1H), 7.85 (s, 1H), 7.70 (dt, *J* = 19.3, 7.2 Hz, 2H), 7.59 (d, *J* = 7.3 Hz, 1H), 7.37 (q, *J* = 8.4 Hz, 5H), 7.15 (t, *J* = 7.7 Hz, 1H), 7.05 (t, *J* = 7.5 Hz, 1H), 6.64 (d, *J* = 7.8 Hz, 1H), 4.96 (s, 2H), 4.75 (s, 2H), 4.07 - 4.00 (m, 1H), 3.99 (s, 2H), 3.40 (s, OH), 3.12 (s, 3H), 1.12 (s, 1H), 1.07 (s, 1H), 0.72 (s, 2H).

### Example 115

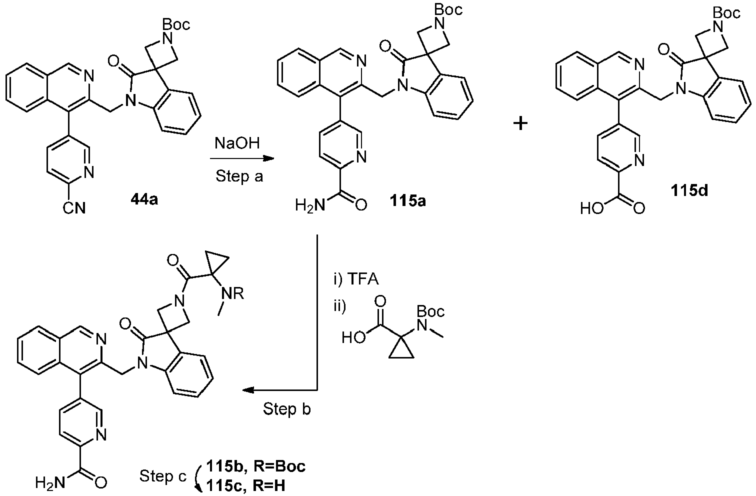

### Step a) tert-butyl 1'-((4-(6-carbamoylpyridin-3-yl)isoguinolin-3-yl)methyl)-2'-oxospiro[azetidine-3,3'-indoline]-1-carboxylate (115a)

1M sodium hydroxide (aq, 3.1 ml) was added to a solution of compound 44a (400 mg, 0.618 mmol) in EtOH. The mixture was stirred at rt over weekend, then warmed at 60 °C for 2.5 hours. The mixture was cooled, acidified with HOAc and concentrated onto silica. The product was purified by silica gel column chromatography eluted with DCM and 2 to 10% MeOH, which gave compound 115a (142 mg, 43%), MS (ES+) 536.5 [M+H]⁺, and compound 115d (66 mg, 20%), MS (ES+) 537.4 [M+H]⁺.

### Step b) tert-butyl (1-(1'-((4-(6-carbamoylpyridin-3-yl)isoquinolin-3-yl)methyl)-2'-oxospiro[azetidine-3,3'-indolin]-1-ylcarbonyl)cyclopropyl)(methyl)carbamate (115b)

N-deprotection: TFA (1.19 g, 10.5 mmol) was added to a solution of compound 115a (70 mg, 0.131 mmol) in DCM (8 mL). The solution was stirred at rt for 90 min, then concentrated in vacuo and co-evaporated twice with toluene.

Acylation: The residue was dissolved in dry DMF (10 mL), 1-((tert-butoxycarbonyl)(methyl)-amino)cyclopropanecarboxylic acid (33.8 mg, 0.157 mmol) and DIEA (169 mg, 1.31 mmol) were added and the solution was cooled in an ice bath and HATU (64.6 mg, 0.170 mmol) was added. The mixture was stirred for 1 h in the ice bath, then water (40 mL) and saturated sodium hydrogen carbonate solution (40 ml) were added and the mixture was extracted three times with EtOAc. The organic phase was washed with brine, dried over sodium sulfate and concentrated under reduced pressure. The product was purified by silica gel chromatography eluted with DCM and 2 to 5% MeOH, which gave the title compound (56 mg, 68%).

### Step c) 5-(3-((1-(1-(methylamino)cyclopropanecarbonyl)-2'-oxospiro[azetidine-3,3'-indolin]-1'-yl)methyl)isoquinolin-4-yl)picolinamide (115c)

TFA (991 mg, 8.69 mmol) was added to a solution of compound 115b (55 mg, 0.087 mmol) in DCM (8 mL). The solution was stirred at rt for 90 min, then concentrated in vacuo and co-evaporated twice with toluene. The residue was dissolved in DCM (5 mL), TEA (0.5 mL) was added and the solution was concentrated. The residue was purified by reverse phase HPLC. Appropriate fractions were pooled and freeze dried from acetonitrile water, which gave the title compound (28 mg, 60%). MS (ES+) 533.4 [M+H]⁺.
¹H NMR (500 MHz, DMSO) δ 9.38 (s, 1H), 8.56 (m, 1H), 8.21 (m, 3H), 8.01 (m, 1H), 7.73 (m, 3H), 7.58 (d, *J=* 6.9 Hz, 1H), 7.26 (m, 1H), 7.17 (m, 1H), 7.06 (m, 1H), 6.71 (d, *J=* 7.8 Hz, 1H), 4.98 (s, 2H), 4.47 (s, 2H), 3.96 (m, 2H), 2.27 (s, 3H), 1.96 (s, 1H), 1.03 (d, *J =* 22.6 Hz, 2H), 0.78 (d, *J* = 6.1 Hz, 2H).
¹³C NMR (126 MHz, DMSO) δ 176.11, 173.33, 165.61, 152.57, 149.80, 148.58, 145.83, 142.86, 138.77, 134.78, 133.66, 131.57, 129.81, 128.44, 127.85, 127.54, 127.00, 126.75, 123.79, 122.69, 122.51, 121.72, 109.17, 60.96, 56.33, 44.13, 42.80, 42.33, 33.26, 15.04, 14.31.

### Example 116

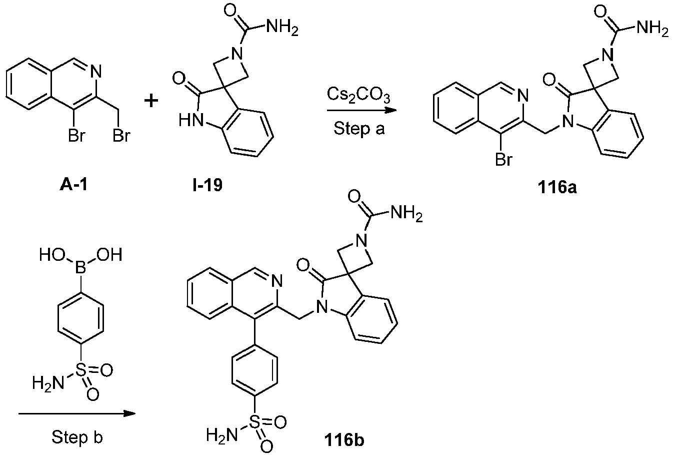

### Step a) 1'-((4-bromoisoquinolin-3-yl)methyl)-2'-oxospiro[azetidine-3,3'-indoline]-1-carboxamide (116a)

A-1 (1.14 g, 3.45 mmol) was reacted with 1-12 (500 mg, 2.30 mmol) according to the procedure described in Ex. 79, which gave the title compound (800 mg, 78%). MS (ES+) 439.05 [M+H]⁺.

### Step b) 2'-oxo-1'-((4-(4-sulfamoylphenyl)isoquinolin-3-yl)methyl)spiro[azetidine-3,3'-indoline]-1-carboxamide (116b)

Compound 116a (250 mg, 0.752 mmol) was reacted with (4-sulfamoylphenyl)boronic acid (149 mg, 0.743 mmol) using the method described for Intermediate 22 step a, which gave the title compound (100 mg, 34%). MS (ES+) 514.20 [M+H]⁺.
¹H NMR (500 MHz, DMSO-*d*₆) δ 9.30 (s, 1H), 8.24 (s, 2H), 8.17 (d, *J=* 7.8 Hz, 1H), 8.01 (d, *J* = 7.7 Hz, 2H), 7.72 (p, *J* = 7.0 Hz, 2H), 7.64 (d, *J* = 7.8 Hz, 2H), 7.55 (d, *J* = 6.6 Hz, 3H), 7.27 (d, *J* = 8.2 Hz, 1H), 7.17 (t, *J* = 7.6 Hz, 1H), 7.09 - 7.02 (m, 1H), 6.68 (d, *J* = 7.8 Hz, 1H), 6.10 (s, 2H), 4.92 (s, 2H), 3.99 (d, *J* = 7.7 Hz, 2H), 3.90 (d, *J* = 8.0 Hz, 2H).

### Example 117

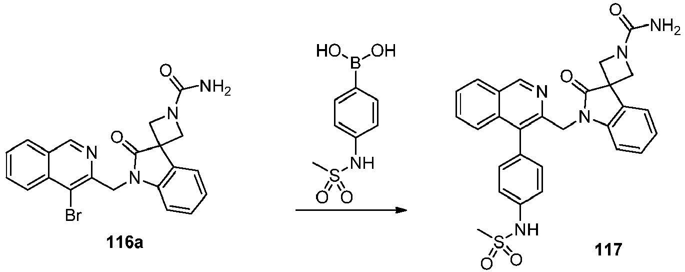

### 1'-((4-(4-(methylsulfonamido)phenyl)isoquinolin-3-yl)methyl)-2'-oxospiro[azetidine-3,3'-indoline]-1-carboxamide (117)

Compound 116a (250 mg, 0.572 mmol) was reacted with (4-(methylsulfonamido)phenyl)boronic acid (160 mg, 0.743 mmol) using the method described in Example 105, which gave the title compound (58 mg, 19%). MS (ES+) 528.22 [M+H]⁺.
¹H NMR (500 MHz, DMSO-*d*₆) δ 9.97 (s, 1H), 9.28 - 9.24 (m, 1H), 8.17 - 8.11 (m, 1H), 7.70 (dddd, *J* = 18.8, 8.0, 6.8, 1.4 Hz, 2H), 7.54 (dd, *J* = 7.4, 1.2 Hz, 1H), 7.42 - 7.32 (m, 5H), 7.14 (td, *J* = 7.7, 1.3 Hz, 1H), 7.04 (td, *J* = 7.5, 1.0 Hz, 1H), 6.63 (d, *J =* 7.8 Hz, 1H), 6.10 (s, 2H), 5.76 (s, 0H), 4.95 (s, 2H), 4.00 (d, *J* = 7.9 Hz, 2H), 3.91 (d, *J =* 7.9 Hz, 2H), 3.37 - 3.33 (m, 1H), 3.13 (s, 3H), 2.54 (s, 0H).

### Example 118

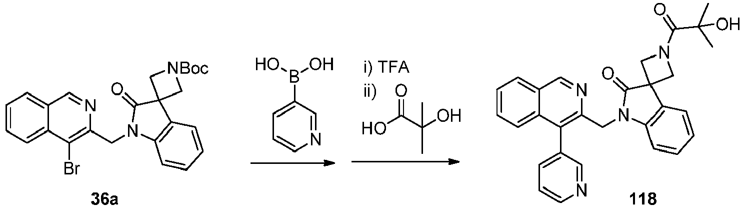

### 1-(2-hydroxy-2-methylpropanoyl)-1'-((4-(pyridin-3-yl)isoquinolin-3-yl)methyl)spiro[azetidine-3,3'-indolin]-2'-one (118)

The title compound was prepared according to the procedure described in Example 51 step a, but using 3-pyridine boronic acid, followed by N-deprotection and acylation with 2-hydroxyisobutyric acid as described in Example 115 step b. Yield 35%. MS (ES+) 479.4 [M+H]⁺. ¹H NMR (500 MHz, DMSO-*d*₆) δ 9.34 (s, 1H), 8.74 - 8.68 (m, 1H), 8.54 (d, *J* = 2.3 Hz, 1H), 8.19 (dd, *J* = 7.5, 1.9 Hz, 1H), 7.86 (s, 1H), 7.86 (dt, *J* = 14.6, 1.9 Hz, 1H), 7.78 - 7.68 (m, 2H), 7.62 - 7.52 (m, 2H), 7.27 (dd, *J* = 8.1, 1.6 Hz, 1H), 7.17 (td, *J* = 7.8, 1.3 Hz, 1H), 7.10 - 7.03 (m, 1H), 6.71 (dd, *J* = 7.9, 5.0 Hz, 1H), 5.25 (s, 1H), 4.97 (qd, *J* = 16.0, 4.7 Hz, 2H), 4.55 (dd, *J* = 9.7, 4.9 Hz, 1H), 4.48 (dd, *J* = 9.7, 4.4 Hz, 1H), 4.03 - 3.93 (m, 2H), 1.34- 1.27 (m, 6H).
¹³C NMR (126 MHz, DMSO-*d*₆) δ 200.48, 176.20, 175.04, 152.29, 149.70, 149.15, 145.77, 143.01, 137.36, 137.34, 134.91, 131.42, 131.02, 129.81, 128.46, 127.79, 127.42, 127.31, 127.03, 123.81, 123.69, 122.67, 122.41, 109.11, 74.69, 73.20, 61.52, 56.35, 43.93, 42.56, 27.64, 27.37.

### Example 119

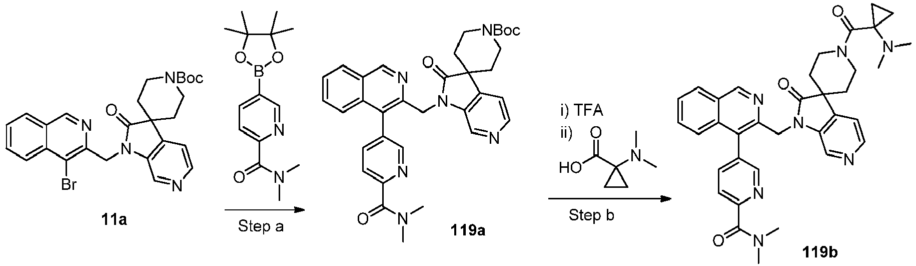

### Step a) tert-butyl 1'-((4-(6-(dimethylcarbamoyl)pyridin-3-yl)isoquinolin-3-yl)methyl)-2'-oxo-1',2'-dihydrospiro[piperidine-4,3'-pyrrolo[2,3-c]pyridine]-1-carboxylate (119a)

2M aq. sodium carbonate (1.46 ml, 2.82 mmol) and bis(triphenylphosphine)Pd(II)Cl₂ (114 mg, 0.16 mmol) were added to a mixture of N,N-dimethyl-5-(4,4,5,5-tetramethyl-1,3,2-dioxaborolan-2-yl)picolinamide (202 mg, 0.73 mmol) and compound 11a (390 mg, 0.74 mmol) in CH₃CN (7 ml) in a microwave vial. The vial was sealed, evacuated and filled with N₂ (x 3), then heated to 80 °C for 16h. The solid was filtered off and the filtrates was concentrated. The product was purified by chromatography on silica using eluting with 1-5% MeOH in DCM, which gave the title compound (501 mg, 116%) contaminated by e.g. triphenylphosphine oxide. The compound was used in the next step without further purification.

### Step b) 5-(3-((1-(1-(dimethylamino)cyclopropanecarbonyl)-2'-oxospiro[piperidine-4,3'-pyrrolo[2,3-c]pyridin]-1'(2'H)-yl)methyl)isoquinolin-4-yl)-N,N-dimethylpicolinamide (119b)

Compound 119a from above was N-deprotected and the afforded amine acylated with 1-(dimethylamino)cyclopropanecarboxylic acid as described in Example 115 step b. Yield over the two steps 46%. MS (ES+) 604.3 [M+H]⁺.
¹H NMR (500 MHz, DMSO-*d*₆) δ 9.36 (d, *J* = 0.8 Hz, 1H), 8.69 (dd, *J* = 2.1, 0.9 Hz, 1H), 8.28 - 8.23 (m, 2H), 8.23 - 8.17 (m, 1H), 8.05 (dd, *J* = 7.9, 2.2 Hz, 1H), 7.81 - 7.70 (m, 3H), 7.60 (dd, *J* = 4.8, 0.8 Hz, 1H), 7.37 (dd, *J* = 8.4, 1.3 Hz, 1H), 5.04 (s, 1H), 5.01 (s, 1H), 3.32 (s, 4H), 3.09 (d, *J=* 11.2 Hz, 6H), 2.21 (s, 6H), 1.70 (pd, *J* = 14.8, 12.0, 5.8 Hz, 4H), 0.88 (s, 2H), 0.77 (s, 2H).

### Example 120

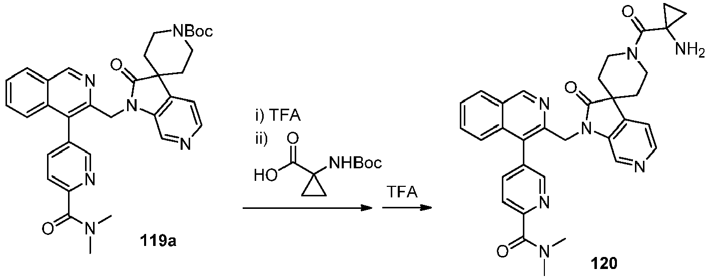

### 5-(3-((1-(1-aminocyclopropanecarbonyl)-2'-oxospiro[piperidine-4,3'-pyrrolo[2,3-c]pyridin]-1'(2'H)-yl)methyl)isoquinolin-4-yl)-N,N-dimethylpicolinamide (120)

Compound 119a from above was N-deprotected and the afforded amine acylated with 1-((tert-butoxycarbonyl)amino)cyclopropanecarboxylic acid as described in Example 115 step b. The afforded Boc protected compound was N-deprotected using TFA in DCM, which gave the title compound (37%). MS (ES+) 576.3 [M+H]⁺.
¹H NMR (500 MHz, DMSO-*d*₆) δ 9.36 (d, *J=* 0.8 Hz, 1H), 8.70 (dd, *J* = 2.2, 0.9 Hz, 1H), 8.29 - 8.21 (m, 2H), 8.20 (dd, *J* = 8.2, 1.3 Hz, 1H), 8.07 (dd, *J* = 7.9, 2.2 Hz, 1H), 7.81 - 7.70 (m, 3H), 7.58 (dd, *J* = 4.8, 0.8 Hz, 1H), 7.37 (dd, *J* = 8.3, 1.2 Hz, 1H), 5.02 (d, *J* = 9.6 Hz, 2H), 3.33 (s, 4H), 3.09 (d, *J* = 8.4 Hz, 6H), 2.30 (s, 2H), 1.76 (s, 2H), 1.71 (s, 3H), 0.88 (q, *J* = 4.3 Hz, 2H), 0.67 (t, *J* = 3.2 Hz, 2H).

### Example 121

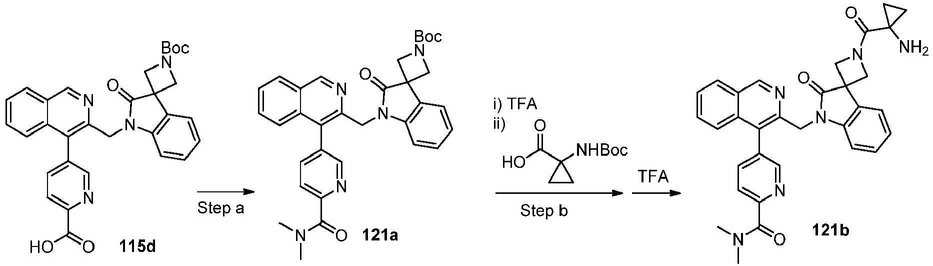

### Step a) tert-butyl 1'-((4-(6-(dimethylcarbamoyl)pyridin-3-yl)isoquinolin-3-yl)methyl)-2'-oxospiro[azetidine-3,3'-indoline]-1-carboxylate (121a)

DIEA (100 µl, 0.57 mmol) was added to a mixture of compound 115d (65 mg, 0.12 mmol), dimethylamine hydrochloride (23 mg, 0.28 mmol) and HATU (52 mg, 0.14 mmol) in DMF (3 ml).The RM was stirred at ambient temperature for 1.5 h, then additional HATU (50 mg, 0.13 mmol) and DIEA (100 µl, 0.57 mmol) was added and the reaction was continued for 1h. The reaction mixture was diluted with EtOAc, washed with aq. NaHCO₃, aq. citric acid and brine. The organic phase was dried over MgSO₄ and concentrated and the product purified by column chromatography on silica eluting with 0-10 % MeOH in DCM, which gave impure title compound (78 mg, 114 %) MS (ES+) 564.4[M+H]⁺.

### Step b) 5-(3-((1-(1-Aminocyclopropanecarbonyl)-2'-oxospiro[azetidine-3,3'-indolin]-1'-yl)methyl)isoquinolin-4-yl)-N,N-dimethylpicolinamide (121b)

Compound 121a (78 mg, 0.138 mmol) was N-deprotected and the afforded amine acylated with 1-((tert-butoxycarbonyl)amino)cyclopropanecarboxylic acid as described in Example 115 step b. The afforded Boc protected compound was N-deprotected using TFA in DCM, which gave the title compound (26 mg, 34%). MS (ES+) 547.3 [M+H]⁺.
¹H NMR (500 MHz, DMSO-*d*₆) δ 9.38 (d, *J* = 0.8 Hz, 1H), 8.64 (s, 0H), 8.54 (s, 1H), 8.24 - 8.18 (m, 1H), 7.96 (s, 1H), 7.89 (s, 1H), 7.81 - 7.68 (m, 3H), 7.59 (dd, *J* = 7.4, 1.2 Hz, 1H), 7.35 - 7.28 (m, 1H), 7.17 (td, *J* = 7.8, 1.3 Hz, 1H), 7.06 (td, *J* = 7.6, 1.0 Hz, 1H), 6.77 (s, 1H), 5.04 (d, *J* = 8.1 Hz, 0H), 4.68 (s, 2H), 4.04 (s, 1H), 3.96 (s, 2H), 3.07 (d, *J* = 4.7 Hz, 6H), 2.25 (s, 2H), 1.13 (s, 1H), 1.06 (s, 1H), 0.72 (s, 2H).

### Example 122

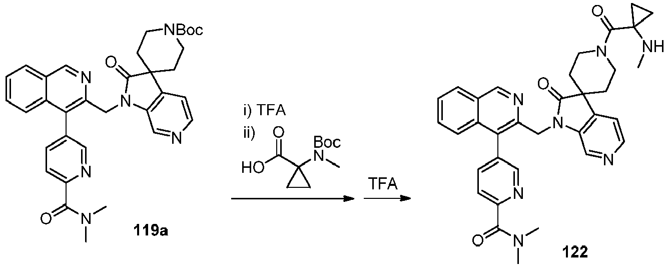

### N,N-dimethyl-5-(3-((1-(1-(methylamino)cyclopropanecarbonyl)-2'-oxospiro[piperidine-4,3'-pyrrolo[2,3-c]pyridin]-1'(2'H)-yl)methyl)isoquinolin-4-yl)picolinamide (122)

Compound 119 a was N-deprotected and the afforded amine acylated with 1-((tert-butoxycarbonyl)amino)cyclopropanecarboxylic acid as described in Example 115 step b. The afforded Boc protected compound was N-deprotected using TFA in DCM, which gave the title compound (11%). MS (ES+) 590.4 [M+H]⁺.
¹H NMR (500 MHz, DMSO-*d*₆) δ 9.38 - 9.34 (m, 1H), 8.70 (dd, *J* = 2.2, 0.9 Hz, 1H), 8.26 (t, *J* = 2.3 Hz, 2H), 8.23 - 8.17 (m, 1H), 8.06 (dd, *J* = 7.9, 2.2 Hz, 1H), 7.81 - 7.70 (m, 4H), 7.61 - 7.56 (m, 1H), 7.37 (dd, *J* = 8.4, 1.2 Hz, 1H), 5.09 - 4.96 (m, 2H), 3.33 (s, 4H), 3.09 (d, *J* = 9.4 Hz, 7H), 2.26 (d, *J* = 4.9 Hz, 3H), 2.18 (d, *J* = 6.0 Hz, 1H), 0.87 (s, 2H), 0.68 (s, 2H).

### Example 123

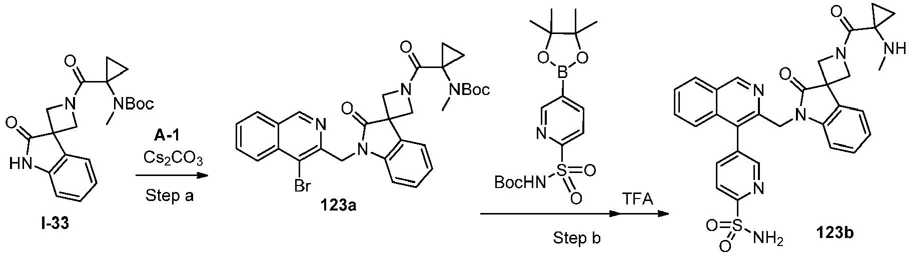

### Step a) tert-butyl (1-(1'-((4-bromoisoquinolin-3-yl)methyl)-2'-oxospiro[azetidine-3,3'-indolin]-1-ylcarbonyl)cyclopropyl)(methyl)carbamate (123a)

Compound I-33 (350 mg, 0.94 mmol) was reacted with Compound A-1 (369 mg, 1.0 mmol) according to the method described in Example 79, which gave the title compound (300 mg, 46 mmol). MS (ES+) 593.2 [M+H]⁺.

### Step b) 5-(3-((1-(1-(methylamino)cyclopropanecarbonyl)-2'-oxospiro[azetidine-3,3'-indolin]-1'-yl)methyl)isoquinolin-4-yl)pyridine-2-sulfonamide (123b)

Compound 123a (250 mg, 0.423 mmol) was reacted with tert-butyl (5-(4,4,5,5-tetramethyl-1,3,2-dioxaborolan-2-yl)pyridin-2-yl)sulfonylcarbamate (216 mg, 0.634 mmol) according to the method described in Example 51 step a. The afforded compound was then N-deprotected by treatment with TFA in DCM at 75 °C for 16h, which gave the title compound (24%). MS (ES+) 569.34 [M+H]+.
¹H NMR (500 MHz, DMSO-*d₆*) δ 9.36 (s, 1H), 8.78 (s, 1H), 8.24 - 8.18 (m, 2H), 8.13 (dd, *J* = 7.9, 0.8 Hz, 1H), 7.76 (dddd, *J* = 20.3, 8.0, 6.9, 1.3 Hz, 2H), 7.60 (d, *J* = 1.2 Hz, 0H), 7.28 - 7.17 (m, 2H), 7.08 (td, *J* = 7.5, 0.9 Hz, 1H), 6.80 (d, *J* = 7.8 Hz, 1H), 4.98 (s, 1H), 4.94 (s, 1H), 4.61 (s, 1H), 4.53 (s, 1H), 2.55 (s, 1H), 2.28 (s, 3H), 1.07 (s, 1H), 1.03 (s, 1H), 0.79 (s, 2H).

### Example 124

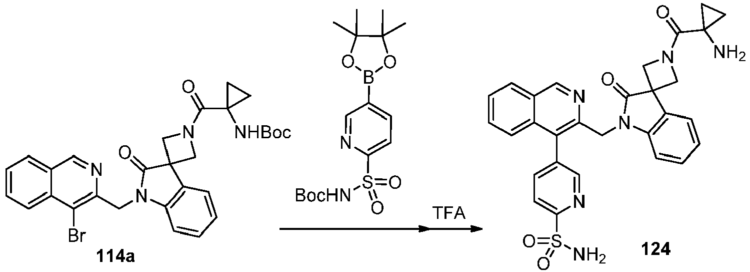

### 5-(3-((1-(1-Aminocyclopropanecarbonyl)-2'-oxospiro[azetidine-3,3'-indolin]-1'-yl)methyl)isoquinolin-4-yl)pyridine-2-sulfonamide (124)

Compound 114a (350 mg, 0.606 mmol) was reacted as described in Example 123 step b, which gave the title compound (23%). MS (ES+) 555.28 [M+H]⁺.
¹H NMR (500 MHz, DMSO-*d*₆) δ 9.37 (s, 1H), 8.79 (s, 1H), 8.71 (s, 0H), 8.24 - 8.18 (m, 1H), 8.12 (d, *J* = 7.9 Hz, 1H), 7.76 (dddd, *J* = 18.6, 8.0, 6.9, 1.3 Hz, 2H), 7.63 (s, 2H), 7.27 - 7.16 (m, 2H), 7.08 (td, *J* = 7.5, 1.0 Hz, 1H), 6.77 (d, *J* = 7.8 Hz, 1H), 5.03 (s, 1H), 4.97 (s, 1H), 4.90 (s, 1H), 4.72 (s, 2H), 3.96 (s, 2H), 3.38 - 3.33 (m, 1H), 2.31 - 2.26 (m, 2H), 1.12 (s, 1H), 1.07 (s, 1H), 0.71 (d, *J* = 4.7 Hz, 2H).

### Example 125

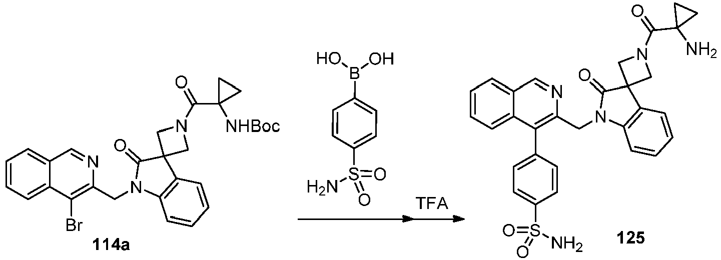

### 4-(3-((1-(1-aminocyclopropanecarbonyl)-2'-oxospiro[azetidine-3,3'-indolin]-1'-yl)methyl)isoquinolin-4-yl)benzenesulfonamide (125)

Compound 114a (400 mg, 0.693 mmol) was reacted with (4-sulfamoylphenyl)boronic acid (181 mg, 0.900 mmol) according to the method described Intermediate 22 step a, then N-deprotected by treatment with TFA in DCM at rt for 7h, which gave the title compound (28%). MS (ES+) 554.14 [M+H]⁺.
¹H NMR (500 MHz, DMSO-*d*₆) δ 9.33 - 9.28 (m, 1H), 8.20 - 8.14 (m, 1H), 8.01 (d, *J* = 7.9 Hz, 2H), 7.72 (dddd, *J* = 18.2, 8.0, 6.9, 1.4 Hz, 2H), 7.65 (s, 2H), 7.60 (dd, *J* = 7.4, 1.2 Hz, 1H), 7.53 (s, 2H), 7.27 (dd, *J* = 8.4, 1.2 Hz, 1H), 7.18 (td, *J* = 7.7, 1.3 Hz, 1H), 7.07 (td, *J* = 7.5, 1.0 Hz, 1H), 6.68 (d, *J* = 7.8 Hz, 1H), 4.97 (s, 1H), 4.91 (s, 1H), 4.72 (s, 2H), 4.01 (s, 1H), 3.96 (s, 1H), 3.39 - 3.33 (m, 0H), 2.26 (s, 2H), 1.13 (s, 1H), 1.07 (s, 1H), 0.71 (d, *J* = 3.9 Hz, 2H).

### Example 127

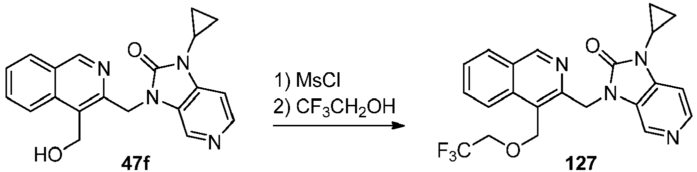

### 1-Cyclopropyl-3-((4-((2,2,2-trifluoroethoxy)methyl)isoquinolin-3-yl)methyl)-1H-imidazo[4,5-c]pyridin-2(3H)-one (127)

Methanesulfonyl chloride (152 mg, 1.0 mmol) was added drop wise at rt under nitrogen to a stirred suspension of compound 47f (400 mg, 1.16 mmol) and triethylamine (351 mg, 3.0 mmol) in DCM (20 mL). The resulting mixture was stirred at rt for 1 h, then diluted with DCM (50 mL) and washed with water (2 x 30 mL). The organic layer was dried over sodium sulfate, filtered and concentrated under reduced pressure. The afforded residue was added at rt to a stirred solution of 2,2,2-trifluoroethanol (94 mg, 0.94 mmol), cesium carbonate (614 mg, 2.0 mmol) and sodium iodide (141 mg, 0.94 mmol) in DMF(3 mL), stirred for 10 minutes. The mixture was stirred for 16 h at rt, then poured into water (30 mL) and extracted with EtOAc (2 X50 mL). The combined organic layers were washed with brine (30 mL), dried over sodium sulfate, filtered and concentrated under reduced pressure. The afforded crude compound was purified by C18 prep-HPLC (10 mM ammonium bicarbonate in H₂O : Acetonitrile). Appropriate fractions were pooled and concentrated. The afforded solid was triturated with water (5 mL) which gave the title compound (60 mg, 15%) as a solid. MS (ES+) 429.61 [M+H]⁺.
¹H NMR (500 MHz, DMSO-*d*₆) δ 9.25 (s, 1H), 8.29 (s, 1H), 8.28 - 8.22 (m, 1H), 8.20 (d, *J* = 5.2 Hz, 1H), 8.12 (dd, *J* = 8.0, 1.4 Hz, 1H), 7.89 (ddd, *J* = 8.4, 6.8, 1.3 Hz, 1H), 7.72 (ddd, *J* = 7.9, 6.8, 0.9 Hz, 1H), 7.24 (dd, *J* = 5.2, 0.8 Hz, 1H), 5.43 (s, 2H), 5.36 (s, 2H), 4.30 (q, *J* = 9.3 Hz, 2H), 3.37 - 3.33 (m, 1H), 2.97 (tt, *J* = 7.1, 3.7 Hz, 1H), 1.06 (td, *J* = 7.3, 5.2 Hz, 2H), 0.94 - 0.87 (m, 2H).

### Example 128

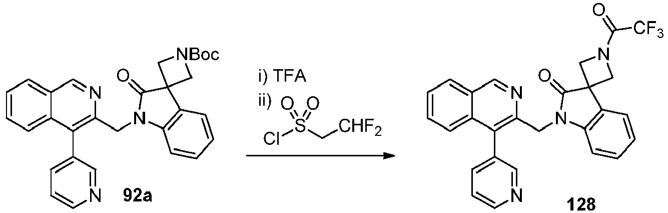

### 1'-((4-(Pyridin-3-yl)isoquinolin-3-yl)methyl)-1-(2,2,2-trifluoroacetyl)spiro[azetidine-3,3'-indolin]-2'-one (128)

TFA was added dropwise at rt to a solution of compound 92a (81 mg, 0.164 mmol) in DCM (2 mL). The solution was stirred at rt for 30 min, then concentrated and co-evaporated with toluene. The residue and DIEA (0.115 mL, 0.658 mmol) were dissolved at rt in DCM (3 mL), then 2,2-difluoroethanesulfonyl chloride (20 µL, 0.197 mmol) was added. The mixture was stirred at rt for 30 min, then concentrated in vacuo. The crude was dissolved in DCM and purified by column chromatography on silica, then further purified by Prep LCMS on a C18 column. Appropriate fractions were pooled and concentrated and the residue lyophilized which gave the title compound (20.3 mg, 25%) MS ES(+): 489.2 [M+H]⁺.
¹H NMR (500 MHz, DMSO-*d*₆) δ 9.35 (s, 1H), 8.75 - 8.70 (m, 1H), 8.58 - 8.53 (m, 1H), 8.22 - 8.15 (m, 1H), 7.88 (tt, *J* = 7.9, 1.8 Hz, 1H), 7.81 - 7.66 (m, 3H), 7.60 (dd, *J* = 7.9, 4.8 Hz, 1H), 7.30 - 7.24 (m, 1H), 7.21 (td, *J* = 7.8, 1.3 Hz, 1H), 7.07 (td, *J* = 7.5, 1.0 Hz, 1H), 6.80-6.74 (m, 1H), 5.05 - 4.88 (m, 2H), 4.65 (dd, *J* = 9.7, 3.7 Hz, 1H), 4.48 (d, *J* = 9.5 Hz, 1H), 4.31 (dd, *J* = 10.4, 4.9 Hz, 1H), 4.16 (dd, *J* = 10.2, 3.5 Hz, 1H).

### Example 129

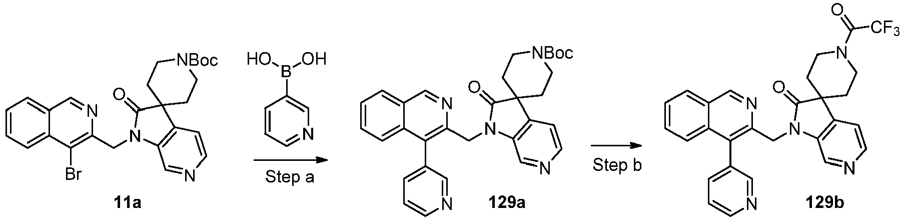

### Stepa)Tert-butyl2'-oxo-1'-((4-(pyridin-3-yl)isoquinolin-3-yl)methyl)-1',2'-dihydrospiro[piperidine-4,3'-pyrrolo[2,3-c]pyridine]-1-carboxylate (129a)

Compound 11a (135 mg, 0.258 mmol) was reacted with pyridine-3-boronic acid (47.6 mg, 0.387 mmol) according to the method described in Example 12 step a, which gave the title compound (135 mg, 97%) MS (ES+) 522.3 [M+H]⁺.

### 1'-((4-(Pyridin-3-yl)isoquinolin-3-yl)methyl)-1-(2,2,2-trifluoroacetyl)spiro[piperidine-4,3'-pyrrolo[2,3-c]pyridin]-2'(1'H)-one (129b)

Compound 129a (50 mg, 0.096 mmol) was reacted as described in Example 128, but reaction temperature was 0 °C, and pyridine was used as base instead of DIPEA, which gave the title compound (49.6 mg, 79%) MS (ES+) 518.3 MS [M+H]⁺.
¹H NMR (500 MHz, DMSO-*d*₆) δ 9.32 (s, 1H), 8.76 (d, *J* = 4.6 Hz, 1H), 8.68 (s, 1H), 8.29 (s, 1H), 8.22 - 8.15 (m, 2H), 7.98 (ddt, *J* = 7.5, 3.6, 1.9 Hz, 1H), 7.74 (dddd, *J* = 19.5, 8.0, 6.9, 1.3 Hz, 2H), 7.65 (q, *J* = 4.3, 3.5 Hz, 2H), 7.36 - 7.30 (m, 1H), 4.99 (qd, *J* = 16.0, 9.2 Hz, 2H), 4.03 (dd, *J* = 10.4, 5.0 Hz, 1H), 3.91 (ddd, *J* = 13.4, 9.4, 3.3 Hz, 1H), 3.85 - 3.72 (m, 2H), 2.05 - 1.89 (m, 2H), 1.78 (ddt, *J* = 19.1, 13.9, 4.5 Hz, 2H).

### Example 130

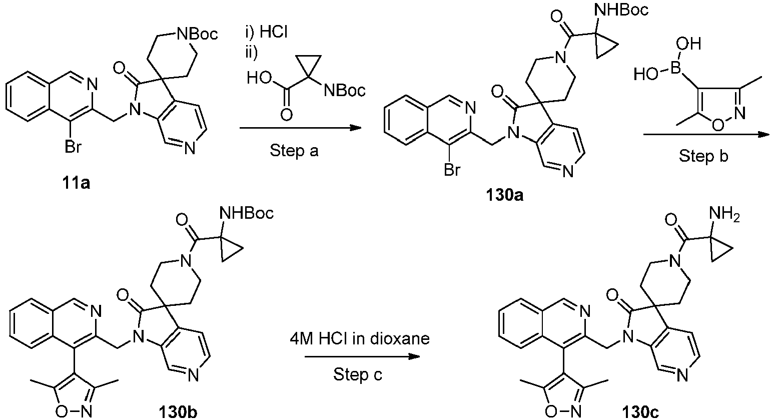

### Step a) Tert-butyl (1-(1'-((4-bromoisoauinolin-3-yl)methyl)-2'-oxo-1',2'-dihydrospiro[piperidine-4.3'-pyrrolo[2.3-c]pyridin]-1-ylcarbonyl)cyclopropyl)carbamate (130a)

To a solution of compound 11a (800 mg, 1.53 mmol) in 1,4-dioxane (20 mL) and MeOH (6 mL) was added conc. aq. HCl (4 mL). The mixture was stirred at ambient temperature for two h, then concentrated and co-evaporated with toluene three times and then dried in vacuum. The afforded di-hydrochloride salt of the amine, 1-(Boc amino)cyclopropanecarboxylic acid (320 mg, 1.59 mmol) and HATU (680 mg, 1.79 mmol) was slurried in DMF (12 mL) and DIEA (1.1 mL, 6.32 mmol) was added. The afforded solution was stirred at ambient temperature for two h, then diluted with EtOAc, washed with aq.NaHCO₃, and brine. The organic phase was dried over MgSO₄ and concentrated. The product was purified by silica gel column chromatography eluted with DCM and 1 to 10% MeOH, which gave the title compound (1.05 g, 112%) contaminated with some DIEA and PF₆.

### Step b) Tert-butyl (1-(1'-((4-(3,5-dimethylisoxazol-4-yl)isoquinolin-3-yl)methyl)-2'-oxo-1',2'-dihydrospiro[piperidine-4,3'-pyrrolo[2,3-c]pyridin]-1-ylcarbonyl)cyclopropyl)carbamate (130b)

Compound 130a (150 mg, 0.247 mmol) and 3,5-dimethylisoxazole-4-boronic acid (68 mg, 0.48 mmol) were coupled together according to the method described in Example 12 step a, which gave the title compound (42 mg, 27%).

### Step c) 1-(1-Aminocyclopropanecarbonyl)-1'-((4-(3,5-dimethylisoxazol-4-yl)isoquinolin-3-yl)methyl)spiro[piperidine-4,3'-pyrrolo[2.3-c]pyridin]1-2'(1'H)-one (130c)

Compound 130b (42 mg, 0.067 mmol) was dissolved in DCM (1.5 mL) and then treated with 4M solution of HCl in dioxane (1.5 mL) according to the method described in Example 39 step b, which gave the title compound (14 mg, 98%).
¹H NMR (500 MHz, DMSO) δ 0.66 (d, 1H), 0.88 (q, 1H), 1.73 (m, 2H), 1.87 (s, 2H), 2.28 (d, 3H), 3.88 (m, 0H), 4.95 (d, 1H), 5.08 (d, 1H), 7.44 (d, 1H), 7.61 (d, 1H), 7.73 (m, 1H), 7.81 (m, 1H), 8.19 (m, 1H), 8.28 (d, 1H).

### Example 131

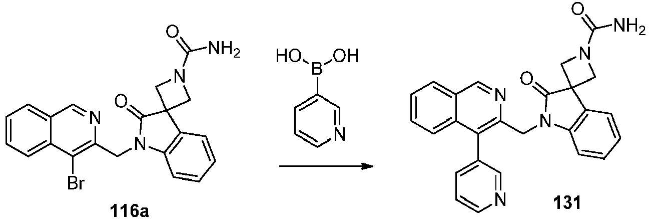

### 2'-Oxo-1'-((4-(pyridin-3-yl)isoquinolin-3-yl)methyl)spiro[azetidine-3,3'-indoline]-1-carboxamide (131)

Pyridine-3-boronic acid (54.8 mg, 0.446 mmol), 1M sodium carbonate solution (1.2 mL) and tetrakis(triphenylphosphine)-palladium (0) were added under argon to a solution of compound 116a (130 mg, 0.297 mmol) in dioxane (8 mL). The mixture was stirred in a sealed tube for 6 h at 90 °C, then cooled, diluted with dioxane and filtered. The filtrate was concentrated under reduced pressure and the residue purified by silica gel chromatography eluted with DCM and 2 to 8% MeOH, which gave the title compound (129 mg, 70%) MS (ES+) 436.4 [M+H]⁺.
¹H NMR (500 MHz, DMSO-*d*₆) δ 9.36 - 9.32 (m, 1H), 8.72 (dd, *J* = 4.9, 1.7 Hz, 1H), 8.53 (d, *J* = 2.2 Hz, 1H), 8.22 - 8.16 (m, 1H), 7.83 (dt, *J* = 7.8, 2.0 Hz, 1H), 7.78 - 7.68 (m, 1H), 7.61 - 7.50 (m, 1H), 7.31 - 7.24 (m, 1H), 7.16 (td, *J* = 7.8, 1.3 Hz, 1H), 7.05 (td, *J* = 7.5, 1.0 Hz, 1H), 6.68

(d, *J* = *7.8* Hz, 1H), 6.11 (s, 1H), 4.97 (d, *J* = 4.0 Hz, 1H), 3.96 (d, *J* = *7.9* Hz, 1H), 3.88 (dd, *J* = 7.9, 2.5 Hz, 1 H).

### Example 132

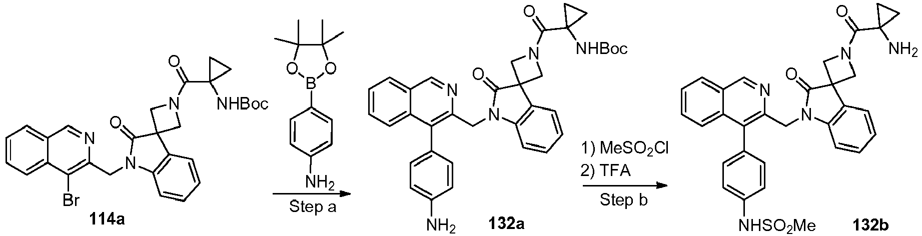

### Step a) tert-butyl (1-(1'-((4-(4-aminophenyl)isoquinolin-3-yl)methyl)-2'-oxospiro[azetidine-3.3'-indolin]-1-ylcarbonyl)cyclopropyl)carbamate (132a)

Compound 114a (400 mg, 0.693 mmol) was reacted with 4-(4,4,5,5-tetramethyl-1,3,2-dioxaborolan-2-yl)aniline (305 mg, 1.38 mmol) according to the method described Example 12 step a but using CH₃CN:water 4:1 as solvent and reaction temperature 100 °C, which gave the title compound (300 mg, 72%). MS (ES+) 591.35 [M+H]⁺.

### Step b) N-(4-(3-((1-(1-aminocyclopropanecarbonyl)-2'-oxospiro[azetidine-3,3'-indolin]-1'-yl)methyl)isoquinolin-4-yl)phenyl)methanesulfonamide (132b)

Et₃N (0.35 mL) and methanesulfonyl chloride (0.09 mL) were added at 0 °C to a stirred solution of compound 132a (300 mg) in DCM (10 mL). The resulting mixture was stirred at rt for 2 h, then concentrated under reduced pressure. The residue was dissolved in 1,4-dioxane (10 mL), the temperature was lowered to 0 °C and 1N NaOH solution (5 mL) was added while stirring. The resulting mixture was stirred at rt for 3 h, then neutralized with 1N HCl solution at 0 °C. The mixture was extracted with EtOAc (2x15 mL), the organic layer was washed with brine, dried over Na₂SO₄, filtered and concentrated under reduced pressure. The afforded solid was suspended in DCM (10 mL), TFA was added at 0 °C and the mixture was then stirred at rt for 6h, then concentrated under reduced pressure and co evaporated with toluene (2x5 mL). The residue was dissolved in DCM (10 mL) and Et₃N (1 mL), concentrated and washed with ethyl acetate (2 X10 mL). The residue was triturated with saturated sodium bicarbonate solution for 2 h, filtered, dried and grained with mortar and pestle for 40 minutes which gave the title compound (83 mg, 27%) as a solid.
¹H NMR (500 MHz, DMSO-*d*₆) δ 9.32 (s, 1H), 8.26 (s, 1H), 8.17 (d, *J=* 8.0 Hz, 1H), 7.73 (dt, *J* = 21.4, 7.7 Hz, 3H), 7.59 (d, *J* = 7.4 Hz, 1H), 7.38 (d, *J* = 8.4 Hz, 1H), 7.16 (q, *J* = 9.4, 8.5 Hz, 2H), 7.06 (t, *J* = 7.5 Hz, 1H), 6.70 (d, *J* = 7.8 Hz, 1H), 5.01 (s, 2H), 4.75 - 4.69 (m, 2H), 4.03 (s, 1H), 3.97 (s, 1H), 3.37 (s, 3H), 1.13 (s, 1H), 1.08 (s, 1H), 0.72 (s, 2H).

### Example 133

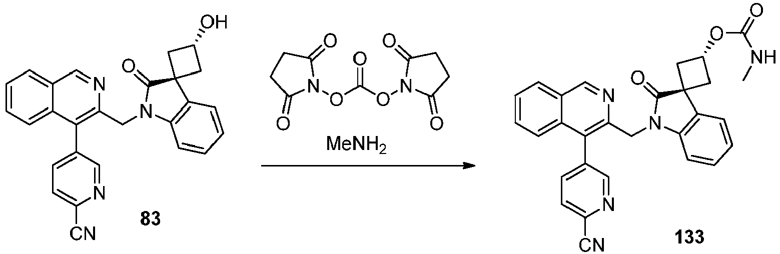

### (1R,3R)-1'-((4-(6-cyanopyridin-3-yl)isoquinolin-3-yl)methyl)-2'-oxospiro[cyclobutane-1,3'-indolin]-3-yl methylcarbamate (133)

Et₃N (0.258 mL), bis(2,5-dioxopyrrolidin-1-yl) carbonate (474 mg) were added at rt to a solution of compound 83 (160 mg) in DCM (5 mL). The reaction mixture was stirred at rt for 2 h, then a 2 M solution of methylamine in THF (57.4 mg) was added and the stirring was continued for 16 h, then concentrated under reduced pressure. The crude product was purified by prep HPLC using which gave the title compound (64 mg, 35%) as a solid. MS (ES+) 490.61 [M+H]⁺.
¹H NMR (500 MHz, DMSO-*d₆*) δ 9.37 (s, 1H), 8.81 - 8.77 (m, 1H), 8.25 (dd, *J* = 8.0, 0.9 Hz, 1H), 8.20 (qd, *J* = 4.2, 1.7 Hz, 2H), 7.74 (ddt, *J* = 9.8, 7.0, 3.6 Hz, 2H), 7.46 - 7.40 (m, 1H), 7.31 - 7.25 (m, 1H), 7.18 - 7.09 (m, 1H), 7.07 - 6.99 (m, 1H), 6.75 (d, *J* = 7.8 Hz, 1H), 5.19 (p, *J* = 7.3 Hz, 1H), 4.93 (d, *J* = 7.7 Hz, 2H), 2.68 - 2.55 (m, 4H), 2.39 (td, *J* = 7.3, 3.8 Hz, 2H).

### Example 134

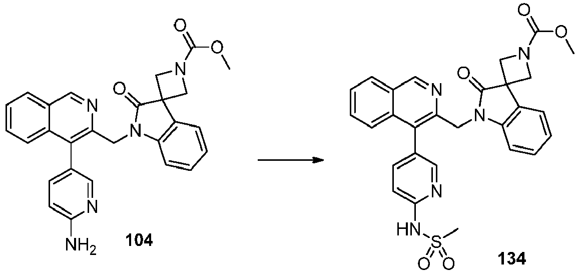

### Methyl 1'-((4-(6-(methylsulfonamido)pyridin-3-yl)isoquinolin-3-yl)methyl)-2'-oxospiro[azetidine-3.3'-indoline]-1-carboxylate (104)

Et₃N (0.33 mL) was added at rt to a stirred solution of compound 104 (220 mg) in DCM (15 mL). The mixture was cooled to 0 °C, methanesulfonyl chloride (0.11 mL) was added and the mixture was then stirred for 3 h at rt and then concentrated. The residue was dissolved in 1,4-dioxane (10 mL), 1N NaOH solution (7 mL) was added and the mixture was stirred at rt for 2 h then concentrated. Water was added and the pH was adjusted to 8-8.5 with 1N HCl solution. The mixture was extracted with DCM (2 x 25 mL), The organic layer was washed with water (10 mL), brine (10 mL), dried over Na₂SO₄ and concentrated. to get crude compound. The afforded crude was purified by column chromatography on silica gel using 1-2% MeOH/DCM as eluent. Pure fractions were collected and concentrated and the residue was triturated with diethyl ether, which gave the title compound (80 mg, 28%) as a solid. MS (ES+) 544.2 [M+H]⁺.
¹H NMR (500 MHz, DMSO-*d*₆) δ 10.86 (s, 1H), 9.32 (s, 1H), 8.25 (s, 1H), 8.17 (d, *J* = 8.0 Hz, 1H), 7.82 (s, 0H), 7.73 (dt, *J* = 13.7, 7.1 Hz, 3H), 7.63 (d, *J=* 7.4 Hz, 1H), 7.37 (d, *J=* 8.3 Hz, 1H), 7.15 (dt, *J* = 16.0, 8.0 Hz, 2H), 7.05 (t, *J* = 7.5 Hz, 1H), 6.72 (d, *J* = 7.8 Hz, 1H), 5.00 (s, 2H), 4.07 (d, *J =* 13.4 Hz, 4H), 3.63 (s, 3H), 3.37 (s, 3H).

### Example 135

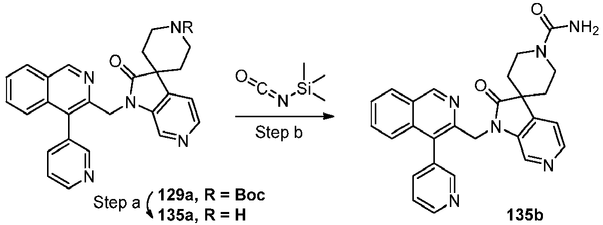

### Step a) 1'-((4-(pyridin-3-yl)isoquinolin-3-yl)methyl)spiro[piperidine-4,3'-pyrrolo[2,3-c]pyridin]-2'(1'H)-one (135a)

TFA (1.2 mL) was added at rt to a stirred solution of compound 129a (400 mg) in DCM (10 mL) The reaction mixture was stirred at rt for 1 h, then concentrated under vacuum. The resulting crude was diluted with DCM (50 mL), washed with saturated sodium bicarbonate solution (40 mL). The organic layer was dried over sodium sulfate, filtered and concentrated under reduced pressure which gave the title compound (325 mg, 90%) as a solid. MS (ES+) 422.2 [M+H]⁺.

### Step b) 2'-oxo-1'-((4-(pyridin-3-yl)isoquinolin-3-yl)methyl)-1',2'-dihydrospiro[piperidine-4,3'-pyrrolo[2.3-c]pyridine]-1-carboxamide (135b)

Compound 135a (100 mg, 0.237 mmol) was dissolved in DCM (10 mL) and triethylamine (0.119 mL) and (trimethylsilyl)isocyanate (0.042 mL) were added at rt. The resulting mixture was stirred at rt for 5 h, then diluted with DCM (50 mL) and washed with water (30 mL). The organic layer was dried over sodium sulphate, filtered and concentrated under reduced pressure. The afford crude compound was purified by prep C18 HPLC. Pure fraction were pooled, concentrated and lyophilized which gave the title compound (50 mg, 44%) as a solid. MS (ES+) 465.46 [M+H]⁺.
¹H NMR (500 MHz, DMSO-*d*₆) δ 9.32 (s, 1H), 8.76 (dd, *J* = 4.9, 1.6 Hz, 1H), 8.67 (d, *J* = 2.1 Hz, 1H), 8.25 (d, *J* = 4.8 Hz, 1H), 8.21 - 8.14 (m, 2H), 7.98 (dt, *J* = 7.8, 2.0 Hz, 1H), 7.74 (dddd, *J* = 19.8, 7.9, 6.9, 1.3 Hz, 2H), 7.65 (dd, *J* = 7.8, 4.8 Hz, 1H), 7.58 (d, *J* = 4.8 Hz, 1H), 7.33 (d, *J* = 8.4 Hz, 1H), 6.02 (s, 2H), 5.01 (d, *J* = 16.0 Hz, 1H), 4.94 (d, *J* = 16.0 Hz, 1H), 3.64 (dt, *J* = 12.3, 5.8 Hz, 2H), 3.57 (dt, *J* = 13.6, 5.5 Hz, 2H), 1.65 (t, *J* = 5.7 Hz, 4H).

### Example 136

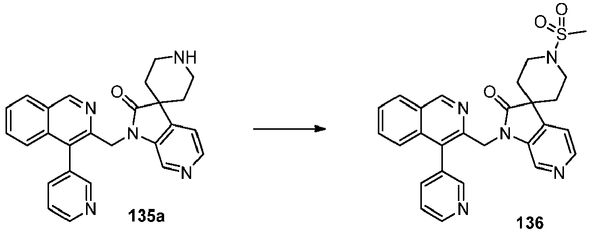

### 1-(methylsulfonyl)-1'-((4-(pyridin-3-yl)isoquinolin-3-yl)methyl)spiro[piperidine-4,3'-pyrrolo[2,3-c]pyridin]-2'(1'H)-one (136)

Compound 135a (80 mg) was dissolved in DCM (3 mL) and triethylamine (0.106 µL) and methanesulfonyl chloride(0.018 µL) were added at 0 °C. The reaction mixture was stirred at rt for 2 h, then diluted with cold water (20 mL) and extracted with DCM(2 x 30 mL). The combined organic layers were washed with brine (30 ml), dried over anhydrous sodium sulfate, filtered and concentrated. The afforded crude compound was purified by prep C18 HPLC, which gave the title compound (45 mg, 47%). MS (ES+) 500.25 [M+H]⁺.
¹H NMR (500 MHz, DMSO-*d*₆) δ 9.31 (s, 1H), 8.76 (d, *J* = 4.7 Hz, 1H), 8.68 (s, 1H), 8.28 (d, *J* = 4.7 Hz, 1H), 8.19 (s, 2H), 7.98 (d, *J* = 7.6 Hz, 1H), 7.74 (dt, *J* = 21.1, 7.1 Hz, 2H), 7.69-7.62 (m, 1H), 7.60 (d, *J* = 4.9 Hz, 1H), 7.34 (d, *J* = 8.3 Hz, 1H), 4.99 (s, 1H), 4.96 (s, 1H), 3.45 (dt, *J* = 22.1, 9.7 Hz, 5H), 2.97 (s, 3H), 1.97 - 1.88 (m, 2H), 1.81 (d, *J =* 13.8 Hz, 2H).

### Example 137

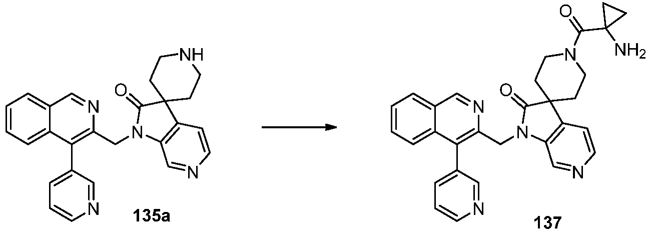

### 1-(methylsulfonyl)-1'-((4-(pyridin-3-yl)isoquinolin-3-yl)methyl)spiro[piperidine-4,3'-pyrrolo[2,3-c]pyridin]-2'(1'H)-one (136)

Compound 135a (125 mg) was acylated with 1-((tert-butoxycarbonyl)amino)-cyclopropanecarboxylic acid as described in Example 115 step b. The afforded Boc protected compound was N-deprotected using TFA in DCM, which gave the title compound (40%). MS (ES+) 505.34 [M+H]⁺.
¹H NMR (500 MHz, DMSO-*d*₆) δ 9.32 (s, 1H), 8.76 (dd, *J* = 4.9, 1.6 Hz, 1H), 8.68 (d, *J* = 2.1 Hz, 1H), 8.26 (d, *J =* 4.7 Hz, 1H), 8.18 (s, 1H), 7.98 (dt, *J* = 7.8, 2.0 Hz, 1H), 7.74 (dddd, *J* = 19.8, 8.0, 6.9, 1.3 Hz, 2H), 7.65 (dd, *J* = 7.8, 4.9 Hz, 1H), 7.58 (d, *J* = 4.7 Hz, 1H), 7.36 - 7.30 (m, 1H), 5.00 (s, 1H), 4.97 (s, 1H), 3.92 (s, 2H), 3.33 (s, 7H), 2.55 (s, 1H), 2.30 (s, 2H), 1.73 (dd, *J* = 27.9, 12.5 Hz, 3H), 0.88 (q, *J=* 4.4 Hz, 1H), 0.76 - 0.63 (m, 2H).

### Example 138

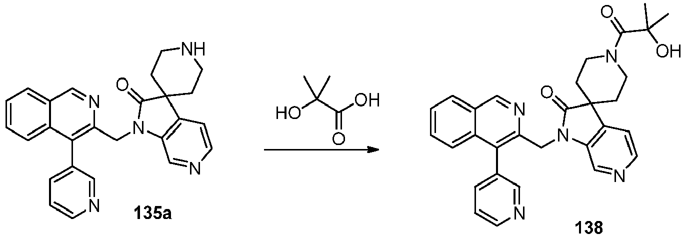

### 1-(2-hydroxy-2-methylpropanoyl)-1'-((4-(pyridin-3-yl)isoquinolin-3-yl)methyl)spiro[piperidine-4,3'-pyrrolo[2,3-c]pyridin]-2'(1'H)-one (138)

Compound 135a was acylated with 2-hydroxyisobutyric acid (23.0 mg, 0.22 mmol) using the method described in Example 115 step b, which gave the title compound (51%). MS (ES+) 508.4 [M+H]⁺.
¹H NMR (500 MHz, DMSO-*d*₆) δ 9.32 (s, 1H), 8.76 (dd, *J* = 4.9, 1.6 Hz, 1H), 8.68 (d, *J* = 2.2 Hz, 1H), 8.27 (d, *J* = 4.7 Hz, 1H), 8.21 - 8.15 (m, 2H), 7.98 (dt, *J* = 7.8, 2.0 Hz, 1H), 7.74 (dddd, *J* = 19.8, 8.0, 6.9, 1.3 Hz, 2H), 7.65 (ddd, *J* = 7.8, 4.9, 0.9 Hz, 1H), 7.58 (d, *J* = 4.8 Hz, 1H), 7.36 - 7.30 (m, 1H), 5.47 (s, 1H), 5.00 (s, 1H), 4.96 (s, 1H), 4.21 (s, 2H), 3.33 (s, 3H), 1.72 (s, 4H), 1.36 (s, 6H).

### Example 139

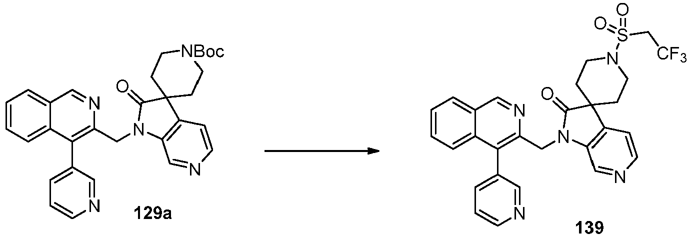

### 1'-((4-(pyridin-3-yl)isoquinolin-3-yl)methyl)-1-((2,2,2-trifluoroethyl)sulfonyl)spiro[piperidine-4,3'-pyrrolo[2,3-c]pyridin]-2'(1'H)-one (139)

Compound 129a (129 mg, 0.247 mmol) was N-deprotected by treatment with HCl in DCM/dioxane. The afforded amine and pyridine (10 eq) were dissolved in dry DCM (3 mL), cooled to 0 °C and 2,2,2-trifluoroethanesulfonyl chloride (5 eq) was added. After 10 min at 0 °C, the reaction was allowed to attain rt and then heated to 35 °C for 72h. The solution was concentrated in vacuo, the crude dissolved in acetonitrile, passed through a filter and then purified by Prep C18 LCMS at neutral pH. Pure fractions were pooled and freeze-dried then dissolved in DCM and purified further by silica chromatography elution with a gradient of DCM:MeOH. Pure fractions containing were pooled, concentrated and lyophilized (water/acetonitrile) which gave the title compound (49.8 mg, 36%) as a solid. MS (ES+) 568.22 [M+H]+.
¹H NMR (500 MHz, DMSO-*d*₆) δ 9.31 (s, 1H), 8.76 (dd, *J* = 4.9, 1.6 Hz, 1H), 8.68 (d, *J* = 2.2 Hz, 1H), 8.29 (d, *J* = 4.7 Hz, 1H), 8.21 - 8.15 (m, 2H), 7.98 (dt, *J* = 7.8, 2.0 Hz, 1H), 7.74 (dddd, *J* = 20.5, 8.0, 6.9, 1.3 Hz, 2H), 7.65 (ddd, *J* = 7.8, 4.9, 0.8 Hz, 1H), 7.59 (d, *J* = 4.8 Hz, 1H), 7.37 - 7.31 (m, 1H), 4.99 (s, 1H), 4.96 (s, 1H), 4.61 (q, *J* = 10.1 Hz, 2H), 3.61 (ddd, *J* = 12.7, 9.0, 3.6 Hz, 2H), 3.52 (dt, *J* = 12.7, 5.0 Hz, 2H), 1.91 (ddd, *J* = 13.3, 8.9, 4.2 Hz, 2H), 1.81 (dp, *J* = 9.6, 3.7 Hz, 2H).

### Example 140

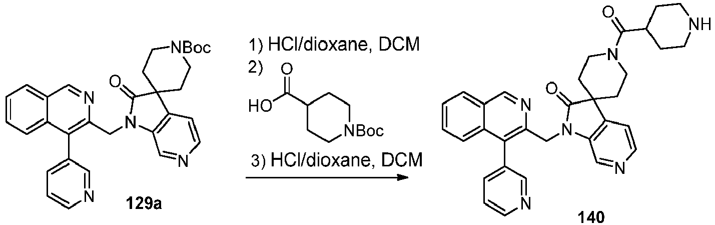

### 1-(piperidine-4-carbonyl)-1'-((4-(pyridin-3-yl)isoquinolin-3-yl)methyl)spiro[piperidine-4,3'-pyrrolo[2,3-c]pyridin]-2'(1'H)-one (140)

Compound 129a (129 mg, 0.247 mmol) was N-deprotected by treatment with HCl in DCM/dioxane. 126 mg (0.237 mmol) of the afforded amine was reacted with 1-(tert-butoxycarbonyl)piperidine-4-carboxylic acid (54 mg, 0.237 mmol) using the method described in Example 115 step b. Removal of the Boc group by treatment with DCM:HCl (4M in dioxane) 1:1 for 30 min at rt followed by concentration in vacuo and purification by Prep C18 LCMS using basic pH, gave the title compound (8.8 mg, 7.2 %). MS (ES+) 533.32 [M+H]⁺.
¹H NMR (500 MHz, DMSO-*d*₆) δ 9.32 (s, 1H), 8.76 (dd, *J* = 4.9, 1.6 Hz, 1H), 8.70 - 8.64 (m, 1H), 8.26 (d, *J* = *4.8* Hz, 1H), 8.21 - 8.15 (m, 1H), 8.18 (s, 1H), 8.01 - 7.94 (m, 1H), 7.74 (dddd, *J* = 19.6, 8.0, 6.9, 1.3 Hz, 2H), 7.65 (dd, *J* = 7.8, 4.9 Hz, 1H), 7.60 (d, *J* = 4.8 Hz, 1H), 7.33 (d, *J* = 8.3 Hz, 1H), 6.29 (s, 1H), 4.98 (s, 1H), 3.86 - 3.80 (m, 2H), 3.68 (s, 1H), 2.96 - 2.88 (m, 2H), 2.76-2.66 (m, 1H), 2.60 (s, 5H), 1.79-1.71 (m, 2H), 1.68 (s, 2H), 1.66 (dd, *J* = 14.7, 9.2 Hz, 1H), 1.57 - 1.50 (m, 4H), 1.46 (dd, *J* = 13.0, 9.2 Hz, 1H), 1.25 (d, *J* = 13.5 Hz, 1H).

### Example 141

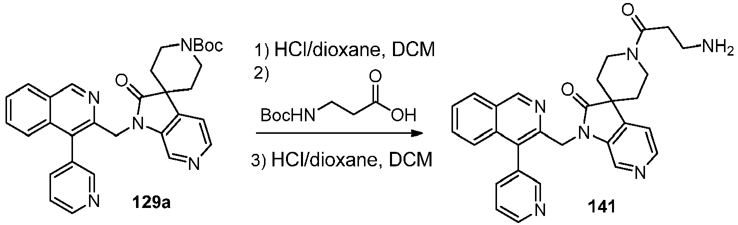

### 1-(3-aminopropanoyl)-1'-((4-(pyridin-3-yl)isoquinolin-3-yl)methyl)spiro[piperidine-4,3'-pyrrolo[2,3-c]pyridin]-2'(1'H)-one (141)

Compound 129a (0.19 mmol) was N-deprotected by treatment with HCl in DCM/dioxane. The afforded amine, 3-((tert-butoxycarbonyl)amino)propanoic acid (40 mg, 0,21 mmol) and HATU (85 mg, 0,22 mmol) were suspended in DMF (3 mL) and DIEA (0,17 ml, 0.98 mmol) was added. The mixture was stirred at rt for 2 h, the then diluted with EtOAc, washed with aq.NaHCO₃, aq. citric acid and brine. The organic phase was dried over MgSO₄, filtered and concentrated. The crude product was purified by column chromatography on silica eluting with 1-10 % MeOH in DCM. Pure fractions were pooled and concentrated. The residue was dissolved in dioxane (5 mL), conc. aq. HCl was added (0,8 ml). added and the mixture was stirred at rt for 1h, then concentrated, co-evaporated with toluene and dried in vacuum. The crude compound was purified by LC-MS, which gave the title compound (33 mg, 34 %). MS (ES+) 493.2 [M+H]⁺.
¹H NMR (500 MHz, DMSO-*d*₆) δ 9.32 (s, 1H), 8.76 (dd, *J* = 4.9, 1.6 Hz, 1H), 8.68 (s, 1H), 8.26 (d, *J= 4.8* Hz, 1H), 8.18 (s, 2H), 7.98 (ddt, *J* = 8.2, 4.2, 1.9 Hz, 1H), 7.74 (dddd, *J* = 19.7, 8.0, 6.9, 1.3 Hz, 2H), 7.68 - 7.61 (m, 1H), 7.59 (d, *J* = 4.8 Hz, 1H), 7.36 - 7.30 (m, 1H), 4.98 (qd, *J* = 16.0, 9.8 Hz, 2H), 3.91 - 3.83 (m, 1H), 3.69 (dd, *J* = 14.5, 8.3 Hz, 2H), 2.82 (t, *J* = 6.5 Hz, 2H), 2.53 (d, *J* = 6.4 Hz, 1H), 1.83 (s, 5H), 1.79 (dd, *J* = 8.2, 3.9 Hz, 1H), 1.79 - 1.60 (m, 2H).

### Example 142

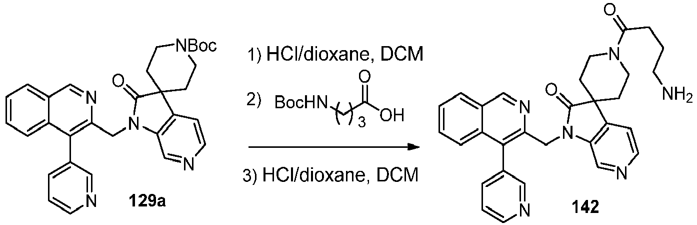

### 1-(4-aminobutanoyl)-1'-((4-(pyridin-3-yl)isoquinolin-3-yl)methyl)spiro[piperidine-4,3'-pyrrolo[2,3-c]pyridin]-2'(1'H)-one (142)

The title compound was prepared as described in example 141 but using the acid 4-((tert-butoxycarbonyl)amino)butanoic acid. Yield 31%. MS (ES+) 607.45 [M+H]⁺.
¹H NMR (500 MHz, DMSO-*d*₆) δ 9.32 (s, 1H), 8.76 (dd, *J* = 4.9, 1.6 Hz, 1H), 8.67 (s, 1H), 8.67 (d, *J* = *4.8* Hz, 0H), 8.26 (d, *J* = *4.8* Hz, 1H), 8.19 (d, *J=* 1.5 Hz, 1H), 8.18 (s, 2H), 7.98 (ddd, *J* = 7.6, 4.8, 2.2 Hz, 1H), 7.74 (dddd, *J* = 19.7, 8.0, 6.9, 1.3 Hz, 2H), 7.68 - 7.61 (m, 1H), 7.59 (d, *J* = 4.8 Hz, 1H), 7.36 - 7.30 (m, 1H), 4.98 (qd, *J* = 16.0, 11.0 Hz, 2H), 3.86 (t, *J* = 9.2 Hz, 1H), 3.69 (ddt, *J* = 17.0, 8.7, 4.5 Hz, 2H), 2.67-2.60 (m, 2H), 2.42 (hept, *J* = 7.8 Hz, 2H), 1.80 (s, 4H), 1.84 - 1.72 (m, OH), 1.66 (p, *J* = 7.3 Hz, 3H).

### Example 143

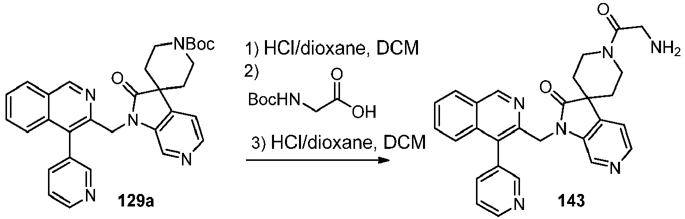

### 1-(2-aminoacetyl)-1'-((4-(pyridin-3-yl)isoquinolin-3-yl)methyl)spiro[piperidine-4,3'-pyrrolo[2,3-c]pyridin]-2'(1'H)-one (143)

The title compound was prepared as described in example 141 but using the acid 2-((tert-butoxycarbonyl)amino)acetic acid. Yield 27%. MS (ES+) 579.45 [M+H]⁺.
¹H NMR (500 MHz, DMSO-*d*₆) δ 9.32 (s, 1H), 8.76 (dd, *J* = 4.9, 1.7 Hz, 1H), 8.68 (s, 1H), 8.68 (d, *J=* 4.8 Hz, 0H), 8.26 (d, *J* = 4.7 Hz, 1H), 8.21 - 8.14 (m, 1H), 8.17 (s, 2H), 7.98 (ddt, *J* = 7.6, 3.9, 1.9 Hz, 1H), 7.74 (dddd, *J* = 19.7, 8.0, 6.9, 1.3 Hz, 3H), 7.65 (ddd, *J* = 7.8, 4.9, 0.9 Hz, 1H), 7.57 (d, *J* = 4.8 Hz, 1H), 7.36 - 7.30 (m, 1H), 5.05 - 4.92 (m, 2H), 3.93 - 3.83 (m, 1H), 3.87 (s, 1H), 3.74-3.64 (m, 3H), 3.60 (d, *J* = 14.1 Hz, 1H), 3.40 (s, 1H), 1.84-1.61 (m, 3H).

### Example 144

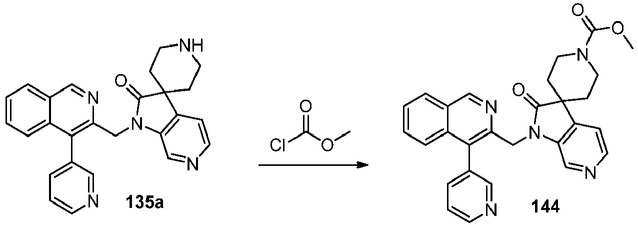

### Methyl 2'-oxo-1'-((4-(pyridin-3-yl)isoquinolin-3-yl)methyl)-1',2'-dihydrospiro[piperidine-4,3'-pyrrolo[2,3-c]pyridine]-1-carboxylate (144)

Triethylamine (0.1 mL) and methyl chloroformate (0.05 mL) were added at 0 °C to a stirred solution of compound 135a (80 mg, 0.190 mmol) in DCM (10 mL). The mixture was stirred at 0 °C for 1 h, then poured into ice water (20 mL) and extracted with DCM (2 x 20 mL). The combined organic layers were dried with anhydrous sodium sulfate, filtered and concentrated under vacuo. The crude product was purified by C18 prep. HPLC which gave the title compound (50 mg, 55%) as a solid. MS (ES+) 480.41 [M+H].
¹H NMR (500 MHz, DMSO-*d*₆) δ 9.32 (d, *J* = 0.8 Hz, 1H), 8.76 (dd, *J* = 4.9, 1.6 Hz, 1H), 8.67 (dd, *J* = 2.2, 0.9 Hz, 1H), 8.26 (d, *J* = 4.8 Hz, 1H), 8.21 - 8.15 (m, 1H), 8.17 (s, 1H), 7.97 (dt, *J* = 7.8, 1.9 Hz, 1H), 7.74 (dddd, *J* = 19.8, 8.0, 6.9, 1.3 Hz, 2H), 7.65 (ddd, *J* = 7.8, 4.9, 0.9 Hz, 1H), 7.60 (dd, *J* = 4.8, 0.8 Hz, 1H), 7.33 (dd, *J* = 8.3, 1.2 Hz, 1H), 4.99 (s, 1H), 4.96 (s, 1H), 3.75 - 3.62 (m, 7H), 1.75 (dt, *J* = 13.1, 6.2 Hz, 2H), 1.66 (dt, *J* = 13.4, 5.0 Hz, 2H).

### Example 145

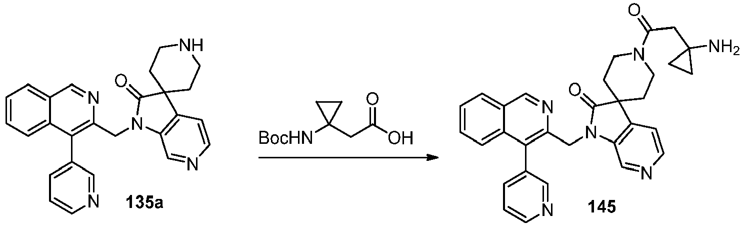

### 1-(2-(1-Aminocyclopropyl)acetyl)-1'-((4-(pyridin-3-yl)isoquinolin-3-yl)methyl)spiro[piperidine-4,3'-pyrrolo[2,3-c]pyridin]-2'(1'H)-one (145)

The title compound was prepared by reaction of the HCl salt of compound 135a (109 mg, 0.238 mmol) with 2-(1-((tert-butoxycarbonyl)amino)cyclopropyl)acetic acid (84.5 mg, 0.393 mol), followed by N-deprotection according to the method described in Example 39 steps a and b. Yield (65 mg, 32%). MS (ES+) 519.4 [M+H].

### Example 146

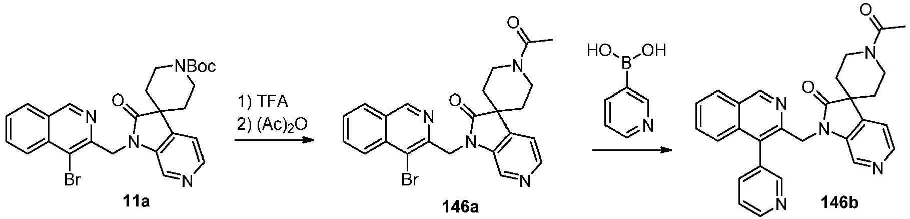

### Step a) 1-Acetyl-1'-((4-bromoisoquinolin-3-yl)methyl)spiro[piperidine-4,3'-pyrrolo[2,3-c]pyridin]-2'(1'H)-one (146a)

To a solution of 11a (3 g) in DCM (30 ml) was added TFA (22 mL), the mixture was stirred at rt for 3 h, then concentrated. The resulting oil was basified with the saturated sodium bicarbonate solution (30 mL), then extracted with DCM (2 x 50 mL). The combined organic layers were dried over sodium sulfate, filtered and concentrated to dryness afford desired product as a pale brown solid (2.4 g, 93.68%). 900 mg of the afforded free amine was dissolved in DCM (15 mL)_and triethyl amine (0.9 mL) was added followed by acetic anhydride (0.3 mL) at 0 °C. The mixture was stirred at rt for 3 h, then diluted with water (30 mL), the aqueous layer was extracted with DCM (2 x 20 mL). The organic layer was dried over sodium sulfate, filtered and concentrated which gave the title compound (750 mg, 70%) as a solid. MS (ES+) 467.02 [M+H].

### Step b) 1-Acetyl-1'-((4-(pyridin-3-yl)isoquinolin-3-yl)methyl)spiro[piperidine-4,3'-pyrrolo[2,3-c]pyridin]-2'(1'H)-one (146b)

To a suspension of compound 146a (0.4 g), pyridin-3-ylboronic acid (0.211 g) in 1,2-dimethoxyethane (10 mL) and water (1 mL) was added sodium carbonate(0.273 g) and [1,1'-bis(diphenylphosphino)ferrocene]palladium(II) chloride (0.126 g), the whole mixture was degassed for 20 min. The resulting mixture was stirred under microwave irradiation at 100 °C for 2h, then filtered through Celite. The filtrate was concentrated and the obtained crude material was combined with another badge (0.42 g) and purified column chromatography on silica eluted with 0 - 5% MeOH in DCM. Pure fractions were pooled, concentrated and 100 mg was further purified by prep. C18 HPLC which gave 60 mg of the title compound. MS (ES+) 465.04 [M+H]. ¹H NMR (500 MHz, DMSO-*d*₆) δ 9.32 (s, 1H), 8.76 (dd, *J* = 4.9, 1.6 Hz, 1H), 8.68 (s, 1H), 8.26 (d, *J* = 4.7 Hz, 1H), 8.21 - 8.15 (m, 1H), 8.17 (s, 1H), 8.02 - 7.94 (m, 1H), 7.74 (dddd, *J* = 19.8, 8.0, 6.9, 1.3 Hz, 2H), 7.65 (ddd, *J* = 7.7, 4.9, 0.8 Hz, 1H), 7.58 (d, *J* = 4.8 Hz, 1H), 7.37 - 7.30 (m, 1H), 4.98 (qd, *J* = 16.0, 10.6 Hz, 2H), 3.91 - 3.81 (m, 1H), 3.77 (ddd, *J* = 12.9, 8.6, 3.7 Hz, 1H), 3.72 - 3.59 (m, 1H), 3.66 (s, 1H), 2.06 (s, 3H), 1.86 - 1.59 (m, 3H).

### Example 147

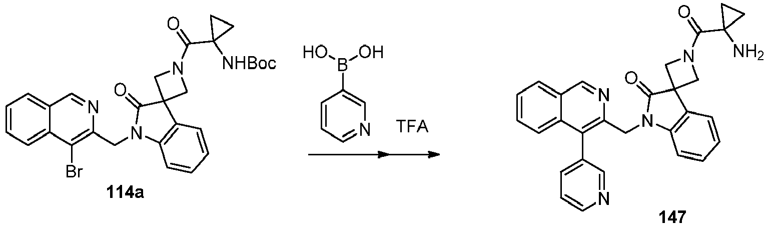

### 1-(1-aminocyclopropanecarbonyl)-1'-((4-(pyridin-3-yl)isoquinolin-3-yl)methyl)spiro[azetidine-3,3'-indolin]-2'-one (147)

Compound 114a (400 mg, 0.693 mmol) was reacted with pyridin-3-ylboronic acid (128 mg, 1.04 mmol) using the method described in Example 146 step b, followed by N-deprotection by treatment with TFA in DCM, which gave the title compound (63 mg, 19%). MS (ES+) 476.6
[M+H].
¹H NMR (500 MHz, DMSO-*d*₆) δ 9.34 (s, 1H), 8.72 (dd, *J* = 4.9, 1.6 Hz, 1H), 8.56 (s, 1H), 8.19 (dd, *J* = 7.4, 1.8 Hz, 1H), 7.86 (d, *J* = 7.8 Hz, 1H), 7.78 - 7.67 (m, 2H), 7.59 (t, *J* = 6.4 Hz, 2H), 7.27 (d, *J* = 8.2 Hz, 1H), 7.21 -7.13 (m, 1H), 7.07 (t, *J* = 7.5 Hz, 1H), 6.71 (d, *J* = 7.8 Hz, 1H), 5.03 - 4.91 (m, 2H), 4.72 (s, 2H), 4.00 (s, 2H), 3.96 (s, 0H), 3.35 (s, 1H), 2.26 (s, 2H), 1.12 (s, 1H), 1.08 (s, 1H), 0.72 (s, 2H).

### Example 148

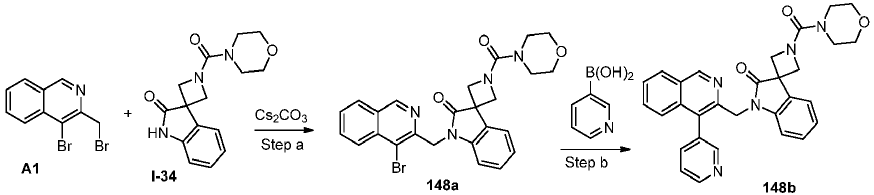

### Step a) 1'-((4-Bromoisoquinolin-3-yl)methyl)-1-(morpholine-4-carbonyl)spiro[azetidine-3,3'-indolin]-2'-one (148a)

Compound A1 (400 mg, 1.33 mmol) was reacted with I-34 (382 mg, 1.33 mmol) using the method described in Example 79 but at rt instead of at 70 °C, which gave the title compound (400 mg, 54%). MS (ES+) 509.1 [M+H].

### Step b) 1-(morpholine-4-carbonyl)-1'-((4-(pyridin-3-yl)isoquinolin-3-yl)methyl)spiro[azetidine-3,3'-indolin]-2'-one (148b)

Compound 148a (380 mg, 0.749 mmol) was reacted with pyridin-3-ylboronic acid (184 mg, 1.50 mmol) according to the method described in Example 22 step a, with the exception that the heating was effected by microwave irradiation and the reaction time was 1h, which gave the title compound (95 mg, 25%). MS (ES+) 506.5 [M+H].
¹H NMR (500 MHz, DMSO-*d*₆) δ 9.33 (s, 1H), 8.71 (dd, *J* = 4.8, 1.6 Hz, 1H), 8.54 (d, *J* = 2.2 Hz, 1H), 8.22 - 8.16 (m, 1H), 7.84 (dt, *J* = 7.8, 2.0 Hz, 1H), 7.78 - 7.68 (m, 2H), 7.62 - 7.52 (m, 2H), 7.27 (d, *J* = 8.0 Hz, 1H), 7.17 (t, *J* = 7.7 Hz, 1H), 7.06 (t, *J* = 7.5 Hz, 1H), 6.70 (d, *J* = 7.8 Hz, 1H), 5.02-4.90 (m, 2H), 4.09 (d, *J* = 8.0 Hz, 2H), 4.00 (dd, *J* = 8.0, 4.9 Hz, 2H), 3.56 (t, *J*= 4.7 Hz, 4H), 3.40 - 3.33 (m, 0H), 3.28 (t, *J* = 4.8 Hz, 4H).

### Example 149

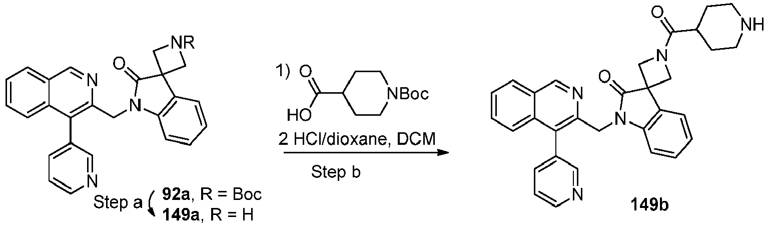

### Step a) 1'-((4-(pyridin-3-yl)isoquinolin-3-yl)methyl)spiro[azetidine-3,3'-indolin]-2'-one (149a)

Compound 92a (1.42 g, 2.88 mmol) was N-deprotected by treatment with TFA in DCM. TFA (11 ml, 144 mmol) was added dropwise, to a solution of compound 92a (1.42 g, 2.88 mmol) in DCM (25 ml). The mixture was stirred for 1 h, then concentrated and co-evaporated with toluene. The crude was dried at reduced pressure for 2 h which gave the title compound.

### Step b) 1-(Piperidine-4-carbonyl)-1'-((4-(pyridin-3-yl)isoquinolin-3-yl)methyl)spiro[azetidine-3,3'-indolin]-2'-one (149b)

Compound 149a (100 mg) was acylated with 1-(tert-butoxycarbonyl)piperidine-4-carboxylic acid (45.3 mg, 0.197 mmol) using the method described in Example 115 step b. The Boc group was removed by treatment with DCM:HCl (4M in dioxane) 1:1 for 30 min at rt followed by concentration in vacuo and purification by Prep C18 LCMS using basic pH, gave the title compound (75 %). MS (ES+) 504.28 [M+H]⁺.
¹H NMR (500 MHz, DMSO-*d₆*) δ 9.34 (s, 1H), 8.72 (dd, *J* = 4.9, 1.6 Hz, 1H), 8.55 (dd, *J* = 10.9, 2.2 Hz, 1H), 8.29 (s, 1H), 8.19 (dd, *J* = 7.4, 1.9 Hz, 1H), 7.86 (ddt, *J* = 10.1, 7.7, 2.0 Hz, 1H), 7.79 - 7.68 (m, 2H), 7.64 - 7.56 (m, 2H), 7.34 - 7.24 (m, 1H), 7.18 (td, *J* = 7.8, 1.3 Hz, 1H), 7.05 (t, *J* = 7.5 Hz, 1H), 6.74 (dd, *J* = 7.9, 1.8 Hz, 1H), 6.29 (s, 0H), 5.04 - 4.89 (m, 2H), 4.37 - 4.26 (m, 2H), 3.98 (d, *J=* 7.0 Hz, 1H), 3.97 (s, 1H), 2.93 (d, *J* = 12.3 Hz, 2H), 2.66 (s, 1H), 2.55 (s, 1H), 2.46 (d, *J* = 13.2 Hz, 2H), 2.40 - 2.30 (m, 1H), 1.57 (dd, *J* = 27.8, 12.6 Hz, 2H), 1.42 (dqt, *J* = 19.2, 14.0, 6.9 Hz, 2H), 1.25 (d, *J* = 13.4 Hz, 0H).

### Example 150

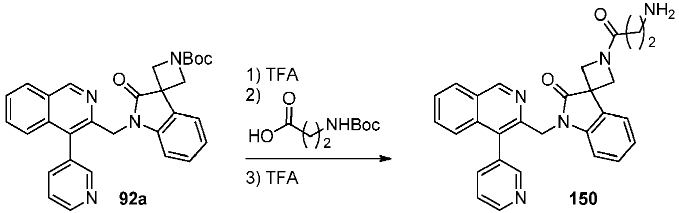

### 1-(3-Aminopropanoyl)-1'-((4-(pyridin-3-yl)isoquinolin-3-yl)methyl)spiro[azetidine-3,3'-indolin]-2'-one (150)

The title compound was prepared from compound 92a as described in example 141 but using TFA for Boc removal. Yield 38%. MS (ES+) 464.2 [M+H]⁺.
¹H NMR (500 MHz, DMSO-*d₆*) δ 9.34 (s, 1H), 8.72 (dd, *J* = 4.9, 1.6 Hz, 1H), 8.54 (dd, *J* = 8.1, 2.5 Hz, 1H), 8.19 (dd, *J* = 7.5, 2.0 Hz, 1H), 7.85 (ddd, *J* = 9.4, 4.7, 2.0 Hz, 1H), 7.72 (dddd, *J* = 14.2, 8.9, 5.2, 1.8 Hz, 2H), 7.66 - 7.56 (m, 2H), 7.27 (d, *J* = 7.8 Hz, 1H), 7.17 (tt, *J* = 7.6, 1.9 Hz, 1H), 7.05 (td, *J* = 7.5, 3.6 Hz, 1H), 6.72 (t, *J* = 7.0 Hz, 1H), 5.04 - 4.90 (m, 2H), 4.35 - 4.21 (m, 2H), 3.98 (d, *J* = 4.3 Hz, 2H), 3.17 (q, *J* = 6.6 Hz, 1H), 2.77 (t, *J* = 6.5 Hz, 1H), 2.29 - 2.14 (m, 2H).

### Example 151

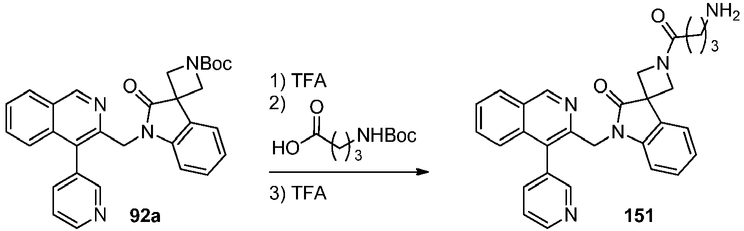

### 1-(4-Aminobutanoyl)-1'-((4-(pyridin-3-yl)isoquinolin-3-yl)methyl)spiro[azetidine-3,3'-indolin]-2'-one (151)

The title compound was prepared from compound 92a as described in example 142 but using TFA for Boc removal. Yield 33%. MS (ES+) 478.2 [M+H]⁺.
¹H NMR (500 MHz, DMSO-*d*₆) δ 9.34 (s, 1H), 8.72 (dd, *J* = 4.9, 1.6 Hz, 1H), 8.54 (dd, *J* = 7.6, 2.4 Hz, 1H), 8.22 - 8.16 (m, 1H), 7.89 - 7.81 (m, 1H), 7.78 - 7.66 (m, 2H), 7.60 (dq, *J* = 8.3, 4.0 Hz, 2H), 7.27 (d, *J* = 8.1 Hz, 1H), 7.21 - 7.13 (m, 1H), 7.05 (td, *J* = 7.6, 2.6 Hz, 1H), 6.72 (d, *J* = 7.7 Hz, 1H), 5.04 - 4.89 (m, 2H), 4.33 - 4.21 (m, 2H), 3.97 (s, 2H), 2.95 (q, *J* = 6.4 Hz, 1H), 2.56 (t, *J* = 6.8 Hz, 1H), 2.21 - 2.03 (m, 2H), 1.69 - 1.54 (m, 2H).

### Example 152

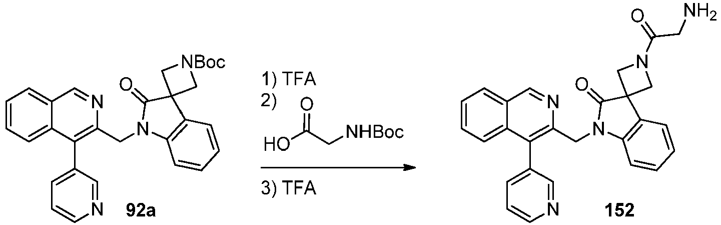

### 1-(2-Aminoacetyl)-1'-((4-(pyridin-3-yl)isoquinolin-3-yl)methyl)spiro[azetidine-3,3'-indolin]1-2'-one (152)

The title compound was prepared from compound 92a as described in example 143 but using TFA for Boc removal. Yield 38%. MS (ES+) 450.2 [M+H]⁺.
¹H NMR (500 MHz, DMSO-*d*₆) δ 9.36 - 9.32 (m, 1H), 8.72 (dd, *J* = 4.9, 1.6 Hz, 1H), 8.54 (dd, *J* = 7.5, 2.2 Hz, 1H), 8.22 - 8.16 (m, 1H), 7.86 (tt, *J* = 7.6, 2.0 Hz, 1H), 7.78 - 7.68 (m, 2H), 7.60 (dt, *J* = 8.0, 3.0 Hz, 2H), 7.30 - 7.24 (m, 1H), 7.18 (td, *J* = 7.8, 1.3 Hz, 1H), 7.06 (td, *J* = 7.6, 1.0 Hz, 1H), 6.75 - 6.67 (m, 1H), 5.03 - 4.89 (m, 2H), 4.35 - 4.23 (m, 2H), 4.01 (s, 2H), 3.19 (s, 2H).

### Example 153

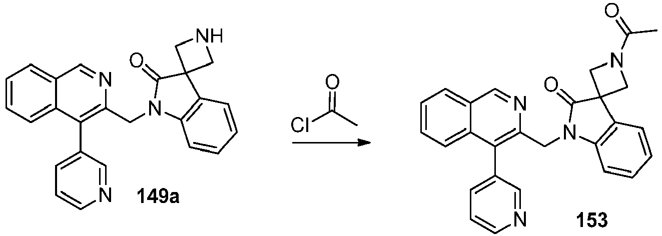

### 1-acetyl-1'-((4-(pyridin-3-yl)isoquinolin-3-yl)methyl)spiro[azetidine-3,3'-indolin]-2'-one (153)

Triethylamine (0.5 mL) was slowly added at 0 °C to a solution of compound 149a (180 mg) in DCM (5 mL). The resulting solution was stirred under nitrogen for 5 minutes, then acetyl chloride (0.06 mg) was added and the solution was stirred for 9 h. The reaction mixture was concentrated under reduced pressure and the afforded oil was diluted with water (20 mL) and extracted with 5% methanol in DCM (2 x 30 mL). The combined organic layers were washed with brine (15 mL), dried over Na₂SO₄, filtered and concentrated under reduced. The crude compound was purified by prep C18 HPLC. Pure fractions were pooled, concentrated to minimum volume and then freeze dried, which gave the title compound as a solid (56 mg, 31%). MS (ES+) 435.17 [M+H]+.
¹H NMR (500 MHz, DMSO-*d*₆) δ 9.37 - 9.32 (m, 1H), 8.72 (dd, J= 4.9, 1.6 Hz, 1H), 8.55 (dd, *J* = 9.9, 2.2 Hz, 1H), 8.22 - 8.16 (m, 1H), 7.86 (ddt, *J* = 9.6, 7.7, 2.0 Hz, 1H), 7.78 - 7.67 (m, 2H), 7.64 - 7.55 (m, 2H), 7.30 - 7.24 (m, 1H), 7.18 (td, J= 7.8, 1.3 Hz, 1H), 7.06 (td, *J* = 7.6, 1.0 Hz, 1H), 6.72 (d, *J* = 7.9 Hz, 1H), 5.04 - 4.89 (m, 2H), 4.31 (dd, *J* **=** 8.3, 3.4 Hz, 1H), 4.25 (d, *J* = 8.1 Hz, 1H), 3.97 (s, 2H), 1.85 (s, 3H).

### Example 154

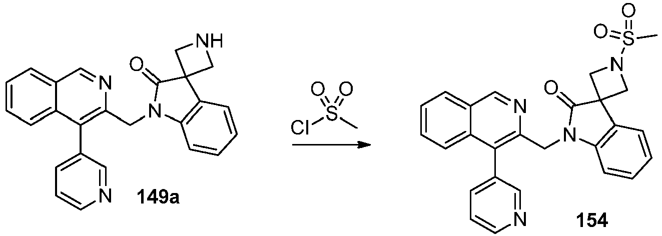

### 1-(Methylsulfonyl)-1'-((4-(pyridin-3-yl)isoquinolin-3-yl)methyl)spiro[azetidine-3,3'-indolin]-2'-one (154)

Compound 149a (180 mg, 0.393 mmol) was sulfonylated with methanesulfonyl chloride using the method described in Example 134 step b, which gave the title compound (42 mg, 22%). MS (ES+) 471.13 [M+H]+.
¹H NMR (500 MHz, DMSO-*d*₆) δ 9.34 (s, 1H), 8.82 (s, 1H), 8.72 (d, *J* = 4.8 Hz, 1H), 8.55 (s, 1H), 8.19 (d, *J* = 7.7 Hz, 1H), 7.93 (s, 0H), 7.87 (d, *J* = 7.7 Hz, 1H), 7.73 (p, *J* = 6.9 Hz, 2H), 7.63 - 7.57 (m, 2H), 7.27 (d, *J* = 8.1 Hz, 1H), 7.20 (t, *J* = 7.8 Hz, 1H), 7.09 (t, *J* = 7.4 Hz, 1H), 6.76 (d, *J* = 7.9 Hz, 1H), 4.98 (s, 1H), 4.95 (s, 1H), 4.07 (s, 4H), 3.40 (s, 1H), 3.17 (s, 3H).

### Example 155

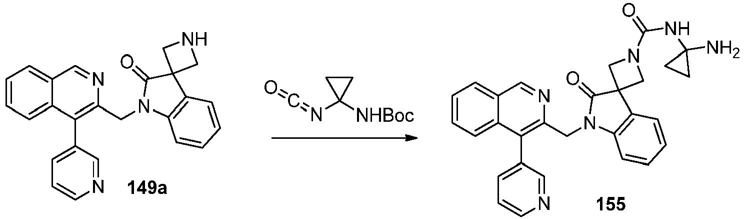

### N-(1-aminocyclopropyl)-2'-oxo-1'-((4-(pyridin-3-yl)isoquinolin-3-yl)methyl)spiro[azetidine-3,3'-indolinel-1-carboxamide (155)

Triethylamine (0.8 mL) and compound 149a (600 mg) were added under nitrogen at 0 °C to a solution of tert-butyl (1-isocyanatocyclopropyl)carbamate (450 mg) in toluene (20 mL). The mixture was stirred at rt for 16 h, then diluted with water (100 mL), extracted with EtOAc (2x200 mL). The combined organic layers were dried over sodium sulfate, filtered and concentrated. The afforded crude was dissolved in DCM (100 mL), TFA (0.8 mL) was added at rt under nitrogen and the solution was stirred for 2h, then concentrated. Saturated sodium bicarbonate solution (100 mL) was added and the mixture was extracted with DCM (2 x 100 mL). The combined organic layers were washed with water (100 mL), dried over sodium sulfate, filtered and concentrated under reduced pressure. The resulting residue was purified by prep C18 HPLC, which gave the title compound as a solid (96 mg, 60%). MS (ES+) 491.2 [M+H]⁺.
¹H NMR (500 MHz, DMSO-*d*₆) δ 9.34 (s, 1H), 8.71 (dd, *J* = 4.9, 1.6 Hz, 1H), 8.52 (d, *J* = 2.2 Hz, 1H), 8.19 (dd, *J* = 7.4, 2.0 Hz, 1H), 7.83 (dt, *J* = 7.8, 2.0 Hz, 1H), 7.78 (s, 1H), 7.78 - 7.67 (m, 2H), 7.58 (dd, *J* = 7.8, 4.9 Hz, 1H), 7.51 (dd, *J* = 7.4, 1.2 Hz, 1H), 7.31 - 7.22 (m, 2H), 7.16 (td, *J* = 7.7, 1.2 Hz, 1H), 7.04 (t, *J* = 7.6 Hz, 1H), 6.68 (d, *J* = 7.8 Hz, 1H), 5.02 - 4.93 (m, 2H), 3.95 (d, *J* = 7.9 Hz, 2H), 3.85 (dd, *J* = 7.9, 2.4 Hz, 2H), 3.38 - 3.32 (m, 1H), 2.56 - 2.52 (m, 2H), 0.71 - 0.65 (m, 3H).

### Example 156

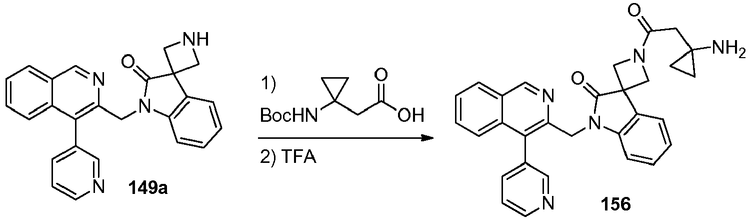

### 1-(2-(1-aminocyclopropyl)acetyl)-1'-((4-(pyridin-3-yl)isoauinolin-3-yl)methyl)spiro[azetidine-3,3'-indolin]-2'-one (156)

The TFA salt of compound 149 a (151 mg, 0.298 mmol) was reacted with 2-(1-((tert-butoxycarbonyl)amino)cyclopropyl)acetic acid (122 mg, 0.556 mmol) using the method described in Example 145, whereafter the N-protecting group was removed by treatment with TFA in DCM, which gave the title compound (58 mg, 40%). MS (ES+) 490.4 [M+H]⁺.
¹H NMR (500 MHz, DMSO-*d*₆) δ 9.34 (s, 1H), 8.72 (dd, *J* = 4.8, 1.6 Hz, 1H), 8.58 - 8.51 (m, 1H), 8.22 - 8.16 (m, 1H), 7.85 (ddt, *J* = 8.1, 4.1, 2.0 Hz, 1H), 7.78 - 7.68 (m, 2H), 7.63 - 7.56 (m, 2H), 7.27 (dd, *J* = 8.1, 1.5 Hz, 1H), 7.18 (td, *J* = 7.7, 1.2 Hz, 1H), 7.05 (td, *J* =7.5, 1.0 Hz, 1H), 6.72 (d, *J* = 7.8 Hz, 1H), 5.03 - 4.90 (m, 2H), 4.34 - 4.23 (m, 2H), 4.00 (s, 1H), 4.00 (d, *J*= 14.9 Hz, 1H), 2.30 (s, 1H), 2.26 (s, 1H), 0.50 - 0.41 (m, 4H).

### Example 157

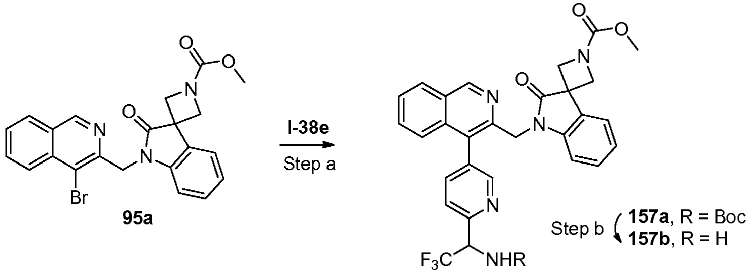

### Step a) Methyl 1'-((4-(6-(1-((tert-butoxycarbonyl)amino)-2,2,2-trifuoroethyl)pyridin-3-yl)isoquinolin-3-yl)methyl)-2'-oxospiro[azetidine-3,3'-indoline]-1-carboxylate (157a)

Compound 95a (92 mg, 0.2 mmol) was reacted with tert-butyl (2,2,2-trifluoro-1-(5-(4,4,5,5-tetramethyl-1,3,2-dioxaborolan-2-yl)pyridin-2-yl)ethyl)carbamate (98 mg, 0.24 mmol) according to the procedure described in Example 12 step a which gave the title compound (11.6 mg, 9%). MS (ES+) 648.3 [M+H]⁺.

### Step b) methyl 1'-((4-(6-(1-amino-2,2,2-trifluoroethyl)pyridin-3-yl)isoquinolin-3-yl)methyl)-2'-oxospiro[azetidine-3,3'-indoline]-1-carboxylate (157b)

Compound 157a (11.6 mg, 0.018 mmol) was dissolved in DCM (2 mL) and then treated with TFA (500µL, 3.5 mmol) according to the procedure described for 106c which gave the title compound as bis TFA salt (18.3 mg, 132%). MS (ES+) 548.3 [M+H]⁺.
¹H NMR (500 MHz, Methanol-*d*₄) δ 9.23 (s, 1H), 9.20 (s, 0H), 8.67 (s, 1H), 8.13 - 8.06 (m, 1H), 8.02 - 7.95 (m, 0H), 7.78 (t, *J* = 8.2 Hz, 1H), 7.64 (tdd, *J* = 7.0, 5.6, 5.1, 2.6 Hz, 2H), 7.50 (s, 0H), 7.50 (dd, *J* = 14.0, 1.2 Hz, 1H), 7.18 (t, *J* = 9.0 Hz, 1H), 7.15 - 7.06 (m, 1H), 7.07 - 6.98 (m, 1H), 6.85 (s, 1H), 6.75 (d, *J* = 7.9 Hz, 0H), 5.69 (d, *J* = 7.2 Hz, 1H), 5.64 (q, *J* = 7.1 Hz, 0H), 4.96 (d, *J* = 16.0 Hz, 1H), 4.89 (d, *J* = 16.1 Hz, 0H), 4.16 (q, *J* = 8.5, 7.8 Hz, 2H), 4.09 - 4.03 (m, 3H), 3.63 (d, *J =* 4.4 Hz, 3H), 1.26 - 1.21 (m, 1H).

### Example 158

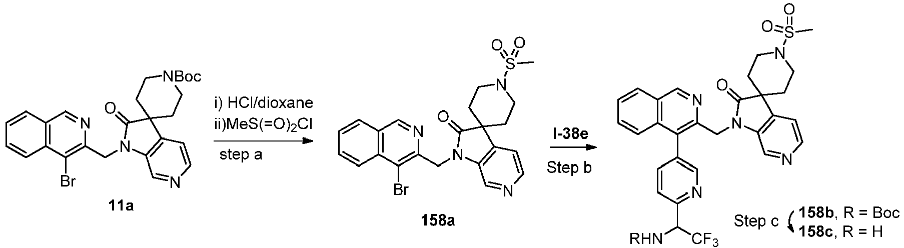

### 1'-((4-bromoisoquinolin-3-yl)methyl)-1-(methylsulfonyl)spiro[piperidine-4,3'-pyrrolo[2,3-c]pyridin]-2'(1'H)-one (158a)

Methanesulfonyl chloride (0.06 ml, 0.77 mmol) was added, dropwise at 0 °C to a suspension of compound 11a (147 mg, 0.32 mmol) and N,N-diisopropylethylamine (0.45 ml, 2.56 mmol) in acetonitrile (2 mL) and the obtained reaction mixture was stirred at 0 °C for 20 min. The reaction was quenched by adding aq. sat NaHCO₃, the product was extracted with DCM, the organic layer was dried over Na₂SO₄ and concentrated at reduced pressure. The product was purified by column chromatography on silica gel, gradient elution with MeOH in DCM. The fractions with the desired compound were combined and the obtained solution was treated with activated charcoal. The solids were filtered off through a pad of Celite and the solution was concentrated at reduced pressure which gave the titled compound (82 mg, 51%). MS (ES+) 501.2; 503.2
[M+H]+.

### tert-Butyl (2,2,2-trifluoro-1-(5-(3-((1-(methylsulfonyl)-2'-oxospiro[piperidine-4,3'-pyrrolo[2,3-c]pyridin]-1'(2'H)-yl)methyl)isoquinolin-4-yl)pyridin-2-yl)ethyl)carbamate (158b)

A mixture of compounds I-38e (98.7 mg, 0,25 mmol), 158a (82 mg, 0,16 mmol), Pd(dppf)Cl₂ (5.98 mg, 0.01 mmol) and 2M aq. potassium carbonate (0.25 mL) in 1,4-dioxane (3 mL) was degassed by passing N₂ gas through for 3-5 min. The mixture was heated in a microwave oven at 130 °C for 1 h, then cooled and filtered through a pad of Celite. The filtrate was concentrated and the product was purified using column chromatography on silica gel (gradient elution with 0-16% MeOH in DCM) and further purified by preparatory HPLC (Phenomenex Gemini-NX 5 µ C18, 110 A, AX, 100 x 30 mm, gradient 13 min, flow 40 ml/min, 50-60% acetonitrile in water with 10 mM ammonium acetate buffer) to give the title compound (15 mg, 13%). MS (ES+) 697.6 [M+H]+.

### 1'-((4-(6-(1-amino-2,2,2-trifluoroethyl)pyridin-3-yl)isoquinolin-3-yl)methyl)-1-(methylsulfonyl)spiro[piperidine-4,3'-pyrrolo[2,3-c]pyridin]-2'(1'H)-one (158c)

Compound 158b (15 mg, 0,02 mmol) was suspended in 1,4-dioxane (0.5 ml). 4M HCl (0.54 ml) was added. Some of methanol was added to obtain a solution and the reaction was stirred for 1 hr. The mixture was concentrated and the crude dried at reduced pressure.

The product was isolated by HPLC (Gemini NX 100x30, gradient 14 minutes, 40-45% acetonitrile in water with 10 mM ammonium acetate buffer) Appropriate fractions were pooled and freeze dried which gave the title compound_(5 mg, 53%), MS (ES+) 597.4 [M+H]⁺.
¹H NMR (500 MHz, DMSO) δ 1.82 (m, 2H, 22', 26'), 1.91 (dtd, J = 15.6, 11.4, 9.9, 6.5 Hz, 2H, 22", 26"), 2.71 (s, 2H, 34), 2.96 (d, J = 2.2 Hz, 3H, 40), 3.43 (ddt, J = 22.6, 11.6, 6.8, 6.8 Hz, 4H, 23, 25), 4.76 (t, J = 8.6, 8.6 Hz, 1H, 33), 4.96 (m, 2H, 7), 6.62 (s, 0H), 7.29 (m, 1H, 21), 7.60 (d, J = 4.8 Hz, 1H, 16), 7.73 (t, J = 7.5, 7.5 Hz, 1H, 19), 7.78 (m, 1H, 20), 7.83 (dd, J = 8.0, 3.8 Hz, 1H, 28), 8.06 (dt, J = 8.0, 2.4, 2.4 Hz, 1H, 27), 8.19 (m, 2H, 13, 18), 8.29 (d, J= 4.8 Hz, 1H, 15), 8.73 (s, 1H, 31), 9.32 (s, 1H, 6). 19F NMR (376 MHz, d2o) δ -71.61 (dd, J = 29.0, 8.6 Hz, 36, 37, 38).

### Example 159

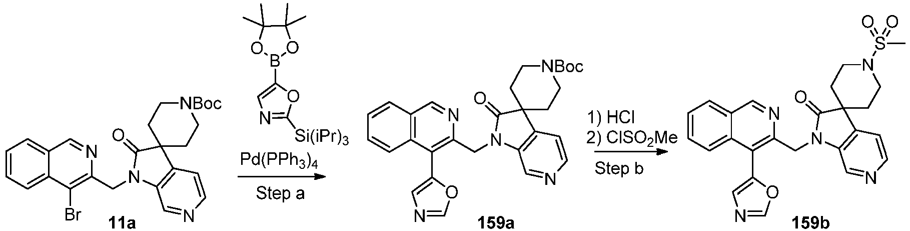

### Step (a) tert-butyl 1'-((4-(oxazol-5-yl)isoquinolin-3-yl)methyl)-2'-oxo-1',2'-dihydrospiro[piperidine-4,3'-pyrrolo[2,3-c]pyridine]-1-carboxylate (159a)

Adapted from literature procedure [N. Primas et al. Tetrahedron 65 (2009), 6348-6353], a mixture of compound 11a (305 mg, 0.58 mmol), 5-(4,4,5,5-tetramethyl-1,3,2-dioxaborolan-2-yl)-2-(triisopropylsilyl)oxazole (277 mg, 0.79 mmol), sodium carbonate (158 mg, 1.48 mmol), and Pd (PPh₃)₄ (55 mg, 0.048 mmol) in dioxane-water 3:1 (9 mL), in a sealed reaction vessel under argon, was heated at 90 to 100 °C. The reaction was monitored by LCMS. After 3.5 h more boronic acid ester (300 mg, 0.85 mmol) in 1.5 mL solvent was added. After 2h, the temperature was increased to 110 to 115 °C. After 3 h more boronic ester (150 mg, 0.43 mmol), Pd catalyst (45 mg, 0.039 mmol), and sodium carbonate (150 mg, 1.42 mmol) were added and heating was continued for 4h until 11a was consumed . At this point the reaction contained a 1/1 mixture of the desired compound and the coupling product with the silyl group still intact.The reaction mixture was diluted with EtOAc (20 mL) and filtered. The filtrate was concentrated under vacuum and the residue was partitioned between 40 mL EtOAc and 40 mL saturated NaCl (aq). The organic phase was washed with 2 x 20 mL NaCl solution, dried (Na₂SO₄), and evaporated *in vacuo.* Purification by silica chromatography with a gradient of MeOH in DCM gave the title compound (212 mg, 70%). MS (ES+) 512.4 [M+H].

### Step (b) 1-(methylsulfonyl)-1'-((4-(oxazol-5-yl)isoquinolin-3-yl)methyl)spiro[piperidine-4,3'-pyrrolo[2,3-c]pyridin]-2'(1'H)-one (159b)

To remove the Boc group, 4M HCl in dioxane (4 mL) and MeOH (2.5 mL) were added to a solution of compound 159a (208 mg, 0.41 mmol) in DCM (4 mL) and stirred for 30 min. The mixture was coevaporated several times with DCM to give the amine HCl salt. Half of the material (0.20 mmol) was stirred for 10-15 min, in an ice bath, with DIEA (0.14 mL, 0.80 mmol) in MeCN (4 mL). A mixture of methanesulfonyl chloride (20 µL, 0.26 mmol) in MeCN (1mL) was added slowly. After 30 min the mixture was concentrated under vacuum and coevaporated several times with DCM. The crude material was redissolved in 30 mL DCM and washed with 15 mL saturated aqueous NaHCO₃. The aqueous phase was extracted with 10 mL more DCM. Organic phases were combined and solvent removed by rotavap to give off-white solids. Purification by preparative C18 HPLC gave the title compound (13.3 mg, 14% yield). ES+ 490.3 [M+H].
¹H NMR (500 MHz, DMSO-*d*₆) δ 9.36 - 9.31 (m, 1H), 8.73 (s, 1H), 8.28 (d, *J* = 4.8 Hz, 1H), 8.19 (dt, *J* = 8.2, 1.0 Hz, 1H), 8.13 (s, 1H), 7.86 (ddd, *J* = 8.3, 6.9, 1.3 Hz, 1H), 7.81 - 7.71 (m, 3H), 7.65 - 7.60 (m, 1H), 5.16 (s, 2H), 3.51 (ddd, *J* = 12.4, 8.9, 3.6 Hz, 2H), 3.47 - 3.39 (m, 2H), 2.98 (s, 3H), 1.96 (ddd, *J* = 13.2, 8.9, 4.1 Hz, 2H), 1.86 (ddt, *J* = 13.6, 6.1, 3.6 Hz, 2H).

### Example 160

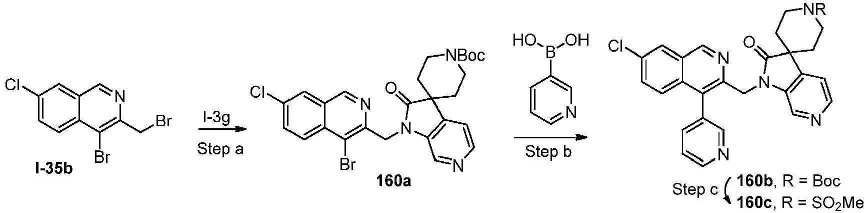

### Step a) Tert-butyl 1'-((4-bromo-7-chloroisoquinolin-3-yl)methyl)-2'-oxo-1',2'-dihydrospiro[piperidine-4,3'-pyrrolo[2,3-c]pyridine]-1-carboxylate (160a)

Compound I-35b (1.64 g, 4.0 mmol) was reacted with I-3g (1.10 g, 4.0 mmol) according to the method described in Example 3 step a, which gave the title compound (1.96 g, 68%). MS (ES+) 559.1 [M+H]+.

### Step b) Tert-butyl 1'-((7-chloro-4-(pyridin-3-yl)isoquinolin-3-yl)methyl)-2'-oxo-1',2'-dihydrospiro[piperidine-4,3'-pyrrolo[2,3-c]pyridine]-1-carboxylate (160b)

A stirred solution of compound 160a (500 mg, 0.896 mmol), pyridin-3-ylboronic acid (176 mg, 1.43 mmol) and sodium carbonate (285 mg, 2.69 mmol) in 1,2-dimethoxyethane (15 mL) and water (2 mL) was purged with argon for 10 minutes, then [1,1'-bis(diphenylphosphino)ferrocene]palladium(II) chloride•CH₂Cl₂ (65 mg, 0.089 mmol) was added the mixture was purged again with argon for 5 minutes. The resulting reaction mixture was stirred in sealed tube at 100 °C for 16 hour, then cooled to rt, filtered through Celite and the filtrate was concentrated in vacuo. The afforded crude compound was purified by flash chromatography on a silica gel columneluted with 3% MeOH in DCM, which gave the title compound (300 mg, 54 %) asa solid. MS (ES+) 556.21 [M+H]⁺.

### Step c) 1'-((7-chloro-4-(pyridin-3-yl)isoquinolin-3-yl)methyl)-1-(methylsulfonyl)spiro[piperidine-4,3'-pyrrolo[2,3-c]pyridin]-2'(1'H)-one (160c)

4 M HCl in dioxane (6 mL) was added dropwise under nitrogen at rt to a solution of compound 160b (300 mg, 0.416 mmol) in DCM (4 mL). The resulting mixture was stirred at rt for 1 h, then concentrated under reduced pressure. The residue was suspended in DCM (5 mL), triethylamine (0.6 mL, 4.32 mmol) and methanesulfonyl chloride (55 mg, 0.48 mmol) were added at 0 °C under nitrogen. The resulting mixture was stirred at rt for 1 h, then diluted with DCM (50 mL), washed with water (2 x 30 mL). The organic layer was dried over sodium sulfate, filtered and concentrated under reduced pressure and the afforded crude compound was purified by C18 prep-HPLC (10 mM ammonium bicarbonate in H₂O : acetonitrile) which gave the title compound (112 mg, 52 %) as a solid. MS (ES+) 534.17 [M+H]⁺.
¹H NMR (500 MHz, DMSO) δ 1.81 (dt, *J* = 13.8, 4.5, 4.5 Hz, 2H), 1.92 (ddd, *J* = 13.4, 8.9, 4.3 Hz, 2H), 2.96 (s, 3H), 3.45 (dddd, *J* = 23.4, 17.6, 10.7, 5.2 Hz, 4H), 4.95 (m, 2H), 7.36 (d, *J* = 9.1 Hz, 1H), 7.61 (m, 1H), 7.66 (ddd, *J* = 7.8, 4.9, 0.9 Hz, 1H), 7.77 (dd, *J* = 9.1, 2.2 Hz, 1H), 7.99 (dt, *J* = 7.8, 2.0, 2.0 Hz, 1H), 8.18 (s, 1H), 8.29 (d, *J* = 4.8 Hz, 1H), 8.32 (d, *J* = 2.2 Hz, 1H), 8.69 (d, 1H), 8.77 (dd, *J* = 4.9, 1.6 Hz, 1H), 9.29 (s, 1H).
¹³C NMR (126 MHz, DMSO) δ 31.19, 31.22, 34.49, 40.67, 43.14, 43.72, 118.53, 124.00, 126.57, 127.53, 127.81, 130.41, 130.62, 131.90, 132.03, 133.45, 137.75, 139.57, 141.17, 143.94, 146.43, 149.62, 150.11, 151.62, 177.45.

### Example 161

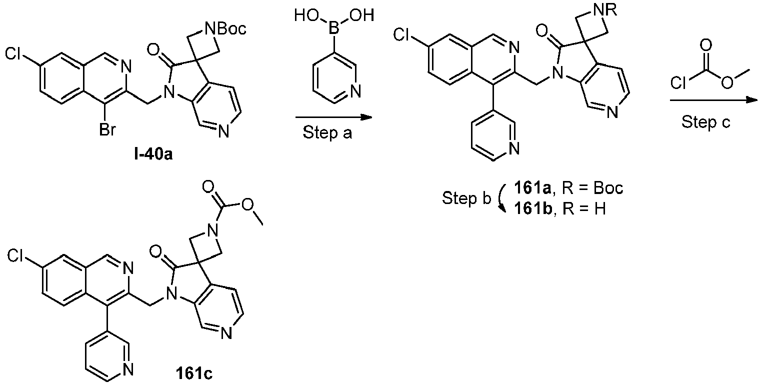

### Step a) Tert-butyl 1'-((7-chloro-4-(pyridin-3-yl)isoquinolin-3-yl)methyl)-2'-oxo-1',2'-dihydrospiro[azetidine-3,3'-pyrrolo[2,3-c]pyridine]-1-carboxylate (161a)

To a mixture of compound 161a (1.1g, 2.08 mmol), pyridin-3-ylboronic acid (330 mg, 2.68 mmol) in DME (45 mL) and water (5 mL) was added sodium carbonate (660 mg, 6.23 mmol). The resulting mixture was degassed with argon for 15 min, then [1,1'-bis(diphenylphosphino)ferrocene]palladium(II) chloride (160 mg, 0.219 mmol) was added and the mixture was again degassed for 5 min. The resulting mixture was stirred at 100 °C for 16 h, then passed through Celite and was concentrated under reduced pressure. The obtained crude compound was purified by column chromatography using silica gel eluted with 3% MeOH in DCM. Fractions containing compound were mixed and concentrated under reduced pressure which gave the title compound (800 mg, 49%) as a solid which was used for next step without further purification. MS (ES+) 528.16 [M+H]⁺.

### Step c) 1'-((7-Chloro-4-(pyridin-3-yl)isoquinolin-3-yl)methyl)spiro[azetidine-3,3'-pyrrolo[2,3-c]pyridin]-2'(1'H)-one (161b)

TFA (5 mL, 65.29 mmol) was added at 0 °C under nitrogen to a stirred solution of compound 161a (800 mg, 1.52 mmol) in DCM (10 mL). The reaction mixture was stirred at rt for 2 h, concentrated and basified with saturated sodium bicarbonate solution to pH 8 and extracted with DCM (2 X 80 mL). The combined organic layers were dried over sodium sulfate, filtered and concentrated under reduced pressure. The obtained solid was triturated with diethylether (20 mL), stirred for 15 min and filtered, which gave the title compound (500 mg, 67%) as a solid. The afforded material was used for next step without any further purification. MS (ES+) 428.15 [M+H]+.

### Step c) methyl 1'-((7-chloro-4-(pyridin-3-yl)isoquinolin-3-yl)methyl)-2'-oxo-1',2'-dihydrospiro[azetidine-3,3'-pyrrolo[2,3-c]pyridine]-1-carboxylate (161c)

Triethylamine (0.8 mL, 5.74 mmol) was added at 0 °C to a stirred solution of compound 161c (250 mg, 0.584 mmol) in DCM (5 mL). After 30 min at 0 °C a stock solution of methyl chloroformate (70 mg, 0.741 mmol) in DCM was added under nitrogen. The resulting mixture was stirred at rt for 2 h, then the mixture was diluted with water (20 mL) and extracted with DCM (2 x 50 mL). The combined organic layers were dried over sodium sulfate, filtered and concentrated under reduced pressure. The obtain solid was purified by prep C18 HPLC, which gave the title compound (120 mg, 42%) as a solid. MS (ES+) 486.35 [M+H]⁺.
¹H NMR (500 MHz, DMSO) δ 3.63 (s, 3H), 4.03 (s, 2H), 4.12 (s, 2H), 5.00 (q, J = 16.0, 16.0, 16.0 Hz, 2H), 7.31 (d, J = 9.1 Hz, 1H), 7.60 (dd, J = 7.8, 4.8 Hz, 1H), 7.74 (d, J = 4.7 Hz, 1H), 7.76 (dd, J = 9.0, 2.2 Hz, 1H), 7.88 (dt, J = 7.8, 2.0, 2.0 Hz, 1H), 8.09 (s, 1H), 8.34 (m, 2H), 8.57 (d, J = 2.2 Hz, 1H), 8.72 (dd, J = 4.9, 1.6 Hz, 1H), 9.34 (s, 1H).
¹³C NMR (126 MHz, DMSO) δ 41.75, 44.11, 52.02, 118.35, 123.73, 126.48, 127.49, 127.74, 130.14, 130.45, 131.88, 131.94, 133.43, 137.39, 137.61, 140.07, 144.40, 146.12, 149.40, 149.73, 151.54, 156.04, 175.27.

### Example 162

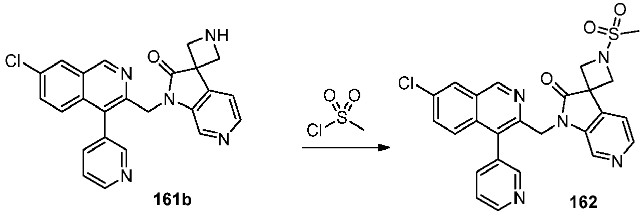

### 1'-((7-chloro-4-(pyridin-3-yl)isoquinolin-3yl)methyl)-1-(methylsulfonyl)spiro[azetidine-3,3'-pyrrolo[2,3-c]pyridin1-2'(1'H)-one (162)

To a solution of compound 161 b (250 mg, 0.584 mmol) in DCM (5 mL) was added triethylamine (0.3 g, 2.96 mmol) at 0 °C, the resulting mixture was stirred at 0 °C for 20 min, then methanesulfonyl chloride (80 mg, 0.701 mmol) was added and the mixture was stirred at rt for 2 h. The reaction mixture was diluted with water (15 mL) and the aqueous layer was extracted with DCM (2 x 10 mL). The combined organic layers were dried over sodium sulfate, filtered and concentrated. The obtained crude compound was purified by prep C18 HPLC. Pure fractions were pooled, concentrated and lyophilized which gave the title compound as a solid (92 mg, 31%) MS (ES+) 506.3 [M+H]⁺.
¹H NMR (500 MHz, DMSO) δ 3.18 (s, 3H), 4.10 (t, 4H), 5.00 (m, 2H), 7.32 (d, 1H), 7.61 (m, 1H), 7.65 (m, 1H), 7.77 (dd, 1H), 7.90 (dt, 1H), 8.13 (s, 1H), 8.35 (d, 1H), 8.38 (d, 1H), 8.59 (m, 1H), 8.72 (dd, 1H), 9.33 (s, 1H).
¹³C NMR (126 MHz, DMSO) δ 33.30, 40.74, 44.11, 57.06, 57.08, 118.16, 123.75, 126.46, 127.42, 127.72, 130.24, 130.45, 131.86, 131.94, 133.40, 137.40, 140.08, 144.43, 146.13, 149.40, 149.75, 151.52, 174.48.

### Example 163

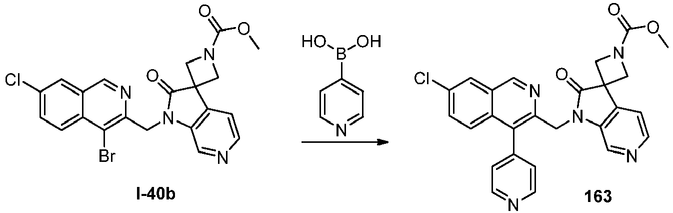

### Methyl 1'-((7-chloro-4-(pyridin-4-yl)isoquinolin-3-yl)methyl)-2'-oxo-1',2'-dihydrospirofazetidine-3,3'-pyrrolo[2,3-c]pyridine1-1-carboxylate (163)

A stirred solution of compound I-40b (350 mg, 0.718 mmol), pyridin-4-ylboronic acid (125 mg, 1.02 mmol) and sodium carbonate (230 mg, 2.17 mmol) in 1,2-dimethoxyethane (10 mL) and water (1.5 mL) was purged with argon for 10 minutes. [1,1'-Bis(diphenylphosphino)ferrocene]palladium(II) chloride•CH₂Cl₂ (50 mg, 0.068 mmol) was added and the mixture was purged again with argon for 5 minutes. The resulting reaction mixture was stirred in sealed tube at 105 °C for 16 h, then cooled to rt, filtered through Celite and concentrated under vacuum. The crude compound was purified by prep C18 HPLC , which gave the title compound (112 mg, 25%) as a solid. MS (ES+) 486.35 [M+H]⁺.
¹H NMR (500 MHz, DMSO) δ 3.63 (s, 3H), 3.99 (d, *J* = 8.4 Hz, 2H), 4.12 (s, 2H), 5.01 (s, 2H), 7.31 (d, *J* = *9.1* Hz, 1H), 7.44 (m, 2H), 7.74 (dd, *J* = 4.7, 0.9 Hz), 7.76 (dd, *J* = 9.1, 2.2 Hz, 1H), 8.01 (s, 1H), 8.34 (d, *J* = 4.7 Hz, 1H), 8.35 (d, *J* = 2.2 Hz, 1H), 8.73 (m, 2H), 9.35 (d, *J* = 0.8 Hz, 1H).
¹³C NMR (126 MHz, DMSO) δ 41.69, 44.12, 52.03, 56.62 (m), 118.37, 124.62, 126.34, 126.53, 127.68, 128.24, 130.00, 131.95, 132.02, 132.47, 137.60, 139.98, 142.67, 144.43, 145.14, 149.97, 151.66, 156.03, 175.22.

### Example 164

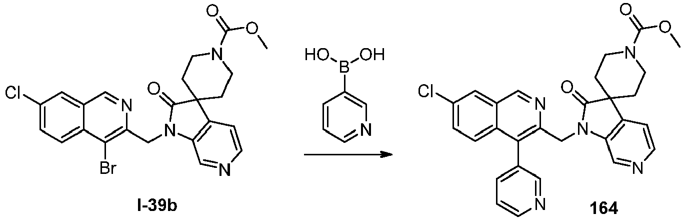

### Methyl 1'-((7-chloro-4-(pyridin-3-yl)isoquinolin-3-yl)methyl)-2'-oxo-1',2'-dihydrospiro[piperidine-4,3'-pyrrolo[2,3-c]pyridine]-1-carboxylate (164)

A stirred solution of compound I-40b (350 mg, 0.718 mmol), pyridin-4-ylboronic acid (125 mg, A mixture of I-39b, 3-pyridineboronic acid, NaHCO₃, and Pd(dppf)Cl₂ complexed with CH₂Cl₂ in degassed solvent (dioxane/water/DMF 6:2:1, 4.75 mL) in a microwave-vial was bubbled with N₂ for 1 min, added 0.2 mL more solvent. The reaction mixture was heated in a microwave reactor at 110 °C for 1h, then cooled to rt and partitioned between water (15 mL) and EtOAc (15 mL). The aqueous phase was extracted with EtOAc (2x 15 mL). Organic phases were combined, dried (Na₂SO₄), and concentrated in vacuo. The afforded crude material was dissolved in MeCN (3 mL) + H₂O (1.5 mL) and purified by C18 prep HPLC._Product fractions were freeze-dried which gave the title compound (66 mg, 45%). MS (ES+) 514.48 [M+H]⁺.
¹H NMR (500 MHz, DMSO) δ 1.67 (dt, 2H), 1.75 (dt, 2H), 3.64 (s, 3H), 3.68 (dq, 4H), 4.96 (m, 2H), 7.36 (d, 1H), 7.60 (d, 1H), 7.65 (m, 1H), 7.77 (dd, 1H), 7.99 (dt, 1H), 8.16 (s, 1H), 8.26 (d, 1H), 8.32 (d, 1H), 8.69 (d, 1H), 8.77 (dd, 1H), 9.29 (s, 1H).
¹³C NMR (126 MHz, DMSO) δ 30.95, 38.54, 43.03, 44.34, 52.26, 118.41, 123.84, 126.43, 126.46, 127.47, 127.69, 130.26, 130.49, 131.78, 131.89, 133.34, 137.63, 139.34, 141.22, 143.79, 146.31, 149.47, 149.99, 151.48, 155.04, 177.41.

### Example 165

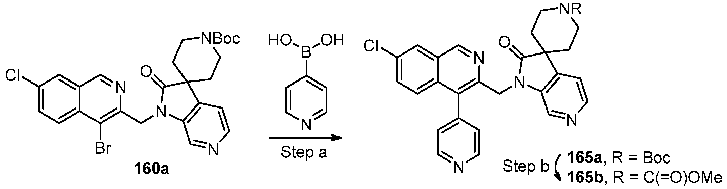

### Step a) tert-butyl 1'-((7-chloro-4-(pyridin-4-yl)isoquinolin-3-yl)methyl)-2'-oxo-1',2'-dihydrospiro[piperidine-4,3'-pyrrolo[2,3-c]pyridine]-1-carboxylate (165a)

160a (500 mg, 0.896 mol) was reacted with pyridin-4-ylboronic acid (200 mg, 1.63 mmol) according to the procedure described in Example 163, which gave the title compound (450 mg, 62%). MS (ES+) 556.44 [M+H]⁺.

### Step b) Methyl 1'-((7-chloro-4-(pyridin-4-yl)isoquinolin-3-yl)methyl)-2'-oxo-1',2'-dihydrospiro[piperidine-4,3'-pyrrolo]2,3-c]pyridine]-1-carboxylate (165b)

HCl 4.0 M in dioxane (5 mL, 20 mmol) was added at 0 °C under nitrogen to a stirred solution of compound 165a (0.45 g, 0.56 mmol) in DCM (6 mL). The resulting reaction mass was stirred at rt for 2 h. when TLC indicated complete consumption of starting material the reaction mixture was concentrated under reduced pressure and co-distilled with 1,4-dioxane (2 x 10 mL). The residue was dissolved in DCM (5 mL), triethylamine (0.80 mL, 5.74 mmol) was added at 0°C and after 30 min a stock solution of methyl chloroformate (67.0 mg, 0.709 mmol) in DCM was added under nitrogen. Resulting reaction mixture was stirred at rt for 1 h, then the reaction mixture was diluted with water (20 mL) and extracted with DCM (2 x 50 mL). The combined organic layers were dried over sodium sulfate, filtered and concentrated under reduced pressure. The obtained crude material was purified by prep C18 HPLC, which gave the title compound (58 mg, 21%) as a solid. MS (ES+) 514.33 [M+H]⁺.
¹H NMR (500 MHz, DMSO) δ 1.67 (dt, 2H), 1.75 (dt, 2H), 3.64 (s, 3H), 3.69 (p, 4H), 4.96 (s, 2H), 7.37 (d, 1H), 7.58 (m, 2H), 7.60 (d, 1H), 7.77 (dd, 1H), 8.14 (s, 1H), 8.26 (d, 1H), 8.32 (d, 1H), 8.81 (m, 2H), 9.29 (s, 1H).
¹³C NMR (126 MHz, DMSO) δ 31.11, 38.65, 43.01, 44.47, 52.38, 118.54, 125.09, 126.49, 126.59, 127.72, 128.41, 130.31, 131.95, 132.08, 132.52, 139.43, 141.34, 142.84, 143.93, 145.47, 150.25, 151.71, 155.16, 177.57.

### Example 166

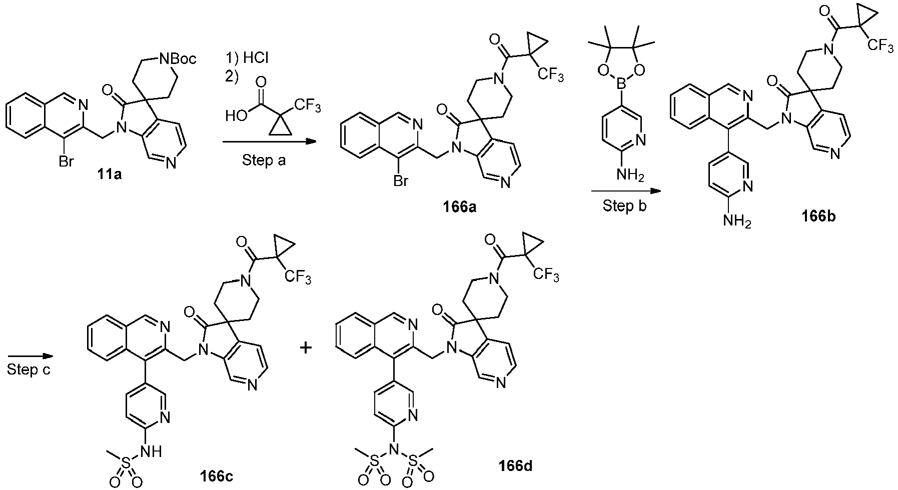

### Step a) 1'-((4-Bromoisoquinolin-3-yl)methyl)-1-(1-(trifluoromethyl)cyclopropanecarbonyl)spiro[piperidine-4,3'-pyrrolo[2,3-c]pyridin]-2'(1'H)-one (166a)

4M HCl in dioxane (1.5 mL) was added to a solution of compound 11a (260 mg, 0.497 mmol) in DCM (2 mL). The solution was stirred for 1h at rt, then concentrated in vacuo and co-evaporated with toluene. The obtained residue was dissolved in DMF (1.5 mL), DIEA (321 mg, 2.48 mmol) was added and the solution was stirred for 15 min then HATU (208 mg, 0.546 mmol) and 1-(trifluoromethyl)cyclopropane-1-carboxylic acid (84.2 mg, 0.546 mmol). The reaction was stirred at rt for 1 h, then concentrated in vacuo onto silica, and then applied onto a silica column. The product was eluted using a gradient of MeOH-DCM, which gave the title compound (134 mg, 48%) MS (ES+) 559.22 [M+H]⁺.

### Step b) 1'-((4-(6-aminopyridin-3-yl)isoquinolin-3-yl)methyl)-1-(1-(trifluoromethyl)cyclopropanecarbonyl)spiro[piperidine-4,3'-pyrrolo[2,3-c]pyridin]-2'(1'H)-one (166b)

5-(4,4,5,5-tetramethyl-1,3,2-dioxaborolan-2-yl)pyridin-2-amine (55.1 mg, 0.250 mmol), bis(triphenylphosphine)palladium(II) dichloride (18 mg, 0.025 mmol) and a 2M solution of potassium carbonate were added under nitrogen at rt to a sealed tube containing a stirred solution of compound 166a (70 mg, 0.125 mmol) in acetonitrile (3 mL). The reaction mixture stirred at 100 °C for 16 h. The reaction mixture was combined with another badge of the same material, filtered and concentrated under reduced pressure. The crude compound was purified by column chromatography on silica gel using 1-6% MeOH in DCM as eluent, which gave the title compound (110 mg, 71%). MS ES(+) 573.3 [M+H]⁺.

### Step c) N-(5-(3-((2'-oxo-1-(1-(trifluoromethyl)cyclopropanecarbonyl)spiro[piperidine-4,3'-pyrrolo[2,3-c]pyridin]-1'(2'H)-yl)methyl)isoquinolin-4-yl)pyridin-2-yl)methanesulfonamide (166c) &

### N-(methylsulfonyl)-N-(5-(3-((2'-oxo-1-(1-(trifluoromethyl)cyclopropanecarbonyl)spiro[piperidine-4,3'-pyrrolo[2,3-c]pyridin]-1'(2'H)-yl)methyl)isoquinolin-4-yl)pyridin-2-yl)methanesulfonamide (166d)

Methanesulfonyl chloride was added (20.9 mg, 0.183 mol) at 0 °C to a solution of compound 166b (95.2 mg, 0.166 mmol) and TEA (37 mg, 0.366 mmol) in DCM (3 mL). The reaction was stirred at 0 °C for 1h, then at rt for 3h and more sulfonyl chloride (2.2 eq.) and TEA (4 eq.) were added, and the solution was stirred at rt over week-end.

The reaction mixture was concentrated in vacuo and the crude was purified by column chromatography on silica gel using a gradient of MeOH in DCM as eluent, which gave the mono sulfonylated and the disulfonyltated compounds. Further purification of the monosulfonylated compound by prep LCMS at pH7 provided, after lyophilization, pure monosulfonyltaed compound (9.3 mg, 7.7%) MS ES(+) 651.5 [M+H]⁺.
¹H NMR (500 MHz, DMSO) δ 1.27 (s, 2H), 1.32 (s, 2H), 1.72 (m, 2H), 1.79 (d, 2H), 3.88 (m, 4H), 5.03 (m, 2H), 7.13 (d, 1H), 7.46 (m, 1H), 7.59 (d, 1H), 7.72 (ddd, 1H), 7.76 (ddd, 1H), 7.83 (dd, 1H), 8.12 (s, 1H), 8.16 (dd, 1H), 8.26 (d, 1H), 8.32 (d, 1H), 9.29 (s, 1H), 11.04 (s, 1H).
¹³C NMR (126 MHz, DMSO) δ 9.78, 26.63 (q), 31.13, 41.49, 43.30, 44.40, 112.34, 118.35, 123.72, 124.06, 125.13 (q), 127.13, 127.37, 127.74, 130.28, 131.31, 135.08, 139.40, 139.98, 141.07, 143.77, 145.86, 147.75, 152.03, 153.11, 162.72, 177.36.
¹⁹F NMR (376 MHz, dmso) δ -66.26.

Further purification of the disulfonylated compound by prep LCMS at pH7 provided, after lyophilization, pure disulfonylated compound (9.5 mg 7.8%). MS ES(+) 729.29 [M+H]⁺.
¹H NMR (500 MHz, DMSO) δ 1.30 (d, 4H), 1.80 (d, 4H), 3.75 (s, 6H), 3.93 (m, 4H), 4.95 (m, 2H), 7.26 (dd, 1H), 7.63 (d, 1H), 7.75 (ddd, 1H), 7.83 (ddd, 1H), 8.00 (dd, 1H), 8.19 (dt, 1H), 8.25 (s, 1H), 8.30 (m, 2H), 8.79 (dd, 1H), 9.31 (m, 1H).
¹³C NMR (126 MHz, DMSO) δ 9.77, 26.62 (q), 31.08, 42.74, 43.89, 44.47, 118.39, 123.44, 125.30 (q), 125.51, 126.02, 126.95, 127.54, 127.92, 130.41, 131.71, 132.53, 134.59, 139.59, 141.03, 141.31, 143.82, 145.73, 148.22, 149.97, 152.76, 162.71, 177.67.
¹⁹F NMR (376 MHz, dmso) δ -66.24.

### Example 167

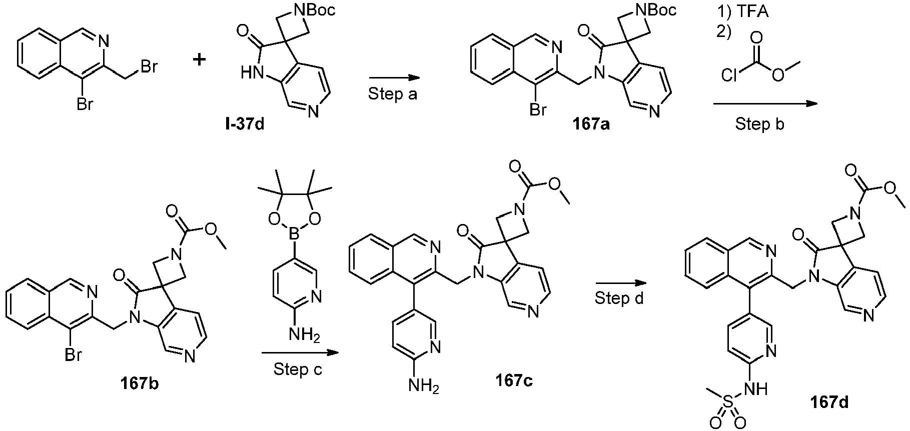

### Step a) 1'-((4-Bromoisoquinolin-3-yl)methyl)-1-(1-(trifluoromethyl)cyclopropanecarbonyl)spiro[piperidine-4,3'-pyrrolo[2,3-c]pyridin]-2'(1'H)-one (167a)

Cesium carbonate was added to a solution of I-37d (140 mg, 0.356 mmol) in DMF (3 mL). The obtained slurry was stirred for 1 h, then a solution of 4-bromo-3-(bromomethyl)isoquinoline in DMF (2 mL) was added. The slurry was stirred for 3 h at rt, then concentrated onto silica and purified by chromatography on silica eluting with 1-8 % MeOH in DCM, which gave the title compound (170 mg, 96%). MS ES(+) 495.2 [M+H]⁺.

### Step b) Methyl 1'-((4-bromoisoquinolin-3-yl)methyl)-2'-oxo-1',2'-dihydrospiro[azetidine-3,3'-pyrrolo[2,3-c]pyridine]-1-carboxylate (167b)

TFA was added dropwise at rt to a solution of compound 166a (170 mg, 0.343 mmol) in DCM (23 mL). After 18h, the reaction was concentrated and co-evaporated twice with toluene. The residue and DIEA (444 mg, 3.43 mmol) were dissolved in DCM (3 mL) at rt, methyl chloroformate was added and the reaction mixture was stired at rt for 1h, then concentrated in vacuo. The crude was dissolved in DCM and purified by silica gel column chromatography using a gradient of MeOH in DCM as gradient, which gave the title compound (158 mg, 102%). MS ES(+) 453.18 [M+H]+.

### Step c) methyl 1'-((4-(6-aminopyridin-3-yl)isoquinolin-3-yl)methyl)-2'-oxo-1',2'-dihydrospiro[azetidine-3,3'-pyrrolo[2,3-c]pyridine]-1-carboxylate (167c)

Compound 167b (158 mg, 0.349 mmol) was reacted with 5-(4,4,5,5-tetramethyl-1,3,2-dioxaborolan-2-yl)pyridin-2-amine (153 mg, 0.697 mmol) according to the procedure described in Example 166 step b, which gave the title compound (160mg, 98%). MS ES(+) 467.3 [M+H]⁺.

### Step d) methyl 1'-((4-(6-(methylsulfonamido)pyridin-3-yl)isoquinolin-3-yl)methyl)-2'-oxo-1',2'-dihydrospiro[azetidine-3,3'-pyrrolo[2,3-c]pyridine]-1-carboxylate (167d)

Methanesulfonyl chloride (43.2 mg, 0.377 mmol) was added to a solution of compound 167c (160 mg, 0.343 mmol) and TEA (76.4 mg, 0.755 mmol) in DCM (3 mL) at 0 °C. The reaction was stirred at 0 °C for 30 min, then concentrated in vacuo.

The crude was purified by column chromatography on silica gel using a gradient of MeOH in DCM as eluent. The product was further purified by prep LCMS at pH 7 followed by lyophilization of pure fractions, which gave the title compound (20.3 mg). MS ES(+) 545.5 [M+H]+.
¹H NMR (500 MHz, DMSO) δ 3.35 (s, 3H), 3.63 (s, 3H), 4.05 (d, 2H), 4.14 (s, 2H), 5.04 (d, 2H), 7.11 (d, 1H), 7.39 (m, 1H), 7.74 (m, 4H), 8.07 (s, 1H), 8.18 (dd, 1H), 8.26 (d, 1H), 8.34 (d, 1H), 9.33 (s, 1H), 10.95 (d, 1H).
¹³C NMR (126 MHz, DMSO) δ 41.61, 41.77, 44.25, 52.00, 56.60, 112.01, 118.34, 124.02, 124.23, 127.13, 127.23, 127.45, 127.76, 130.14, 131.37, 135.15, 137.58, 139.90, 140.12, 144.34, 145.70, 152.10, 152.41, 156.04, 175.29.

### Example 168

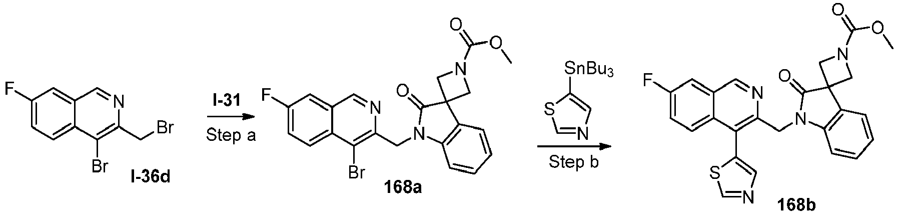

### Step a) Methyl 1'-((4-bromo-7-fluoroisoquinolin-3-yl)methyl)-2'-oxospiro[azetidine-3.3'-indoline]-1-carboxylate (168a)

Compound I-36d (1.50 g, 4.22 mmol) was reacted with I-31 (1.00 g, 4.22 mmol) using the method described in Intermediate 41 step a, which gave the title compound (1.60 g, 76%). MS (ES+) 471.9 [M+H]+.

### Step b) methyl 1'-((7-fluoro-4-(thiazol-5-yl)isoquinolin-3-yl)methyl)-2'-oxospiro[azetidine-3,3'-indoline]-1-carboxylate (168b)

A solution of 5-(tributylstannyl)thiazole (480 mg, 1.27 mmol), in MeCN (10 mL) and sodium carbonate (2.5 g, 1.910 mmol) were added under argon to a solution of compound 168a (300 mg, 0.637 mmol) in MeCN (20 mL). The reaction mixture was purged with argon for 15 min, then bis(tri-tert-butylphosphine)palladium(0) (30 mg, 0.057 mmol) was added and the reaction mixture was stirred at 130 °C for 18h. The reaction mixture was filtered through Celite and concentrated in vacuo. The crude along with another batch (100 mg) was purified by prep-HPLC. Appropriate fraction were collected and further purified by SFC. Fractions containing desired compound were collected, concentrated under vacuum triturated with water and filtered off, which gave the title compound (52 mg) as a solid. MS (ES+) 476.29 [M+H]⁺. [M+H]⁺.
¹H NMR (500 MHz, DMSO) δ 3.64 (s, 3H), 4.08 (d, 2H), 4.17 (s, 2H), 5.06 (s, 2H), 7.54 (dd, 1H), 7.72 (td, 1H), 7.76 (dd, 1H), 8.02 (dd, 1H), 8.05 (d, 1H), 8.08 (s, 1H), 8.35 (d, 1H), 9.35 (s, 1H), 9.41 (d, 1H).
¹³C NMR (126 MHz, DMSO) δ 41.76, 44.08, 52.01, 56.65, 110.94 (d), 118.40, 120.40, 122.09 (d), 127.21 (d), 127.82 (d), 128.85, 129.99, 133.05, 137.70, 140.06, 144.25, 144.42, 147.02 (d), 152.47 (d), 156.04, 156.62, 160.30 (d), 175.31.
¹⁹F NMR (376 MHz, dmso) δ -111.41 (m).

### Example 169

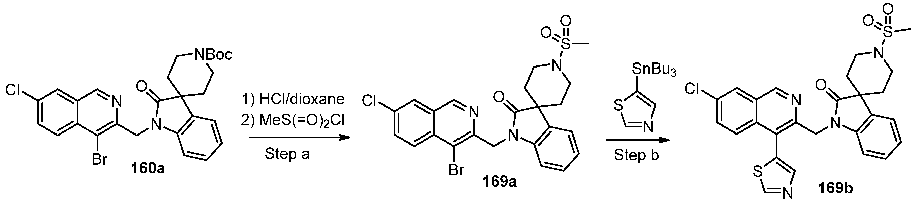

### Step a) 1-((4-Bromo-7-chloroisoquinolin-3-yl)methyl)-1'-(methylsulfonyl)spiro[indoline-3,4'-piperidin]-2-one (169a)

HCl 4.0 M in dioxane (5 mL, 20 mmol) was added at 0 °C under nitrogen to a stirred solution of compound 160a (0.3 g, 0.48 mmol) in DCM (3 mL). The resulting mixture was stirred at rt for 2 h, then concentrated under reduced pressure and co-distilled with 1,4-dioxane (2 x 10 mL). The residue was dissolved in DCM (5 mL), triethylamine (0.4 mL, 2.83 mmol) and methanesulfonyl chloride (0.1 mL, 0.85 mmol) were added at 0 °C under nitrogen and the mixture was stirred at rt for 3 h. The reaction mixture was poured into water (20 mL) and extracted with DCM (2 x 30 mL). The organic layer was washed with brine (10 mL), dried over sodium sulfate, filtered and concentrated under reduced pressure. The crude compound was purified by flash chromatography on silica gel eluted with 15% MeOH in DCM, which gave the title compound (130 mg, 50%) as a solid. MS (ES+) 535.06 [M+H]⁺.

### Step b) 1-((7-chloro-4-(thiazol-5-yl)isoquinolin-3-yl)methyl)-1'-(methylsulfonyl)spiro[indoline-3,4'-piperidi]-2-one (169b)

Compound 169 a (110 mg, 0.203 mmol) was reacted with 5-(tributylstannyl)thiazole (134 mg, 0.358 mmol) according to the procedure described in Example 168 step b. The afforded crude compound was combined with additional crude compounds obtained using the same method. Purifcation of the combined crude compounds gave the title compound (40 mg). MS (ES+) 540.11 [M+H]+.
¹H NMR (500 MHz, DMSO) δ = 1.85 (dt, 2H), 1.96 (ddd, 2H), 2.97 (s, 3H), 3.42 (dt, 2H), 3.49 (td, 2H), 5.03 (s, 2H), 7.55 (d, 1H), 7.63 (d, 1H), 7.83 (dd, 1H), 8.17 (s, 1H), 8.18 (s, 1H), 8.30 (d, 1H), 8.32 (d, 1H), 9.29 (s, 1H), 9.46 (s, 1H).

In addition, the compounds listed in TABLE 2 were prepared using procedures in line with those described hereinabove using commercially available building blocks or building blocks prepared according to literature procedures or as described herein.

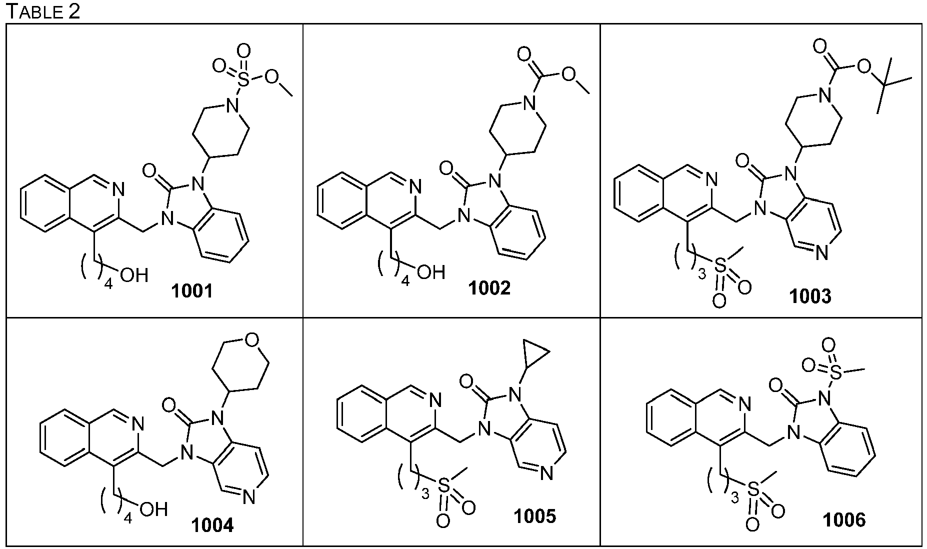

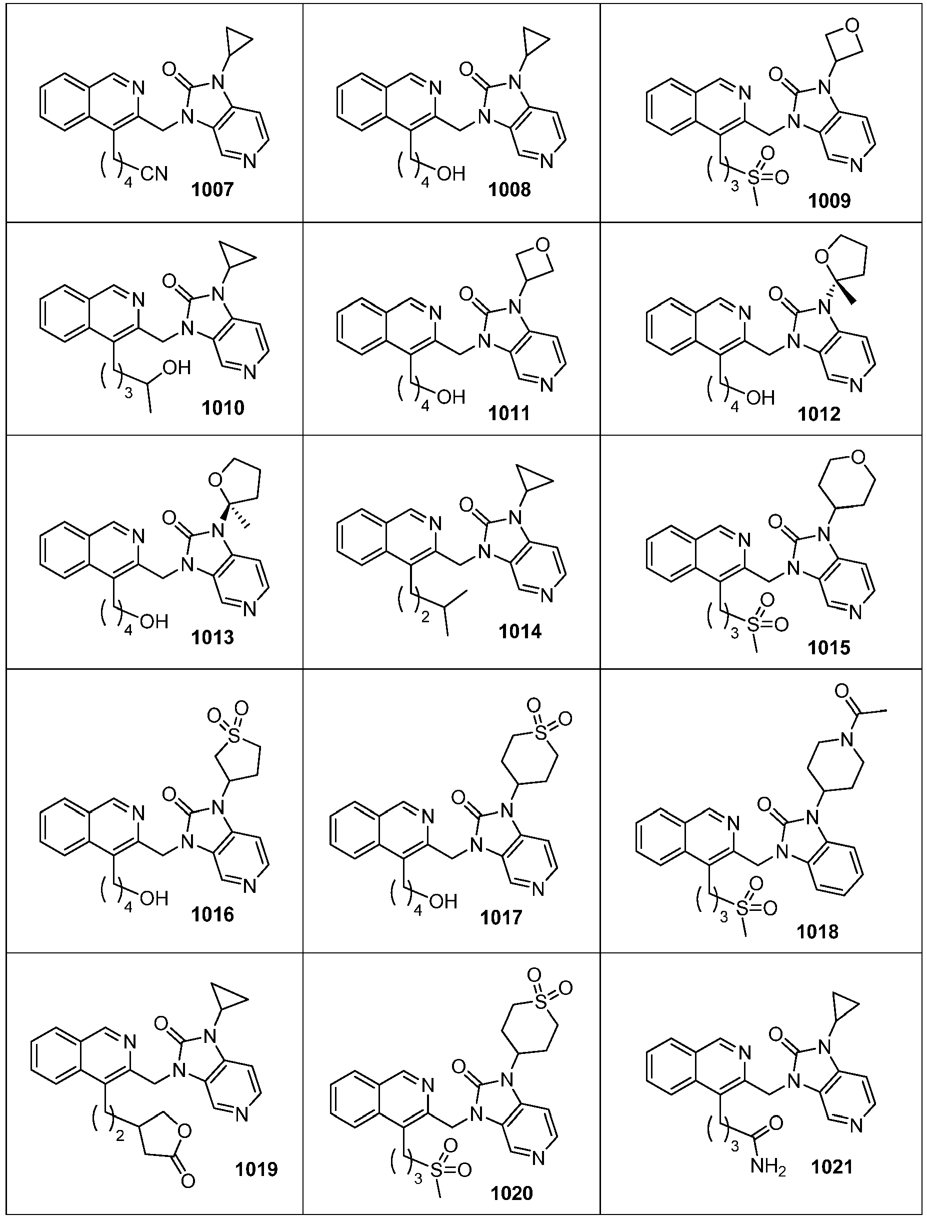

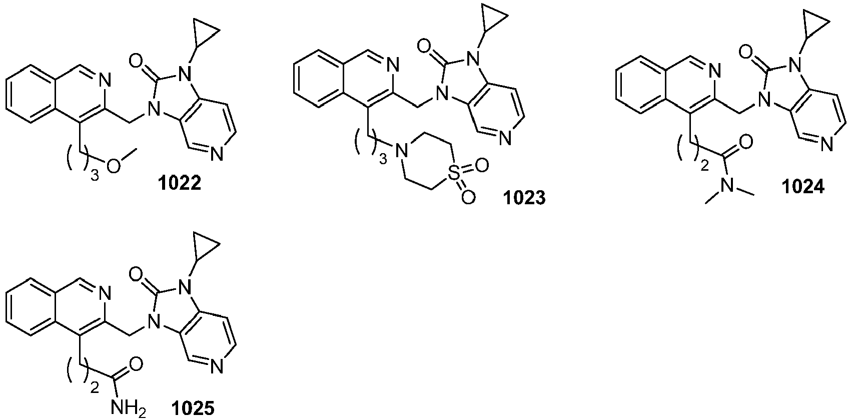

**TABLE 2**

| Compound | Analytical data |
|---|---|
| 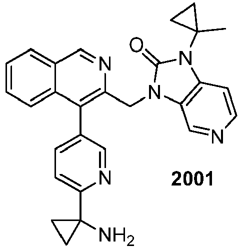 | ¹H NMR (500 MHz, DMSO-*d*₆) δ 9.30 (s, 1H), 8.38 (d, *J* = 2.3 Hz, 1H), 8.21 - 8.13 (m, 2H), 8.08 (s, 1H), 7.93 - 7.87 (m, 1H), 7.79 - 7.65 (m, 3H), 7.31 (dd, *J* = 8.3, 1.3 Hz, 1H), 7.22 (d, *J* = 5.2 Hz, 1H), 5.17 - 5.09 (m, 2H), 2.47 (s, 1H), 1.37 - 1.29 (m, 5H), 1.05 (q, *J* = 3.4 Hz, 2H), 1.01 - 0.90 (m, 4H). |
| 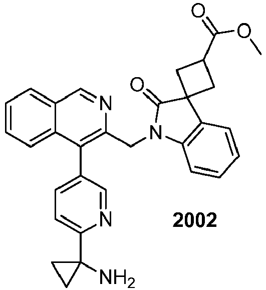 | ¹H NMR (500 MHz, DMSO-*d*₆) δ 9.30 (s, 1H), 8.36 (s, 1H), 8.17 (dd, *J* = 7.6, 1.7 Hz, 1H), 7.93 (d, *J* = 8.1 Hz, 1H), 7.79 -7.66 (m, 4H), 7.63 (dd, *J* = 7.4, 1.2 Hz, 1H), 7.29 (dd, *J =* 8.2, 1.3 Hz, 1H), 7.17 (td, *J* = 7.8, 1.2 Hz, 1H), 7.05 (td, *J* = 7.5, 1.0 Hz, 1H), 6.69 (d, *J* = 7.8 Hz, 1H), 4.99 (d, *J* = 16.0 Hz, 1H), 4.93 (d, *J* = 16.0 Hz, 1H), 4.05 (d, *J* = 8.1 Hz, 3H), 3.64 (s, 3H), 3.36 (s, 1H), 2.56 - 2.51 (m, 1H), 1.33 (q, *J* = 3.2 Hz, 2H), 1.06 (q, *J* = 3.3 Hz, 2H). |
| 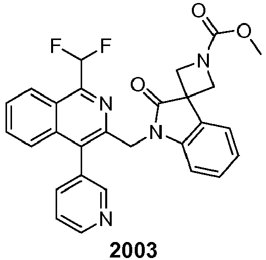 | ¹H NMR (500 MHz, DMSO-*d*₆) δ 8.82 - 8.60 (m, 1H), 8.64 - 8.47 (m, 1H), 8.53 - 8.35 (m, 1H), 7.96 - 7.72 (m, 3H), 7.73 - 6.95 (m, 6H), 6.72 (d, *J* = 7.8 Hz, 1H), 5.12 - 4.87 (m, 2H), 4.06 (s, 4H), 3.64 (s, 3H), 1.70 (s, 1H). |
| 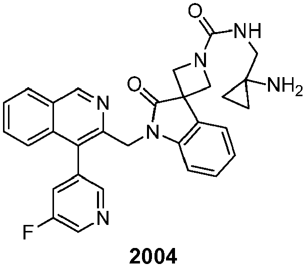 | ¹H NMR (500 MHz, DMSO-*d*₆) δ 9.38 (s, 1H), 8.71 *(d, J* = 2.8 Hz, 1H), 8.36 (s, 1H), 8.21 (dd, *J* = 7.1, 2.0 Hz, 1H), 7.93 (dt, *J* = 9.4, 2.2 Hz, 1H), 7.74 (pd, *J* = 7.0, 1.5 Hz, 2H), 7.54 (d, *J* = 7.3 Hz, 1H), 7.31 (d, *J* = 7.9Hz, 1H), 7.21 - 7.13(m, 1H), 7.06 (t, *J* = 7.5 Hz, 1H), 6.76 (d, *J* = 7.9 Hz, 1H), 6.60 (t, *J* = 5.8 Hz, 1H), 5.05 (d, *J* = 15.9 Hz, 1H), 4.94 (d, *J* = 16.0 Hz, 1H), 4.00 (dd, *J* = 15.5, 7.9 Hz, 2H), 3.90 (dd, *J* = 7.9, 2.0 Hz, 2H), 3.07 (d, *J* = 5.7 Hz, 2H), 2.01 (s, 2H), 0.45 (q, *J* = 3.9, 3.4 Hz, 2H), 0.37 (q, *J* = 3.8 Hz, 2H). |
| 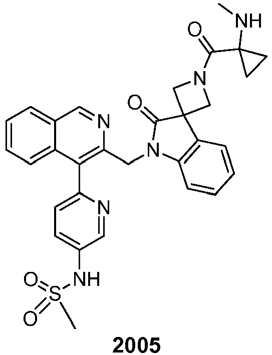 | |
| 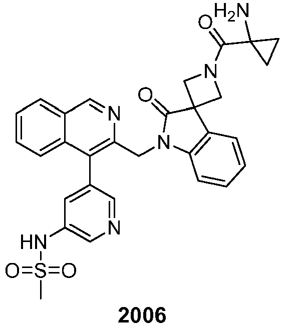 | ¹H NMR (500 MHz, DMSO-*d*₆) δ 9.35 (s, 1H), 8.55 (d, *J* = 2.5 Hz, 1H), 8.19 (dd, *J* = 7.7, 1.7 Hz, 1H), 7.74 (dddd, *J* = 16.4, 8.1, 6.9, 1.4 Hz, 2H), 7.64 (s, 1H), 7.59 (dd, *J* = 7.4, 1.2 Hz, 1H), 7.36 - 7.30 (m, 1H), 7.17 (td, *J* = 7.7, 1.2 Hz, 1H), 7.08 (d, *J* = 0.9 Hz, 0H), 7.07 (s, 0H), 6.74 (d, *J* = 7.8 Hz, 1H), 5.08 (d, *J* = 15.9 Hz, 1H), 4.90 (d, *J* = 15.9 Hz, 1H), 4.70 (s, 2H), 3.98 (s, 2H), 3.42 (s, 1H), 3.18 (s, 3H), 2.45 (s, 1H), 1.12 (s, 1H), 1.08 (s, 1H), 0.75 - 0.70 (m, 2H). |
| 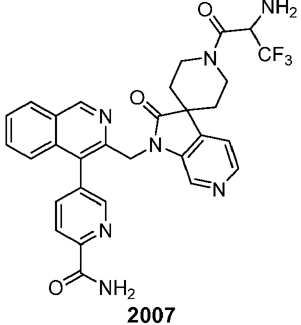 | ¹H NMR (500 MHz, DMSO-*d*₆) δ 9.30 (d, *J* = 1.8 Hz, 1H), 8.61 (ddd, *J* = 5.5, 2.2, 0.9 Hz, 1H), 8.20 (d, *J* = 12.9 Hz, 1H), 8.13 (s, 1H), 7.73 - 7.64 (m, 2H), 7.53 (dd, *J* = 5.2, 2.4 Hz, 1H), 7.45 (d, *J* = 4.8 Hz, 1H), 7.30 - 7.24 (m, 1H), 6.79 (s, 0H), 5.06 - 4.90 (m, 2H), 4.91 - 4.81 (m, 1H), 4.58 (tt, *J* = 10.2, 6.0 Hz, 1H), 3.94 (dd, *J* = 13.4, 5.1 Hz, 1H), 3.89 - 3.66 (m, 2H), 3.53 (ddt, *J* = 12.8, 9.1, 5.4 Hz, 1H), 2.32 - 2.23 (m, 2H), 1.79 - 1.65 (m, 1H), 1.61 (p, *J* = 8.2, 6.8 Hz, 3H). |
| 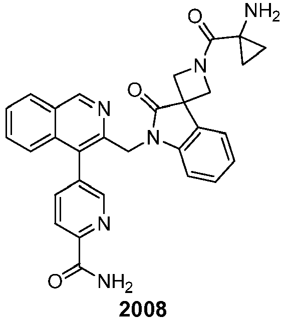 | ¹H NMR (500 MHz, DMSO-*d*₆) δ 9.40 - 9.35 (m, 1H), 8.59 (s, 1H), 8.25 - 8.15 (m, 2H), 8.01 (s, 1H), 7.78 - 7.69 (m, 3H), 7.58 (dd, *J* = 7.4, 1.2 Hz, 1H), 7.30 - 7.24 (m, 1H), 7.17 (td, *J* = 7.7, 1.3 Hz, 1H), 7.06 (td, *J* = 7.5, 1.0 Hz, 1H), 6.72 (d, *J* = 7.8 Hz, 1H), 5.02 (s, 1H), 4.97 (s, 1H), 4.66 (s, 2H), 3.97 (s, 2H), 2.21 (s, 2H), 1.12 (s, 1H), 1.07 (s, 1H), 0.72 (s, 2H). |
| 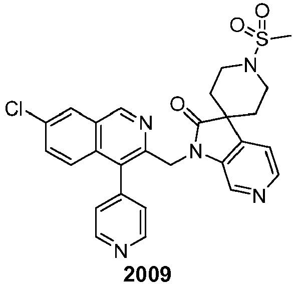 | ¹H NMR (500 MHz, DMSO-*d*₆) δ 9.29 (s, 1H), 8.85 - 8.80 (m, 2H), 8.32 (d, *J* = 2.2 Hz, 1H), 8.29 (d, *J* = 4.8 Hz, 1H), 8.16 (s, 1H), 7.77 (dd, *J* = 9.1, 2.3 Hz, 1H), 7.64 - 7.56 (m, 3H), 7.38 (d, *J* = 9.1 Hz, 1H), 4.96 (s, 2H), 3.52 - 3.37 (m, 4H), 2.97 (s, 3H), 1.93 (ddd, *J* = 13.2, 8.6, 4.1 Hz, 2H), 1.81 (dt, *J* = 9.9, 3.4 Hz, 2H). |
| 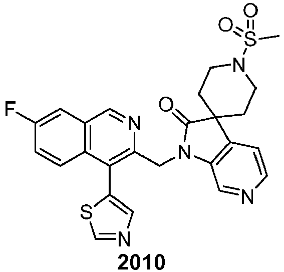 | ¹H NMR (500 MHz, DMSO-*d*₆) δ 9.46 (d, *J* = 0.8 Hz, 1H), 9.29 (s, 1H), 8.30 (d, *J* = 4.8 Hz, 1H), 8.20 - 8.12 (m, 2H), 7.99 (dd, *J* = 9.0, 2.7 Hz, 1H), 7.74 (td, *J* = 9.0, 2.7 Hz, 1H), 7.65 - 7.56 (m, 2H), 5.02 (s, 2H), 3.54 - 3.38 (m, 4H), 2.97 (s, 3H), 1.96 (ddd, *J* = 13.3, 8.9, 4.2 Hz, 2H), 1.85 (ddt, *J* = 13.7, 6.0, 3.7 Hz, 2H). |
| 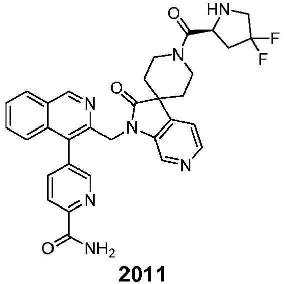 | ¹H NMR (500 MHz, DMSO-*d*₆) δ 9.40 - 9.33 (m, 1H), 8.77 - 8.70 (m, 1H), 8.72 - 8.65 (m, 1H), 8.28 (s, 1H), 8.24 (s, 0H), 8.20 (s, 1H), 7.81 - 7.69 (m, 4H), 7.66 - 7.57 (m, 1H), 7.53 (d, *J* = 4.8 Hz, 1H), 7.34 (dd, *J* = 7.8, 1.7 Hz, 1H), 5.08 (dd, *J* = 15.9, 5.7 Hz, 1H), 5.00 (s, 1H), 4.93 (dd, *J* = 15.9, 9.8 Hz, 1H), 4.23 (t, *J* = 8.0 Hz, 1H), 4.00 (d, *J* = 13.5 Hz, 1H), 3.88 (dd, *J* = 13.4, 5.4 Hz, 1H), 3.77 (dd, *J* = 9.3, 5.4 Hz, 0H), 3.72 - 3.51 (m, 1H), 3.32 - 3.13 (m, 2H), 3.05 - 2.94 (m, 1H), 2.57 - 2.37 (m, 0H), 2.41 - 2.26 (m, 0H), 1.93 - 1.70 (m, 3H). |
| 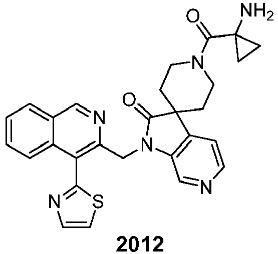 | ¹H NMR (500 MHz, DMSO-*d*₆) δ 9.38 (s, 1H), 8.25 (d, *J* = 4.8 Hz, 1H), 8.25 - 8.14 (m, 4H), 7.83 (ddd, *J* = 8.4, 6.8, 1.4 Hz, 1H), 7.76 (ddd, *J* = 8.0, 6.8, 1.1 Hz, 1H), 7.61 - 7.56 (m, 1H), 7.54 - 7.48 (m, 1H), 5.04 (s, 2H), 3.95 (s, 2H), 3.33 (s, 4H), 2.32 (s, 2H), 1.80 (d, *J* = 14.6 Hz, 4H), 0.89 (q, *J* = 4.3 Hz, 2H), 0.67 (t, *J* = 3.3 Hz, 2H). |
| 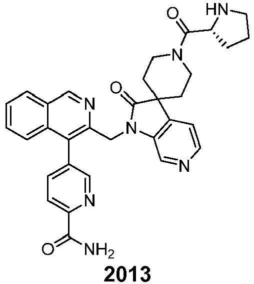 | ¹H NMR (500 MHz, DMSO-*d*₆) δ 9.37 (dd, *J* = 6.3, 3.6 Hz, 1H), 8.75 - 8.67 (m, 1H), 8.31 - 8.11 (m, 7H), 7.80 - 7.73 (m, 2H), 7.62 (t, *J* = 4.1 Hz, 1H), 7.56 (d, *J* = 4.8 Hz, 1H), 7.34 (d, *J* = 8.0 Hz, 1H), 5.11 - 4.90 (m, 2H), 3.96 (dt, *J* = 11.1, 4.8 Hz, 1H), 3.91 - 3.75 (m, 2H), 3.71 (tq, *J* = 15.0, 9.6, 7.0 Hz, 1H), 3.58 (ddt, *J* = 13.4, 9.7, 4.7 Hz, 1H), 3.04 - 2.97 (m, 1H), 2.67 - 2.58 (m, 1H), 2.01 (s, 1H), 2.07 - 1.95 (m, 1H), 1.84 (ddd, *J* = 13.2, 8.7, 4.2 Hz, 1H), 1.70 (s, 2H), 1.79 - 1.51 (m, 2H). |
| 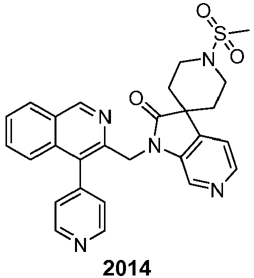 | ¹H NMR (500 MHz, DMSO-*d*₆) δ 9.33 - 9.28 (m, 1H), 8.84 - 8.79 (m, 2H), 8.28 (d, *J* = 4.8 Hz, 1H), 8.21 - 8.15 (m, 1H), 8.17 (s, 1H), 7.74 (dddd, *J* = 20.8, 8.0, 6.9, 1.3 Hz, 2H), 7.59 (ddd, *J* = 16.2, 4.6, 1.2 Hz, 3H), 7.38 - 7.32 (m, 1H), 4.97 (s, 2H), 3.48 (ddd, *J* = 12.3, 8.8, 3.6 Hz, 2H), 3.45 - 3.37 (m, 2H), 2.97 (s, 3H), 1.93 (ddd, *J* = 13.2, 8.8, 4.2 Hz, 2H), 1.81 (ddt, *J* = 13.6, 6.1, 3.6 Hz, 2H). |
| 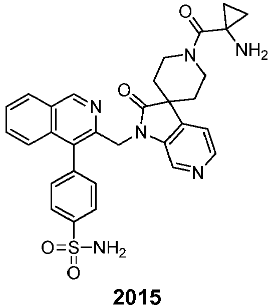 | ¹H NMR (500 MHz, DMSO-*d*₆) δ 9.31 - 9.27 (m, 1H), 8.27 (d, *J* = 4.8 Hz, 1H), 8.20 - 8.14 (m, 1H), 8.12 (s, 1H), 8.08 - 8.03 (m, 2H), 7.80 - 7.68 (m, 3H), 7.62 - 7.56 (m, 1H), 7.53 (s, 2H), 7.33 (dd, *J* = 8.4, 1.2 Hz, 1H), 4.95 (s, 2H), 2.33 - 2.29 (m, 2H), 1.80 - 1.68 (m, 4H), 0.88 (q, *J* = 4.4 Hz, 2H), 0.66 (t, *J* = 3.3 Hz, 2H). |
| 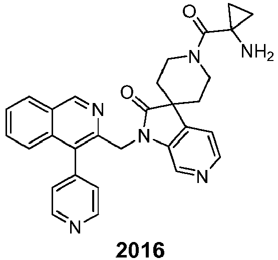 | ¹H NMR (500 MHz, DMSO-*d*₆) δ 9.35 - 9.30 (m, 1H), 8.84 - 8.78 (m, 2H), 8.26 (d, *J* = 4.7 Hz, 1H), 8.21 - 8.15 (m, 1H), 8.16 (s, 1H), 7.74 (dddd, *J* = 19.8, 8.1, 6.9, 1.3 Hz, 2H), 7.58 (ddd, *J* = 6.0, 4.5, 1.2 Hz, 3H), 7.38 - 7.32 (m, 1H), 4.97 (s, 2H), 3.92 (s, 2H), 3.33 (s, 6H), 2.30 (s, 2H), 1.77 (s, 1H), 1.72 (s, 2H), 0.88 (q, *J* = 4.3 Hz, 2H), 0.67 (t, *J* = 3.3 Hz, 2H). |
| 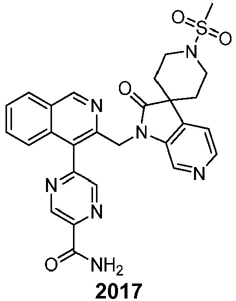 | ¹H NMR (500 MHz, DMSO-*d*₆) δ 9.47 (s, 1H), 9.38 (d, *J* = 1.4 Hz, 1H), 9.02 (d, *J* = 1.4 Hz, 1H), 8.46 - 8.41 (m, 1H), 8.29 - 8.21 (m, 2H), 8.01 - 7.97 (m, 1H), 7.76 (dt, *J* = 6.4, 3.4 Hz, 2H), 7.55 (d, *J* = 4.7 Hz, 1H), 7.41 (dd, *J* = 6.3, 3.3 Hz, 1H), 5.07 (s, 2H), 3.46 - 3.32 (m, 4H), 2.94 (s, 3H), 1.91 (ddd, *J* = 13.7, 9.4, 4.4 Hz, 2H), 1.74 (dt, *J* = 13.9, 4.2 Hz, 2H). |
| 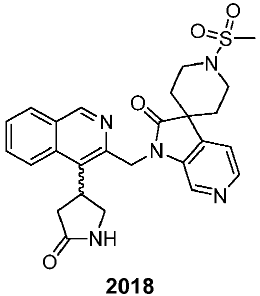 | ¹H NMR (500 MHz, DMSO-*d*₆) δ 9.10 (s, 1H), 8.41 (s, 1H), 8.30 (d, *J* = 4.8 Hz, 1H), 8.12 (dd, *J* = 8.2, 1.3 Hz, 1H), 8.07 (s, 1H), 7.93 (d, *J* = 8.6 Hz, 1H), 7.85 (ddd, *J* = 8.5, 6.8, 1.4 Hz, 1H), 7.73 - 7.59 (m, 2H), 5.36 (s, 2H), 4.77 - 4.63 (m, 1H), 3.79 (t, *J* = 10.1 Hz, 1H), 3.64 (dd, *J* = 10.0, 7.4 Hz, 1H), 3.61 - 3.48 (m, 2H), 3.51 - 3.39 (m, 2H), 2.99 (s, 3H), 2.78 - 2.59 (m, 2H), 1.96 (dtd, *J* = 28.5, 11.3, 9.3, 4.7 Hz, 4H). |
| 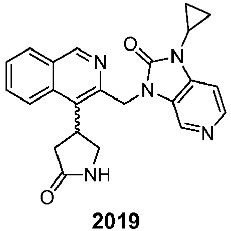 | ¹H NMR (500 MHz, DMSO-*d*₆) δ 9.15 (s, 1H), 8.43 - 8.35 (m, 1H), 8.34 (s, 1H), 8.20 (d, *J* = 5.2 Hz, 1H), 8.14 (d, *J* = 8.1 Hz, 1H), 8.06 (s, 1H), 7.90 (d, *J* = 8.6 Hz, 1H), 7.85 (t, *J* = 7.7 Hz, 1H), 7.70 (t, *J* = 7.3 Hz, 1H), 7.25 (d, *J* = 5.1 Hz, 1H), 5.46 (s, 2H), 4.75 (p, *J* = 10.1, 9.7 Hz, 1H), 3.73 (t, *J* = 10.1 Hz, 1H), 3.60 (t, *J* = 8.6 Hz, 1H), 3.03 - 2.95 (m, 1H), 2.61 (dt, *J* = 13.0, 7.4 Hz, 2H), 2.32 (s, 1H), 1.06 (d, *J* = 6.8 Hz, 2H), 0.89 (s, 2H), 0.81 (s, 0H). |
| 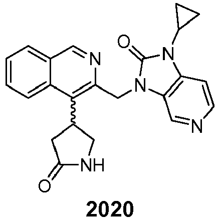 | ¹H NMR (500 MHz, DMSO-*d*₆) δ 9.15 (s, 1H), 8.33 (s, 1H), 8.20 (d, *J* = 5.2 Hz, 1H), 8.14 (d, *J* = 8.1 Hz, 1H), 8.06 (s, 1H), 7.94 - 7.80 (m, 2H), 7.70 (t, *J* = 7.4 Hz, 1H), 7.25 (d, *J* = 5.2 Hz, 1H), 5.46 (d, *J* = 2.1 Hz, 2H), 4.75 (qd, *J* = 10.2, 7.5 Hz, 1H), 3.73 (t, *J* = 10.1 Hz, 1H), 3.60 (dd, *J* = 9.9, 7.5 Hz, 1H), 2.99 (tt, *J* = 7.0, 3.6 Hz, 1H), 2.69 - 2.53 (m, 2H), 1.06 (td, *J* = 7.2, 5.1 Hz, 2H), 0.89 (p, *J* = 5.3, 4.8 Hz, 2H). |
| 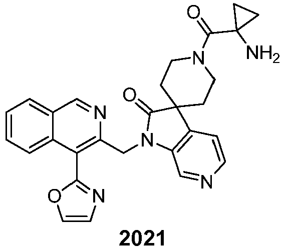 | ¹H NMR (500 MHz, DMSO-*d*₆) δ 9.35 (s, 1H), 8.70 (s, 1H), 8.65 (d, *J* = 5.5 Hz, 1H), 8.55 (d, *J* = 0.9 Hz, 1H), 8.22 (t, *J* = 7.5 Hz, 2H), 8.08 - 8.02 (m, 1H), 7.92 (ddd, *J* = 8.5, 6.8, 1.3 Hz, 1H), 7.79 (ddd, *J* = 8.0, 6.9, 1.1 Hz, 1H), 7.68 (d, *J* = 0.9 Hz, 1H), 5.40 (s, 2H), 4.10 (dt, *J* = 13.7, 4.4 Hz, 2H), 2.17 (dd, *J* = 24.5, 4.3 Hz, 1H), 2.17 (s, 1H), 1.86 (dt, *J* = 13.9, 3.8 Hz, 2H), 1.34 (s, 2H), 1.25 (q, *J* = 6.5, 5.9 Hz, 2H). |
| 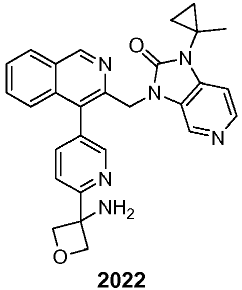 | ¹H NMR (500 MHz, DMSO-*d*₆) δ 9.33 (d, *J* = 0.8 Hz, 1H), 8.60 (dd, *J* = 2.2, 0.9 Hz, 1H), 8.23 - 8.14 (m, 2H), 8.14 (s, 1H), 7.88 (dd, *J* = 8.1, 2.3 Hz, 1H), 7.82 (dd, *J* = 8.1, 0.9 Hz, 1H), 7.79 - 7.69 (m, 2H), 7.32 (dd, *J* = 8.3, 1.3 Hz, 1H), 7.22 (dd, *J* = 5.2, 0.8 Hz, 1H), 5.13 (d, *J* = 3.0 Hz, 2H), 4.99 (dd, *J* = 6.8, 5.6 Hz, 2H), 4.62 (dd, *J* = 5.6, 1.3 Hz, 2H), 2.78 (s, 2H), 1.35 (s, 3H), 1.01 - 0.89 (m, 4H). |
| 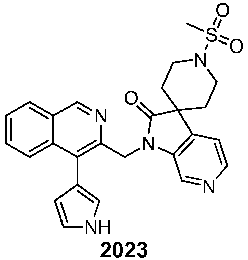 | ¹H NMR (500 MHz, DMSO-*d*₆) δ 11.29 (s, 1H), 9.08 (s, 1H), 8.24 (d, *J* = 4.8 Hz, 1H), 8.04 (dd, *J* = 8.1, 1.3 Hz, 1H), 7.97 - 7.88 (m, 2H), 7.72 (ddd, *J* = 8.4, 6.8, 1.3 Hz, 1H), 7.67 - 7.57 (m, 2H), 7.13 (q, *J* = 2.0 Hz, 1H), 7.06 (q, *J* = 2.4 Hz, 1H), 6.33 (q, *J* = 2.3 Hz, 1H), 5.13 (s, 2H), 3.54 (ddd, *J* = 12.3, 8.8, 3.7 Hz, 2H), 3.47 - 3.39 (m, 2H), 2.98 (s, 3H), 1.96 (ddd, *J* = 13.1, 8.8, 4.1 Hz, 2H), 1.88 (ddd, *J* = 13.6, 6.0, 3.8 Hz, 2H). |
| 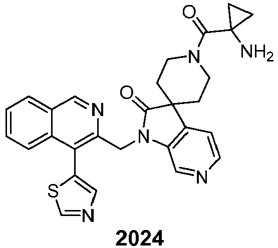 | ¹H NMR (500 MHz, DMSO-*d*₆) δ 9.46 (s, 1H), 9.33 (s, 1H), 8.31 - 8.27 (m, 1H), 8.21 - 8.12 (m, 3H), 7.82 (ddd, *J* = 8.3, 6.8, 1.3 Hz, 1H), 7.74 (ddd, *J* = 8.0, 6.9, 1.1 Hz, 1H), 7.60 (d, *J* = 4.7 Hz, 1H), 7.53 (d, *J* = 8.5 Hz, 1H), 5.05 (s, 2H), 4.71 (s, 2H), 3.98 - 3.91 (m, 2H), 3.85 (s, 2H), 1.93 - 1.73 (m, 4H), 0.99 (q, *J* = 4.7 Hz, 2H), 0.84 (q, *J* = 4.5 Hz, 2H). |
| 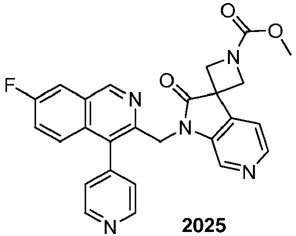 | ¹H NMR (500 MHz, DMSO-*d*₆) δ 9.35 (d, *J* = 0.8 Hz, 1H), 8.73 (d, *J* = 4.9 Hz, 2H), 8.34 (d, *J* = 4.8 Hz, 1H), 8.03 (dd, *J* = 9.0, 2.7 Hz, 1H), 8.01 (s, 1H), 7.74 (d, *J* = 4.6 Hz, 1H), 7.67 (td, *J* = 9.0, 2.7 Hz, 1H), 7.46 - 7.40 (m, 2H), 7.36 (dd, *J* = 9.3, 5.1 Hz, 1H), 5.01 (s, 2H), 4.11 (s, 2H), 3.99 (d, *J* = 8.5 Hz, 2H), 3.63 (s, 3H). |
| 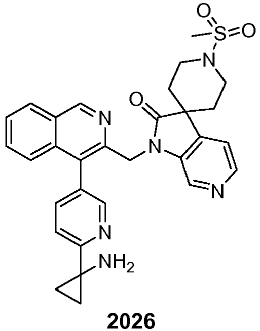 | ¹H NMR (500 MHz, DMSO-*d*₆) δ 9.31 - 9.26 (m, 1H), 8.51 - 8.46 (m, 1H), 8.28 (d, *J* = 4.8 Hz, 1H), 8.20 - 8.13 (m, 1H), 8.12 (s, 1H), 8.01 - 7.95 (m, 1H), 7.88 (dd, *J* = 8.1, 2.3 Hz, 1H), 7.73 (dddd, *J* = 21.4, 8.0, 6.9, 1.3 Hz, 2H), 7.59 (dd, *J* = 4.8, 0.8 Hz, 1H), 7.39 - 7.33 (m, 1H), 5.00 (s, 1H), 4.97 (s, 1H), 3.51 - 3.35 (m, 3H), 3.43 (s, 2H), 2.96 (s, 3H), 2.56 (s, 2H), 1.92 (ddt, *J* = 13.4, 9.0, 4.4 Hz, 2H), 1.79 (ddt, *J* = 13.7, 9.7, 4.5 Hz, 2H), 1.40 - 1.29 (m, 2H), 1.07 (d, *J* = 3.0 Hz, 2H). |
| 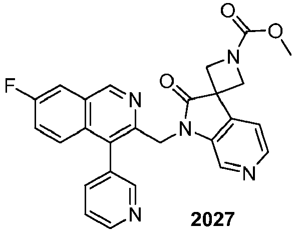 | ¹H NMR (500 MHz, DMSO-*d*₆) δ 9.34 (d, *J* = 0.8 Hz, 1H), 8.72 (dd, *J* = 4.9, 1.7 Hz, 1H), 8.56 (dd, *J* = 2.3, 0.9 Hz, 1H), 8.34 (d, *J* = 4.7 Hz, 1H), 8.08 (d, *J* = 0.8 Hz, 1H), 8.02 (dd, *J* = 9.1, 2.7 Hz, 1H), 7.88 (dt, *J* = 7.8, 1.9 Hz, 1H), 7.74 (dd, *J* = 4.7, 0.9 Hz, 1H), 7.67 (td, *J* = 9.0, 2.7 Hz, 1H), 7.59 (ddd, *J* = 7.8, 4.9, 0.9 Hz, 1H), 7.35 (dd, *J* = 9.3, 5.1 Hz, 1H), 5.01 (s, 1H), 4.98 (s, 1H), 4.12 (s, 2H), 3.63 (s, 3H), 3.31 (d, *J* = 11.9 Hz, 1H). |
| 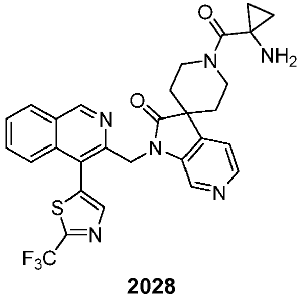 | ¹H NMR (500 MHz, DMSO-*d*₆) δ 9.41 (s, 1H), 8.37 (d, *J* = 1.2 Hz, 1H), 8.30 - 8.19 (m, 3H), 7.84 (ddd, *J* = 8.3, 6.9, 1.4 Hz, 1H), 7.77 (ddd, *J* = 8.0, 6.9, 1.1 Hz, 1H), 7.63-7.55 (m, 2H), 5.10 (s, 2H), 3.93 (s, 2H), 2.34 (s, 2H), 1.76 (d, *J* = 33.7 Hz, 4H), 0.89 (q, *J* = 4.4 Hz, 2H), 0.67 (t, *J* = 3.2 Hz, 2H). |
| 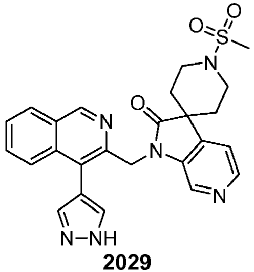 | ¹H NMR (500 MHz, DMSO-*d*₆) δ 13.33 (s, 1H), 9.16 (s, 1H), 8.26 (d, *J* = 4.8 Hz, 1H), 8.17 (s, 1H), 8.09 (d, *J* = 8.1 Hz, 1H), 8.00 (s, 1H), 7.82 - 7.71 (m, 3H), 7.68 (ddd, *J* = 8.0, 5.8, 2.1 Hz, 1H), 7.61 (d, *J* = 4.8 Hz, 1H), 5.09 (s, 2H), 3.53 (ddd, *J* = 12.4, 9.0, 3.7 Hz, 2H), 3.43 (dt, *J* = 11.8, 4.9 Hz, 2H), 2.98 (s, 3H), 1.96 (ddd, *J* = 13.3, 8.8, 4.1 Hz, 2H), 1.86 (dt, *J* = 13.9, 4.4 Hz, 2H). |
| 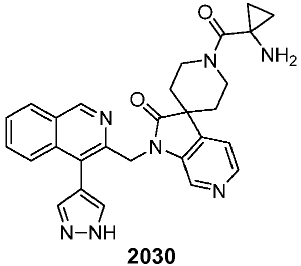 | ¹H NMR (500 MHz, DMSO-*d*₆) δ 13.32 (s, 1H), 9.16 (s, 1H), 8.24 (d, *J* = 4.8 Hz, 1H), 8.10 (dt, *J* = 8.2, 1.0 Hz, 1H), 7.98 (s, 2H), 7.80 - 7.71 (m, 2H), 7.68 (ddd, *J* = 8.0, 5.8, 2.1 Hz, 1H), 7.59 (dd, *J* = 4.8, 0.8 Hz, 1H), 5.10 (s, 2H), 3.93 (s, 4H), 2.31 (s, 2H), 1.78 (p, *J* = 7.5 Hz, 4H), 0.89 (q, *J* = 4.3 Hz, 2H), 0.67 (t, *J* = 3.3 Hz, 2H). |
| 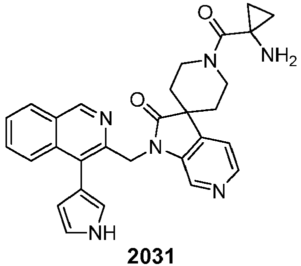 | ¹H NMR (500 MHz, DMSO-*d*₆) δ 11.30 (q, *J* = 2.5 Hz, 1H), 9.08 (s, 1H), 8.22 (d, *J* = 4.8 Hz, 1H), 8.05 (dd, *J* = 8.1, 1.3 Hz, 1H), 7.95-7.89 (m, 1H), 7.90 (s, 1H), 7.73 (ddd, *J* = 8.4, 6.8, 1.3 Hz, 1H), 7.64 (ddd, *J* = 8.0, 6.8, 1.1 Hz, 1H), 7.61 - 7.56 (m, 1H), 7.13 (q, *J* = 2.0 Hz, 1H), 7.06 (q, *J* = 2.4 Hz, 1H), 6.34 (q, *J* = 2.3 Hz, 1H), 5.14 (s, 2H), 2.39 - 2.33 (m, 3H), 1.79 (s, 4H), 0.90 (q, *J* = 4.3 Hz, 2H), 0.70 - 0.64 (m, 2H). |
| 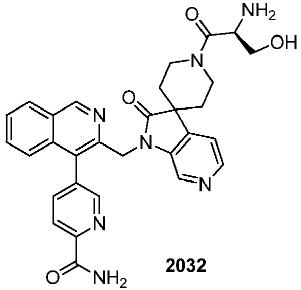 | ¹ H NMR (500 MHz, DMSO-*d*₆) δ 9.37 (s, 1H), 8.74 (s, 1H), 8.69 (s, 1H), 8.28 (s, 1H), 8.26 (s, 1H), 7.80 - 7.70 (m, 3H), 7.53 (dd, *J* = 13.3, 4.8 Hz, 1H), 7.37 - 7.31 (m, 1H), 5.07 (dd, *J* = 15.9, 4.0 Hz, 1H), 5.02 - 4.89 (m, 2H), 4.75 (s, 1H), 4.70 (s, 1H), 3.95 - 3.87 (m, 1H), 3.80 (t, *J* = 5.7 Hz, 2H), 3.75 (t, *J* = 6.4 Hz, 1H), 3.69 - 3.60 (m, 1H), 3.46 (s, 1H), 1.80 (s, 1H), 1.74 (s, 2H), 1.71 (d, *J* = 14.7 Hz, 1H), 1.64 (s, 1H). |
| 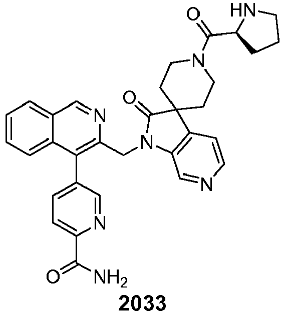 | ¹H NMR (500 MHz, DMSO-*d*₆) δ 9.37 (dd, *J* = 6.3, 3.6 Hz, 2H), 8.75 - 8.67 (m, 2H), 8.25 (s, 2H), 8.20 (s, 0H), 8.17 (d, *J* = 5.7 Hz, 1H), 7.76 (d, *J* = 5.8 Hz, 1H), 7.63 (t, *J* = 4.1 Hz, 1H), 7.56 (d, *J* = 4.8 Hz, 1H), 7.34 (dd, *J* = 8.7, 3.1 Hz, 2H), 5.11 - 4.90 (m, 3H), 3.96 (dd, *J* = 12.8, 5.7 Hz, 1H), 3.90 - 3.64 (m, 4H), 3.58 (tt, *J* = 13.3, 3.9 Hz, 1H), 3.01 (h, *J* = 4.7 Hz, 2H), 2.69 - 2.59 (m, 1H), 2.08 - 1.94 (m, 2H), 1.85 (d, *J* = 14.5 Hz, 1H), 1.79 - 1.57 (m, 3H), 1.61 - 1.52 (m, 0H), 1.56 (s, 1H), 1.23 (s, 0H). |
| 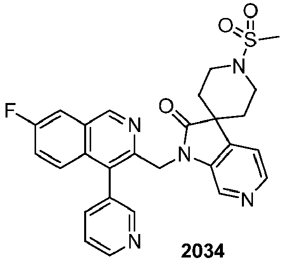 | ¹H NMR (500 MHz, DMSO-*d*₆) δ 9.29 (s, 1H), 8.77 (dd, *J* = 4.9, 1.7 Hz, 1H), 8.69 (dd, *J* = 2.1, 0.9 Hz, 1H), 8.29 (d, *J* = 4.7 Hz, 1H), 8.18 (s, 1H), 7.99 (ddd, *J* = 9.8, 4.5, 2.3 Hz, 2H), 7.72 - 7.62 (m, 2H), 7.63 - 7.58 (m, 1H), 7.41 (dd, *J* = 9.3, 5.2 Hz, 1H), 4.98 (s, 1H), 4.95 (s, 1H), 3.44 (dddd, *J* = 23.0, 17.6, 10.7, 5.2 Hz, 4H), 2.97 (s, 3H), 1.92 (ddd, *J* = 13.4, 8.9, 4.3 Hz, 2H), 1.85 - 1.76 (m, 2H). |
| 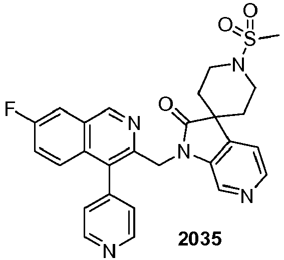 | ¹H NMR (500 MHz, DMSO) δ = 1.81 (d, 2H), 1.92 (m, 2H), 2.97 (s, 3H), 3.44 (m, 4H), 4.96 (s, 2H), 7.42 (dd, 1H), 7.58 (d, 2H), 7.61 (d, 1H), 7.69 (dt, 1H), 8.00 (d, 1H), 8.16 (s, 1H), 8.29 (d, 1H), 8.82 (d, 2H), 9.29 (s, 1H). |
| 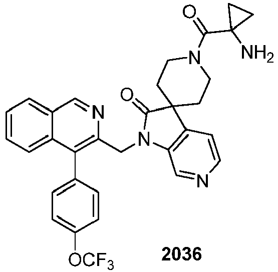 | ¹H NMR (500 MHz, DMSO-*d*₆) δ 9.29 (s, 1H), 8.26 (d, *J* = 4.7 Hz, 1H), 8.19 - 8.13 (m, 1H), 8.13 (s, 1H), 7.73 (dddd, *J* = 24.8, 8.0, 6.8, 1.3 Hz, 2H), 7.69 - 7.56 (m, 4H), 7.35 (dd, *J* = 8.4, 1.2 Hz, 1H), 4.96 (s, 2H), 3.33 (s, 8H), 2.31 (s, 2H), 1.74 (t, *J* = 16.4 Hz, 3H), 0.88 (q, *J* = 4.4 Hz, 2H), 0.67 (t, *J* = 3.2 Hz, 2H). |
| 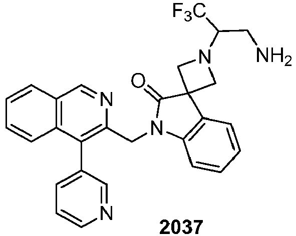 | ¹H NMR (500 MHz, DMSO-*d*₆) δ 9.33 (t, *J* = 1.0 Hz, 1H), 8.70 (dt, *J* = 4.9, 1.5 Hz, 1H), 8.53 (ddd, *J* = 6.5, 2.3, 0.9 Hz, 1H), 8.21 - 8.14 (m, 1H), 7.86 - 7.79 (m, 1H), 7.77 - 7.67 (m, 2H), 7.66 (dd, *J* = 7.4, 1.3 Hz, 1H), 7.61 - 7.54 (m, 1H), 7.29 - 7.23 (m, 1H), 7.18 - 7.11 (m, 1H), 7.05 (td, *J* = 7.5, 1.0 Hz, 1H), 6.67 (dd, *J* = 7.8, 2.6 Hz, 1H), 5.01 - 4.89 (m, 2H), 3.75 (d, *J =* 6.6 Hz, 1H), 3.62 (dd, *J* = 6.7, 3.7 Hz, 1H), 3.54 (dt, *J* = 19.1, 6.4 Hz, 2H), 3.12 - 3.03 (m, 1H), 2.86 (dd, *J* = 13.9, 5.0 Hz, 1H), 2.79 (dd, *J* = 14.1, 5.0 Hz, 1H). |
| 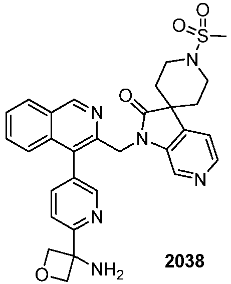 | ¹H NMR (500 MHz, DMSO-*d*₆) δ 9.32 (s, 1H), 8.70 (d, *J* = 2.2 Hz, 1H), 8.28 (d, *J* = 4.8 Hz, 1H), 8.19 (s, 2H), 8.18 (d, *J* = 1.4 Hz, 0H), 7.99 (dd, *J* = 8.1, 2.3 Hz, 1H), 7.90 (d, *J* = 8.1 Hz, 1H), 7.80 - 7.69 (m, 2H), 7.60 (d, *J* = 4.8 Hz, 1H), 7.37 (d, *J* = 8.4 Hz, 1H), 5.02 (dd, *J* = 18.0, 6.1 Hz, 4H), 4.65 (d, *J =* 5.6 Hz, 2H), 3.52 - 3.36 (m, 4H), 2.96 (s, 3H), 2.90 (s, 2H), 1.92 (ddd, *J* = 13.5, 8.9, 4.5 Hz, 2H), 1.81 (dt, *J* = 14.0, 4.5 Hz, 2H). |
| 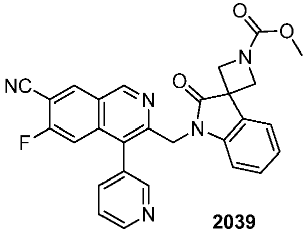 | ¹H NMR (500 MHz, DMSO-*d*₆) δ 9.42 (s, 1H), 9.04 (d, *J* = 6.6 Hz, 1H), 8.74 (dd, *J* = 4.9, 1.6 Hz, 1H), 8.59 (d, *J* = 2.2 Hz, 1H), 7.88 (dt, *J* = 7.8, 2.0 Hz, 1H), 7.68 - 7.57 (m, 2H), 7.23 - 7.14 (m, 2H), 7.07 (td, *J* = 7.5, 0.9 Hz, 1H), 6.72 (d, *J* = 7.8 Hz, 1H), 5.03 - 4.91 (m, 2H), 4.09 (s, 2H), 4.05 (s, 2H), 3.63 (s, 3H). |
| 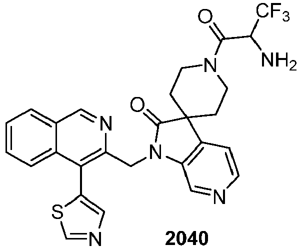 | ¹H NMR (500 MHz, DMSO-*d*₆) δ 9.45 (d, *J* = 1.6 Hz, 1H), 9.32 (s, 1H), 8.28 (dd, *J* = 8.1, 4.8 Hz, 1H), 8.21 - 8.13 (m, 3H), 7.82 (ddd, *J* = 8.4, 6.8, 1.3 Hz, 1H), 7.74 (ddd, *J* = 8.0, 6.9, 1.1 Hz, 1H), 7.64 (d, *J* = 4.8 Hz, 1H), 7.58 - 7.50 (m, 2H), 5.07 - 4.98 (m, 2H), 4.68 (s, 1H), 4.07 - 3.92 (m, 1H), 3.94 - 3.80 (m, 2H), 3.71 (ddd, *J* = 13.3, 9.5, 3.6 Hz, 1H), 2.36 (d, *J* = 9.5 Hz, 1H), 2.35 (s, 2H), 1.91 (dtd, *J* = 14.5, 10.0, 9.5, 4.0 Hz, 1H), 1.85 - 1.71 (m, 2H), 0.98 (s, 1H). |
| 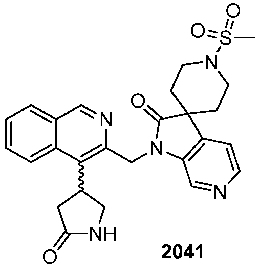 | ¹H NMR (500 MHz, DMSO-*d*₆) δ 9.10 (s, 1H), 8.41 (s, 1H), 8.30 (d, *J* = 4.8 Hz, 1H), 8.12 (d, *J* = 8.1 Hz, 1H), 8.07 (s, 1H), 7.93 (d, *J* = 8.7 Hz, 1H), 7.85 (ddd, *J* = 8.4, 6.7, 1.3 Hz, 1H), 7.73 - 7.62 (m, 2H), 5.36 (s, 2H), 4.70 (p, *J* = 9.9 Hz, 1H), 3.79 (t, *J* = 10.1 Hz, 1H), 3.64 (dd, *J* = 10.0, 7.4 Hz, 1H), 3.55 (dq, *J* = 11.9, 3.5 Hz, 2H), 3.44 (dt, *J* = 11.9, 5.1 Hz, 2H), 2.99 (s, 3H), 2.78 - 2.63 (m, 2H), 2.04 - 1.87 (m, 4H). |
| 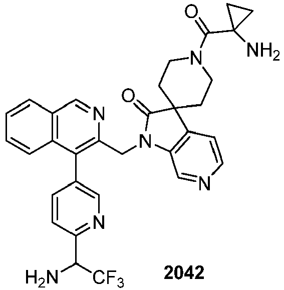 | ¹H NMR (500 MHz, DMSO-*d*₆) δ 9.32 (d, *J* = 2.4 Hz, 1H), 8.74 (t, *J* = 2.7 Hz, 1H), 8.27 (d, *J* = 4.8 Hz, 1H), 8.18 (t, *J* = 4.0 Hz, 2H), 8.08 (dt, *J* = 8.0, 2.0 Hz, 1H), 7.87 - 7.69 (m, 3H), 7.59 (d, *J* = 4.8 Hz, 1H), 7.33 - 7.27 (m, 1H), 5.00 (dd, *J* = 16.1, 5.9 Hz, 1H), 4.93 (dd, *J* = 16.1, 4.5 Hz, 1H), 4.76 (p, *J* = 8.0 Hz, 1H), 3.91 (s, 3H), 1.88 (s, 2H), 1.78 (s, 1H), 1.75 - 1.68 (m, 1H), 0.88 (t, *J* = 3.4 Hz, 2H), 0.66 (t, *J* = 3.2 Hz, 2H). |
| 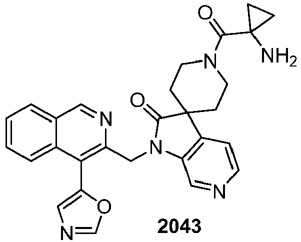 | ¹H NMR (500 MHz, DMSO-*d*₆) δ 9.34 (s, 1H), 8.73 (s, 1H), 8.26 (d, *J* = 4.7 Hz, 1H), 8.22 - 8.16 (m, 1H), 8.12 (s, 1H), 7.86 (ddd, *J* = 8.3, 6.9, 1.3 Hz, 1H), 7.81 - 7.71 (m, 3H), 7.61 (d, *J* = 4.7 Hz, 1H), 5.17 (s, 2H), 3.33 (s, 9H), 1.81 (s, 2H), 1.81 - 1.73 (m, 2H), 0.90 (p, *J* = 4.1, 3.4 Hz, 2H), 0.67 (t, *J* = 3.3 Hz, 2H). |

### Example A: RSV cytopathic effect

Compounds of the invention are initially tested in a cytopathic effect (CPE)-based viral replication assay using immortalized cells and a laboratory strain of RSV (Long). This assay evaluates the ability of a compound to inhibit viral replication.

### Procedure:

Assay plates are prepared by seeding 2,500 HEp-2 cells (ATCC) per well of a 384-well black clear-bottom plate (Greiner Bio-One) in 20 µL of assay media (defined as DMEM supplemented with 2% heat-inactivated fetal bovine serum and 1% Penicillin/Streptomycin). Assay plates are incubated overnight at 37°C in an incubator containing 5% CO₂. The following day, a 10-point serial dilution of test compound is prepared in DMSO. Compounds are subsequently diluted with assay media and 20 µL of diluted compound (containing 1.5% DMSO) is transferred to an assay plate for evaluation of antiviral activity.

For the CPE assay, cells are infected at a Multiplicity of Infection (MOI) of 0.015 using 20 µL of RSV Long (ATCC) diluted in assay media. The DMSO concentration is constant throughout the assay plate, including the negative and positive controls. The assay plate is incubated for 3 days at 37°C in an incubator containing 5% CO₂. Cell viability is evaluated with the addition of 10 µL of CellTiter-Glo (ProMega). Luminescence is measured using an EnVision plate reader (Perkin Elmer). EC₅₀ values are calculated using the raw data from the CPE assays.

The compounds of the invention were tested in the assay described in Example A, the results are as shown in the TABLE 3.

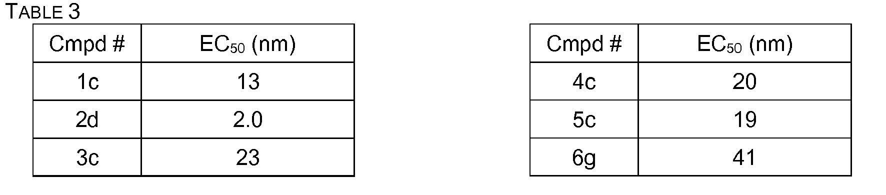

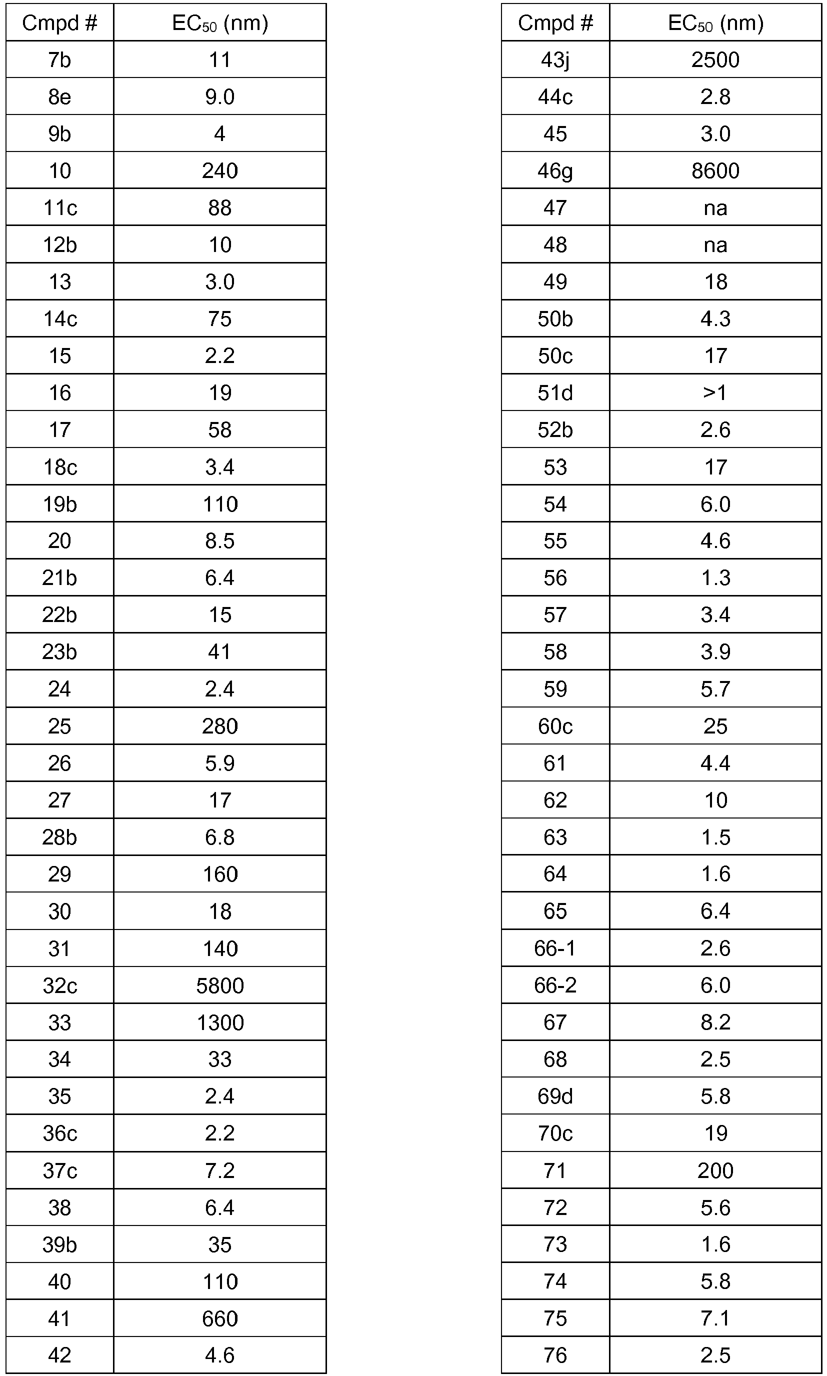

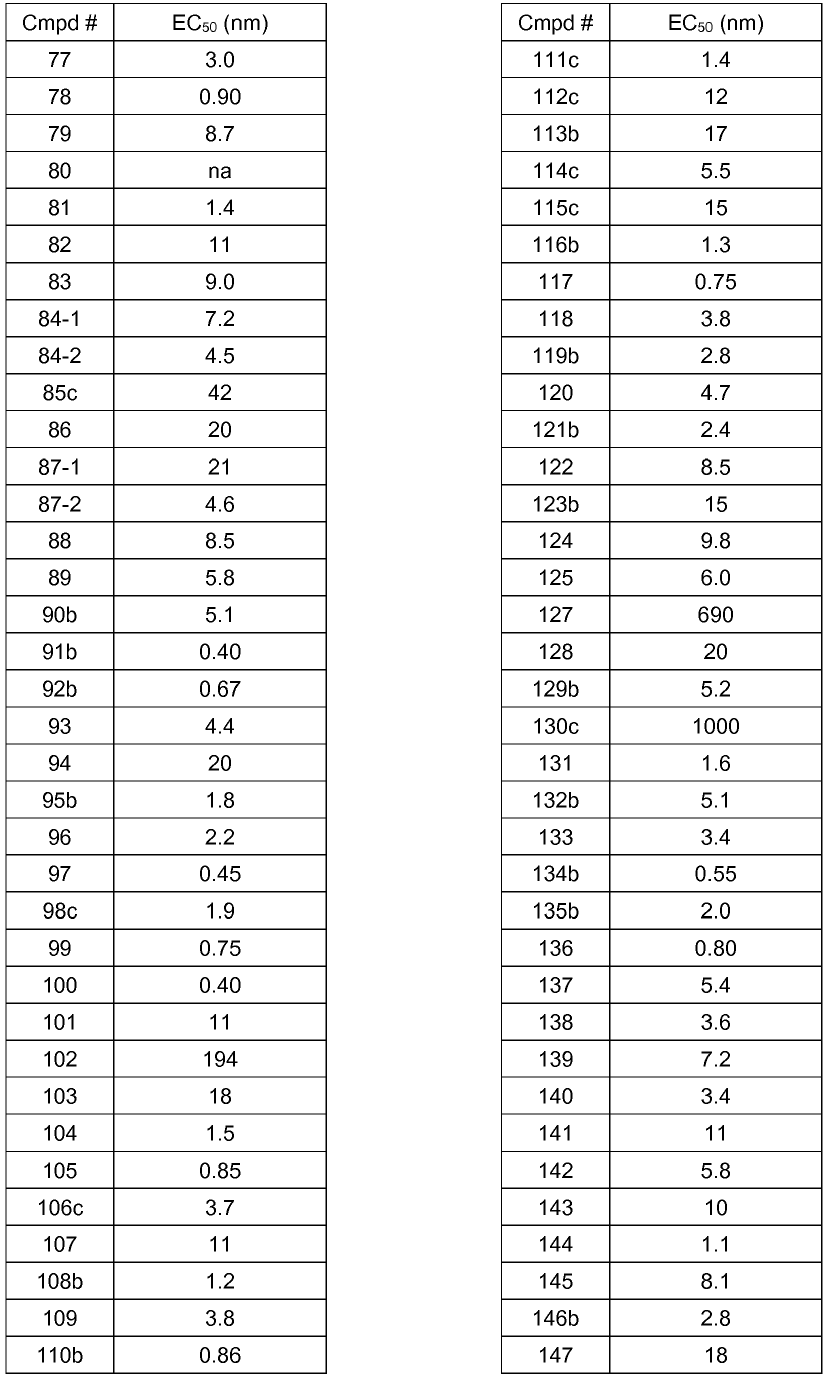

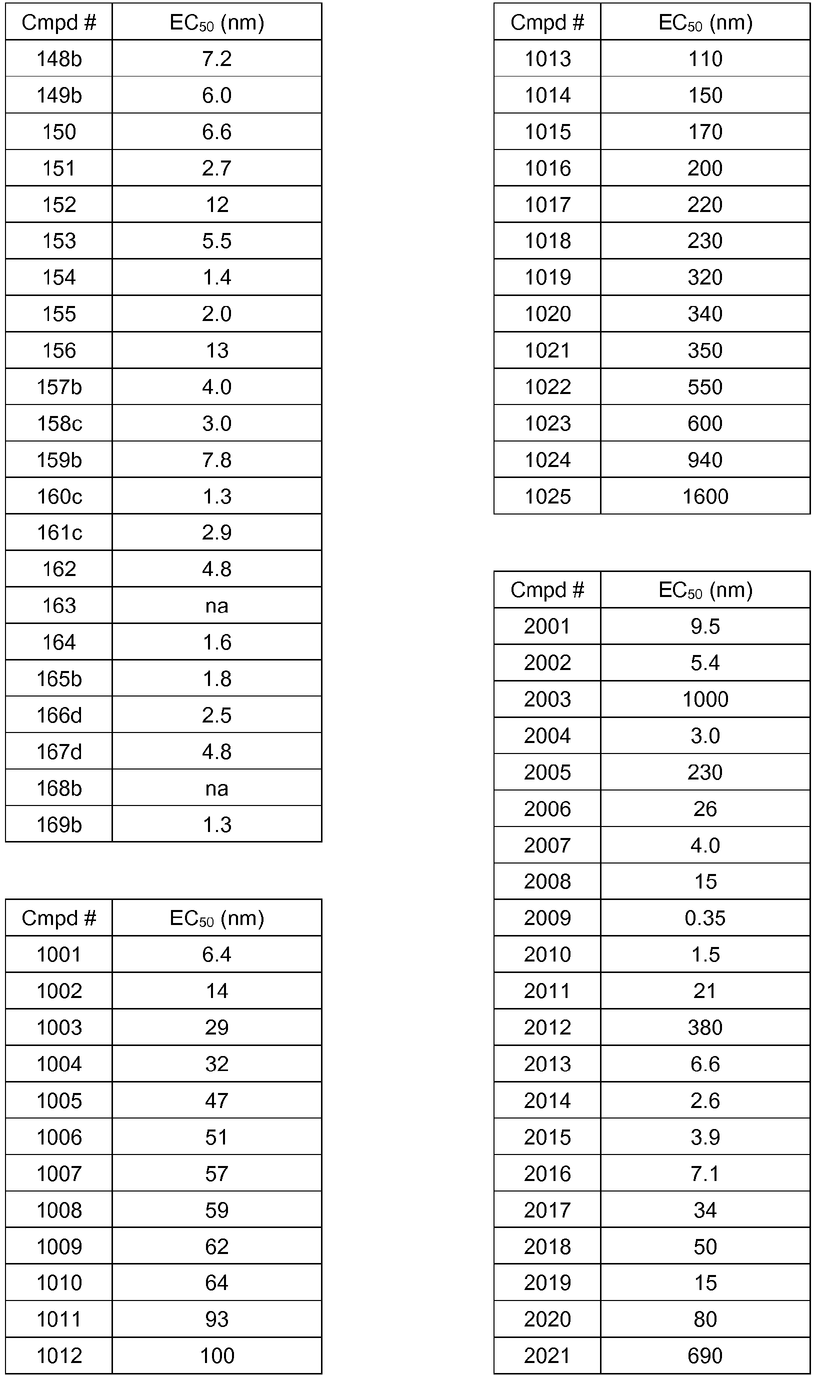

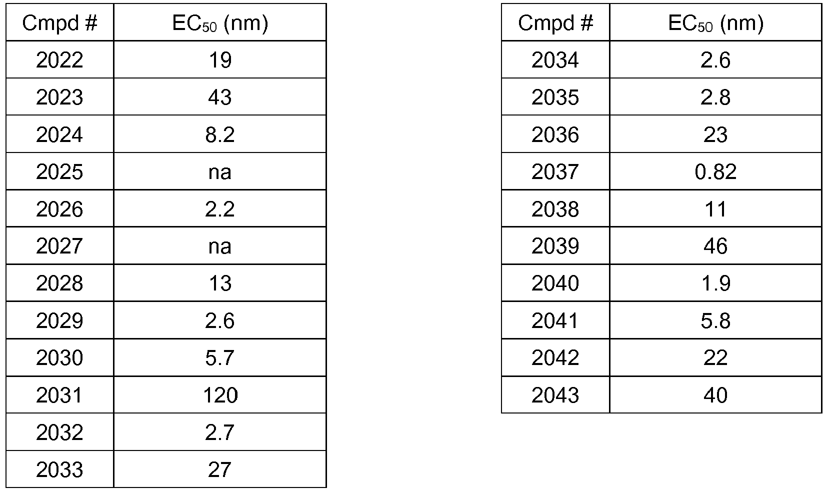

## Claims

1. A compound having Formula (I): 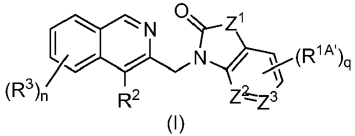 wherein
Z¹ is NR^{1A}, CHR^{1A}, CR^{1B}R^{1B};
one of Z² and Z³ is CH or CR^{1A'}, the other is N, CH or CR^{1A'};
R^{1A} is C₁-C₆alkyl, C₃-C₇cycloalkyl, S(=O)₂R^{1C}, aryl, heteroaryl, heterocyclyl or a 7 or 8-membered spiroheterocyclyl, wherein each said alkyl, cycloalkyl, aryl, heteroaryl, heterocyclyl and spiroheterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1C}, -NR^{1D}R^{1D'}, -C(=O)OH, -C(=O)R^{1C}, -C(=O)C₁-C₆alkyleneNH₂, -C(=O)OR^{1C}, -C(=O)NHR^{1C}, -C(=O)NR^{1D}R^{1D'}, -S(=O)₂R^{1C}, S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} or -NHS(=O)₂R^{1C};
the two R^{1B} together with the carbon atom to which they are attached combine and form a C₃-C₆cycloalkyl or heterocyclyl, wherein the cycloalkyl and heterocyclyl are optionally mono-, di- or tri-substituted with substituents each independently selected from the group consisting of C₁-C₆alkyl, C₁-C₆haloalkyl, halo, C₁-C₆alkoxy, hydroxy, cyano, amino, -NHR^{1c}, -NR^{1D}R^{1D}', -C(=O)OH, -C(=O)R^{1C}, -C(=O)OR^{1C}, -C(=O)NHR^{1C}, -C(=O)NR^{1D}R^{1D'}, -S(=O)₂R^{1C}, S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} or -NHS(=O)₂R^{1C};
each R^{1A'} is independently selected from halo, hydroxy, cyano, C₁-C₃haloalkyl, C₁-C₃alkoxy;
R^{1C} is C₁-C₆alkyl, C₃-C₇cycloalkyl or heterocyclyl, any of which is optionally substituted with one or two substituents independently selected from halo, hydroxy, cyano, amino, trifluoromethyl, C₁-C₃alkyl, C₁-C₃alkoxy, C₁-C₃haloalkyl, C₁-C₃alkylamino and C₁-C₃dialkylamino;
R^{1D} and R^{1D'} are each independently H or C₁-C₆alkyl, or
R^{1D} and R^{1D'} together with the nitrogen atom to which they are attached form a 4 to 6 membered ring which ring is optionally substituted with one or two substituents independently selected from halo, hydroxy, cyano and amino;
R² is C₁-C₆alkyl which is substituted with one, two or three substituents each independently selected from halo, hydroxy, cyano, trifluoromethyl, amino, -NHR^{2A}, -NR^{2B}R^{2B'}, C₁-C₃alkoxy, S(=O)₂R^{2A}, C₃-C₄cycloalkoxy and heterocycloxy, wherein each said alkoxy, cycloalkoxy and heterocycloxy is optionally mono-, di- or tri-substituted with substituents each independently selected from oxo, halo, hydroxy, cyano, amino, C₁-C₃alkyl, C₁-C₃haloalkyl, C₁-C₃alkoxy, C₁-C₃haloalkoxy, hydroxyC₁-C₃alkoxy, C₁-C₃alkylamino and -S(=O)₂R^{2A}, or
R² is C₂-C₆alkyl, C₃-C₇cycloalkylC₀-C₅alkyl, heterocyclylC₀-C₅alkyl, arylC₀-C₅alkyl or heteroarylC₀-C₅alkyl wherein heterocyclyl is a 4 to 8 membered saturated mono-, bi- or spirocyclic ring, and wherein each said cycloalkyl, heterocyclyl, aryl and heteroaryl is optionally mono-, di- or tri-substituted with substituents each independently selected from oxo, halo, hydroxy, cyano, amino, C₁-C₃alkyl, C₁-C₃haloalkyl, C₁-C₃hydroxyalkyl, C₁-C₃alkoxy, C₁-C₃haloalkoxy, hydroxyC₁-C₃alkoxy, C₁-C₃alkylamino, C₃-C₄cycloalkyl, oxetanyl, -S(=O)₂R^{2A}, -S(=O)₂NH₂, -NHS(=O)₂R^{2A} and -C(=O)NH₂, and the cycloalkyl and oxetanyl is optionally substituted with amino or methyl;
R^{2A} is C₁-C₃alkyl, C₃-C₄cycloalkyl, amino, aryl, heteroaryl or heterocyclyl;
R^{2B} and R^{2B'} are each independently C₁-C₃alkyl, or
R^{2B} and R^{2B'} together with the nitrogen atom to which they are attached combine and form a 4 to 6 membered heterocyclyl, which heterocyclyl is optionally substituted with one or two substituents independently selected from amino, halo, C₁-C₃alkyl and trifluoromethyl;
R³ is each independently selected from the group consisting of halo, hydroxy, cyano, amino, C₁-C₆alkyl, C₁-C₆alkoxy, C₃-C₇cycloalkylC₀-C₂alkyl or heterocyclylC₀-C₂alkyl wherein the alkyl, alkoxy, cycloalkyl and heterocyclyl is optionally substituted with 1, 2 or 3 substituents independently selected from -NR^{3A}R^{3B}, halo, hydroxy and trifluoromethyl;
R^{3A} and R^{3B} are each independently H or C₁-C₆alkyl, wherein the alkyl is optionally substituted with one or two halo;
n is 0, 1 or 2;
q is 0, 1 or 2;
heterocyclyl is a saturated 4 to 7 membered mono- or bi- cyclic ring containing 1, 2 or 3 heteroatoms each independently selected from O, S and N, unless otherwise specified; or a salt thereof.

2. The compound according to claim 1, wherein Z¹ is CR^{1B}R^{1B}.

3. The compound according to any one of claims 1 to 2, wherein q is 0.

4. The compound according to any one of claims 1 to 3, wherein Z² is CH, Z³ is N.

5. The compound according to any one of claims 1 to 3, wherein Z² and Z³ both are CH.

6. The compound according to any one of claims 1 to 5, wherein n is 1 and R³ is C₁-C₃alkyl, halo or trifluoromethyl.

7. The compound according to claim 6, wherein R³ is methyl, chloro, fluoro or trifluoromethyl.

8. The compound according to claim 6 or 7, wherein R³ is located in the 7-position of the isoquinoline moiety, thus providing compounds of the general formula:

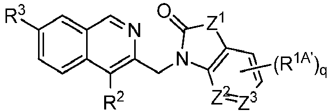

9. The compound according to any one of claims 1 to 8, wherein R² is heteroaryl which is optionally substituted with one or two substituents.

10. The compound according to claim 9, wherein R² is thiazolyl or optionally substituted pyridinyl.

11. The compound according claim 10, wherein R² is pyridin-3-yl or pyridin-4-yl any of which is optionally substituted.

12. The compound according to claim 1, having the structure IIb' or IIb" 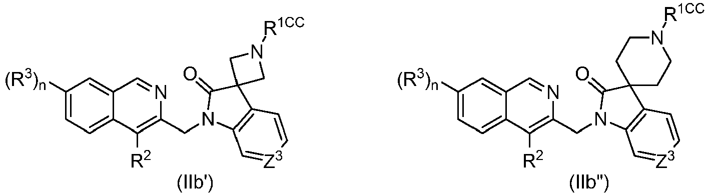 wherein
Z³ is N or CH;
R^{1CC} is -C(=O)R^{1C}, -C(=O)OR^{1C}, -S(=O)₂R^{1C}, wherein
R^{1C} is C₁-C₄alkyl or C₃-C₆cycloalkyl any of which is optionally substituted with methyl, amino or trifluoromethyl;
R² is thiazolyl, pyridinyl or pyridinyl which is substituted with cyano, -NHS(=O)₂Me, C(=O)NH₂, S(=O)₂NH₂ or fluoro;
R³ is C₁-C₃alkyl, halo, cyano or C₁-C₃haloalkyl;
n is 0 or 1.

13. The compound according to claim 12, wherein
R^{1C} is methyl or cyclopropyl wherein cyclopropyl is optionally substituted with methyl, amino or trifluoromethyl;
R² is thiazol-5-yl, pyrid-3-yl or pyrid-4-yl;
R³ is methyl, chloro, fluoro or trifluoromethyl.

14. The compound according to any one of claims 1 to 13, or a pharmaceutically acceptable salt thereof, for use in a method for the treatment or prevention of RSV infection in a human being.

15. A pharmaceutical composition comprising a compound according to any one of claims 1 to 13, or a pharmaceutically acceptable salt thereof, and a pharmaceutically acceptable carrier.

## Patentansprüche

1. Verbindung mit der Formel (I): 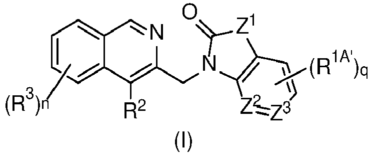 wobei
Z¹ NR^{1A}, CHR^{1A}, CR^{1B}R^{1B} darstellt;
eines von Z² und Z³ ist CH oder CR^{1A'}, das andere ist N, CH oder CR^{1A} ;
R^{1A} ist C₁-C₆-Alkyl, C₃-C₇-Cycloalkyl, S(=O)₂R^{1C}, Aryl, Heteroaryl, Heterocyclyl oder ein 7- oder 8-gliedriges Spiroheterocyclyl, wobei jedes Alkyl, Cycloalkyl, Aryl, Heteroaryl, Heterocyclyl und Spiroheterocyclyl gegebenenfalls mit Substituenten mono-, di- oder trisubstituiert ist, jeweils unabhängig ausgewählt aus der Gruppe, bestehend aus C₁-C₆-Alkyl, C₁-C₆-Haloalkyl, Halo, C₁-C₆-Alkoxy, Hydroxy, Cyano, Amino, -NHR^{1C}, -NR^{1D}R^{1D'}, -C(=O)OH, -C(=O)R^{1C}, -C(=O)C₁-C₆-Alkylen-NH₂, -C(=O)OR^{1C}, -C(=O)NHR^{1C}, -C(=O)NR^{1D}R ^{1D'}, -S(=O)₂R^{1C}, S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} oder -NHS(=O)₂R^{1C};
die zwei R^{1B} sind kombiniert und bilden zusammen mit dem Kohlenstoffatom, mit dem sie verknüpft sind, ein C₃-C₆-Cycloalkyl oder Heterocyclyl, wobei das Cycloalkyl und Heterocyclyl gegebenenfalls mit Substituenten mono-, di- oder trisubstituiert ist, jeweils unabhängig ausgewählt aus der Gruppe, bestehend aus C₁-C₆-Alkyl, C₁-C₆-Haloalkyl, Halo, C₁-C₆-Alkoxy, Hydroxy, Cyano, Amino, -NHR^{1C}, -NR^{1D}R^{1D'}, -C(=O)OH, -C(=O)R^{1C}, -C(=O)OR^{1C}, -C(=O)NHR^{1C}, -C(=O)NR^{1D}R^{1D'}, -S(=O)₂R^{1C}, S(=O)₂NHR^{1C}, -S(=O)(=NH)R^{1C}, -OC(=O)R^{1C}, -OC(=O)NHR^{1C}, -NHC(=O)R^{1C}, -NHC(=O)NHR^{1C}, -NHC(=O)OR^{1C} oder -NHS(=O)₂R^{1C};
jedes R^{1A'} ist unabhängig ausgewählt aus Halo, Hydroxy, Cyano, C₁-C₃-Haloalkyl, C₁-C₃-Alkoxy;
R^{1C} ist C₁-C₆-Alkyl, C₃-C₇-Cycloalkyl oder Heterocyclyl, wobei jedes hiervon gegebenenfalls mit einem oder zwei Substituenten substituiert ist, unabhängig ausgewählt aus Halo, Hydroxy, Cyano, Amino, Trifluormethyl, C₁-C₃-Alkyl, C₁-C₃-Alkoxy, C₁-C₃-Haloalkyl, C₁-C₃-Alkylamino und C₁-C₃-Dialkylamino;
R^{1D} und R^{1D'} sind jeweils unabhängig H oder C₁-C₆-Alkyl, oder
R^{1D} und R^{1D'} bilden zusammen mit dem Stickstoffatom, mit dem sie verknüpft sind, einen 4- bis 6-gliedrigen Ring, wobei der Ring gegebenenfalls mit einem oder zwei Substituenten substituiert ist, unabhängig ausgewählt aus Halo, Hydroxy, Cyano und Amino;
R² ist C₁-C₆-Alkyl, das mit einem, zwei oder drei Substituenten substituiert ist, jeweils unabhängig ausgewählt aus Halo, Hydroxy, Cyano, Trifluormethyl, Amino, -NHR^{2A}, -NR^{2B}R^{2B'}, C₁-C₃-Alkoxy, S(=O)₂R^{2A}, C₃-C₄-Cycloalkoxy und Heterocycloxy, wobei jedes Alkoxy, Cycloalkoxy und Heterocycloxy gegebenenfalls mit Substituenten mono-, di- oder trisubstituiert ist, jeweils unabhängig ausgewählt aus Oxo, Halo, Hydroxy, Cyano, Amino, C₁-C₃-Alkyl, C₁-C₃-Haloalkyl,C₁-C₃-Alkoxy, C₁-C₃-Haloalkoxy, Hydroxy-C₁-C₃-alkoxy, C₁-C₃-Alkylamino und -S(=O)₂R^{2A}, oder
R² ist C₂-C₆-Alkyl, C₃-C₇-Cycloalkyl-C₀-C₅-alkyl, Heterocyclyl-C₀-C₅-alkyl, Aryl-C₀-C₅-alkyl oder Heteroaryl-C₀-C₅-alkyl, wobei das Heterocyclyl einen 4- bis 8-gliedrigen gesättigten mono-, bi- oder spirocyclischen Ring darstellt, und wobei jedes Cycloalkyl, Heterocyclyl, Aryl und Heteroaryl mit Substituenten mono-, di- oder trisubstituiert ist, jeweils unabhängig ausgewählt aus Oxo, Halo, Hydroxy, Cyano, Amino, C₁-C₃-Alkyl, C₁-C₃-Haloalkyl,C₁-C₃-Hydroxyalkyl, C₁-C₃-Alkoxy, C₁-C₃-Haloalkoxy, Hydroxy-C₁-C₃-alkoxy, C₁-C₃-Alkylamino, C₃-C₄-Cycloalkyl, Oxetanyl, -S(=O)₂R^{2A}, -S(=O)₂NH₂, -NHS(=O)₂R^{2A} und -C(=O)NH₂, und das Cycloalkyl und Oxetanyl ist gegebenenfalls mit Amino oder Methyl substituiert;
R^{2A} ist C₁-C₃-Alkyl, C₃-C₄-Cycloalkyl, Amino, Aryl, Heteroaryl oder Heterocyclyl;
R^{2B} und R^{2B'} sind unabhängig C₁-C₃-Alkyl, oder
R^{2B} und R^{2B'} sind kombiniert und bilden zusammen mit dem Stickstoffatom, mit dem sie verknüpft sind, ein 4- bis 6-gliedriges Heterocyclyl, wobei das Heterocyclyl gegebenenfalls mit einem oder zwei Substituenten substituiert ist, unabhängig ausgewählt aus Amino, Halo, C₁-C₃-Alkyl und Trifluormethyl;
R³ ist jeweils unabhängig ausgewählt aus der Gruppe, bestehend aus Halo, Hydroxy, Cyano, Amino, C₁-C₆-Alkyl, C₁-C₆-Alkoxy, C₃-C₇-Cycloalkyl-C₀-C₂-alkyl oder Heterocyclyl-C₀-C₂-alkyl wobei das Alkyl, Alkoxy, Cycloalkyl und Heterocyclyl gegebenenfalls mit 1, 2 oder 3 Substituenten substituiert ist, unabhängig ausgewählt aus -NR^{3A}R^{3B}, Halo, Hydroxy und Trifluormethyl;
R^{3A} und R^{3B} sind jeweils unabhängig H oder C₁-C₆-Alkyl, wobei das Alkyl gegebenenfalls mit einem oder zwei Halo substituiert ist;
n ist 0, 1 oder 2;
q ist 0, 1 oder 2;
Heterocyclyl ist ein gesättigter 4- bis 7-gliedriger mono- oder bicyclischer Ring, enthaltend 1, 2 oder 3 Heteroatome, jeweils unabhängig ausgewählt aus O, S und N, sofern nicht anders angegeben;
oder ein Salz hiervon.

2. Verbindung nach Anspruch 1, wobei Z¹ CR^{1B}R^{1B} darstellt.

3. Verbindung nach irgendeinem der Ansprüche 1 bis 2, wobei q 0 ist.

4. Verbindung nach irgendeinem der Ansprüche 1 bis 3, wobei Z² CH darstellt, Z³ ist N.

5. Verbindung nach irgendeinem der Ansprüche 1 bis 3, wobei Z² und Z³ beide CH sind.

6. Verbindung nach irgendeinem der Ansprüche 1 bis 5, wobei n 1 ist und R³ stellt C₁-C₃-Alkyl, Halo oder Trifluormethyl dar.

7. Verbindung nach Anspruch 6, wobei R³ Methyl, Chlor, Fluor oder Trifluormethyl ist.

8. Verbindung nach Anspruch 6 oder 7, wobei R³ sich in der 7-Position des Isochinolinrests befindet, was somit Verbindungen der allgemeinen Formel 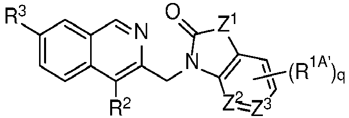 bereitstellt.

9. Verbindung nach irgendeinem der Ansprüche 1 bis 8, wobei R² Heteroaryl darstellt, welches gegebenenfalls mit einem oder zwei Substituenten substituiert ist.

10. Verbindung nach Anspruch 9, wobei R² Thiazolyl darstellt oder gegebenenfalls substituiertes Pyridinyl.

11. Verbindung nach Anspruch 10, wobei R² Pyridin-3-yl oder Pyridin-4-yl darstellt, wobei jedes hiervon gegebenenfalls substituiert ist.

12. Verbindung nach Anspruch 1 mit der Struktur IIb' oder IIb'' 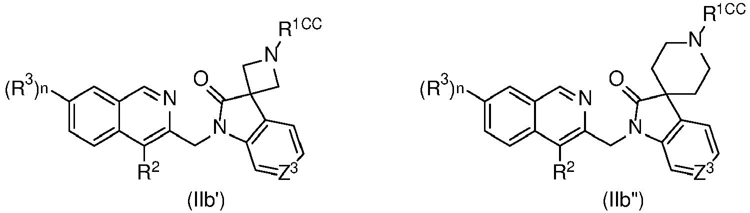 wobei
Z³ N oder CH darstellt;
R^{1CC} ist -C(=O)R^{1C}, -C(=O)OR^{1C}, -S(=O)₂R^{1C}, wobei
R^{1C} C₁-C₄-Alkyl oder C₃-C₆-Cycloalkyl darstellt, wobei jedes hiervon gegebenenfalls mit Methyl, Amino oder Trifluormethyl substituiert ist;
R² ist Thiazolyl, Pyridinyl oder Pyridinyl, welches mit Cyano, -NHS(=O)₂Me, C(=O)NH₂, S(=O)₂NH₂ oder Fluor substituiert ist;
R³ ist C₁-C₃-Alkyl, Halo, Cyano oder C₁-C₃-Haloalkyl;
n ist 0 oder 1.

13. Verbindung nach Anspruch 12, wobei
R^{1C} Methyl oder Cyclopropyl darstellt, wobei Cyclopropyl gegebenenfalls mit Methyl, Amino oder Trifluormethyl substituiert ist;
R² ist Thiazol-5-yl, Pyrid-3-yl oder Pyrid-4-yl;
R³ ist Methyl, Chlor, Fluor oder Trifluormethyl.

14. Verbindung nach irgendeinem der Ansprüche 1 bis 13 oder ein pharmazeutisch akzeptables bzw. annehmbares Salz hiervon zur Verwendung in einem Verfahren zur Behandlung oder Prävention bzw. Vorbeugung von RSV-Infektion in einem Menschen.

15. Pharmazeutische Zusammensetzung, umfassend eine Verbindung nach irgendeinem der Ansprüche 1 bis 13 oder ein pharmazeutisch akzeptables bzw. annehmbares Salz hiervon und einen pharmazeutisch akzeptablen bzw. annehmbaren Träger.

## Revendications

1. Composé de formule (I) : 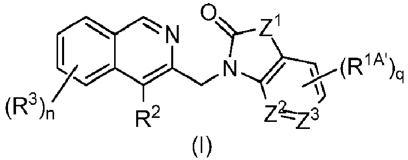 dans lequel
Z¹ est NR^{1A}, CHR^{1A}, CR^{1B}R^{1B} ;
l'un parmi Z² et Z³ est CH ou CR^{1A'} ; l'autre est N, CH ou CR^{1A'} ;
R^{1A} est C₁-C₆ alkyle, C₃-C₇ cycloalkyle, S(=O)₂R^{1C}, aryle, hétéroaryle, hétérocyclyle ou un spirohétérocyclyle à 7 ou 8 chaînons, dans lequel chacun desdits alkyle, cycloalkyle, aryle, hétéroaryle, hétérocyclyle et spirohétérocyclyle est facultativement mono, di ou trisubstitué par des substituants sélectionnés chacun indépendamment à partir du groupe consistant en C₁-C₆ alkyle, C₁-C₆ halogénoalkyle, halogéno, C₁-C₆ alcoxy, hydroxy, cyano, amino, -NHR^{1C},-NR^{1D}R^{1D'}, -C(=O)OH, -C(=O)R^{1C}, -C(=O)C₁-C₆ alkylène-NH₂,-C(=O)OR^{1C}, -C(=O)NHR^{1C}, -C(=O)NR^{1D}R^{1D'}, -S(=O)₂R^{1C}, S(=O)₂NHR^{1C}, -S(=O)(=NH)R^{1C}, -OC(=O)R^{1C}, -OC(=O)NHR^{1C},-NHC(=O)R^{1C}, -NHC(=O)NHR^{1C}, -NHC(=O)OR^{1C} ou -NHS(=O)₂R^{1C} ;
les deux R^{1B} conjointement avec l'atome de carbone auquel ils sont rattachés se combinent et forment un C₃-C₆ cycloalkyle ou hétérocyclyle, dans lequel le cycloalkyle et l'hétérocyclyle sont facultativement mono, di ou trisubstitués par des substituants sélectionnés chacun indépendamment à partir du groupe consistant en C₁-C₆ alkyle, C₁-C₆ halogénoalkyle, halogéno, C₁-C₆ alcoxy, hydroxy, cyano, amino, -NHR^{1C}, - NR^{1D}R^{1D'}, -C(=O)OH, -C(=O)R^{1C}, -C(=O)OR^{1C}, -C(=O)NHR^{1C},-C(=O)NR^{1D}R^{1D'}, -S(=O)₂R^{1C}, S(=O)₂NHR^{1C}, -S(=O)(=NH)R^{1C},-OC(=O)R^{1C}, -OC(=O)NHR^{1C}, -NHC(=O) R^{1C}, -NHC(=O)NHR^{1C},-NHC(=O)OR^{1C} ou -NHS(=O)₂R^{1C} ;
chaque R^{1A'} est sélectionné indépendamment à partir d'halogéno, hydroxy, cyano, C₁-C₃ halogénoalkyle, C₁-C₃ alcoxy ;
R^{1C} est C₁-C₆ alkyle, C₃-C₇ cycloalkyle ou hétérocyclyle, l'un quelconque d'entre eux est facultativement substitué par un ou deux substituants sélectionnés indépendamment à partir d'halogéno, hydroxy, cyano, amino, trifluorométhyle, C₁-C₃ alkyle, C₁-C₃ alcoxy, C₁-C₃ halogénoalkyle, C₁-C₃ alkylamino et C₁-C₃ dialkylamino ;
R^{1D} et R^{1D'} sont chacun indépendamment H ou C₁-C₆ alkyle, ou
R^{1D} et R^{1D'} conjointement avec l'atome d'azote auquel ils sont rattachés forment un cycle à 4 à 6 chaînons, lequel cycle est facultativement substitué par un ou deux substituants sélectionnés indépendamment à partir d'halogéno, hydroxy, cyano et amino ;
R² est C₁-C₆ alkyle qui est substitué par un, deux ou trois substituants sélectionnés chacun indépendamment à partir d'halogéno, hydroxy, cyano, trifluorométhyle, amino, -NHR^{2A}, -NR^{2B}R^{2B'}, C₁-C₃ alcoxy, S (=O) ₂R^{2A}, C₃-C₄ cycloalcoxy et hétérocycloxy, dans lequel chacun desdits alcoxy, cycloalcoxy et hétérocycloxy est facultativement mono, di ou trisubstitué par des substituants sélectionnés chacun indépendamment à partir d'oxo, halogéno, hydroxy, cyano, amino, C₁-C₃ alkyle, C₁-C₃ halogénoalkyle, C₁-C₃ alcoxy, C₁-C₃ halogénoalcoxy, hydroxyC₁-C₃alcoxy, C₁-C₃ alkylamino et -S (=O) ₂R^{2A}, ou
R² est C₂-C₆ alkyle, C₃-C₇ cycloalkyle-Co-Csalkyle, hétérocyclylCo-Csalkyle, arylC₀-C₅alkyle ou hétéroarylCo-Csalkyle, dans lequel l'hétérocyclyle est un cycle mono, bi ou spirocyclique saturé à 4 à 8 chaînons, et dans lequel chacun desdits cycloalkyle, hétérocyclyle, aryle et hétéroaryle est facultativement mono, di ou trisubstitué par des substituants sélectionnés chacun indépendamment à partir d'oxo, halogéno, hydroxy, cyano, amino, C₁-C₃ alkyle, C₁-C₃ halogénoalkyle, C₁-C₃ hydroxyalkyle, C₁-C₃ alcoxy, C₁-C₃ halogénoalcoxy, C₁-C₃ hydroxyalcoxy, C₁-C₃ alkylamino, C₃-C₄ cycloalkyle, oxétanyle, -S (=O) ₂R^{2A}, -S (=O) ₂NH₂, - NHS(=O)₂R^{2A} et -C(=O)NH₂, et le cycloalkyle et l'oxétanyle sont facultativement substitués par amino ou méthyle ;
R^{2A} est C₁-C₃ alkyle, C₃-C₄ cycloalkyle, amino, aryle, hétéroaryle ou hétérocyclyle ;
R^{2B} et R^{2B'} sont chacun indépendamment C₁-C₃ alkyle, ou
R^{2B} et R^{2B'} conjointement avec l'atome d'azote auquel ils sont rattachés se combinent et forment un hétérocyclyle à 4 à 6 chaînons, lequel hétérocyclyle est facultativement substitué par un ou deux substituants sélectionnés indépendamment à partir d'amino, halogéno, C₁-C₃ alkyle et trifluorométhyle ;
chaque R³ est sélectionné indépendamment à partir du groupe consistant en halogéno, cyano, amino, C₁-C₆ alkyle, C₁-C₆ alcoxy, C₃-C₇ cycloalkyle-C₀-C₂alkyle ou C₀-C₂ hétérocyclylalkyle, dans lequel l'alkyle, l'alcoxy, le cycloalkyle et l'hétérocyclyle sont facultativement substitués par 1, 2 ou 3 substituants sélectionnés indépendamment à partir de -NR^{3A}R^{3B}, halogéno, hydroxy et trifluorométhyle ;
R^{3A} et R^{3B} sont chacun indépendamment H ou C₁-C₆ alkyle, dans lequel l'alkyle est facultativement substitué par un ou deux halogéno ;
n vaut 0, 1 ou 2 ;
q vaut 0, 1, ou 2 ;
l'hétérocyclyle est un cycle mono- ou bi-cyclique à 4 à 7 chaînons contenant 1, 2 ou 3 hétéroatomes sélectionnés chacun indépendamment à partir de O, S et N, sauf indication contraire ;
ou un sel de celui-ci.

2. Composé selon la revendication 1, dans lequel Z¹ est CR^{1B}R^{1B}.

3. Composé selon l'une quelconque des revendications 1 et 2, dans lequel q vaut 0.

4. Composé selon l'une quelconque des revendications 1 à 3, dans lequel Z² est CH, Z³ est N.

5. Composé selon l'une quelconque des revendications 1 à 3, dans lequel Z² et Z³ sont tout deux CH.

6. Composé selon l'une quelconque des revendications 1 à 5, dans lequel n vaut 1 et R³ est C₁-C₃ alkyle, halogéno ou trifluorométhyle.

7. Composé selon la revendication 6, dans lequel R³ est méthyle, chloro, fluoro ou trifluorométhyle.

8. Composé selon la revendication 6 ou 7, dans lequel R³ est situé dans la position 7 du fragment isoquinoline, fournissant ainsi des composés répondant à la formule générale :

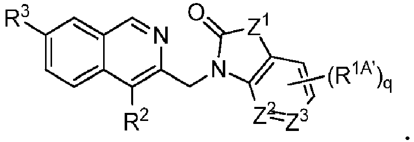

9. Composé selon l'une quelconque des revendications 1 à 8, dans lequel R² est hétéroaryle qui est facultativement substitué par un ou deux substituants.

10. Composé selon la revendication 9, dans lequel R² est thiazolyle ou pyridinyle facultativement substitué.

11. Composé selon la revendication 10, dans lequel R2 est pyridin-3-yle ou pyridin-4-yle, l'un quelconque d'entre eux est facultativement substitué.

12. Composé selon la revendication 1, présentant la structure IIb' ou IIb" 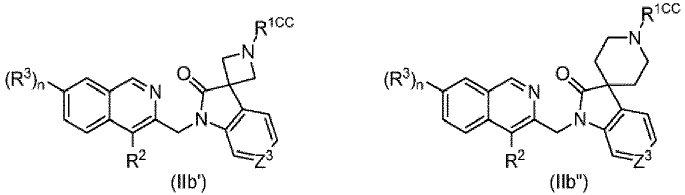 dans lequel
Z³ est N ou CH ;
R^{1CC} est -C(=O)R^{1C}, -C (=O) OR^{1C}, -S(=O)₂R^{1C}, dans lequel
R^{1C} est C₁-C₄ alkyle ou C₃-C₆ cycloalkyle, l'un quelconque d'entre eux est facultativement substitué par méthyle, amino ou trifluorométhyle ;
R² est thiazolyle, pyridinyle ou pyridinyle qui est substitué par cyano, -NHS(=O)₂Me, C(=O)NH₂, S(=O)₂NH₂ ou fluoro ;
R3 est C₁-C₃ alkyle, halogéno, cyano ou C₁-C₃ halogénoalkyle ;
n vaut 0 ou 1.

13. Composé selon la revendication 12, dans lequel
R^{1C} est méthyle ou cyclopropyle, dans lequel cyclopropyle est facultativement substitué par méthyle, amino ou trifluorométhyle ;
R² est thiazol-5-yle, pyrid-3-yle ou pyrid-4-yle ;
R³ est méthyle, chloro, fluoro ou trifluorométhyle.

14. Composé selon l'une quelconque des revendications 1 à 13, ou sel pharmaceutiquement acceptable de celui-ci, pour son utilisation dans une méthode pour le traitement ou la prévention d'une infection au RSV chez un être humain.

15. Composition pharmaceutique comprenant un composé selon l'une quelconque des revendications 1 à 13, ou un sel pharmaceutiquement acceptable de celui-ci, et un véhicule pharmaceutiquement acceptable.
